# EUROPEAN PATENT APPLICATION

(11) **EP 2 634 176 A1**
(43) Date of publication of application: **04.09.2013**
(21) Application number: 11836395.1
(22) Date of filing: 27.10.2011
(51) Int. Cl.: C07D 213/84, A61K 31/4427, A61K 31/4439, A61K 31/444, A61K 31/4545, A61K 31/496, A61K 31/541, A61P 17/00, A61P 27/00, A61P 29/00, A61P 35/00, A61P 35/02, A61P 37/00, A61P 37/02, A61P 37/08, A61P 43/00, C07D 401/14

(54) **PYRIDINE DERIVATIVE AND MEDICINAL AGENT**

(30) Priority: 02.09.2011 JP 2011191449; 28.10.2010 JP 2010242624
(71) Applicant: Nippon Shinyaku Co., Ltd., Kyoto-shi Kyoto 601-8550 (JP)
(72) Inventor: FUJIHARA, Hidetaka, Kyoto-shi Kyoto 601-8319 (JP); SUGIYAMA, Hiroyuki, Kyoto-shi Kyoto 612-8443 (JP); TSUJI, Takashi, Kyoto-shi Kyoto 600-8865 (JP); INO, Takara, Kyoto-shi Kyoto 612-8225 (JP); HARUTA, Yoshinari, Nagaokakyo-shi Kyoto 617-0826 (JP)
(74) Representative: Vossius & Partner
(86) International application number: PCT/JP2011/074813
(87) International publication number: WO 2012/057262

(57) **Abstract**

[Problem to be Solved by the Invention]

A main object of the present invention is to provide a novel pyridine derivative or a pharmaceutically acceptable salt thereof.

[Means of Solving the Problems]

A pyridine derivative or a pharmaceutically acceptable salt represented by the following genaral formula [1] or a pharmaceutically acceptable salt thereof. 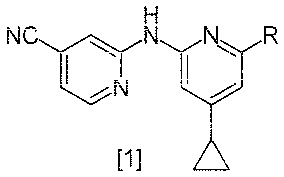 In formula [1], R represents an aryl group or a heteroaryl group, wherein each of the aryl group and the heteroaryl group may be substituted by one or two substituents independently selected from the group consisting of an optionally substituted alkyl group, a hydroxy group, a halogen atom and a group represented by general formula [2]. In formula [2],

-L¹-L²-L³-R^{A}

L1 and L3 independently represent a single bond, an alkylene group or a cycloalkylene group; L2 represents a single bond, O, or NRB; RB represents H or an optionally substituted alkyl group; RA represents H, an amino group, a cyano group, a hydroxy group, an alkoxy group, an aryl group, a monoalkylamino group, a dialkylamino group, a carbamoyl group, an alkyloxycarbonyl group, a monoalkylaminocarbonyl group, a dialkylaminocarbonyl group, an alkylcarbonylamino group, a saturated heterocyclic group, an optionally substituted alkyl group, an optionally substituted cycloalkyl group or an optionally substituted heteroaryl group.

## Description

### [Technical Field]

The present invention relates to a novel pyridine derivative and a pharmaceutical composition containing the same as an active ingredient.

### [Background Art]

Peripheral T-cell lymphoma not-otherwise specified (PTCL-NOS) is a disease caused by uncontrolled proliferation of T-lymphocytes. PTCL-NOS is a hematologic tumor of high malignant potential. There is no effective treatment method for PTCL-NOS at present, and its poor prognosis is problematic (see, for example, NPL 1). Therefore, a therapeutic agent for the disease has been awaited.
In 2006, Itk-Syk caused by chromosomal translocation was found in cells of patients with PTCL-NOS (see, for example, NPL 2). Further, it is shown that mice expressing Itk-Syk develop a T-cell lymphoma, and therefore, it has become evident that Itk-Syk causes tumors (see, for example, NPL 3 and NPL 4). From the previous studies, it is presumed that the intracellular signal transduction pathway is activated by Itk-Syk activation to enhance cell cycle progression (see, for example, NPL 5). Therfore it is considered that the inhibition of Itk-Syk can stop cell growth, it being possible to suppress progression and exacerbation of tumors. Further, it has been reported that overexpression of the Syk gene was observed with high probability even in peripheral T-cell lymphoma not-otherwise specified, in which Itk-Syk is not expressed, angioimmunoblastic T-cell lymphoma, anaplastic large-cell lymphoma, cutaneous anaplastic large-cell lymphoma, mycosis fungoides, enteropathy-associated T-cell lymphoma, extranodal NK-T-cell lymphoma, hepatosplenic T-cell lymphoma, and subcutaneous panniculitis-like T-cell lymphoma (see, for example, NPL 6). Therefore, a therapeutic effect on these diseases by inhibiting Syk tyrosine kinase can also be expected.
Syk tyrosine kinase is a kinase expressed in lymphoid cells, and regulates intracellular signaltransduction from B-cell receptor to promote cell growth and differentiation (see, for example, NPL 7). It is considered that in B-cell lymphomas such as diffuse large-cell lymphoma, follicular lymphoma, and small-cell lymphoma, and B-cell leukemias such as chronic lymphocytic leukemia, cell growth can be suppressed by a Syk tyrosine kinase inhibitor, and clinical trials are currently underway (see, for example, NPL 8).
It has been revealed that Syk tyrosine kinase is expressed in B cells that produce a antibodies or mast cells that release chemical mediators, and is involved in an immune response by positively regulating the production of an antibody in response to an antigen stimulation, the release of histamine in response to a Fc receptor stimulation, and so on (see, for example, NPL 9). Therefore, it is considered that by inhibiting Syk tyrosine kinase, chronic rheumatoid arthritis or idiopathic thrombocytopenic purpura, which is an autoimmune disease, can be suppressed by suppressing the production of an antibody, or asthma can be suppressed by suppressing the release of histamine. In fact, a Syk tyrosine kinase inhibitor is used in a clinical trial as a therapeutic agent for an inflammatory disease (see, for example, NPL 10).

As described above, Syk tyrosine kinase is involved in transmission of an extracellular stimulation. Therefore, a compound having a Syk tyrosine kinase inhibitory activity can be expected to have a therapeutic effect on various diseases such as hematologic cancer, inflammatory diseases, and autoimmune diseases.

As the Syk tyrosine kinase inhibitor, for example, an aminopyridine compound has been reported in PTL 1.

### [Citation List]

### [Patent Literature]

[PTL 1] WO 2006/093247

### [Non Patent Literature]

[NPL 1] Kerry J. Savage, 2007, Blood Reviews, 21, 201-216.
[NPL 2] B Streubel, et al., 2006, Leukemia, 20, 313-318.
[NPL 3] Dierks C, et al., Cancer Res, 70, 6193-6204.
[NPL 4] Pechloff K, et al., 2010, J Exp Med, 207, 1031-1044.
[NPL 5] C Dierks, et al., 2010, Cancer Res, 70, 6193-6204.
[NPL 6] Feldman AL, et al., 2008, Leukemia, 6, 1139-1143.
[NPL 7] Mocsai A, et al., 2010, Nature Review Immunology, 6, 387-402.
[NPL 8] Friedberg JW, et al., 2010, Blood, 115, 2578-2585.
[NPL 9] Zhang J, et al., 1996, Journal of Experimental Medicine, 184, 71-79.
[NPL 10] Weinblatt ME, et al., 2010, New England Journal of Medicine, 363, 1303-1312.

### [Summary of Invention]

### [Problem to be Solved by the Invention]

A main object of the present invention is to provide a novel pyridine derivative or a pharmaceutically acceptable salt thereof. Another main object of the present invention is to provide a pharmaceutical composition containing such a pyridine derivative or a pharmaceutically acceptable salt thereof as an active ingredient.

### [Means of Solving the Problems]

The present inventors found that a novel pyridine derivative or a pharmaceutically acceptable salt thereof, which will be described below, has an excellent Syk tyrosine kinase inhibitory activity, and thus completed the present invention.

The present invention includes, for example, the following (A) to (C).
(A) A pyridine derivative represented by the following general formula [1] (hereinafter referred to as "the compound of the present invention") or a pharmaceutically acceptable salt thereof: 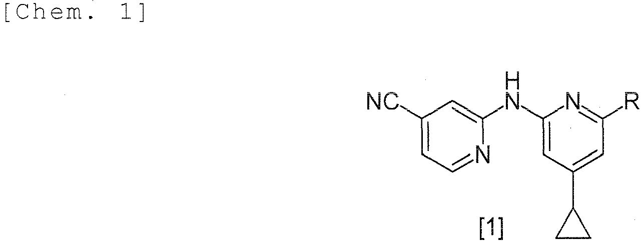 wherein
   R represents aryl or heteroaryl, each of which may be substituted with one or two substituents selected from the group consisting of alkyl which may be substituted with hydroxy, hydroxy, halogen, and a group represented by the following general formula [2]:

   [Chem. 2] -L¹-L²-L³-R^{A}

   (wherein
   L¹ and L³ each independently represent a single bond, alkylene, or cycloalkylene;
   L² represents a single bond, O, or NR^{B};
   R^{B} represents alkyl which may be substituted with hydroxy;
   R^{A} represents:
   (1) H,
   (2) amino,
   (3) cyano,
   (4) hydroxy,
   (5) alkoxy,
   (6) aryl,
   (7) monoalkylamino,
   (8) dialkylamino,
   (9) carbamoyl,
   (10) alkyloxycarbonyl,
   (11) monoalkylaminocarbonyl,
   (12) dialkylaminocarbonyl,
   (13) alkylcarbonylamino,
   (14) alkyl which may be substituted with one or two hydroxy groups,
   (15) heteroaryl which may be substituted with hydroxy or alkyl,
   (16) cycloalkyl which may be substituted with one or two substituents selected from halogen, alkoxy, alkylcarbonyloxy, hydroxy, and hydroxyalkyl, or
   (17) a 4- to 7-membered saturated heterocyclic group, which has one or two heteroatoms, and may be substituted with one or two substituents selected from the group consisting of cyano, hydroxy, oxo, halogen, alkylcarbonyl, amino, monoalkylamino, dialkylamino, alkylcarbonylamino, carbamoylamino, monoalkylaminocarbonylamino, alkyl, hydroxyalkyl, hydroxycarbonylalkyl, carbamoylalkyl, monoalkylaminocarbonylalkyl, dialkylaminocarbonylalkyl, hydroxycarbonyl, carbamoyl, monoalkylaminocarbonyl, dialkylaminocarbonyl, aminothiocarbonyl, alkylsulfonyl, and aryl may be substituted with halogen).
(B) A pharmaceutical composition, containing the compound of the present invention or a pharmaceutically acceptable salt thereof as an active ingredient.
(C) A Syk tyrosine kinase inhibitor, containing the compound of the present invention or a pharmaceutically acceptable salt thereof as an active ingredient.

The compound of the present invention is preferably such that aryl or heteroaryl represented by R is phenyl, pyridinyl, pyrimidinyl, benzimidazolyl, indazolyl, or isoquinolyl.
Further, the compound of the present invention is preferably such that R^{A} is cycloalkyl which may be substituted with hydroxy or hydroxyalkyl, or a 4- to 7-membered saturated heterocyclic group, which has one or two heteroatoms, and is substituted with one or two substituents selected from the group consisting of cyano, hydroxy, oxo, halogen, alkylcarbonyl, amino, monoalkylamino, dialkylamino, alkylcarbonylamino, carbamoylamino, monoalkylaminocarbonylamino, alkyl, hydroxyalkyl, hydroxycarbonylalkyl, carbamoylalkyl, monoalkylaminocarbonylalkyl, dialkylaminocarbonylalkyl, hydroxycarbonyl, carbamoyl, monoalkylaminocarbonyl, dialkylaminocarbonyl, aminothiocarbonyl, alkylsulfonyl, and aryl which may be substituted with halogen.

Specific examples of the compound of the present invention may include compounds selected from the group consisting of the following compounds (1) to (170).
(1) 2-{[4-cyclopropyl-6'-(morpholin-4-yl)-2,3-bipyridin-6-yl]amino}pyridine-4-carbonitrile,
(2) 2-({4-cyclopropyl-6'-[(2-hydroxyethyl)amino]-2,3'-bipyridin-6-yl}amino)pyridine-4-carbonitrile,
(3) 2-{[4-cyclopropyl-6'-(1,1-dioxidethiomorpholin-4-yl)-2,3'-bipyridin-6-yl]amino}pyridine-4-carbonitrile,
(4) 2-{[4-cyclopropyl-6'-(4-oxopiperidin-1-yl)-2,3'-bipyridin-6-yl]amino}pyridine-4-carbonitrile,
(5) 2-{[4-cyclopropyl-6'-(3-oxopiperazin-1-yl)-2,3'-bipyridin-6-yl]amino}pyridine-4-carbonitrile,
(6) 2-{[4-cyclopropyl-6'-(3-hydroxyazetidin-1-yl)-2,3'-bipyridin-6-yl]amino}pyridine-4-carbonitrile,
(7) 2-(4-{6-[(4-cyanopyridin-2-yl)amino]-4-cyclopropyl-2,3'-bipyridin-6'-yl}piperazin-1-yl)acetamide,
(8) 2-({4-cyclopropyl-6'-[4-hydroxy-2-oxopyrrolidin-1-yl]-2,3'-bipyridin-6-yl}amino)pyridine-4-carbonitrile,
(9) 2-{[6'-(4-acetyl-1,4-diazepan-1-yl)-4-cyclopropyl-2,3'-bipyridin-6-yl]amino}pyridine-4-carbonitrile,
(10)4-{6-[(4-cyanopyridin-2-yl)amino]-4-cyclopropyl-2,3'-bipyridin-6'-yl}-1,4-diazepane-1-carboxamide,
(11) 2-({4-cyclopropyl-6'-[(trans-4-hydroxycyclohexyl)amino]-2,3'-bipyridin-6-yl}amino)pyridine-4- carbonitrile),
(12) 2-({6-[1-(2-aminoethyl)-1H-benzimidazol-5-yl]-4-cyclopropylpyridin-2-yl}amino)pyridine-4- carbonitrile,
(13) 2-({4-cyclopropyl-6-[1-(trans-4-hydroxycyclohexyl)-1H-benzimidazol-5-yl]pyridin-2-yl}amino)pyridine-4-carbonitrile,
(14) 2-{[4-cyclopropyl-6-(1-methyl-1H-benzimidazol-5-yl)pyridin-2-yl]amino}pyridine-4-carbonitrile,
(15) 2-({4-cyclopropyl-6-[1-(tetrahydro-2H-pyran-4-yl)-1H-benzimidazol-5-yl]pyridin-2-yl}amino)pyridine-4-carbonitrile,
(16) 6-[(4-cyanopyridin-2-yl)amino]-4-cyclopropyl-2,3'-bipyridine-6'-carbonitrile
(17) 2-[(6'-amino-4-cyclopropyl-2,3'-bipyridin-6-yl)amino]pyridine-4-carbonitrile
(18) 2-({6'-[(2-aminoethyl)amino]-4-cyclopropyl-2,3'-bipyridin-6-yl}amino)pyridine-4-carbonitrile)
(19) 2-{[4-cyclopropyl-6'-(4-hydroxypiperidin-1-yl)-2,3'-bipyridin-6-yl]amino}pyridine-4-carbonitrile,
(20) 2-({4-cyclopropyl-6'-[(2-methoxyethyl)amino]-2,3'-bipyridin-6-yl}amino)pyridine-4-carbonitrile,
(21) 2-({4-cyclopropyl-6'-[3-hydroxypyrrolidin-1-yl]-2,3'-bipyridin-6-yl}amino)pyridine-4-carbonitrile,
(22) 2-({4-cyclopropyl-6'-[(3-hydroxypropyl)amino]-2,3'-bipyridin-6-yl}amino)pyridine-4-carbonitrile,
(23) 2-({4-cyclopropyl-6'-[(3-hydroxy-2,2-dimethylpropyl)amino]-2,3'-bipyridin-6-yl}amino)pyridine-4- carbonitrile,
(24) 2-({4-cyclopropyl-6'-[4-(hydroxymethyl)piperidin-1-yl]-2,3'-bipyridin-6-yl}amino)pyridine-4-carbonitrile,
(25) 2-{[4-cyclopropyl-6'-(thiomorpholin-4-yl)-2,3'-bipyridin-6- yl]amino}pyridine-4-carbonitrile,
(26) 2-{[6'-(4-aminopiperidin-1-yl)-4-cyclopropyl-2,3'-bipyridin-6-yl]amino}pyridine-4-carbonitrile,
(27) 2-({4-cyclopropyl-6'-[4-(methylamino)piperidin-1-yl]-2,3'-bipyridin-6-yl}amino)pyridine-4-carbonitrile,
(28) 2{[4-cyclopropyl-6'-(4-fluoropiperidin-1-yl)-2,3'-bipyridin-6-yl]amino}pyridine-4-carbonitrile,
(29) 2-({6'-[bis(2-hydroxyethyl)amino]-4-cyclopropyl-2,3'-bipyridin-6-yl} amino)pyridine-4-carbonitrile
(30) 2-{[4-cyclopropyl-6'-(1,4-diazepan-1-yl)-2,3'-bipyridin-6-yl]amino}pyridine-4-carbonitrile,
(31) 2-({4-cyclopropyl-6'-[3-hydroxypiperidin-1-yl]-2,3'-bipyridin-6-yl}amino)pyridine-4-carbonitrile,
(32) 2-({4-cyclopropyl-6-[2-(4-hydroxypiperidin-1-yl)pyrimidin-5-yl)pyridin-2-yl}amino)pyridine-4-carbonitrile,
(33) 2-{[4-cyclopropyl-6-(1-methyl-1H-indazol-5-yl)pyridin-2-yl]amino}pyridine-4-carbonitrile,
(34) 2-{[4-cyclopropyl-6-(1-methyl-1H-indazol-6-yl)pyridin-2-yl]amino}pyridine-4-carbonitrile,
(35) 2-{[4-cyclopropyl-6'-(4-methyl-1,4-diazepan-1-yl)-2,3'-bipyridin-6-yl]amino}pyridine-4-carbonitrile,
(36) 1-{6-[(4-cyanopyridin-2-yl)]-4-aminocyclopropyl-2,3'-bipyridin-6'-yl}-N-methylpyrrolidine-2-carboxamide,
(37) 1-{6-[(4-cyanopyridin-2-yl)amino]-4-cyclopropyl-2,3'-bipyridin-6'-yl}pyrrolidine-2-carboxamide,
(38) 2-({4-cyclopropyl-6-[4-(4-hydroxypiperidin-1-yl)phenyl)pyridin-2-yl}amino)pyridine-4-carbonitrile,
(39) N-[1-{6-[(4-cyanopyridin-2-yl)amino]-4-cyclopropyl-2,3'-bipyridin-6'-yl}pyrrolidin-3-yl]acetamide,
(40) 4-{6-[(4-cyanopyridin-2-yl)amino]-4-cyclopropyl-2,3'-bipyridin-6'-yl)-N-ethylpiperazine-1-carboxamide,
(41) 4-{6-[(4-cyanopyridin-2-yl)amino]-4-cyclopropyl-2,3'-bipyridin-6'-yl}-N-(propan-2-yl)piperazine-1-carboxamide,
(42) 1-[1-{6-[(4-cyanopyridin-2-yl)amino]-4-cyclopropyl-2,3'-bipyridin-6'-yl}pyrrolidin-3-yl]urea,
(43) 4-{6-[(4-cyanopyridin-2-yl)amino]-4-cyclopropyl-2,3'-bipyridin-6'-yl}piperazine-1-carbothioamide,
(44) 2-({4-cyclopropyl-6'-[2-(hydroxymethyl)pyrrolidin-1-yl]-2,3'-bipyridin-6-yl}amino)pyridine-4-carbonitrile,
(45) 2-({6'-[3-aminopyrrolidin-1-yl]-4-cyclopropyl-2,3'-bipyridin-6-yl}amino)pyridine-4-carbonitrile,
(46) 2-({4-cyclopropyl-6'-[3-fluoropyrrolidin-1-yl]-2,3'-bipyridin-6-yl}amino)pyridine-4-carbonitrile,
(47) 2-({4-cyclopropyl-6'-[3-(dimethylamino)pyrrolidin-1-yl]-2,3'-bipyridin-6-yl}amino)pyridine-4-carbonitrile
(48) 2{[4-cyclopropyl-6'-(2-oxopyrrolidin-1-yl)-2,3'-bipyridin-6-yl]amino}pyridine-4-carbonitrile,
(49) 2-[(4-cyclopropyl-6'-methyl-2,3'-bipyridin-6-yl)amino]pyridine-4-carbonitrile,
(50) 2-{[4-cyclopropyl-6-(1H-indazol-6-yl)pyridin-2-yl]amino}pyridine-4-carbonitrile,
(51) 2 {[4-cyclopropyl-6-(1H-indazol-5-yl)pyridin-2-yl]amino}pyridine-4-carbonitrile,
(52) 4-{6-[(4-cyanopyridin-2-yl)amino]-4-cyclopropyl-2,3'-bipyridin-6'-yl}piperazine-1-carboxamide,
(53) 2-({4-cyclopropyl-6'-[4-(methanesulfonyl)piperazin-1-yl]-2,3'-bipyridin-6-yl}amino)pyridine-4- carbonitrile,
(54) 2-{[6'-(4-acetylpiperazin-1-yl)-4-cyclopropyl-2,3'-bipyridin-6-yl]amino}pyridine-4-carbonitrile,
(55) 1-{6-[(4-cyanopyridin-2-yl)amino]-4-cyclopropyl-2,3'-bipyridin-6'-yl} piperidine-4-carbonxylic acid,
(56) 1-{6-[(4-cyanopyridin-2-yl)amino]-4-cyclopropyl-2,3'-bipyridin-6'-yl}piperidine-4-carboxamide,
(57) 1-{6-[(4-cyanopyridin-2-yl)amino]-4-cyclopropyl-2,3'-bipyridin-6'-yl}-4-(4-fluorophenyl)piperidine-4-carboxamide,
(58) 2-(4-{6-[(4-cyanopyridin-2-yl)amino]-4-cyclopropyl-2,3'-bipyridin-6'-yl}piperazin-1-yl)-Nethylacetamide,
(59) 1-(1-{6-[(4-cyanopyridin-2-yl)amino]-4-cyclopropyl-2,3'-bipyridin-6'-yl}piperidin-4-yl)urea,
(60) 4-({6-[(4-cyanopyridin-2-yl)amino]-4-cyclopropyl-2,3'-bipyridin-6'-yl}amino)piperidine-1- carboxamide,
(61) 2-{[4-cyclopropyl-6'-(2-oxopiperazin-1-yl)-2,3'-bipyridin-6-yl]amino}pyridine-4-carbonitrile,
(62) 4-{6-[(4-cyanopyridin-2-yl)amino]-4-cyclopropyl-2,3'-bipyridin-6'-yl}-3-oxopiperazine-1-carboxamide,
(63) 2-{[4-cyclopropyl-5'-(1,1- dioxidethiomorpholin-4-yl)-2,3'-bipyridin-6-yl]amino}pyridine-4-carbonitrile,
(64) 4-{6-[(4-cyanopyridin-2-yl)amino]-4-cyclopropyl-2,3'-bipyridin-5'-yl}-1,4-diazepane-1-carboxamide,
(65) 2-({4-cyclopropyl-6'-[(3-oxopiperazin-1-yl)methyl]-2,3'-bipyridin-6-yl}amino)pyridine-4-carbonitrile,
(66) 2-{[4-cyclopropyl-6'-(hydroxymethyl)-2,3'-bipyridin-6-yl]amino}pyridine-4-carbonitrile,
(67) 2-({4-cyclopropyl-6'-[(1,1-dioxidethiomorpholin-4-yl)methyl]-2,3'-bipyridin-6-yl}amino)pyridine-4-carbonitrile,
(68) 2-({4-cyclopropyl-5'-[(3-oxopiperazin-1-yl)methyl]-2,3'-bipyridin-6-yl}amino)pyridine-4-carbonitrile,
(69) 2-({4-cyclopropyl-5'-[(1,1-dioxidethiomorpholin-4-yl)methyl]-2,3'-bipyridin-6-yl}amino)pyridine-4-carbonitrile
(70) 4-({6-[(4-cyanopyridin-2-yl)amino]-4-cyclopropyl-2,3'-bipyridin-5'-yl}methyl])-1,4-diazepane-1-carboxamide,
(71) 4-{6-[(4-cyanopyridin-2-yl)amino]-4-cyclopropyl-2,4'-bipyridin-2'-yl}-1,4-diazepane-1-carboxamide,
(72) 4-({6-[(4-cyanopyridin-2-yl)amino]-4-cyclopropyl-2,3'-bipyridin-5'-yl}amino)piperidine-1-carboxamide,
(73) 4-(3-{6-[(4-cyanopyridin-2-yl)amino]-4-cyclopropylpyridin-2-yl}phenyl)-1,4-diazepane-1-carboxamide,
(74) 2-[(4-cyclopropyl-6-{3-[(3-oxopiperazin-1-yl)methyl]phenyl}pyridin-2-yl)amino]pyridine-4-carbonitrile,
(75) 2-[(4-cyclopropyl-6-{4-[(3-oxopiperazin-1-yl)methyl]phenyl}pyridin-2-yl]amino]pyridine-4-carbonitrile,
(76) 2-{[4-cyclopropyl-5'-(piperazin-1-ylmethyl)-2,3'-bipyridin-6-yl] amino}pyridine-4-carbonitrile,
(77) 2-({5'-[(4-acetylpiperazin-1-yl)methyl]-4-cyclopropyl-2,3'-bipyridin-6-yl}amino)pyridine-4-carbonitrile,
(78) 4-({6-[(4-cyanopyridin-2-yl)amino]-4-cyclopropyl-2,3'-bipyridin-5'-yl}methyl)piperazine-1-carboxamide,
(79) 2-({4-cyclopropyl-6-[3-(piperazin-1-ylmethyl) phenyl]pyridin-2-yl}amino)pyridine-4-carbonitrile,
(80) 2-{[4 cyclopropyl-5'-(piperidin-4-ylamino)-2,3'-bipyridin-6-yl]amino}pyridine-4-carbonitrile,
(81) 2-{[4-cyclopropyl-2'-(piperazin-1-ylmethyl)-2,4'-bipyridin-6-yl]amino}pyridine-4-carbonitrile,
(82) 2-({4-cyclopropyl-2'-[(3-oxopiperazin-1-yl)methyl]-2,4'-bipyridin-6-yl}amino)pyridine-4-carbonitrile,
(83) 2-({4-cyclopropyl-5'-[pyrrolidin-3-ylamino]-2,3'-bipyridin-6-yl}amino)pyridine-4-carbonitrile,
(84) 2-({5'-[(2-aminoethyl)amino]-4-cyclopropyl-2,3'-bipyridin-6-yl}amino)pyridine-4-carbonitrile,
(85) 2-({4-cyclopropyl-5'-[(piperidin-4-yloxy)methyl]-2,3'-bipyridin-6-yl}amino)pyridine-4-carbonitrile,
(86) 2-[(4-cyclopropyl-5'-{[N-methyl-N-(piperidin-4-yl)amino]methyl}-2,3'-bipyridin-6-yl)amino]pyridine-4-carbonitrile,
(87) 2-({4-cyclopropyl-5'-[(piperidin-4-ylamino)methyl]-2,3'-bipyridin-6-yl}amino)pyridine-4-carbonitrile,
(88) 2-({4-cyclopropyl-5'-[(piperidin-4-ylmethyl)amino]-2,3'-bipyridin-6-yl}amino)pyridine-4-carbonitrile
(89) 2-{[5'-(azetidin-3-ylamino)-4-cyclopropyl-2,3'-bipyridin-6-yl]amino}pyridine-4-carbonitrile,
(90) 2-{[4-cyclopropyl-5'-(piperidin-4-yloxy)-2,3'-bipyridin-6-yl]amino}pyridine-4-carbonitrile,
(91) 2-[(4-cyclopropyl-5'-{[pyrrolidin-3-ylmethyl]amino}-2,3'-bipyridin-6-yl)amino]pyridine-4-carbonitrile,
(92) 2-{[4-cyclopropyl-5'-(1,4-diazepan-1-yl)-2,3'-bipyridin-6-yl]amino}pyridine-4-carbonitrile,
(93) 2-({4-cyclopropyl-5'-[(1-methylpiperidin-4-yl)oxy]-2,3'-bipyridin-6-yl}amino)pyridine-4-carbonitrile,
(94) 2-({4-cyclopropyl-5'-[2-(piperazin-1-yl)ethoxy]-2,3'-bipyridin-6-yl}amino)pyridine-4-carbonitrile,
(95) 2-({4-cyclopropyl-5'-[2-(morpholin-4-yl)ethoxy]-2,3'-bipyridine-6-yl}amino)pyridine-4-carbonitrile,
(96) 2-{[5'-(azetidin-3-yloxy)-4-cyclopropyl-2,3'-bipyridin-6-yl]amino}pyridine-4-carbonitrile,
(97) 2-[4-({6-[(4-cyanopyridin-2-yl)amino]-4-cyclopropyl-2,3'-bipyridine-5'-yl}oxy)-piperidine-1-yl]acetamide,
(98) 4-({6-[(4-cyanopyridin-2-yl)amino]-4-cyclopropyl-2,3'-bipyridin-5'-yl}oxy)piperidine-1-carboxamide,
(99) 2-({4-cyclopropyl-6-[2-(1,1-dioxidethiomorpholin-4-yl)pyrimidin-5-yl]pyridin-2-yl}amino)pyridine-4-carbonitrile,
(100) 4-(5-{6-[(4-cyanopyridin-2-yl)amino]-4-cyclopropylpyridin-2-yl}pyrimidin-2-yl)-1,4-diazepane-1-carboxamide,
(101) 2-({4-cyclopropyl-5'-[2-(dimethylamino)ethoxy]-2,3'-bipyridin-6-yl}amino)pyridine-4-carbonitrile,
(102) 2-({4-cyclopropyl-5'-[2-(dimethylamino)-2-methylpropoxy]-2,3'-bipyridin-6-yl}amino)pyridine-4-carbonitrile,
(103) 2-({6-[(4-cyanopyridin-2-yl)amino]-4-cyclopropyl-2,3'-bipyridin-5'-yl}oxy)acetamide,
(104) 2-{[5'-(4-acetyl-1,4-diazepan-1-yl)-4-cyclopropyl-2,3'-bipyridin-6-yl]amino}pyridine-4-carbonitrile,
(105) 2-{[4-cyclopropyl-5'-(3-hydroxypyrrolidin-1-yl)-2,3'-bipyridin-6-yl]amino}pyridine-4-carbonitrile,
(106) 2-({4-cyclopropyl-5'-[3-hydroxypiperidin-1-yl]-2,3'-bipyridine-6-yl}amino)pyridine-4-carbonitrile,
(107) 2-{[4-cyclopropyl-5'-(1-methyl-1H-pyrazol-4-yl)-2,3'-bipyridin-6-yl]amino}pyridine-4-carbonitrile,
(108) 2-{[4-cyclopropyl-5'-(3-oxopiperazin-1-yl)-2,3'-bipyridin-6-yl]amino}pyridine-4-carbonitrile,
(109) 2-{[4-cyclopropyl-5'-(4-hydroxypiperidin-1-yl)-2,3'-bipyridin-6-yl]amino}pyridine-4-carbonitrile,
(110) 2-({4-cyclopropyl-6-[1-(1,3-dihydroxypropan-2-yl)-1H-benzimidazol-5-yl)pyridin-2-yl}amino) pyridine-4-carbonitrile,
(111) 2-({4-cyclopropyl-6-[1-(2-hydroxy-2-methylpropyl)-1H-benzimidazol-5-yl)pyridin-2-yl}amino)pyridine-4-carbonitrile
(112) 2-({4-cyclopropyl-6-[1-(3-hydroxy-2,2-dimethylpropyl)-1H-benzimidazol-5-yl)pyridin-2-yl}amino)pyridine-4-carbonitrile,
(113) 2-[(4-cyclopropyl-6-{1-[(1-hydroxycyclohexyl)methyl]-1H-benzimidazol-5-yl}pyridin-2-yl]amino] pyridine-4-carbonitrile,
(114) 2-({4-cyclopropyl-6-[1-(3-hydroxypropyl)-1H-benzimidazol-5-yl)pyridin-2-yl}amino)pyridine-4-carbonitrile,
(115) 2-({4-cyclopropyl-6-[1-(trans-4-hydroxycyclohexyl)-1H-benzimidazol-6-yl)pyridin-2-yl}amino)pyridine-4-carbonitrile,
(116) Ethyl 3-(5-{6-[(4-cyanopyridine-2-yl)amino]-4-cyclopropylpyridin-2-yl}-1H-benzimidazol-1-yl)propanonate,
(117) 2-({4-cyclopropyl-6-[1-(3-hydroxypropyl)-1H-benzimidazol-6-yl)] pyridin-2-yl}amino)pyridine-4-carbonitrile,
(118) 3-(6-{6-[(4-cyanopyridin-2-yl)amino]-4-cyclopropylpyridin-2-yl}-1H-benzimidazol-1-yl)-N,N-dimethylpropanamide,
(119) methyl 3-(6-{6-[(4-cyanopyridin-2-yl)amino]-4-cyclopropylpyridin-2-yl}-1H-benzimidazol-1-yl) propanonate,
(120) 2-({4-cyclopropyl-6-[1-(4-hydroxybutyl)-1H-benzimidazol-6-yl]pyridin-2-yl}amino)pyridine-4-carbonitrile,
(121) 2-({4-[1-cyclopropyl-6-(4-hydroxybutyl)-1H-benzimidazol-5-yl)pyridin-2-yl}amino)pyridine-4-carbonitrile,
(122) 3-(5-{6-[(4-cyanopyridin-2-yl)amino]-4-cyclopropylpyridin-2-yl}-1H-benzimidazol-1-yl)propanamide,
(123) 2-({4-cyclopropyl-6-[1-(pyridin-3-ylmethyl)-1H-benzimidazol-6-yl]pyridin-2-yl}amino)pyridine-4-carbonitrile,
(124) 3-(5-{6-[(4-cyanopyridin-2-yl)amino]-4-cyclopropylpyridin-2-yl}-1H-benzimidazol-1-yl)-N,N-dimethylpropanamide,
(125) 3-(5-{6-[(4-cyanopyridin-2-yl)amino]-4-cyclopropylpyridin-2-yl}-1H-benzimidazol-1-yl)-N-methylpropanamide,
(126) 2-({4-cyclopropyl-6-[1-(pyridin-4-ylmethyl)-1H-benzimidazol-6-yl)pyridin-2-yl}amino)pyridine-4-carbonitrile,
(127) 2-({6-[1-(2-cyanoethyl)-1H-benzimidazol-5-yl]-4-cyclopropylpyridin-2-yl}amino)pyridine-4- carbonitrile,
(128) 2-[(4-cyclopropyl-6-{1-[1-(hydroxymethyl)cyclohexyl]-1H-benzimidazol-6-yl}pyridin-2-yl)amino]pyridine-4- carbonitrile,
(129) 2-({4-cyclopropyl-6-[1-(4,4-difluorocyclohexyl)-1H-benzimidazol-6-yl]pyridine-2-yl}amino)pyridine-4-carbonitrile,
(130) 2{[6-(1-benzyl-1H-benzimidazol-5-yl)-4-cyclopropylpyridine-2-yl]amino}pyridin-4-carbonitrile,
(131) 2-({4-cyclopropyl-6-[1-(trans-4-methoxycyclohexyl)-1H-benzimidazol-6-yl)pyridin-2-yl}amino)pyridine-4-carbonitrile,
(132) 2-({4-cyclopropyl-6-[1-(tetrahydro-2H-pyran-4-yl)-1H-benzimidazol-6-yl)pyridin-2-yl}amino)pyridine -4-carbonitrile,
(133) 2-[(4-cyclopropyl-6-{1-[trans-2-hydroxycyclopentyl]-1H-benzimidazol-6-yl}pyridine-2-yl)amino]pyridine-4-carbonitrile,
(134) trans-2-(6-{6-[(4-cyanopyridin-2-yl)amino]-4-cyclopropylpyridin-2-yl}-1H-benzimidazol-1-yl)cyclopentyl acetate,
(135) 2-{[4-cyclopropyl-6-(1-ethyl-1H-benzimidazol-6-yl)pyridin-2-yl]amino}pyridine-4-carbonitrile,
(136) 2-({4-cyclopropyl-6-[1-(1,3-dihydroxydimethylmethan-2-yl)-1H-benzimidazol-6-yl]pyridin-2-yl}amino)pyridine-4-carbonitrile,
(137) 2-({4-cyclopropyl-6-[1-(2-hydroxy-2-methylpropyl)-1H-benzimidazol-6-yl)pyridin-2-yl}amino)pyridine-4-carbonitrile,
(138) 2-({4-cyclopropyl-6-[1-(cis-4-hydroxycyclohexyl)-1H-benzimidazol-6-yl]pyridin-2-yl}amino)pyridine -4-carbonitrile,
(139) 2-[(4-cyclopropyl-6-{1-[trans-2-hydroxycyclohexyl]-1H-benzimidazol-6-yl}pyridin-2-yl]amino]pyridine-4-carbonitrile,
(140) 2-({4-cyclopropyl-6-[1-(2-hydroxyethyl)-1H-benzimidazol-5-yl)pyridin-2-yl}amino)pyridine-4-carbonitrile,
(141) 2-({4-cyclopropyl-6-[1-(2-ethoxyethyl)-1H-benzimidazol-5-yl]pyridin-2-yl}amino)pyridin-4-carbonitrile,
(142) 2-{[4-cyclopropyl-6-(1-ethyl-1H-benzimidazol-5-yl)pyridin-2-yl]amino}pyridine-4-carbonitrile,
(143) 2-{[4-cyclopropyl-6-(1-cyclopropyl-1H-benzimidazol-5-yl)pyridin-2-yl]amino}pyridin-4-carbonitrile,
(144) N-[2-(5-{6-[(4-cyanopyridin-2-yl)amino]-4-cyclopropylpyridin-2-yl}-1H-benzimidazol-1-yl)ethyl]acetamide,
(145) 2-[(4-cyclopropyl-6-{1-[trans-2-hydroxycyclohexyl]-1H-benzimidazol-5-yl}pyridin-2-yl]amino]pyridine-4-carbonitrile,
(146) 2-{[4-cyclopropyl-6-(1-methyl-1H-benzimidazol-6-yl)pyridin-2-yl]amino}pyridine-4-carbonitrile,
(147) 4-({6-[(4-cyanopyridine-2-yl)amino]-4-cyclopropyl-2,3'-bipyridin-6'-yl}methyl)-1,4-diazepane-1-carboxamide,
(148) 2-([4-cyclopropyl-6'-(1,1-dioxidethiomorpholin-4-yl)-4'-methyl-2,3'-bipyridin-6-yl]amino}pyridine-4-carbonitrile,
(149) 2-({4-cyclopropyl-6-[3-(trans-4-hydroxycyclohexyl)-2-oxo-2,3-dihydro-1H-benzimidazol-5-yl] pyridin- 2-yl}amino)pyridine-4-carbonitrile,
(150) 2-({4-cyclopropyl-6-[2-ethyl-1-(trans-4-hydroxycyclohexyl)-1H-benzimidazol-6-yl)pyridin-2-yl} amino)pyridine-4-carbonitrile,
(151) 2-({4-cyclopropyl-6-[1-(3-oxopiperazin-1-yl)isoquinolin-7-yl]pyridin-2-yl}amino)pyridine-4-carbonitrile,
(152) 2-({4-cyclopropyl-6-[1-(trans-4-hydroxycyclohexyl)-2-methyl-1H-benzimidazol-6-yl]pyridine-2-yl}amino)pyridine -4 - carbonitrile,
(153) 2-({4-cyclopropyl-6-[1-(trans-4-hydroxycyclohexyl)-2-(propan-2-yl)-1H-benzimidazol-6-yl)pyridin-2-yl}amino)pyridine-4-carbonitrile,
(154) 2-({4-cyclopropyl-6-[1-(trans-4-hydroxycyclohexyl)-2-(hydroxymethyl)-1H-benzimidazol-6-yl)pyridin-2-yl}amino)pyridine-4-carbonitrile,
(155) 2-({4-cyclopropyl-6-[1-(piperazin-1-yl)isoquinolin-7-yl]pyridin-2-yl}amino)pyridine-4-carbonitrile,
(156) 2-({4-cyclopropyl-6-[1-(4-hydroxypiperidin-1-yl)isoquinolin-7-yl]pyridin-2-yl}amino)pyridine-4-carbonitrile,
(157) 2-[(4-cyclopropyl-6-{1-[3-hydroxypyrrolidin-1-yl]isoquinolin-7-yl}pyridin-2-yl]amino]pyridine-4-carbonitrile,
(158) 2-({4-cyclopropyl-6-[1-(3-hydroxyazetidin-1-yl)isoquinolin-7-yl]pyridin-2-yl}amino)pyridine-4-carbonitrile,
(159) 2-[(4-cyclopropyl-6-{1-[2-(hydroxymethyl)pyrrolidin-1-yl]isoquinolin-7-yl}pyridin-2-yl]amino]pyridine-4-carbonitrile,
(160) 2-[(4-cyclopropyl-6-{1-[3-fluoropyrrolidin-1-yl]isoquinolin-7-yl}pyridin-2-yl)amino]pyridine-4-carbonitrile,
(161) 2-[(6-{1-[(3R)-3-aminopyrrolidin-1-yl]isoquinolin-7-yl}-4-cyclopropylpyridin-2-yl)amino]pyridine-4-carbonitrile,
(162) 2-({6-[1-(4-cyanopiperidin-1-yl)isoquinolin-7-yl]-4-cyclopropylpyridin-2-yl}amino)pyridine-4-carbonitrile,
(163) 2-({4-cyclopropyl-6-[1-(2-oxo-imidazolidin-1-yl)isoquinolin-7-yl]pyridin-2-yl}amino)pyridine-4-carbonitrile,
(164) 2-({6-[1-(trans-4-aminocyclohexyl)-1H-benzimidazol-6-yl]-4-cyclopropylpyridin-2-yl}amino)pyridine-4-carbonitrile,
(165) 2-({4-[1-cyclopropyl-6-(piperidin-4-yl)-1H-benzimidazol-6-yl)pyridin-2-yl}amino)pyridine-4-carbonitrile,
(166) 2-[(4-cyclopropyl-6-{1-(6-oxo-1,6-dihydropyridin-3-yl)methyl]-1H-benzimidazol-6-yl}pyridin-2-yl)amino]pyridine-4-carbonitrile,
(167) 2-({4-cyclopropyl-6-[2-(trans-4-hydroxycyclohexyl)-1H-benzimidazol-6-yl)pyridin-2-yl}amino)pyridine-4-carbonitrile,
(168) 2-({6-[1-(trans-4-cyanocyclohexyl)-1H-benzimidazol-6-yl]-4-cyclopropylpyridin-2-yl}amino)pyridine-4-carbonitrile,
(169) 2-{[4-cyclopropyl-6'-(1,1-dioxidethiomorpholin-4-yl)-5'-fluoro-2,3'-bipyridin-6-yl]amino}pyridine-4-carbonitrile,
(170) 2-(14-cyclopropyl-6-[1-(trans-4-hydroxycyclohexyl)-1H-benzimidazol-5-yl)pyridin-2-yl}amino)pyridine-4-carbonitrile.

Some of the compounds of the present invention exist in the form of tautomers, and these respective isomers and mixtures thereof are also included in the present invention.
The tautomers in the compound of the present invention refer to, for example, keto and enol isomers based on a carbonyl group present in the molecule.

Hereinafter, the respective terms in the present description will be described in detail.
Examples of the "halogen" may include fluorine, chlorine, bromine, and iodine.

Examples of the "alkyl" may include linear or branched alkyl having 1 to 8 carbon atoms, and specific examples thereof may include methyl, ethyl, n-propyl, isopropyl, n-butyl, isobutyl, sec-butyl, tert-butyl, n-pentyl, isopentyl, n-hexyl, isohexyl, n-heptyl, isoheptyl, and n-octyl. Among these, alkyl having 1 to 6 carbon atoms is preferred, and alkyl having 1 to 3 carbon atoms is more preferred.
Examples of an alkyl moiety in the "alkylsulfonyl", "alkylcarbonyl", "alkyloxycarbonyl", "alkylcarbonylamino", "alkylcarbonyloxy", "hydroxyalkyl", "monoalkylamino", "dialkylamino", "monoalkylaminocarbonyl", "dialkylaminocarbonyl", "carbamoylalkyl", "monoalkylaminocarbonylalkyl", "dialkylaminocarbonylalkyl", "hydroxycarbonylalkyl", "monoalkylaminocarbonylamino", or "dialkylaminocarbonylamino" may include the same groups as the above-described "alkyl".

Examples of the "alkylene" may include linear or branched alkylene having 1 to 8 carbon atoms, and specific examples thereof may include methylene, ethylene, propylene, butylene, pentylene, hexylene, heptylene, and octylene.

Examples of the "cycloalkyl" may include cycloalkyl having 3 to 8 carbon atoms, and specific examples thereof may include cyclopropyl, cyclobutyl, cyclopentyl, cyclohexyl, cycloheptyl, and cyclooctyl.
Examples of the "cycloalkylene" may include cycloalkylene having 3 to 8 carbon atoms, and specific examples thereof may include 1,1-cyclopropylene, 1,2-cyclopropylene, 1,1-cyclobutylene, 1,2-cyclobutylene, 1,3-cyclobutylene, 1,1-cyclopentylene, 1,2-cyclopentylene, 1,3-cyclopentylene, 1,1-cyclohexylene, 1,2-cyclohexylene, 1,3-cyclohexylene, 1,4-cyclohexylene, 1,1-cycloheptylene, 1,2-cycloheptylene, 1,3-cycloheptylene, 1,4-cycloheptylene, 1,1-cyclooctylene, 1,2-cyclooctylene, 1,3-cyclooctylene, 1,4-cyclooctylene, and 1,5-cyclooctylene.

Examples of the "alkoxy" may include linear or branched alkoxy having 1 to 8 carbon atoms, and specific examples thereof may include methoxy, ethoxy, n-propoxy, isopropoxy, n-butoxy, isobutoxy, sec-butoxy, t-butoxy, n-pentyloxy, n-hexyloxy, n-heptyloxy, and n-octyloxy.

Examples of the "4- to 7-membered saturated heterocyclic group, which has one or two heteroatoms" may include a 4-to 7-membered saturated heterocyclic group, which has one or two atoms selected from S, N, and O as a ring constituting atom, and specific examples thereof may include 1-azetidinyl, 3-azetidinyl, 1-pyrrolidinyl, 3-pyrrolidinyl, 2-tetrahydropyranyl, 3-tetrahydropyranyl, 4-tetrahydropyranyl, piperidino, 4-piperidinyl, 1-piperazinyl, morpholino, thiomorpholino, 1-homopiperazinyl, and 4-tetrahydropyranyl.

Examples of the "aryl" may include aryl having 6 to 10 carbon atoms, and specific examples thereof may include phenyl, 1-naphthyl, and 2-naphthyl. Among these, phenyl is preferred.
Examples of the "heteroaryl" may include a 5- or 6-membered monocyclic aromatic heterocyclic group having 1 to 4 atoms selected from N, O, and S as a ring constituting atom, and a fused bicyclic heterocyclic group having 8 to 10 ring constituting atoms and having 1 to 4 atoms selected from N, O, and S as a ring constituting atom, and specific examples thereof may include furyl (e.g., 2-furyl and 3-furyl), thienyl (e.g., 2-thienyl and 3-thienyl), pyrrolyl (e.g., 1-pyrrolyl, 2-pyrrolyl, and 3-pyrrolyl), imidazolyl (e.g., 1-imidazolyl, 2-imidazolyl, and 4-imidazolyl), pyrazolyl (e.g., 1-pyrazolyl, 3-pyrazolyl, and 4-pyrazolyl), triazolyl (e.g., 1,2,4-triazol-1-yl, 1,2,4-triazol-3-yl, and 1,2,4-triazol-4-yl), tetrazolyl (e.g., 1-tetrazolyl, 2-tetrazolyl, and 5-tetrazolyl), oxazolyl (e.g., 2-oxazolyl, 4-oxazolyl, and 5-oxazolyl), isoxazolyl (e.g., 3-isoxazolyl, 4-isoxazolyl, and 5-isoxazolyl), oxadiazolyl (e.g., 1,3,4-oxadiazol-2-yl), thiazolyl (e.g., 2-thiazolyl, 4-thiazolyl, and 5-thiazolyl), thiadiazolyl, isothiazolyl (e.g., 3-isothiazolyl, 4-isothiazolyl, and 5-isothiazolyl), pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), pyrazinyl (e.g., 2-pyrazinyl), benzimidazolyl (e.g., 1-benzimidazolyl, 2-benzimidazolyl, 4-benzimidazolyl, 5-benzimidazolyl, 6-benzimidazolyl, and 7-benzimidazolyl), indazolyl (e.g., 1-indazolyl, 3-indazolyl, 4-indazolyl, 5-indazolyl, 6-indazolyl, and 7-indazolyl), and isoquinolyl (e.g., 1-isoquinolyl, 3-isoquinolyl, 4-isoquinolyl, 5-isoquinolyl, 6-isoquinolyl, 7-isoquinolyl, and 8-isoquinolyl). As the "heteroaryl" represented by R, pyridyl (e.g., 2-pyridyl, 3-pyridyl, and 4-pyridyl), pyrimidinyl (e.g., 2-pyrimidinyl, 4-pyrimidinyl, and 5-pyrimidinyl), benzimidazolyl (e.g., 1-benzimidazolyl, 2-benzimidazolyl, 4-benzimidazolyl, 5-benzimidazolyl, 6-benzimidazolyl, and 7-benzimidazolyl), indazolyl (e.g., 1-indazolyl, 3-indazolyl, 4-indazolyl, 5-indazolyl, 6-indazolyl, and 7-indazolyl), and isoquinolyl (e.g., 1-isoquinolyl, 3-isoquinolyl, 4-isoquinolyl, 5-isoquinolyl, 6-isoquinolyl, 7-isoquinolyl, and 8-isoquinolyl) are preferred. As the "heteroaryl" represented by R^{A}, imidazolyl (e.g., 1-imidazolyl, 2-imidazolyl, and 4-imidazolyl) is preferred.

### [Brief Description of Drawings]

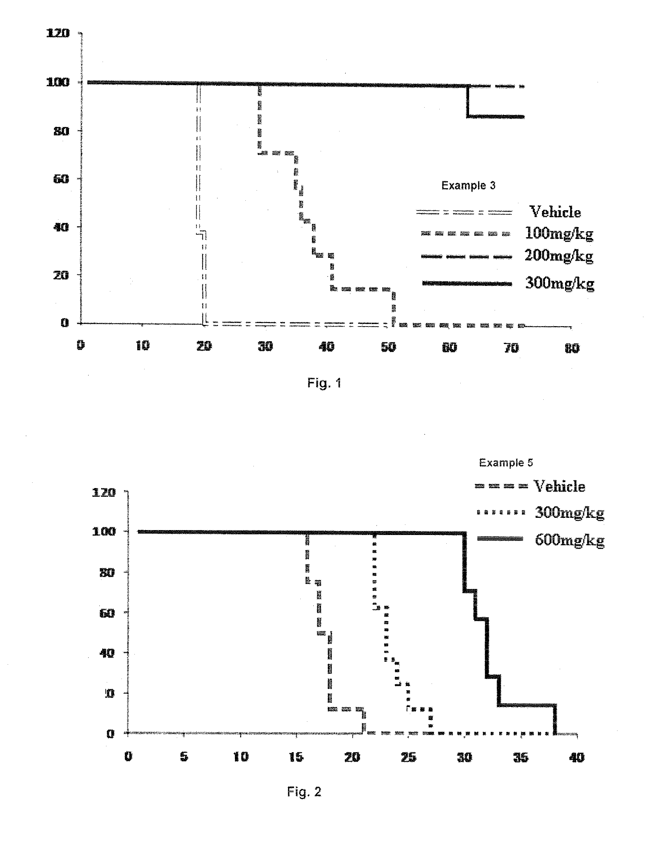

[Fig. 1] Fig. 1 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.
[Fig. 2] Fig. 2 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.

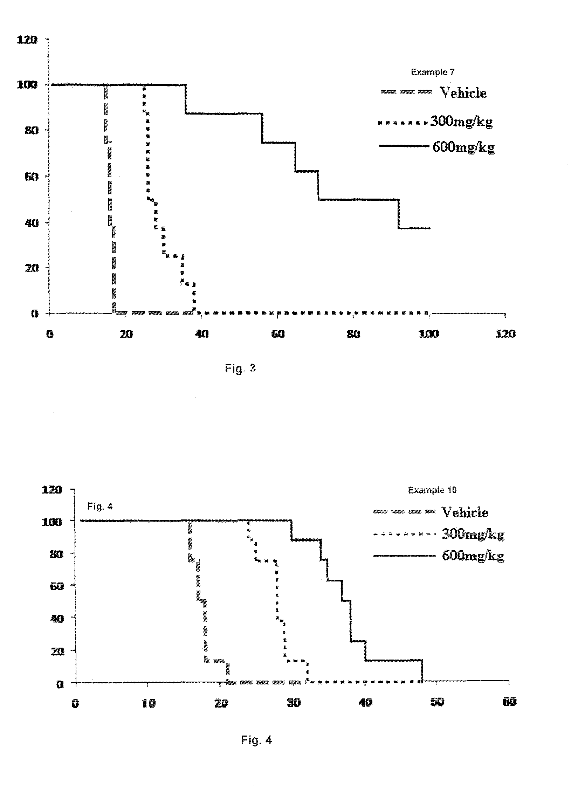

[Fig. 3] Fig. 3 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.
[Fig. 4] Fig. 4 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.

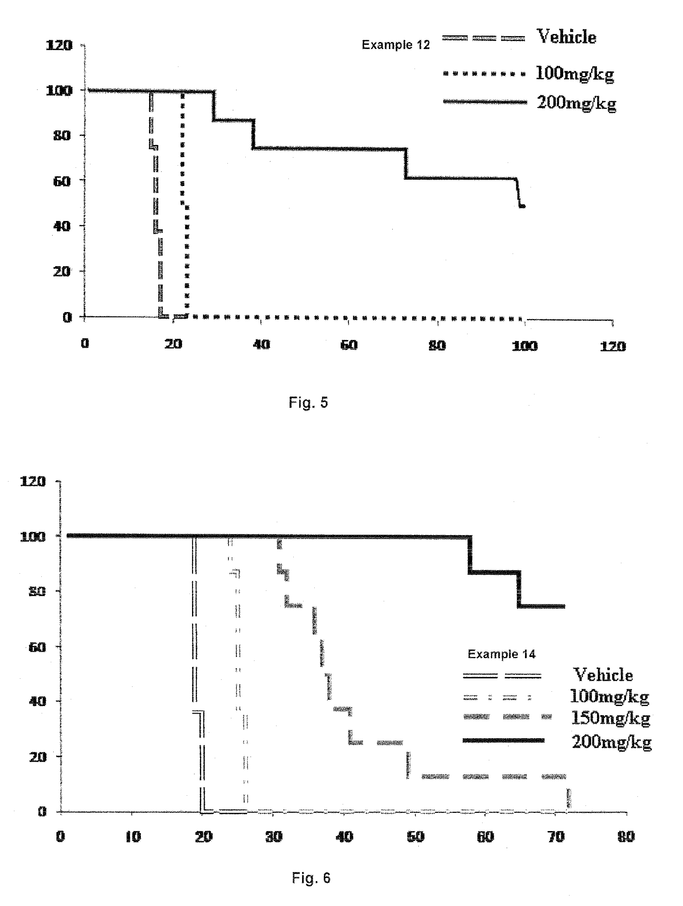

[Fig. 5] Fig. 5 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.
[Fig. 6] Fig. 6 shows evaluation of a drug efficacy (life-prolonging effect) using a mouse model of leukemia obtained by transplantation of Ba/F3 cells that express TEL-Syk fusion protein into a nude mouse. The vertical axis indicates a survival rate (%), and the horizontal axis indicates the number of days after the Ba/F3 cells that express TEL-Syk fusion protein were transplanted into the nude mouse.

### [Description of Embodiments]

The compound of the present invention can be produced according to, for example, the following method from a known compound or an intermediate which can be easily synthesized. In the production of the compound of the present invention, in the case where a starting material has a substituent which affects a reaction, the reaction is generally performed after the starting material is protected with a suitable protecting group according to a known method in advance. The protecting group can be removed by a known method after the reaction.

### Production Method 1

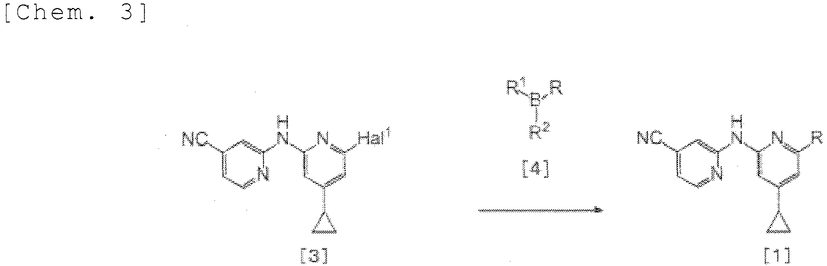

(In the formulae, R has the same definition as described above; R¹ and R² each represent hydroxy or are combined together to represent -O-C(CH₃)₂-C(CH₃)₂-O-, -O-(CH₂)₃-O-, or -O-CH₂-C(CH₃)₂-CH₂-O-; and Hall represents halogen.)
This reaction can be performed using a compound [3], an organoboron compound [4], and a palladium catalyst by a method known as a cross-coupling reaction using a palladium catalyst. This reaction can be performed, for example, in the presence of a palladium catalyst and a base in an appropriate solvent at a temperature ranging from 20°C to 200°C. Examples of the usable palladium catalyst may include tetrakis(triphenylphosphine)palladium, dichlorobis(triphenylphosphine)palladium, 1,1'-bis(diphenylphosphino)ferrocene-palladium(II) dichloride-dichloromethane complex, tris (dibenzylideneacetone) (chloroform) dipalladium(0), tris(dibenzylideneacetone)dipalladium(0), and palladium(II) acetate. An appropriate amount of the usable palladium catalyst is in a range of from 0.001 mol to 0.3 mol with respect to 1 mol of halogenated aryl. Examples of a ligand for the usable palladium catalyst may include 1,1'-bis(diphenylphosphino) ferrocene, 4,5-bis(diphenylphosphino)-9,9'-dimethylxanthene, 2-dicyclohexylphosphino-2',4',6'-triisopropylbiphenyl, 2-dicyclohexylphosphino-2',6'-dimethoxy-1,1'-biphenyl, (±)-2,2'-bis (diphenylphosphino)-1,1'-binaphthyl, 2-(di-t-butylphosphino)biphenyl, bis [2-(diphenylphosphino) phenyl] ether, and tri-tert-butylphosphine. The usable reaction solvent is not particularly limited as long as it is inert in the reaction, however, examples thereof may include ethers such as tetrahydrofuran, 1,4-dioxane, and 1,2-dimethoxyethane; alcohol-based solvents such as methanol and ethanol; amide-based solvents such as N,N-dimethylformamide and N,N-dimethylacetamide; hydrocarbon-based solvents such as benzene and toluene; water; and mixed solvents thereof. Examples of the usable base may include sodium hydroxide, potassium carbonate, sodium carbonate, and potassium phosphate. The reaction time varies depending on the type of a starting material to be used and the reaction temperature, however, in general, a reaction time ranging from 30 minutes to 24 hours is appropriate.
As the organoboron compound [4] to be used in this reaction, a purified compound or a compound in a state of a crude product can be used. Further, the compound [1] can also be produced by putting the compound [3] directly in a reaction system in which the organoboron compound [4] has been prepared.

The compound [3], which is a starting material compound, can be produced according to, for example, the following method. 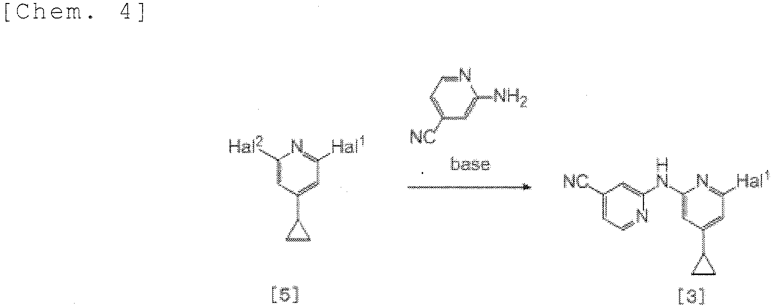 (In the formulae, Hall has the same definition as described above; and Hal² represents halogen.)
This reaction is an addition reaction to be performed in the presence of a base using a compound [5] and 2-amino-4-cyanopyridine, and can be performed by a known method per se. For example, this reaction can be performed in the presence of a base in an appropriate solvent at a temperature ranging from room temperature to 200°C. Examples of the usable base may include sodium hydride, sodium tert-butoxide, and potassium phosphate. The usable solvent is not particularly limited as long as it is inert in the reaction, examples thereof may include hydrocarbon-based solvents such as toluene and xylene; ether-based solvents such as 1,4-dioxane and tetrahydrofuran; amide-based solvents such as N,N-dimethylformamide, N,N-dimethylacetamide, N-methyl-2-pyrrolidone, and 1,3-dimethyl-2-imidazolidinone; dimethyl sulfoxide; and mixed solvents thereof. The reaction time varies depending on the type of a starting material to be used and the reaction temperature, in general, the reaction time ranging from 30 minutes to 24 hours is appropriate.

Further, the compound [4] which is a starting material compound can be produced according to, for example, the following method. 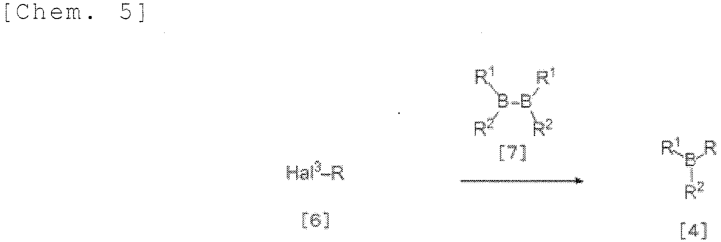 (In the formulae, R, R¹, and R² have the same definitions as described above; and Hal³ represents halogen.)
This reaction can be performed in the same manner as the above-described Production Method 1 using a compound [6], an organoboron compound [7], and a palladium catalyst.

### Production Method 2

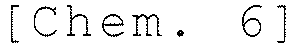

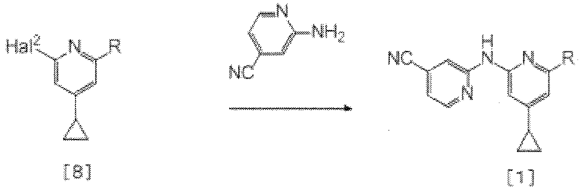

(In the formulae, R and Hal² have the same definitions as described above.)
This reaction is a condensation reaction between a compound [8] and 2-amino-4-cyanopyridine using a palladium catalyst, and therefore can be performed by a known method per se as a condensation reaction. For example, this reaction can be performed in the presence of a palladium catalyst and a base in an appropriate solvent at a temperature ranging from 20°C to 200°C. The usable solvent is not particularly limited as long as it is inert in the reaction, examples thereof may include hydrocarbon-based solvents such as toluene and xylene; ether-based solvents such as 1,4-dioxane and tetrahydrofuran; amide-based solvents such as N,N-dimethylformamide, N,N-dimethylacetamide, and N-methyl-2-pyrrolidone; and mixed solvents thereof. Examples of the usable palladium catalyst may include tris(dibenzylideneacetone)(chloroform)dipalladium(0), tris(dibenzylideneacetone)dipalladium(0), and palladium(II) acetate. An appropriate amount of the usable palladium catalyst is in a range of from 0.001 mol to 1.0 mol with respect to 1 mol of halogenated aryl. Examples of a ligand for the usable palladium catalyst may include 1,1'-bis(diphenylphosphino)ferrocene, 4,5-bis(diphenylphosphino)-9,9'-dimethylxanthene, 2-dicyclohexylphosphino-2',4',6'-triisopropylbiphenyl, (±)-2,2'-bis(diphenylphosphino)-1,1'-binaphthyl, 2-(di-t-butylphosphino)biphenyl, bis [2-(diphenylphosphino) phenyl] ether, and tri-t-butylphosphine. Examples of the usable base may include sodium tert-butoxide, potassium phosphate, and cesium carbonate. The reaction time varies depending on the type of a starting material to be used and the reaction temperature, In general, a reaction time ranging from 10 minutes to 24 hours is appropriate.

Further, the compound [8] which is a starting material compound can be produced according to, for example, the following method. 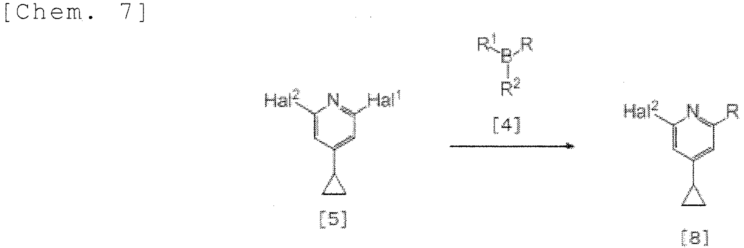 (In the formulae, R, R¹, R², Hall, and Hal² have the same definitions as described above.)
This reaction can be performed in the same manner as the above-described Production Method 1 using a compound [5], an organoboron compound [4], and a palladium catalyst.

Further, compounds of the present invention described below can also be produced on the basis of production methods described below.
A compound of the present invention having a "-CO₂H" moiety can be produced by producing a compound of the present invention having a "-CO₂R³" (wherein R³ represents alkyl) moiety corresponding thereto according to any of the above-described Production Methods 1 to 3, followed by a hydrolysis reaction.
A compound of the present invention having a "-CONR⁴(R⁵)" (wherein R⁴ and R⁵ are the same or different and represent H or alkyl) moiety can be produced by subjecting a compound of the present invention having "-CO₂H" corresponding thereto and a compound represented by NHR⁴ (R⁵) (wherein R⁴ and R⁵ have the same definitions as described above) to an amidation reaction.
A compound of the present invention having a "-N(COR⁶) -" (wherein R⁶ represents alkyl) moiety can be produced by reacting a compound of the present invention having a "-NH-" moiety corresponding thereto with, for example, a compound represented by (R⁶CO)₂O (wherein R⁶ has the same definition as described above) or a compound represented by R⁶CO-Hal⁴ (wherein R⁶ has the same definition as described above, and Hal⁴ represents halogen.).
A compound of the present invention having a "-N (CONHR⁷) -" (wherein R⁷ represents alkyl) moiety can be produced by reacting a compound of the present invention having a "-NH-" moiety corresponding thereto with, for example, a compound represented by R⁷-N=C=O (wherein R⁷ has the same definition as described above).
A compound of the present invention having a "-N (CONH₂) -" moiety can be produced by reacting a compound of the present invention having a "-NH-" moiety corresponding thereto with, for example, trimethylsilyl isocyanate.
A compound of the present invention having a "-N (CSNHR⁸) -" (wherein R⁸ represents alkyl) moiety can be produced by reacting a compound of the present invention having a "-NH-" moiety corresponding thereto with, for example, a compound represented by R⁸-N=C=S (wherein R⁸ has the same definition as described above).
A compound of the present invention having a "-N(CSNH₂)-" moiety can be produced by reacting a compound of the present invention having a "-NH-" moiety corresponding thereto with, for example, trimethylsilyl thioisocyanate.
A compound of the present invention having a "-N (SO₂R⁹) -" (wherein R⁹ represents alkyl) moiety can be produced by reacting a compound of the present invention having a "-NH-" moiety corresponding thereto with, for example, a compound represented by (R⁹SO₂-Hal⁵ (wherein R⁹ has the same definition as described above, and Hal⁵ represents halogen.).
A compound of the present invention having a "-OCOR¹⁰" (wherein R¹⁰ represents alkyl) moiety can be produced by reacting a compound of the present invention having a "-OH" moiety corresponding thereto with, for example, a compound represented by (R¹⁰CO)₂O (wherein R¹⁰ has the same definition as described above) or a compound represented by R¹⁰ CO-Hal⁶ (wherein R⁹ has the same definition as described above, and Hal⁶ represents halogen.).

The compound of the present invention can be used as a medicinal agent as it is, but can also be used after it is converted into a pharmaceutically acceptable salt by a known method. Examples of such a salt may include salts of mineral acids such as hydrochloric acid, hydrobromic acid, sulfuric acid, and phosphoric acid; and salts of organic acids such as acetic acid, citric acid, tartaric acid, maleic acid, succinic acid, fumaric acid, p-toluenesulfonic acid, benzenesulfonic acid, and methanesulfonic acid.
For example, a hydrochloride of the compound of the present invention can be obtained by reacting the compound of the present invention with hydrochloric acid, or an alcohol solution, an ethyl acetate solution, a 1,4-dioxane solution, or a diethyl ether solution of hydrogen chloride.

Some of the compounds of the present invention have an asymmetric carbon, and the respective optical isomers and mixtures thereof are all included in the present invention. The optical isomers can be produced by, for example, optical resolution of racemates using an optically active acid (such as tartaric acid, dibenzoyltartaric acid, mandelic acid, or 10-camphorsulfonic acid) utilizing its basicity according to a known method, or by using an optically active compound prepared in advance as a starting material. In addition, the optical isomers can also be produced by optical resolution using a chiral column or by asymmetric synthesis.

The compound of the present invention or a pharmaceutically acceptable salt thereof has a high Syk tyrosine kinase inhibitory activity as shown in the following test examples, and can be used as a preventive agent or a therapeutic agent for a disease associated with Syk tyrosine kinase, for example, an allergic disease (e.g., bronchial asthma, allergic rhinitis, allergic dermatitis, or allergic conjunctivitis), an autoimmune disease (e.g., chronic rheumatoid arthritis, idiopathic thrombocytopenic purpura, systemic lupus erythematosus, or multiple sclerosis) or a malignant tumor (e.g., a B-cell lymphoma (e.g., small-cell lymphoma), a B-cell leukemia (e.g., chronic lymphocytic leukemia), peripheral T-cell lymphoma not-otherwise specified, angioimmunoblastic T-cell lymphoma, anaplastic large-cell lymphoma, cutaneous anaplastic large-cell lymphoma, mycosis fungoides, enteropathy-associated T-cell lymphoma, extranodal NK-T-cell lymphoma, hepatosplenic T-cell lymphoma, subcutaneous panniculitis-like T-cell lymphoma, diffuse large-cell lymphoma, or follicular lymphoma).

When the compound of the present invention or a pharmaceutically acceptable salt thereof is administered as a medicinal agent, the compound of the present invention or a pharmaceutically acceptable salt thereof is administered to mammals including humans as it is or as a pharmaceutical composition containing it at, for example, 0.001% to 99.5%, preferably 0.1% to 90% in a pharmaceutically acceptable nontoxic and inactive carrier.
As the carrier, at least one member selected from solid, semi-solid, or liquid excipients, fillers, and other auxiliaries for pharmaceutical formulation is used. The pharmaceutical composition according to the present invention is desirably administered in a unit dosage form. The pharmaceutical composition can be administered by interstitial administration, oral administration, intravenous administration, topical administration (such as transdermal administration, instillation, intraperitoneal administration, or intrathoracic administration), or transrectal administration. It is needless to say that the composition is administered in a dosage form suitable for these administration methods.
The dose as the medicinal agent is desirably determined in consideration of the conditions of a patient, such as age, body weight, and the type and severity of a disease, administration route, the type of the compound of the present invention (a racemate or an optically active substance), whether or not the compound is a salt, if so, the type of the salt, etc. However, in general, in the case of oral administration, a dose as an active ingredient of the compound of the present invention or a pharmaceutically acceptable salt thereof for an adult is suitably in a range of from 0.01 mg to 5 g/day/adult, preferably from 1 mg to 1 g/day/adult. In some cases, a dose lower than the above range may be sufficient, or conversely, a dose higher than the above range may be required. In general, the daily dose may be administered in a single dose or in several divided doses. In the case of intravenous administration, the medicinal agent can be promptly administered or continuously administered in, for example, 24 hours.

### [Examples]

Hereinafter, the present invention will be described in more detail with reference to Reference Examples, Examples, Test Examples, and Formulation Examples, however, the invention is not limited only thereto.

### Reference Example 1 2,6-dichloropyridin-4-yl trifluoromethanesulfonate

To a solution of 2,6-dichloropyridine-4-ol (24.6g) (J. Am. Chem. Soc. 2003, 125, 7792-7793) and triethylamine (50.2 mL) in dichloromethane (450 mL) was added dropwise trifluoromethane sulfonic anhydride (30.3 mL) over 40 min. at 0 °C. 4-Dimethylaminopyridine (hereinafter referred to as DMAP) was added to the resulting solution, and the reaction mixture was stirred at room temperature for an hour. The reaction mixture was diluted with 500 mL of water and then extracted with 150 mL of chloroform. The organic layer was dried over anhydrous magnesium sulfate, evaporated under reduced pressure.The resulting residue was purified by column chromatography to give 36.5 g of the title compound as a pale yellow solid.

### Reference Example2 2,6-dichloro-4-cyclopropylpyridine

To a solution of 2,6-dichloropyridin-4-yl trifluoromethanesulfonate (38.5 g) obtained in Reference Example 1 in toluene (220 mL) was added cyclopropylboronic acid (11.4 g), cesium carbonate (53.1g), water (110 mL), 1,1'-bis(diphenylphosphino)ferrocene-palladium dichloride-dichloromethane complex (hereinafter referred to as PdCl₂(dppf)·CH₂Cl₂) (4.3 g) successively and the interior of a vessel was purged with argon. After the mixture was stirred for an hour at 70 °C, the reaction mixture was diluted with ethyl acetate, followed by extraction, the organic layer was washed with water.The organic layer was dried over anhydrous magnesium sulfate and evaporated under reduced pressure. The resulting residue was purified by column chromatography to give 21.7 g of the title compound as a white solid.

### Reference Example3 2-[(6-chloro-4-cyclopropylpyridin-2-yl)amino]pyridine-4-carbonitrile

To a suspension of sodium hydride (6.7 g) in 1,3-dimethyl-2-imidazolidinone (130mL) was added portionwise 2-amino-4-cyanopyridine (10.0 g), and the mixture was stirred at 0 °C for 30 minutes. 2,6-Dichloro-4-cyclopropylpyridine (13.2 g) obtained in Reference Example 2 in 1,3-dimethyl-2-imidazolidinone (70 mL) was added to the resulting mixture, and the reaction mixture was stirred at 100 °C for 2 hours. After cooling, 500 mL of water was added gradually at 0 °C, and the reaction mixture was stirred at room temperature for an hour. The precipitate was collected by filtration, washed with water to give 17.3 g of the title compound as a pale brown solid.

### Reference Example4 tert-butyl (6-chloro-4-cyclopropylpyridin-2-yl) (4-cyanopyridin-2-yl) carbamate

To a suspension of 2-[(6-chloro-4-cyclopropylpyridin-2-yl)amino]pyridine-4-carbonitrile obtained in Reference Example 3 in dichloromethane (60 mL) was successively added pyridine (3.6 mL), Di-*tert*-butyl dicarbonate (hereinafter referred to as Boc₂O) (8.7 mL), DMAP (489 mg), and the reaction mixture was stirred at room temperature for 30 minutes. After the solvent was evaporated under reduced pressure, the obtained residue was purified by column chromatography to give 8.0 g of the title compound as a pale yellow solid.

### Reference Example 5 5-bromo-N-(2-{[tert-butyl(dimethyl)silyl]oxy}ethyl)pyridine-2-amine

### Step 1 Production of 2-[(5-bromopyridin-2-yl)amino] ethanol

A mixture of 5-bromo-2-chloropyridine (1.0 g) and 2-aminoethanol (6mL) was stirred at 130 °C for 24 hours. The reaction mixture was diluted with water, followed by carrying out an operation of extraction twice with ethyl acetate, and evaporated under reduced pressure. The resulting residue was purified by column chromatography to give 970 mg of the title compound as a white powder.

### Step 2 Production of 5-bromo-N-(2-{[tert-butyl (dimethyl) silyl]oxy}ethyl)pyridine-2-amine

2-[(5-bromopyridin-2-yl)amino] ethanol (490 mg) obtained in Step 1 was dissolved in N,N-dimethylformamide (hereinafter referred to as DMF) (10 mL), and imidazole (170 mg) and tert- butyldimethyl chloro silane (hereinafter referred to as TBSCl) (410 mg) were sequentially added, and the reaction mixutre was stirred at room temperature for 2 hours. The reaction mixture was diluted with ethyl acetate, and water, then extracted. The organic layer was wased with saturated brine, evaporated under reduced pressure. The resulting residue was purified by column chromatography to give 718 mg of the title compound as a yellow oil.

### Reference Example 6 4-(5-bromopyridin-2-yl)thiomorpholine 1,1-dioxide

2,5-Dibromopyridine (500 mg)and thiomorpholine-1,1-dioxide (860 mg) were dissolved in 2-methoxyethanol (3 mL), and the mixture was stirred at 120 °C for 3 days. The reaction mixture was diluted with ethyl acetate, washed successively with water and saturated brine, and the solvent was evaporated under reduced pressure. The resulting residue was purified by column chromatography to give 200 mg of the title compound as a white solid.

### Reference Example 7 1-(5-bromopyridin-2-yl)piperidin-4-on

### Step 1 Production of 1-(5-bromopyridin-2-yl)piperidin-4-ol

A mixture of 2,5-dibromopyridine (10.0 g) and piperidin-4-ol (13.0 g) were stirred at 130 °C for 4 hours. The reaction mixture was diluted with water, extracted with ethyl acetate and the solvent was evaporated under reduced pressure. The resulting residue was purified by column chromatography to give 10.4 g of the title compound as a white solid.

### Step 2 Production of 1-(5-bromopyridin-2-yl)piperidin-4-on

1-(5-Bromopyridin-2-yl)piperidin-4-ol (300 mg) obtained in step 1 was dissolved in dichloromethane (10 mL), and then 1,1,1-tris(acetyloxy)-1λ⁵,2-benziodoxol-3(1H)-on (Dess-Martin reagent) (990 mg) was added. The mixutre was stirred at room temperature for an hour. The reaction mixture was diluted with saturated aqueous sodium hydrogen carbonate solution, extracted with ethyl acetate and the solvent was evaporated under reduced pressure. The resulting residue was purified by column chromatography to give 245 mg of the title compound as a white solid.

### Reference Example 8 tert-Butyl 4-(5-bromopyridin-2-yl)-2-oxo-piperazine-1-carboxylate

### Step 1 Production of 4-(5-bromopyridin-2-yl)piperazin-2-on

2,5-Dibromopyridine (500 mg) and piperazin-2-on (634 mg) were dissolved in 2-ethoxyethanol (1 mL), and then N,N-diisopropylethylamine (1.1 mL) was added. The mixutre was stirred at 120 °C for 17 hours.The reaction mixure was diluted with water, followed by extraction with chloroform/methanol (5/1), and then the solvent was evaporated under reduced pressure. The resulting residue was purified by column chromatography to give 318 mg of the title compound as a pale yellow solid.

### Step 2 Production of tert-Butyl 4-(5-bromopyridin-2-yl)-2-oxo-piperazine-1-carboxylate

4-(5-Bromopyridin-2-yl)piperazin-2-on (182 mg) obtained in step 1 was suspended in dichloromethane (6 mL), Boc₂O (233 mg), DMAP (10 mg), triethylamine (0.2 mL) were added, and the mixture was stirred at room temperature. DMF was added to the reaction mixture, and the insolubles were dissolved, and then the mixture was stirred at room temperature for 15 hours. The reaction mixure was diluted with ethyl acetate, washed with saturated brine, and then the solvent was evaporated under reduced pressure. The resulting residue was purified by column chromatography to give 237 mg of the title compound as a white solid.

### Reference Example 9 5-Bromo-2-(3-{[tert-butyl(dimethyl)silyl]oxy}azetidin-1-yl)pyridine

Step1 Production of 1-(5-bromopyridin-2-yl)azetidin-3-ol To a mixture of 5-bromo-2-fluoropyridine (517 mg) and azetidin-3-ol hydrochloride (644 mg) was added 1,8-diazabicyclo[5.4.0]undec-7-en (hereinafter referred to as DBU) (2.2 mL), and the reaction mixture was stirred at 160 °C for an hour. The reaction mixture was diluted with water, extracted with ethyl acetate, and the solvent was evaporated under reduced pressure. The resulting residue was purified by column chromatography to give 630 mg of the title compound as a white solid.

### Step 2 Production of 5-bromo-2-(3-{[tert-butyl(dimethyl)silyl]oxy}azetidin-1-yl)pyridine

1-(5-Bromopyridine-2-yl)azetidin-3-ol (630 mg) obtained in Reference Example 9 step 1 was dissolved in DMF (10 mL), imidazole (225 mg) and TBSCl (500 mg) were sequentially added, and the reaction mixutre was stirred at room temperature for 17 hours. The reaction mixture was diluted with ethyl acetate, washed successively with water and saturated brine, and the solvent was evaporated under reduced pressure. The resulting residue was purified by column chromatography to give 950 mg of the title compound as a white solid.

### Reference Example 10 tert-butyl 4-(5-bromopyridin-2-yl)piperazin-1-carboxylate

To a 2,5-dibromopyridine (2.0 g) in 2-methoxyethanol (2 mL) was added N,N-diisopropylethylamine (4.4 mL), tert-butyl piperazine-1-carboxylate (4.8 g) and the mixture was stirred at 120 °C overnight. The reaction mixture was diluted with water, and extracted three times with ethyl acetate. The organic layer was washed with saturated brine and dried over Magnesium sulfate. The solvent was evaporated under reduced pressure. The resulting residue was purified by column chromatography to give 2.9 g of the title compound as a white solid.

### Reference Example 11 (4R)-1-(5-bromopyridin-2-yl)-4-{[tert-butyl(dimethyl)silyl]oxy}pyrrolidin-2-on

### Step1 Production of (4R)-4-{[tert-butyl(dimethyl)silyl]oxy}pyrrolidin-2-on

(4R)-4-Hydroxy-pyrrolidin-2-on (505 mg) was dissolved in DMF (3 mL), imidazole (510 mg) and TBSCl (830 mg) were sequentially added, and the reaction mixutre was stirred at room temperature overnight. Water (20 mL) was added to the reaction mixture at 0 °C, and the mixture was stirred at room temperature for an hour. The precipitated solid was collected by filtration and washed with water to give 1.0g of the title compound as a white solid.

### Step2 Production of (4R)-1-(5-bromopyridin-2-yl)-4-{[tert- butyl(dimethyl)silyl]oxy}pyrrolidin-2-on

To a mixture of 2,5-dibromopyridine (400 mg), (4R)-4-{[tert-butyl(dimethyl)silyl]oxy}pyrrolidin-2-on obtained in step 1 (400 mg), 4,5-bis(diphenylphosphino)-9,9-dimethylxanthene (hereinafter referred to as Xantphos) (98 mg), potassium phosphate (1.08 g) and tris(dibenzylideneacetone)dipalladium-chloroform complex (hereinafter referred to as Pd₂(dba)₃·CHCl₃) (88 mg) was added 1,4-dioxane (10 mL), and the interior of a vessel was purged with argon. The reaction mixture was stirred at 100 °C for 1.5 hours. The reaction mixture was filtered through Celite, and the filtrate was concentrated under reduced pressure. The resulting residue was purified by column chromatography to give 400 mg of the title compound as a pale yellow solid.

### Reference Example 12 1-[4-(5-bromopyridin-2-yl)-1,4-diazepan-1-yl]ethanone

To a mixture of 2,5-dibromopyridine (500 mg) and 1-(1,4-diazepan-1-yl)ethanone (900 mg) was added N,N-diisopropylethylamine (1.1 mL), and the mixture was stirred at 125 °C overnight. The reaction mixture was diluted with water, extracted with ethyl acetate, and the solvent was evaporated under reduced pressure. The resulting residue was purified by column chromatography to give 580 mg of the title compound as a colorless oil.

### Reference Example 13 tert-butyl-4-(5-bromopyridine-2-yl)-1,4-diazepane-1-carboxylate

To a mixture of 2,5-dibromopyridine (1.3 g) and tert-butyl 1,4-diazepane-1-carboxylate (3.3 g) was added N,N-diisopropylethylamine (2.9 mL), and the mixture was stirred at 130 °C overnight. The reaction mixture was diluted with water, and extracted three times with ethyl acetate. The organic layer was washed with saturated brine and dried over magnesium sulfate. The solvent was evaporated under reduced pressure. The resulting residue was purified by column chromatography to give 2.0 g of the title compound as a brown oil.

### Reference Example 14 (4S)-1-(5-bromopyridin-2-yl)-4-{[tert- butyl(dimethyl)silyl]oxy}pyrrolidin-2-on

### Step1 Production of (4S)-4-{[tert-butyl(dimethyl)silyl]oxy}pyrrolidin-2-on

(4S)-4-Hydroxy-pyrrolidin-2-on (505 mg) was dissolved in DMF (3mL), imidazole (510 mg) and TBSCl (830 mg) were sequentially added, and the reaction mixutre was stirred at room temperature for 17 hours. The reaction mixture was added with water at 0 °C, followed by stirring at room temperature for an hour. The precipitated solid was collected by filtration and washed with water to give 925 mg of the title compound as a white solid.

### Step2 Production of (4S)-1-(5-bromopyridin-2-yl)-4-{[tert- butyl(dimethyl)silyl]oxy}pyrrolidin-2-on

To a mixture of 2,5-dibromopyridine (400 mg), (4S)-4-{[tert-butyl(dimethyl)silyl]oxy}pyrrolidin-2-on obtained in step 1 (437 mg), Xantphos (98 mg), potassium phosphate (1.1 g) and Pd₂(dba)₃·CHCl₃ (88 mg) was added 1,4-dioxane (10 mL) and the interior of a vessel was purged with argon. The reaction mixture was stirred at 100 °C for 1.5 hours. The reaction mixture was filtered through Celite, and the filtrate was concentrated under reduced pressure. The resulting residue was purified by column chromatography to give 412 mg of the title compound as a pale yellow solid

### Reference Example 15 Production of 5-bromo-N-(trans-4-{[tert-butyl(dimethyl)silyl]oxy}cyclohexyl)pyridine-2-amine

### Step1 Production of trans-4-[5-(bromopyridin-2-yl)amino]cyclohexanol

To a mixture of 2,5-dibromopyridine (1.0 g) and trans-4-aminocyclohexanol (1.5g) were added 2-ethoxyethanol(1 mL) and DBU (1.9 mL) successively, and the mixture was stirred at 160 °C for 2 hours. The reaction mixture was diluted with saturated brine, and extracted with ethyl acetate two times. The solvent was evaporated under reduced pressure. The resulting residue was purified by column chromatography to give 380 mg of the title compound as a yellow solid.

### Step2 Production of 5-bromo-N-(trans-4-{[tert-butyl(dimethyl)silyl]oxy}cyclohexyl)pyridine-2-amine

trans-4-[5-(Bromopyridin-2-yl)amino]cyclohexanol (380 mg) obtained in step 1 was dissolved in DMF(3 mL), imidazole (191 mg) and TBSCl (423 mg) were successively added, and the reaction mixutre was stirred at room temperature for 2 days. The reaction mixture was diluted with ethyl acetate, washed with saturated brine, and the solvent was evaporated under reduced pressure. The resulting residue was purified by column chromatography to give 440 mg of the title compound as a colorless oil.

### Reference Example 16 tert-butyl [2-(5-bromo-1H-benzimidazol-1-yl)ethyl]carbamate

1,4-Dibromo-2-nitrobenzene (500mg), tert-butyl (2-aminoethyl)carbamate (1.53 g) and potassium carbonate (790 mg) was suspended in tert-butanol (2 mL), and the mixture was reacted under microwave irradiation (Biotage INITIATOR at 120 °C for 20 minutes, at 140 °C for 40 minutes).The reaction mixture was diluted with ethyl acetate, washed with water, and the solvent was evaporated under reduced pressure. The resulting residue was dissolved in ethanol-water (4 :1, 19 mL), iron (1.1 g) and ammonium chloride (53 mg) were added, and the reaction mixutre was stirred at 80 °C overnight. The reaction mixture was filtered through Celite, and the filtrate was concentrated under reduced pressure. To the resulting residue was added methyl orthoformate (2 mL) and pyridinium p-toluenesulfonate (528 mg), and the mixture was stirred at 100 °C for 5 hours. The reaction mixture was diluted with ethyl acetate, and washed with water and dried over sodium sulfate. The solvent was evaporated under reduced pressure. The resulting residue was purified by column chromatography to give 600 mg of the title compound as a white amorphous form.

### Reference Example 17 5-Bromo-1-(trans-4-{[tert-butyl(dimethyl)silyl]oxyl}cyclohexyl)-1H-benzimidazole

### Step 1 Production of trans-4-(5-bromo-1H-benzimidazol-1-yl)cyclohexanol

A mixture of 1,4-dibromo-2-nitrobenzene (1.0 g), trans-4-aminocyclohexanol (820 mg), N,N-diisopropylethylamine (3.1 mL) was dissolved in N-methylpyrrolidone (8 mL), and the mixture was reacted under microwave irradiation (Biotage INITIATOR,at 200 °C for 30 minutes). The reaction mixture was diluted with ethyl acetate, washed successively with water and saturated brine. The organic layer was dried over magnesium sulfate and the solvent was evaporated under reduced pressure. The resulting residue was purified by column chromatography to give crude nitro compound. The resulting nitro compound thus obtained was dissolved in acetic acid (2.5 mL) and ethanol (2.5 mL) and iron (303 mg) was added, and the reaction mixutre was stirred at 80 °C for 30 minutes. Acetic acid (1.0 mL), ethanol (1.0 mL), iron (303 mg) was added to the mixture again, and the mixture was stirred at 80 °C for 2.5 hours. Iron (303 mg) was further added, and the mixture was stirred at 80 °C for 30 minutes. To the resulting mixture was added methyl orthoformate (396 µL), and the mixture was stirred at 80 °C for 45 minutes. The reaction mixture was filtered through Celite, and to the filtrate were added ethyl acetate and 2N aqueous sodium hydroxide solution for extraction. The organic layer was washed with saturated brine, dried over magnesium sulfate, and the solvent was evaporated under reduced pressure. The resulting residue was purified by column chromatography to give 431 mg of the title compound as a pale brown amorphous form.

### Step 2 Production of 5-Bromo-1-(trans-4-{[tert-butyl(dimethyl)silyl]oxy}cyclohexyl)-1H-benzimidazole

To a solution of trans-4-(5-bromo-1H-benzimidazol-1-yl)cyclohexanol obtained in Reference Example 17 step 1 (500 mg)and imidazole (345 mg) in DMF (5 mL) was added TBSCl (383 mg), and the mixture was stirred at room temperature for 3 days. The reaction mixture was diluted with ethyl acetate, washed successively with water and saturated brine. The organic layer was dried over magnesium sulfate and the solvent was evaporated under reduced pressure. The resulting residue was purified by column chromatography to give 507 mg of the title compound as a pale brown solid.

### Reference Example 18 5-bromo-1-methyl-1H-benzimidazole

Methanol (80 mL) and 40% methylamine solution in methanol (170 mL) was added successively to 1,4-dibromonitrobenzene (50 g), and the mixture was stirred in a sealed tube at 100 °C for 19 hours. 40% methylamine solution in methanol (50 mL) was added to the mixture again, and the mixture was stirred at 100 °C for 4 hours. The solvent was evaporated under reduced pressure, the resulting red solid was dissolved in acetic acid (250 mL) and ethanol (250 mL). The reaction mixture was heated at 80 °C, then was added with iron (49 g) gradually and stirred at 80 °C for 10 minutes. Methyl orthoformate (105 mL) was added in ten portions. After the mixture was stirred at 80 °C for 10 minutes, the solvent was evaporated under reduced pressure. 4N aqueous sodium hydroxide solution was added to the residue, and the insoluble material was separated by filteration through celite, washed successively with methanol and ethyl acetate. The filtrate and the washing solution were combined, and the combined solvent was evaporated under reduced pressure. To the resulting residue were added ethyl acetate and 4N aqueous sodium hydroxide solution for extraction. The organic layer was washed with saturated brine, dried over sodium sulfate, and the solvent was evaporated under reduced pressure to give 38 g of the title compound as a brown solid.

### Reference Example 19 5-bromo-1-(tetrahydro-2H-pyran-4-yl)-1H-benzimidazole

The title compound was prepared as a pale brown amorphous form (480 mg) according to the aforementioned procedure described in Reference Example 17, step1.

### Reference Example 20 tert-butyl{2-[(5-bromopyridin-2-yl)amino]ethyl}carbamate

To a mixture of 5-bromo-2-chloropyridine (1.5 g) and tert- Butyl (2-aminoethyl)carbamate (3.8 g) was added N,N-diisopropylethylamine (4.0 mL), and the mixture was stirred at 125 °C for 1 day. The reaction mixture was diluted with water, extracted with ethyl acetate, and the solvent was evaporated under reduced pressure. The resulting residue was purified by column chromatography to give 670 mg of the title compound as a pale yellow solid.

### Reference Example 21 5-bromo-2-(4-{[tert-butyl(dimethyl)silyl]oxy}piperidin-1-yl)pyridine

The title compound was prepared as a white solid (662 mg) according to the aforementioned procedure described in Reference Example 5.

### Reference Example 22 5-bromo-N-(2-methoxyethyl)pyridin-2-amine

The title compound was prepared as a white solid (240 mg) according to the aforementioned procedure described in Reference Example 7 Step1.

### Reference Example 23 5-bromo-2-[(3R)-3-{[tert-butyl(dimethyl)silyl]oxy}pyrrolidine-1-yl]pyridine

### Step1 Production of (3R)-1-(5-Bromopyridin-2-yl)pyrrolidin-3-ol

The title compound was prepared as a white solid (1.5 g) according to the aforementioned procedure described in Reference Example 8 Step1.

### Step2 Production of 5-Bromo-2-[(3R)-3-{[tert-butyl(dimethyl)sily}loxy]pyrrolidin-1-yl]pyridine

(3R)-1-(5-bromopyridin-2-yl)pyrrolidin-3-ol (1.5 g) obtained in Reference Example 22 step 1, and TBSCl (1.1 g) were dissolved in dichloromethane (30 mL) N,N-Diisopropylethylamine (1.4 mL) and DMAP (75 mg) were successively added, and the reaction mixutre was stirred at room temperature for 70 hours. The reaction mixture was diluted with saturated aqueous ammonium chloride solution, extracted with ethyl acetate, and the solvent was evaporated under reduced pressure. The resulting residue was purified by column chromatography to give 2.2 g of the title compound as a white solid.

### Reference Example 24 5-bromo-N-(3-{[tert-butyl(dimethyl)silyl]oxy}propyl)pyridine-2-amine

The title compound was prepared as a pale yellow solid (900 mg) according to the aforementioned procedure described in Reference Example 5, using 2,5-dibromopyridine instead of 5-bromo-2-chloropyridine, and using 3-aminopropanol instead of 2-aminoethanol.

### Reference example 25 5-bromo-N-(3-{[tert-butyl(dimethyl)silyl]oxy}-2,2-dimethylpropyl)pyridine-2-amine

The title compound was prepared as a pale yellow oil (800 mg) according to the aforementioned procedure described in Reference Example 5, using 2,5-dibromopyridine instead of 5-bromo-2-chloropyridine, and using 3-amino-2,2-dimethylpropan-1-ol instead of 2-aminoethanol.

### Reference Example 26 5-bromo-2-[(3S)-3-{[tert-butyl(dimethyl)silyl]oxy}pyrrolidin-1-yl}pyridine

The title compound was prepared as a white solid (1.7 g) according to the aforementioned procedure described in Reference Example 23, using (S)-pyrrolidin-3-ol.

### Reference Example 27 5-bromo-2-[4-({[tert-butyl(dimethyl)silyl]oxy}methyl)piperidin-1-yl]pyridine

The title compound was prepared as a white solid (235 mg) according to the aforementioned procedure described in Reference Example 5, using 2,5-dibromopyridine instead of 5-bromo-2-chloropyridine, and using piperidin-4-ylmethanol instead of 2-aminoethanol.

### Reference Example 28 4-(5-bromopyridin-2-yl)thiomorpholine

The title compound was prepared as a brown oil (485 mg) according to the aforementioned procedure described in Reference Example 7, Step1 using thiomorpholine instead of piperidin-4-ol.

### Reference Example 29 tert-butyl [1-(5-bromopyridin-2-yl)piperidin-4-yl]carbamate

The title compound was prepared as a white solid (1.1 g) according to the aforementioned procedure described in Reference Example 8 Step1 using tert-butyl piperidin-4-ylcarbamate instead of piperazin-2-on.

### Reference Example 30 tert-butyl [1-(5-bromopyridin-2-yl)piperidin-4-yl] methylcarbamate

To a suspension of sodium hydride (113 mg) in DMF (3 mL) was added a solution of tert-butyl [1-(5-bromopyridin-2-yl)piperidin-4-yl] carbamate obtained in Reference Example 29 (400 mg) in DMF (10 mL) and the mixture was stirred at room temperature for an hour. Iodomethane (84µL) was added, and the mixture was further stirred at room temperature for 3 hours. The reaction mixture was diluted with water, extracted with ethyl acetate, and the solvent was evaporated under reduced pressure. The resulting residue was purified by column chromatography to give 410 mg of the title compound as a colorless oil.

### Reference Example 31 5-bromo-2-(4-fluoropiperidin-1-yl)pyridine

The title compound was prepared as a yellow oil (370 mg) according to the aforementioned procedure described in Reference Example 8, Step1 using 4-fluoropiperidine instead of piperazin-2-on.

### Reference Example 32 5-bromo-N,N-bis(2-{[tert-butyl(dimethyl)silyl]oxy}ethyl)pyridin-2-amine

### Step1 Production of 2,2'-[(5-bromopyridin-2-yl)imino]diethanol

2,5-Dibromopyridine (1.5 g) and 2,2 '-azanediyldiethanol (5 mL) were dissolved in 2-ethoxyethanol (3 mL), and the mixture was stirred at 130 °C for 24 hours. The reaction mixture was diluted with water, extracted with ethyl acetate, and the solvent was evaporated under reduced pressure. The resulting residue was purified by column chromatography to give 1.4 g of the title compound as a pale yellow oil.

### Step 2 Production of 5-bromo-N,N-bis(2-{[tert-butyl(dimethyl)silyl]oxy}ethyl)pyridin-2-amine

The title compound was prepared as a colorless oil (560 mg) according to the aforementioned procedure described in Reference Example 5, Step2, using 2,2'-[(5-bromopyridin-2-yl)imino]diethanol obtained in Reference Example 32 step 1 instead of 2-[(5-bromopyridin-2-yl)amino]ethanol.

### Reference Example 33 tert-butyl-4-(5-bromopyridin-2-yl)-1,4-diazepane-1-carboxylate

The title compound was prepared as a pale yellow oil (780 mg) according to the aforementioned procedure described in Reference Example 12 using tert-butyl 1,4-diazepane-1-carboxylate instead of 1-(1,4-diazepan-1-yl)ethanone.

### Reference example 34 5-bromo-2-[(3S)-3-{[tert-butyl(dimethyl)silyl]oxy}piperidin-1-yl]pyridine

### Step1 Production of (3S)-1-(5-bromopyridine-2-yl)piperidin-3-ol

The title compound was prepared as a pale yellow oil (530 mg) according to the aforementioned procedure described in Reference Example 8 Step1 using (S)-piperidin-3-ol instead of piperazin-2-on.

### Step 2 Production of 5-bromo-2-[(3S)-3-{[tert-butyl(dimethyl)silyl]oxy}piperidin-1-yl]pyridine

The title compound was prepared as a yellow oil (604 mg) according to the aforementioned procedure described in Reference Example 5 Step2 using (3S)-1-(5-bromopyridine-2-yl)piperidin-3-ol obtained in Reference Example 34 Step 1 instead of 2-[(5-bromopyridin-2-yl)amino]ethanol

### Reference Example 35 5-bromo-2-[(3R)-3-{[tert-butyl(dimethyl)silyl]oxy}piperidin-1-yl]pyridine

The title compound was prepared as a pale yellow oil (313 mg) according to the aforementioned procedure described in Reference Example 34 using (R)-piperidin-3-ol instead of (S)-piperidin-3-ol.

### Reference Example 36 5-bromo-2-(4-{[tert-butyl(dimethyl)silyl]oxy}piperidin-1-yl)pyrimidine

The title compound was prepared as a white solid (780 mg) according to the aforementioned procedure described in Reference Example 5, using 5-bromo-2-chloropyrimidine instead of 5-bromo-2-2-chloropyridine, and using piperidin-4-ol instead of 2-aminoethanol.

### Reference Example 37 1-(5-bromopyridin-2-yl)-4-methyl-1,4-diazepane

The title compound was prepared as a white oil (550 mg) according to the aforementioned procedure described in Reference Example 12, using 1-methyl-1,4-diazepane instead of 1-(1,4-diazepan-1-yl)ethanone.

### Reference Example 38 (S)-tert- butyl1-(5-bromopyridin-2-yl)pyrrolidine-2-carboxylate

The title compound was prepared as a pale yellow solid (595 mg) according to the aforementioned procedure described in Reference Example 12, using (S)-tert-butyl 1-pyrrolidine-2-carboxylate instead of 1-(1,4-diazepan-1-yl)ethanone.

### Reference Example 39 1-(4-bromophenyl)-4-{[tert-butyl(dimethyl)silyl]oxy}piperidine

### Step 1 Production of 1-(4-bromophenyl)piperidin-4-ol

To a mixture of 1,4-dibromobenzene (1.3 g), piperidin-4-ol (506 mg), (±)-2,2'-bis(diphenylphosphino)-1,1'-binaphthyl (hereinafter referred to as (±)-BINAP) (467 mg), sodium tert-butoxide (hereinafter referred to as NaOtBu)(625 mg) and Pd₂(dba)₃·CHCl₃ (129 mg) was added toluene (30 mL), and the interior of a vessel was purged with argon. The reaction mixture was stirred at 120 °C for an hour. The reaction mixture was diluted with ethyl acetate, filtered through Celite, and the filtrate was evaporated under reduced pressure. The resulting residue was purified by column chromatography to give 711 mg of the title compound as a white solid.

### Step 2 Production of 1-(4-bromophenyl)-4-{[tert-butyl(dimethyl)silyl]oxy}piperidine

The title compound was prepared as a white solid (454 mg) according to the aforementioned procedure described in Reference Example 5 Step 2, using 1-(4-bromophenyl)piperidin-4-ol obtained in Reference Example 39 Step 1 (307 mg) instead of 2-[(5-bromopyridin-2-yl)amino] ethanol.

### Reference Example 40 N-[(3S)-1-(5-bromopyridin-2-yl)pyrrolidin-3-yl]acetamide

The title compound was prepared as a white solid (440 mg) according to the aforementioned procedure described in Reference Example 8 Step 1, using (S)-N-(pyrrolidin-3-yl)acetamide (541 mg) instead of piperazin-2-on.

### Reference Example 41 tert-butyl [(3S)-1-(5-bromopyridin-2-yl)pyrrolidin-3-yl] carbamate

The title compound was prepared as a white solid (1.3 g) according to the aforementioned procedure described in Reference Example 8 Step 1, using (S)-tert-butyl pyrrolidin-3-yl carbamate (1.57 g) instead of piperazin-2-on.

### Reference Example 42 (R)-1-(5-bromopyridin-2-yl)pyrrolidin-2-carboxamide

The title compound was prepared as a pale yellow solid (80 mg) according to the aforementioned procedure described in Reference Example 8 Step 1, using (R)-pyrrolidine-2-carboxamide (200 mg) instead of piperazin-2-on.

### Reference Example 43 5-bromo-2-[(2S)-2-({[tert-butyl(dimethyl)silyl]oxy}methyl)pyrrolidin-1-yl]pyridine

### Step 1 Production of [(2S)-1-(5-bromopyridin-2-yl)pyrrolidin-2-yl]methanol

The title compound was prepared as a yellow oil (630 mg) according to the aforementioned procedure described in Reference Example 8 Step 1, using (S)-pyrrolidin-2-ylmethanol (477 mg) instead of piperazin-2-on.

### Step 2 Production of 5-bromo-2-[(2S)-2-({[tert-butyl(dimethyl)silyl]oxy}methyl)pyrrolidin-1-yl]pyridine

The title compound was prepared as a colorless oil (930 mg) according to the aforementioned procedure described in Reference Example 5 Step2 using (2S)-1-(5-bromopyridine-2-yl)pyrrolidin-2-yl]methanol (630 mg) obtained in Reference Example 43 step 1 instead of 2-[(5-bromopyridin-2-yl)amino]ethanol.

### Reference Example 44 tert-butyl [(3R)-1-(5-bromopyridin-2-yl)pyrrolidin-3-yl]carbamate

The title compound was prepared as a yellow oil (660 mg) according to the aforementioned procedure described in Reference Example 8 Step 1, using (R)-tert- butyl pyrrolidin-3-yl carbamate (1.18 g) instead of piperazin-2-on, and using 1-butanol instead of 2-ethoxyethanol.

### Reference Example 45 N-[(3R)-1-(5-bromopyridin-2-yl)pyrrolidin-3-yl]acetamide

The title compound was prepared as a yellow oil (530 mg) according to the aforementioned procedure described in Reference Example 8 Step 1, using (R)-N-(pyrrolidin-3-yl)acetamide (811 mg) instead of piperazin-2-on, and using 1-butanol instead of 2-ethoxyethanol.

### Reference Example 46 5-bromo-2-[(3R)-3-fluoropyrrolidin-1-yl]pyridine

The title compound was prepared as a white solid (385 mg) according to the aforementioned procedure described in Reference Example 8 Step 1 using (R)-3-fluoropyrrolidine (795 mg) instead of piperazin-2-on, and using 1-butanol instead of 2-ethoxyethanol.

### Reference Example 47 (3R)-1-(5-bromopyridin-2-yl)-N,N-dimethylpyrrolidin-3-amine

The title compound was prepared as a brown amorphous form (470 mg) according to the aforementioned procedure described in Reference Example 8 Step 1 using (R)-N,N-dimethylpyrrolidin-3-amine (723 mg) instead of piperazin-2-on, and using 1-butanol instead of 2-ethoxyethanol.

### Reference Example 48 1-(5-bromopyridin-2-yl)pyrrolidin-2-on

The title compound was prepared as a pale yellow amorphous form (375 mg) according to the aforementioned procedure described in Reference Example 11 Step 2 using pyrrolidin-2-on (178 µL) instead of (4R)-4-{[tert-butyl(dimethyl)silyl]oxy}pyrrolidin-2-on.

### Reference Example 49 1-(5-bromopyridin-2-yl)-4-(methanesulfonyl)piperazine

### Step 1 Production of 1-(5-bromopyridin-2-yl)piperazine

To a solution of tert- butyl 4-(5-bromopyridin-2-yl)piperazine-1-carboxylate(900 mg) in dichloromethane (10 mL) was added trifluoroacetic acid (hereinafter referred to as TFA) (3.0 mL), and the mixture was stirred at room temperature for 2 hours. 1 N aqueous sodium hydroxide solution was added to the reaction mixture, and pH of the aqueous layer was adjusted to 10-11. The aqueous layer was extracted with chloroform-methanol (10: 1).The organic layer was dried over Magnesium sulfate, and was evaporated under reduced pressure to give 447 mg of the title compound as a pale yellow solid.

### Step 2 Production of 1-(5-bromopyridin-2-yl)-4-(methanesulfonyl)piperazine

To asolution of 1-(5-bromopyridin-2-yl)piperazine obtained in Step 1 (210 mg) in dichloromethane (3 mL) was successively added triethylamine (313 µL) and methanesulfonyl chloride (87 µL) at 0 °C and the mixture was stirred at room tempetrature. The reaction mixture was washed with water and the organic layer was dried over magnesium sulfate. The solvent was evaporated under reduced pressure. The resulting residure was washed with hexane-ethyl acetate (20:1) to give 240 mg of the title compound as a brown solid.

### Reference Example 50 1-[4-(5-bromopyridin-2-yl)-piperazin-1-yl]ethanone

The title compound was prepared as a pale yellow solid (195 mg) according to the aforementioned procedure described in Reference Example 49 Step 2 using acetyl chloride (92 µL) instead of methanesulfonyl chloride.

### Reference Example 51 2-(trimethylsilyl)ethyl 1-(5-bromopyridin-2-yl)piperidine-4-carboxylate

### Step 1 Production of ethyl 1-(5-bromopyridin-2-yl)piperidine-4-carboxylate

The title compound was prepared as a pale yellow amorphous (1.96 g) according to the aforementioned procedure described in Reference Example 7 Step 1 using ethyl piperidin-4-carboxylate (3.0 g) instead of piperidin-4-ol.

### Step 2 Production of 1-(5-bromopyridin-2-yl)piperidine-4-carbonxylic acid

To a solution of ethyl 1-(5-bromopyridin-2-yl)piperidine-4-carboxylate obtained in Reference Example 51 Step 1 (1.0 g) in methanol (20 mL) was added 2N aqueous sodium hydroxide solution (6 mL), and the mixture was stirred at 80 °C for 2 hours. The solvent was evaporated under reduced pressure, and water was added. The mixture was adjusted to about pH3-4 with 2 N aqueous hydrochloric acid solution. The precipitated was collected by filtration, washed with water to give 693 mg of the title compound as a white solid.

### Step 3 Prodution of 2-(trimethylsilyl)ethyl 1-(5-bromopyridin-2-yl)piperidine-4-carboxylate

To a suspension of 1-(5-bromopyridin-2-yl)piperidine-4-carbonxylic acid obtained in Reference example 51 Step 2 (190 mg), trimethylsilylethanol (191 µL) and N,N-diisopropylethylamine (232 µL) in dichloromethane (4 mL) were successively added 1-ethyl-3-(3-dimethylaminopropyl)carbodiimide hydrochloride (hereinafter referred to as WSCD·HCl) (206 mg) and DMAP (82 mg), and the mixture was stirred at room temperature for an hour. The reaction mixture was added with water, followed by carrying out an operation of extraction. The organic layer was dried over magnesium sulfate, evaporated under reduced pressure. The resulting residue was purified by column chromatography to give 212 mg of the title compound as a white solid.

### Reference Example 52 1-(5-bromopyridin-2-yl)-4-(4-fluorophenyl)piperidine-4-carboxamide

### Step 1 Production of 1-(5-bromopyridin-2-yl)-4-(4-fluorophenyl)piperidine-4-carbonitrile

A mixture of 2,5-dibromopyridine (1.0 g) and 4-(4-fluorophenyl)piperidine-4-carbonitrile (2.6 g) was stirred at 120 °C for 19 hours. The reaction mixture was diluted with water, and extracted with ethyl acetate three times. The organic layer was washed with saturated brine anddried over magnesium sulfate., The solvent was evaporated under reduced pressure. The resulting residue was purified by column chromatography to give 1.42 g of the title compound as a white powder.

### Step 2 Production of 1-(5-bromopyridin-2-yl)-4-(4-fluorophenyl)piperidine-4-carboxamide

Concentrated sulfuric acid (14 mL) was added to 1-(5-bromopyridin-2-yl)-4-(4-fluorophenyl)piperidine-4-carbonitrile obtained in Step 1 (500 mg), and the mixture was stirred at room temperature for 20 hours. Aqueous sodium hydroxide solution was added to the reaction mixture, and pH of the reaction mixture was adjusted to 10-11. The precipitated solid was collected by filtration, washed with water to give 354 mg of the title compound as a white powder.

### Reference Example 53 4-[(5-bromopyridin-2-yl)amino]piperidine-1-carboxamide

### Step 1 Production of tert-butyl 4-[(5-bromopyridin-2-yl)amino]piperidine-1-carboxylate

The title compound was prepared as a white solid (400 mg) according to the aforementioned procedure described in Reference Example 8 Step1 using tert-butyl 4-aminopiperidine-1-carboxylate (2.1 g) instead of piperazin-2-on.

### Step 2 Production of 4-[(5-bromopyridin-2-yl)amino]piperidine-1-carboxamide

To a solution of tert-butyl 4-[(5-bromopyridin-2-yl)amino]piperidine-1-carboxylate (400 mg) in tetrahydrofuran (hereinafter referred to as THF) (6 mL) obtained in Reference Example 53 Step 1 was added 4N hydrogen chloride-ethyl acetate solution (4 mL), and the mixture was stirred at room temperature for 17 hours. Afrer evaporation of the solvent under reduced pressure, THF(6.0 mL), triethylamine (470 µL) and trimethylsilyl isocyanate (194 µL) was successively added to the resulting residue, and the mixture was stirred at room temperature for 1 day. The solvent was evaporated under reduced pressure, and the resulting residue was purified by column chromatography to give 357 mg of the title compound as a pale yellow amorphous form.

### Reference Example 54 tert-butyl 4-(5-bromopyridin-2-yl)-3-oxopiperazine-1-carboxylate

The title compound was prepared as a white solid (1.45 g) according to the aforementioned procedure described in Reference Example 11 Step 2 using tert-butyl 3-oxopiperazine-1-carboxylate (930 mg) instead of (4R)-4-{[tert-butyl(dimethyl)silyl]oxy}pyrrolidin-2-on.

### Reference Example 55 4-(5-bromopyridin-2-yl)-3-oxopiperazine-1-carboxamide

To a solution of tert-butyl 4-(5-bromopyridin-2-yl)-3-oxopiperazine-1-carboxylate obtained in Reference Example 54 (420 mg) in dichlorometnane (2 mL) was added TFA (1 mL), and the mixture was stirred at room temperature for 3 hours. The solvent was evaporated under reduced pressure, and the resulting residue was added with saturated aqueous sodium hydrogen carbonate solution and extracted with chloroform. sodium hydrogen carbonate,. The organic layer was dried over anhydrous magnesium sulfate and the solvent was evaporated under reduced pressure. Dichloromethane (3 mL), trimethylsilyl isocyanate (0.21 ml) triethylamine (0.15 mll) was successively added to the resulting residue, and the mixture was stirred at room temperature for 17 hours. The reaction mixture was added with water, and the mixture was extracted with chloroform. The solvent was dried over magnesium sulfate, and evaporated under reduced pressure. The resulting residue was purified by column chromatography to give 130 mg of the title compound as a white solid.

### Reference Example 56 4-(5-bromopyridine-3-yl)thiomorpholine 1,1-dioxide

To a mixture of 3,5-dibromopyridine (500 mg), thiomorpholine 1,1-dioxide (343 mg), Xantphos (123 mg), sodium tert-butoxide (305 mg) and Pd₂(dba)₃·CHCl₃ (110 mg) was added toluene (6 mL), and the interior of a vessel was purged with argon. The reaction mixture was stirred at 100 °C for an hour. The reaction mixture was filtered through Celite, and the filtrate was concentrated under reduced pressure. The resulting residue was purified by column chromatography to give 324 mg of the title compound as a brown solid.

### Reference Example 57 4-(5-bromopyridin-3-yl)-1,4-diazepane-1-carboxamide

### Step 1 Production of tert-butyl 4-(5-bromopyridin-3-yl)-1,4-diazepane-1-carboxylate

The title compound was prepared as a red oil (860 mg) according to the aforementioned procedure described in Reference Example 56 using tert-butyl 1,4-diazepane-1-carboxylate (930 mg) instead of thiomorpholine 1,1-dioxideStep 2 Production of 4-(5-bromopyridin-3-yl)-1,4-diazepane-1-carboxamide

2N Hydrogen chloride-ethanol solution (10 mL) was added to tert-butyl 4-(5-bromopyridin-3-yl)-1,4- diazepane-1-carboxylate obtained in Reference Example 57 Step 1 (860 mg), and the mixture was stirred at room temperature for 17 hours. Afrer evaporation of the solvent under reduced pressure, ethanol (10 mL), Potassium cyanate (392 mg)- and acetic acid (0.28 ml) was successively added to the resulting residue, and the mixture was stirred at room temperature for 1 day. The mixture was added with saturated aqueous sodium hydrogen carbonatesodium hydrogen carbonate solution to, and extracted with chloroform. The solvent was evaporated under reduced pressure, and the resulting residue was purified by column chromatography to give 550 mg of the title compound as a pale yelow solid.

### Reference Example 58 4-[(5-bromopyridin-2-yl)methyl]piperazin-2-on

To a solution of 5-bromo-2-formylpyridine (300 mg), piperazin-2-on (485 mg) in methanol (5.0 mL) was added thionyl chloride (0.1 mL), and the mixture was stirred at room temperature for 4 hours. Sodium triacetoxyborohydride (1 g) was added to the reaction mixture, the mixture was further stirred for an 17 hours. Saturated aqueous sodium hydrogen carbonatesodium hydrogen carbonate solution was added to the mixture, and the mixture was extracted with chloroform. The solvent was evaporated under reduced pressure, and the resulting residue was purified by column chromatography to give 227 mg of the title compound as a pale yellow solid.

### Reference Example 59 4-[(5-bromopyridin-2-yl)methyl]-1,4-diazepane-1-carboxamide

### Step 1 Production of tert- butyl 4-[ (5-bromopyridin-2-yl)methyl]1-1,4-diazepane-1-carboxylate

To a solution of tert-butyl 1,4-diazepane-1-carboxylate (810 mg) in methanol (8 mL) were added 5-bromo-2-formylpyridine (500 mg) and sodium triacetoxyborohydride (1.1 g), and the mixture was stirred at room temperature for 17 hours. Saturated aqueous sodium hydrogen carbonatesodium hydrogen carbonate solution was added to the mixture, and the mixture was extracted with chloroform. The organic layer was washed with saturated brine,the solvent was evaporated under reduced pressure. The resulting residue was purified by column chromatography to give 660 mg of the title compound as a colorles

### Step 2 Production of 4-[(5-bromopyridin-2-yl)methyl]-1,4-diazepane-1-carboxamide

2N Hydrogen chloride-ethanol solution (9 mL) was added to tert-butyl 4-[(5-bromopyridin-2-yl)methyl]1-1,4-diazepane-1-carboxylate obtained in Reference Example 59 Step 1 (660 mg), and the mixture was stirred at room temperature for 1 day. The reaction mixture was neutralized with saturated aqueous sodium hydrogen carbonatesodium hydrogen carbonate solution, and the aqueous layer was extracted with chloroform. The organic layer was dried over Magnesium sulfate, and the solvent was evaporated under reduced pressure. To the resulting residue was successively added ethanol (9 mL), potassium cyanate (290 mg) and acetic acid (204 µL), and the mixture was stirred at room temperature for 2 days. Saturated aqueous sodium hydrogen carbonatesodium hydrogen carbonate solution was added to the mixture, and the mixture was extracted with chloroform. The solvent was evaporated under reduced pressure, and the resulting residue was purified by column chromatography to give 162 mg of the title compound as a pale white solid.

### Reference Example 60 (5-bromopyridin-2-yl) methanol

To a solution of 5-bromo-2-formylpyridine (300 mg) in methanol (5 mL) was added sodium triacetoxyborohydride (684 mg), and the mixture was stirred at room temperature for 17 hours. Saturated aqueous sodium hydrogen carbonatesodium hydrogen carbonate solution was added to the mixture, and the mixture was extracted with ethyl acetate. The solvent was evaporated under reduced pressure, and the resulting residue was purified by column chromatography to give 260 mg of the title compound as a pale yelow solid.

### Reference Example 61 4-[(5-bromopyridin-2-yl)methyl] thiomorpholine 1,1-dioxide

### Step 1 Production of 5-bromo-2-(chloromethyl)pyridine hydrochloride

To (5-bromopyridin-2-yl)methanol obtained in Reference Example 60 (1.9 g) was slowly added thionyl choride (7.4 mL) at 0 °C, and the mixture was stirred at 60 °C for a hour. The mixture was added with diethyl ether (10 mL) at 0 °C, and the mixture was stirred at the same temperature for 30 minutes. The precipitated solid was collected by filtration to give 1.5 g of the title compound as a white solid.

### Step 2 Production of 4-[(5-bromopyridin-2-yl)methyl]thiomorpholine 1,1-dioxide

To a solution of 5-bromo-2-(chloromethyl)pyridine hydrochloride obtained in Reference Example 61 Step 1 (300 mg) and thiomorpholine 1,1-dioxide (500 mg) in DMF (3.5 mL) was added triethylamine (0.52 mL), and the mxture was stirred at room temperature for 20 hours. The reaction mixture was diluted with ethyl acetate, washed with saturated brine three times. The solvent was evaporated under reduced pressure. The resulting residue was purified by column chromatography to give 275 mg of the title compound as a white solid.

### Reference Example 62 4-[(5-bromopyridin-3-yl)methyl]piperazin-2-on

### Step 1 Production of (5-bromopyridin-3-yl)methanol

To a solution of ethyl 5-bromonicotinate (2.3 g) in methanol (35 mL) was added sodium borohydride (1.5 g) at 0 °C, and the mixture was stirred for 2 hours. Water was added to the reaction mixture, and the mixture was extracted with dichloromethane three times. The solvent was evaporated under reduced pressure, and the resulting residue was purified by column chromatography to give 1.0 g of the title compound as a pale yelow oil.

### Step 2 Production of 3-bromo-5-(chloromethyl)pyridine hydrochloride

To (5-bromopyridin-3-yl)methanol (1.0 g) was slowly added thionyl choride (3.9 mL) at 0 °C, and the mixture was stirred at 70 °C for an hour.The mixture was added with diethyl ether (10 mL) at 0 °C, and was added, and the mixture was stirred at the same temperature for 30 minutes. The precipitated solid was collected by filtration to give 1.1 g of the title compound as a white solid.

### Step 3 Production of 4-[(5-bromopyridin-3-yl)methyl]piperazin-2-on

To a solution of 3-bromo-5-(chloromethyl)pyridine hydrochloride obtained in Reference Example 62 Step 2 (250 mg) and piperazin-2-on (206 mg) in DMF (1.0 mL) was added triethylamine (0.43 mL), and the mixture was stirred at room temperature for 17 hours. The solvent was evaporated under reduced pressure, and the resulting residue was purified by column chromatography to give 230 mg of the title compound as a white solid.

### Reference Example 63 4-[(5-bromopyridin-3-yl)methyl]thiomorpholine 1,1-dioxide

The title compound was prepared as a colorless oil (352 mg) according to the aforementioned procedure described in Reference Example 62 Step 3, using thiomorpholine 1,1-dioxide (500 mg) instead of piperazin-2-on.

### Reference Example 64 4-[(5-bromopyridin-3-yl)methyl]-1,4-diazepane-1-carboxamide

The title compound was prepared as a white solid (375 mg) according to the aforementioned procedure described in Reference Example 59, using 5-bromo-3-formylpyridine (500 mg) instead of 5-bromo-2-formylpyridine .

### Reference Example 65 4-(4-bromopyridin-2-yl)-1,4-diazepane-1-carboxamide

A solution of 4-bromo-2-chloropyridine (770 mg) and 1,4-diazepane (1.2 g) in acetonitrile (2 mL) was stirred at 80 °C for 3 hours. After cooling, 10% aqueous sodium hydroxide solution (12 mL) was added to the mixture, and the mixture was extracted with chloroform three times. The organic layer was dried over Magnesium sulfate, and evaporated under reduced pressure. To the resulting residue was successively added ethanol (12 mL), potassium cyanate (650 mg), acetic acid (0.46 ml), and the mixture was stirred at room temperature for 2 days. Saturated aqueous sodium hydrogen carbonate solution was added to the mixture, and the mixture was extracted with chloroform. The solvent was evaporated under reduced pressure, and the resulting residue was purified by column chromatography to give 60 mgof the title compound as a white solid.

### Reference Example 66 4-[(5-chloropyridin-3-yl)amino]piperidine-1-carboxamide

### Step 1 Production of tert- butyl 4-[(5-chloropyridin-3-yl)amino]piperidine-1-carboxylate

To a mixture of 3,5-dichloropyridine (500 mg), tert-butyl 4-aminopiperidine-1-carboxylate (745 mg), Xantphos (196 mg), sodium tert-butoxide (488 mg) and Pd₂(dba)₃·CHCl₃ (155 mg) was added toluene (9 mL), and the interior of a vessel was purged with argon. The reaction mixture was stirred at 100 °C for an hour. The reaction mixture was filtered through Celite, and the filtrate was concentrated under reduced pressure. The resulting residue was purified by column chromatography to give 450 mg of the title compound as a pale yellow solid.

### Step 2 Production of 4-[(5-chloropyridin-3-yl)amino]piperidine-1-carboxamide

To a solution of tert- butyl 4-[(5-chloropyridin-3-yl)amino]piperidine-1-carboxylate obtained in Reference Example 66 Step 1 (450 mg) in THF (5 mL) was added 4N hydrogen chloride-ethyl acetate solution (4 mL) and the mixture was stirred at room temperature for 17 hours. Afrer evaporation of the solvent under reduced pressure, ethanol (5 mL), potassium cyanate (352 mg) and acetic acid (0.25 ml) was successively added to the resulting residue, and the mixture was stirred at room temperature for 1 day. Saturated aqueous sodium hydrogen carbonate solution was added to the mixture, and the mixture was extracted with chloroform. The solvent was evaporated under reduced pressure, and the resulting residue was purified by column chromatography to give 180 mg of the title compound as a pale yelow solid.

### Reference Example 67 4-(3-bromophenyl)-1,4-diazepane-1-carboxamide

### Step 1 Production of tert- buty 4- (3-bromophenyl)-1,4-diazepane-1-carboxylate

To a mixture of 1,3-dibromobenzene (700 mg), tert-buty 1,4-diazepane-1-carboxylate (624 mg), Xantphos (172 mg), sodium tert-butoxide (428 mg) and Pd₂(dba)₃·CHCl₃ (154 mg) was added toluene (15 mL), and the interior of a vessel was purged with argon. The reaction mixture was stirred at 100 °C for an hour. The reaction mixture was filtered through Celite, and the filtrate was evaporated under reduced pressure. The resulting residue was purified by column chromatography to give 606 mg of the title compound as a yellow amorphos form.

### Step 2 Production of 4-(3-bromophenyl)-1,4-diazepane-1-carboxamide

The title compound was prepared as a pale yellow solid (420 mg) according to the aforementioned procedure described in Reference Example 57 Step 2, using tert-buty 4-(3-bromophenyl)-1,4-diazepane-1-carboxylate obtained in Reference Example 67 Step 1 (606 mg) instead of tert-butyl 4-(5-bromopyridin-3-yl)-1,4-diazepane-1-carboxylate.

### Reference Example 68 4-(3-bromobenzyl)piperazin-2-on

The title compound was prepared as a white solid (260 mg) according to the aforementioned procedure described in Reference Example 62 Step 3, using 1-bromo-3-bromomethylbenzene (250 mg) instead of 3-bromo-5-(chloromethyl) pyridine hydrochloride.

### Reference Example 69 4-(4-bromobenzyl)piperazin-2-on

The title compound was prepared as a white solid (280 mg) according to the aforementioned procedure described in Reference Example 62 Step 3, using 1-bromo-4-bromomethylbenzene (250 mg) instead of 3-bromo-5-(chloromethyl)pyridine hydrochloride.

### Reference Example 70 tert-Butyl 4-[(5-bromopyridin-3-yl)methyl]piperazine-1-carboxylate

To a solution of 5-bromo-3-formylpyridine (500 mg) in dichloromethane (5 mL) was added tert-butyl piperazine-1-carboxylate (751 mg) and Sodium triacetoxyborohydride (1.14 g), and the mixture was stirred at room temperature for 1 day. Saturated aqueous sodium hydrogen carbonate solution was added to the mixture, and the mixture was extracted with ethyl acetate. The organic layer was washed with saturated brine,the solvent was evaporated under reduced pressure. The resulting residue was purified by column chromatography to give 860 mg of the title compound as a colorless oil.

### Reference Example71 tert- butyl 4-(3-bromobenzyl)piperazine-1-carboxylate

To a solution of 1-bromo-3-bromomethylbenzene (250 mg) and tert-butyl piperazine-1-carboxylate (205 mg) in dichloromethane (1 mL) was added triethylamine (0.21 mL), and the mixture was stirred at room temperature for 2 hours. The reaction solution was directly purified by column chromatography to give 320 mg of the title compound as a colorless oil.

### Reference Example 72 tert- butyl 4-[(5-bromopyridin-3-yl)amino]piperidine-1-carboxylate

The title compound was prepared as a pale yellow solid (500 mg) according to the aforementioned procedure described in Reference Example 56, using tert-butyl 4-aminopiperidine-1-carboxylate (540 mg) instead of thiomorpholine 1,1-dioxide.

### Reference Example 73 tert-butyl 4-[(4-bromopyridin-2-yl)methyl]piperazine-1-carboxylate

### Step 1 Production of (4-bromopyridin-2-yl)methanol

To a suspension of lithium aluminium hydride (232 mg) in THF (12 mL) was added a solution of methyl 4-bromopicolinate (1.1 g) in THF (18 mL) at 0 °C, and the mixture was stirred at room temperature for 2 hours. Saturated aqueous ammonium chloride solution was added to the reaction mixture at 0 °C, and the mixture was extracted with ethyl acetate. The organic layer was dried over Magnesium sulfate, and evaporated under reduced pressure. The resulting residue was purified by column chromatography to give 270 mg of the title compound as a pale brown oil.

### Step 2 Production of 4-bromo-2-(chloromethyl)pyridine hydrochloride

To a (4-bromopyridin-2-yl)methanol (270 mg) was slowly added thionyl chloride (1.0 mL) at 0 °C and the mixture was stirred at 70 °C for 30 minutes. The mixture was added with diethylether at 0 °C, and the mixture was stirred at the same temperature for 30 minutes. The precipitated solid was collected by filtration to give 275 mg of the title compound as a brown solid.

### Step 3 Production of tert-butyl 4-[(4-bromo pyridin-2-yl)methyl]piperazine-1-carboxylate

To a solution of 4-bromo-2-(chloromethyl)pyridine hydrochloride obtained in Reference Example 73 Step 2 (133 mg) and tert-butyl piperazine-1-carboxylate (153 mg) in DMF (0.4 mL) was added triethylamine (0.23 mL), and the mixture was stirred at room temperature for 2 hours. Saturated aqueous sodium hydrogen carbonate solution was added to the mixture, and the mixture was extracted with ethyl acetate. The solvent was evaporated under reduced pressure, and the resulting residue was purified by column chromatography to give 193 mg of the title compound as a pale yelow oil.

### Reference Example 74 4-[(4-bromopyridine-2-yl)methyl]piperazin-2-on

The title compound was prepared as a pale yellow oil (138 mg) according to the aforementioned procedure described in Reference Example 74 Step 3, using piperaazin-2-on (114 mg) instead of tert-butyl piperazine-1-carboxylate.

### Reference Example 75 tert-butyl (3R)-3-[(5-bromopyridin-3-yl)amino]pyrrolidine-1-carboxylate

The title compound was prepared as a pale yellow solid (450 mg) according to the aforementioned procedure described in Reference Example 56, using (R)-tert- butyl 3-aminopyrrolidine-1-carboxylate (436 mg) instead of thiomorpholine-1,1-dioxide.

### Reference Example 76 tert- butyl (3S)-3-[(5-bromopyridin-3-yl)amino]pyrrolidin-1-carboxylate

The title compound was prepared as a pale yellow solid (264 mg) according to the aforementioned procedure described in Reference Example 56, using (S)-tert- butyl 3-aminopyrrolidine-1-carboxylate (350 mg) instead of thiomorpholine-1,1-dioxide.

### Reference Example 77 tert- butyl {2-[(5-bromopyridin-3-yl)amino]ethyl}carbamate

To a mixture of 3,5-Dibromopyridine (766 mg), tert-butyl (2-aminoethyl)carbamate (570 mg), Xantphos (188 mg), cesium carbonate (1.69g) and Pd₂(dba)₃ (148 mg) was added toluene (15 mL), and the interior of a vessel was purged with argon. The reaction mixture was stirred at 100 °C for 5 hours. The reaction mixture was filtered through Celite, and the filtrate was concentrated under reduced pressure. The resulting residue was purified by column chromatography to give 540 mg of the title compound as a pale yellow solid.

### Reference Example 78 tert-Butyl 4-[(5-bromopyridin-3-yl)methoxy]piperidine-1-carboxylate

To a solution of 3-bromo-5-(chloromethyl)pyridine hydrochloride obtained in Reference Example 62 Step 2 (150 mg) and tert-butyl 4-hydroxypiperidine-1-carboxylate (280 mg) in DMF (2 mL) was added sodium hydride (62 mg), and the mixture was stirred at room temperature for 17 hours. The reaction mixture was diluted with ethyl acetate, and successively washed with water and saturated brine, and the solvent was evaporated under reduced pressure. The resulting residue was purified by column chromatography to give 146 mg of the title compound as a colorless oil.

### Reference Example 79 tert-butyl 4-{[(5-bromopyridin-3-yl)methyl](methyl)amino}piperidine-1- carboxylate

### Step 1 Production of tert-butyl 4-{[(5-bromopyridin-3-yl)methyl]amino}piperidine-1-carboxylate

The title compound was prepared as a pale yellow oil (480 mg) according to the aforementioned procedure described in Reference Example 62 Step 3, using tert-butyl 4-aminopiperidine-1-carboxylate (833 mg) instead of piperazin-2-on.

### Step 2 Production of tert-butyl 4-{[(5-bromopyridin-3-yl)methyl](methyl)amino}piperidine-1- carboxylate

To a solution of tert-butyl 4-{[(5-bromopyridin-3-yl)methyl]amino}piperidine-1-carboxylate obtained in Reference Example 79 Step 1 (250 mg) in dichloromethane (1 mL) was added 36% aqueous formaldehyde solution (0.21 mL) and sodium triacetoxyborohydride (290 mg), and the mixture was stirred at room temperature for an hour. The reaction solution was directly purified by column chromatography to give 230 mg of the title compound as a colorless oil.

### Reference Example 80 tert-butyl 4-{[(5-bromopyridin-3-yl)methyl](tert-butoxycarbonyl)amino}piperidine-1-carboxylate

To a solution of tert-butyl 4-{[(5-bromopyridin-3-yl)methyl]amino}piperidine-1-carboxylate obtained in Reference Example 79 Step 1 (225 mg) in dichloromethane (2 mL) was successively added BOC₂O (199 mg) and triethylamine (0.17 mL), and the mixture was stirred at room temperature for 4 hours. The reaction solution was directly purified by column chromatography to give 240 mg of the title compound as a white amorphous form.

### Reference Example 81 tert-butyl 4-{[(5-bromopyridin-3-yl)amino]methyl}piperidine-1-carboxylate

The title compound was prepared as a pale yellow solid (295 mg) according to the aforementioned procedure described in Reference Example 56, using tert-butyl 4-(aminomethyl)piperidine-1-carboxylate (295 mg) instead of thiomorpholine-1,1-dioxide.

### Reference Example 82 tert-butyl 3-[(5-bromopyridin-3-yl)amino]azetidine-1-carboxylate

To a mixture of 3,5-dibromopyridine (708 mg), tert-butyl 3-aminoazetidine-1-carboxylate (566 mg), (±)-BINAP (280 mg), sodium tert-butoxide (575 mg) and Pd₂(dba)₃ (274 mg) was added toluene (15 mL), and the interior of a vessel was purged with argon. The reaction mixture was stirred at 100 °C for 5 hours. The reaction mixture was filtered through Celite, and the filtrate was concentrated under reduced pressure. The resulting residue was purified by column chromatography to give 317 mg of the title compound as a brown amorphous form.

### Reference Example 83 tert-butyl 4-[(5-bromopyridin-3-yl)oxy]piperidine-1-carboxylate

To a solution of 5-bromopyridin-3-ol (500 mg), tert-butyl 4-hydroxypiperidine-1-carboxylate (870 mg) and triphenylphosphine (1.13 g) in toluene (15 mL) was added diethyl azodicarboxylate (hereinafter referred to as DEAD)(2.2 mol/l in toluene solution, 2.0 mL), and the mixture was stirred at 90 °C for 2 hours. The solvent was evaporated under reduced pressure. The resulting residue was purified by column chromatography to give 965 mg of the title compound as a colorless oil.

### Reference Example 84 tert-butyl (3R)-3-{[(5-bromopyridin-3-yl)amino]methyl}pyrrolidine-1-carboxylate

The title compound was prepared as a yellow amorphous form (250 mg) according to the aforementioned procedure described in Reference Example 56, using (R)-tert-butyl 3-(aminomethyl)pyrrolidine-1-carboxylate (353 mg) instead of thiomorpholine-1,1-dioxide.

### Reference Example 85 tert-butyl 4-(5-bromopyridin-3-yl)-1,4-diazepane-1-carboxylate

The title compound was prepared as a brown oil (245 mg) according to the aforementioned procedure described in Reference Example 56, using tert-butyl 1,4-diazepane-1-carboxylate (233 mg) instead of thiomorpholine-1,1-dioxide.

### Reference Example 86 3-bromo-5-[(1-methylpiperidin-4-yl)oxy]pyridine

The title compound was prepared as a colorless oil (268 mg) according to the aforementioned procedure described in Reference Example 83, using 1-methylpiperidin-4-ol (250 mg) instead of tert-butyl 4-hydroxypiperidine-1-carboxylate.

### Reference Example 87 tert-butyl 4-{2-[(5-bromopyridin-3-yl)-oxy]ethyl}piperazine-1-carboxylate

The title compound was prepared as a pale brown oil (975 mg) according to the aforementioned procedure described in Reference Example 83, using tert-butyl 4-(2-hydroxyethyl)piperazine-1-carboxylate (993 mg) instead of tert-butyl 4-hydroxypiperidine-1-carboxylate.

### Reference Example 88 4-{2-[(5-bromopyridin-3-yl)-oxy]ethyl}morpholine

The title compound was prepared as a colorless oil (400 mg) according to the aforementioned procedure described in Reference Example 83, using 2-morpholinoethanol (0.52 mL) instead of tert-butyl 4-hydroxypiperidine-1-carboxylate.

### Reference Example 89 tert-butyl 3-[(5-bromopyridin-3-yl)oxy]azetidine-1-carboxylate

The title compound was prepared as a pale brown solid (820 mg) according to the aforementioned procedure described in Reference Example 83, using tert-butyl 3-hydroxyazetidine-1-carboxylate (747 mg) instead of tert-butyl 4-hydroxypiperidine-1-carboxylate.

### Reference Example 90 2-{4-[(5-bromopyridin-3-yl)oxy]piperidin-1-yl}acetamide

### Step 1 Production of 3-bromo-5-(piperidin-4-yloxy)pyridine hydrochloride

To a solution of tert-butyl 4-[(5-bromopyridin-3-yl)oxy]piperidine-1-carboxylate obtained in Reference Example 83 (863 mg) in ethanol (1 mL) was added 2N hydrogen chloride-ethanol solution (5 mL), and the mixture was stirred at room temperature for 17 hours. The precipitated solid was collected by filtration and washed with ethanol to give 700 mg of the title compound as a white solid.

### Step 2 Production of 2 {4-[(5-bromopyridin-3-yl)-oxy]piperidine-1-yl}acetamide

A suspension of 3-bromo-5-(piperidin-4- yloxy)pyridine hydrochloride (294 mg), 2-bromoacetamide (166 mg) and potassium carbonate (415 mg) in ethanol (3 mL) was stirred at 90 °C for 1 day. Water was added to the reaction mixture, and the mixture was extracted with chloroform three times. The solvent was evaporated under reduced pressure, and the resulting residue was purified by column chromatography to give 165 mg of the title compound as a white solid.

### Reference Example 91 4-[(5-bromopyridin-3-yl)oxy]piperidine-1-carboxamide

To a suspension of 3-bromo-5-(piperidin-4-yloxy)pyridine hydrochloride obtained in Refernce Example 90 Step1(294 mg) and potassium cyanate (244 mg) in ethanol (2 mL) was added acetic acid (172 µL), and the mixture was stirred at room temperature for 1 day. Saturated aqueous sodium hydrogen carbonate solution was added to the mixture, and the mixture was stirred for an hour. The precipitated solid was collected by filtration and washed with diethylether to give 193 mg of the title compound as a white solid.

### Refence Example 92 4-(5-bromopyrimidin-2-yl)thiomorpholine 1,1-dioxide

5-bromo-2-chloropyrimidine (500 mg) and thiomorpholine-1,1-dioxide (525 mg) were dissolved in 2-ethoxyethanol (1 mL), and N,N-diisopropylethylamine (1.1 mL) was added to the mixture. The mixutre was stirred at 130 °C for 17 hours. The reaction mixure was diluted with ethyl acetate, washed with saturated brine, and the solvent was evaporated under reduced pressure. The resulting residue was purified by column chromatography to give 750 mg of title compound as pale yellow solid.

### Reference Example 93 4-(5-bromopyrimidin-2-yl)-1,4-diazepane-1-carboxamide

### Step 1 Production of tert-butyl 4-(5-bromopyrimidin-2-yl)-1,4- diazepane-1-carboxylate

The title compound was prepared as a white solid (840 mg) according to the aforementioned procedure described in Reference Example 92, using tert-butyl 1,4-diazepane-1-carboxylate (777 mg) instead of thiomorpholine-1,1-dioxide.

### Step 2 4-(5-bromopyrimidin-2-yl)-1,4-diazepane-1-carboxamide

To a solution of tert-butyl 4-(5-bromopyrimidin-2-yl)-1,4-diazepane-1-carboxylate obtained in Reference Example 93 Step 1 (200 mg) in THF (3 mL) was added 4N hydrogen chloride-ethyl acetate solution (2 mL), and the mixture was stirred at room temperature for 17 hours. Afrer evaporation of the solvent under reduced pressure, dichloromethane (10 mL), trimethylsilyl isocyanate (97 µL) and triethylamine (235 µL) was successively added to the resulting residue, and the mixture was stirred at room temperature for 1 day. Water was added to the reaction mixture, and the mixture was extracted with chloroform. The solvent was dried over Magnesium sulfate, and evaporated under reduced pressure. The resulting residue was purified by column chromatography to give 166 mg of title compound as a white solid.

### Reference Example 94 2-[(5-bromopyridin-3-yl)oxy]-N,N-dimethylethanamine

The title compound was prepared as a pale yellow oil (130 mg) according to the aforementioned procedure described in Reference Example 83, using 2-(dimethylamino)ethanol (116 mg) instead of tert-butyl 4-hydroxypiperidine-1-carboxylate.

### Reference Example 95 1-[(5-chloropyridin-3-yl)oxy]-N,N, 2 -trimethylpropan-2-amine

The title compound was prepared as a pale brown oil (420 mg) according to the aforementioned procedure described in Reference Example 83, using 5-chloropyridin-3-ol (400 mg) instead of 5-bromopyridin-3-ol, and using 2-(dimethylamino)-2-methylpropan-1-ol (543 mg) instead of tert-butyl 4-hydroxypiperidine-1-carboxylate.

### Reference Example 96 2-[(5-bromopyridin-3-yl)oxy]acetamide

A suspension of 5-bromopyridin-3-ol (250 mg) and 2-bromoacetamide (240 mg) and cesium carbonate (1.4 g) in DMF (2 mL) was stirred at 100 °C for 2 hours. The reaction mixture was diluted with ethyl acetate, washed with saturated brine, and the solvent was evaporated under reduced pressure. The resulting residue was purified by column chromatography to give 72 mg of the title compound as a pale yellow solid.

### Reference Example 97 1-[4-(5-bromopyridin-3-yl)-1,4-diazepan-1-yl]ethanone

The title compound was prepared as a yellow amorphous form (145 mg) according to the aforementioned procedure described in Reference Example 56 using 1-(1,4-diazepan-1-yl)ethanone (198 mg) instead of thiomorpholine 1,1-dioxide.

### Reference Example 98 3-bromo-5-(3-{[tert-butyl(dimethyl)silyl]oxy}pyrrolidin-1-yl)pyridine

### Step 1 Production of 1-(5-bromopyridin-3-yl)pyrrolidin-3-ol

The title compound was prepared as a pale brown solid (407 mg) according to the aforementioned procedure described in Reference Example 82 using pyrrolidin-3-ol. (174 mg) instead of tert-butyl 3-aminoazetidine-1-carboxylate.

### Step 2 Production of 3-bromo-5-(3-{[tert-butyl(dimethyl)silyl]oxy}pyrrolidin-1-yl)pyridine

The title compound was prepared as a yellow oil (570 mg) according to the aforementioned procedure described in Reference Example 5 Step 2 using 1-(5-bromopyridin-3-yl)pyrrolidin-3-ol (406 mg) obtained in Reference Example 98 Step 1 instead of 2-[(5-bromopyridin-2-yl)amino] ethanol.

### Reference Example 99 3-bromo-5-[(3S)-3-{[tert-butyl(dimethyl)silyl]oxy}piperidin-1-yl]pyridine

### Step 1 Production of (3S)-3 -{[tert-butyl(dimethyl)silyl]oxy}piperidine

To a suspension of (S)-piperidin-3-ol (1.0 g) in dichloromethane (8 mL) was added successively triethylamine (4.1 mL), DMAP (44 mg), TBSCl (1.31 g), and the mixture was stirred at room temperature for 1 day. The reaction mixture was diluted with water, extracted with dichloromethane, and the solvent was evaporated under reduced pressure. The resulting residue was purified by column chromatography to give 1.59 g of the title compound as a yellow oil.

### Step 2 Production of 3-bromo-5-[(3S)-3-{[tert-butyl(dimethyl)silyl]oxy}piperidin-1-yl]pyridine

The title compound was prepared as a yellow oil (331 mg) according to the aforementioned procedure described in Reference Example 56 using (3S)-3-{[tert-butyl(dimethyl)silyl]oxy}piperidine obtained in Reference Example 99 Step 1 (646 mg) instead of thiomorpholine 1,1-dioxide

### Reference Example 100 3-bromo-5-[(3R)-3-{[tert-butyl(dimethyl)silyl]oxy}piperidin-1-yl]pyridine

The title compound was prepared as a yellow oil (493 mg) according to the aforementioned procedure described in Reference Example 99 using (R)-piperidin-3-ol (500 mg) instead of (S)-piperidin-3-ol.

### Reference Example 101 3-bromo-5-(1-methyl-1H-pyrazol-4-yl)pyridine

To a mixture of 3,5-dibromopyridine (474 mg), 1-methyl -4 -(4,4,5,5-tetramethyl-1,3,2-dioxaborolan-2-yl)-1H-pyrazole (458 mg), potassium phosphate (1.27 g) and PdCl₂(dppf)·CH₂Cl₂ (163 mg) was added water (5.0 mL) and 1,4-dioxane (15 mL), and the interior of a vessel was purged with argon, and the reaction mixture was stirred at 100 °C for 5 hours. The reaction mixture was diluted with ethyl acetate, washed with water, and the solvent was evaporated under reduced pressure. The resulting residue was purified by column chromatography to give 242 mg of the title compound as a yellow solid.

### Reference Example 102 tert-butyl-4-(5-bromopyridin-3-yl)-2-oxopiperazine-1-carboxylate

To a mixture of 3,5-dibromopyridine (474 mg) and piperazin-2-on (601 mg) was added N-methyl-2-pyrrolidone (1 mL) and the mixture was reacted under microwave irradiation (Biotage INITIATOR, at 200 °C for 30 minutes). The reaction mixture was dissolved in ethyl acetate and water, and extracted with ethyl acetate five times. The solvent was evaporated under reduced pressure. The resulting yellow solid was dissolved in DMF (4 mL), triethylamine (585 µL), Boc2O (689 µL) and DMAP (24 mg) were successively added, and the mixture was stirred at room temperature for 17 hours. The reaction mixture was diluted with water and saturated brine. The reaction mixture was diluted with ethyl acetate and washed with water and saturated brine. The organic layer was dried over magnesium sulfate and evaporated under reduced pressure. The resulting residue was purified by column chromatography to give 74 mg of the title compound as a yellow oil.

### Reference Example 103 3-bromo-5-(4-{[tert-butyl(dimethyl)silyl]oxy}piperidin-1-yl)pyridine

The title compound was prepared as a colorless oil (603 mg) according to the aforementioned procedure described in Reference Example 98 using piperidin-3-ol (202 mg) instead of pyrrolidin-3-ol.

### Reference Example 104 5-bromo-1-{1,3-bis[tert-butyl(dimethyl)siloxy]propan-2-yl}-1H-benzimidazole

### Step 1 Production of 2-[(4-bromo-2-nitrophenyl)amino]propane-1,3-diol

To a suspension of 1,4-dibromo-2-nitrobenzene (2.0 g) and 2-aminopropane-1,3-diol (1.29 g) in ethanol (5 mL) was added N,N-diisopropylethylamine (1.84 g), and the mixture was stirred in a sealed tube at 150 °C overnight. 1N aqueous hydrochloric acid solution was added to the mixture, and extracted with ethyl acetate. The organic layer was washed with saturated aqueous sodium hydrogen carbonate solution, and dried over anhydrous magnesium sulfate. The solvent was evaporated under reduced pressure, and the resulting residue was purified by column chromatography to give 722 mg of the title compound as brown solid.

### Step 2 Production of 2-(5-bromo-1H-benzimidazol-1-yl)propane-1,3-diol

To a suspension of 2-[(4-bromo-2-nitrophenyl)amino]propane-1,3-diol obtained in Reference Example 104 Step 1 (500 mg) in ethanol (5 mL) was added 1% Platinum + 0.1% Copper on Activated Carbon (Degussa type CF 105 R/W, manufactured by Wako Pure Chemical Industries, Ltd.)(hereinafter referred to as CF 105 R/W) (50 mg), and the mixture was stirred at room temperature for 6 hours under 3 atm hydrogen atmosphere. The reaction mixture was filtered through Celite, and the filtrate was concentrated under reduced pressure. To the resulting residue was successively added methyl orthoformate (3.5 mL) and TFA (2.0 mL), and the mixture was stirred at 80 °C for an hour. The solvent was evaporated under reduced pressure, and the resulting residue was purified by column chromatography to give 323 mg of the title compound as a pale yellow solid.

### Step 3 Production of 5-bromo-1-{1,3-bis[tert-butyl(dimethyl)siloxy]propan-2-yl}-1H-benzimidazole

The title compound was prepared as a yellow oil (284 mg) according to the aforementioned procedure described in Reference Example 5 Step 2 using 2-(5-bromo-1H-benzimidazol-1-yl)propan-1,3-diol (323 mg) obtained in Reference Example 104 Step 2 instead of 2-[(5-bromopyridin-2-yl)amino] ethanol.

### Reference Example 105 2-Methyl-l-[5-(4,4,5,5-tetramethyl-1,3,2-dioxaborolan-2-yl)-1H-benzimidazol-1-yl]propan-2-ol

### Step 1 Production of 1-amino- 2-methylpropan-2-ol hydrochloride

To a suspension of lithium aluminium hydride (1.78 g) in THF (30 mL) was slowly added a solution of cyanoacetohydrin (2.0 g) in THF (10 mL) at 0 °C, and the mixture was stirred at room temperature overnight. The reaction mixture was diluted with diethyl ether at 0 °C, and then water (1.8 mL), 15 % aqueous sodium hydroxide solution (1.8 mL), water (5.4 mL) was added, and the mixture was stirred at room temperature for an hour. The reaction mixture was filtered through Celite, and 2N hydrochloric acid-eyhanol solution was added to the filtrate. The mixture was concentrated under reduced pressure to give 1.65 g of the title compound as a pale yellow oil.

### Step 2 Production of 1-(5-Bromo-1H-benzimidazol-1-yl)-2-methylpropan-2-ol

The title compound was prepared as a brown amorphous form (635 mg) according to the aforementioned procedure described in Reference Example 104 Step 1, 2 using 1-amino- 2-methylpropan-2-ol hydrochloride (820 mg) obtained in Reference Example 105 Step 1 instead of 2-aminopropane-1,3-diol.

### Step 3 Production of 2-Methyl-1-[5-(4,4,5,5-tetramethyl-1,3,2-dioxaborolan-2-yl)-1H-benzimidazol-1-yl] propan-2-ol

To a mixture of 1-(5-bromo-1H-benzimidazol-1-yl)-2-methylpropan-2-ol (431 mg), bis(pinacolato)diboron (hereinafter referred to as Pin₂B₂) (528 mg), potassium acetate (785 mg) and PdCl₂(dppf)·CH₂Cl₂ (65 mg) was added 1,4-dioxane (10 mL), and the interior of a vessel was purged with argon, and the reaction mixture was stirred at 100 °C overnight. The reaction mixture was filtered through Celite, and the filtrate was concentrated under reduced pressure. The resulting residue was purified by column chromatography to give 488 mg of the title compound as a pale brown amorphous form.

### Reference Example 106 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole

### Step 1 Production of 5-bromo-1-(3-{[tert-butyl(dimethyl)silyl]oxy}-2,2-dimethylpropyl)-1H-benzimidazole

The title compound was prepared as a pale brown oil (486 mg) according to the aforementioned procedure described in Reference Example 104 using 3-amino-2,2-dimethylpropan-1-ol (734 mg) instead of 2-aminopropane-1,3-diol.

### Step 2 Production of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole

The title compound was prepared as a pale brown oil (708 mg) according to the aforementioned procedure described in Reference Example 105 Step 3 using 5-bromo-1-(3-{[tert-butyl(dimethyl)silyl]oxy}-2,2-dimethylpropyl)-1H-benzimidazole (486 mg) obtained in Reference Example 106 Step 1 instead of 1-(5-bromo-1H-benzimidazol-1-yl)-2-methylpropan-2-ol.

### Reference Example 107 1-{[5-(4,4,5,5-tetramethyl-1,3,2-dioxaborolan-2-yl)-1H-benzimidazol-1-yl]methyl}cyclohexanol

The title compound was prepared as a pale brown amorphous form (514 mg) according to the aforementioned procedure described in Reference Example 104 Step 1 and Step 2, and Reference Example 105 Step 3 using 1-(aminomethyl)cyclohexanol (960 mg) instead of 2-aminopropane-1,3-diol

### Reference Example 108 1-(3-{[tert-butyl(dimethyl)silyl]oxy}propyl)-5-(4,4,5,5-tetramethyl-1,3,2- dioxaborolan-2-yl)-1H-benzimidazole

The title compound was prepared as a yellow solid (320 mg) according to the aforementioned procedure described in Reference Example 104 and Reference Example 105 Step 3 using 3-amino-1-propanol (535 mg) instead of 2-aminopropane-1,3-diol.

### Refence Wxample 109 1-(trans-4-{[tert-butyl(dimethyl)silyl]oxy}cyclohexyl)-6-(4,4,5,5-tetramethyl-1,3,2- dioxaborolan-2-yl)-1H-benzimidazole

The title compound was prepared as a brown oil (121 mg) according to the aforementioned procedure described in Reference Example 104 and Reference Example 105 Step 3 using 2,4-dibromo-1-nitrobenzene (400 mg) instead of 1,4-dibromo-2-nitrobenzene and using trans-4-amino cyclohexanol (327 mg) instead of 2-aminopropane-1,3-diol.

### Reference Example 110 ethyl 3-[5-(4,4,5,5-tetramethyl-1,3,2-dioxaborolan-2-yl)-1H-benzimidazol-1-yl]propanoate

### Step 1 N-(4-bromo-2-nitrophenyl)-β-alanine

The title compound was prepared as a brown solid (1.0 g) according to the aforementioned procedure described in Reference Example 104 Step 1 using 3-aminopropanoic acid (634 mg) instead of 2-aminopropane-1,3-diol.

### Step 2 Production of ethyl N-(4-bromo-2-nitrophenyl)-β-alaninate

To a suspension of N-(4-bromo-2-nitrophenyl)-β-alanine obtained in Reference Example 110 Step 1 (1.0 g) in ethanol (15 mL) was added concentrated sulfuric acid (0.5 mL), and the mixture was stirred under reflux for 4 hours. The solvent was evaporated under reduced pressure, and 0.5 N aqueous sodium hydroxide solution was added to the residue,and extracted with ethyl acetate. The organic layer was washed with saturated brine and dried over magnesium sulfate. The solvent was evaporated under reduced pressure. The resulting residue was purified by column chromatography to give 333 mg of the title compound as a brown amorphous form.

### Step 3 Production of ethyl 3-(5-bromo-1H-benzimidazol-1-yl)propanoate

The title compound was prepared as a pale brown oil (96 mg) according to the aforementioned procedure described in Reference Example 104 Step 2 using ethyl N-(4-bromo-2-nitrophenyl)-β-alaninate obtained in Reference Example 110 Step 2 (150 mg) instead of 2-[(4-bromo-2-nitrophenyl)amino]propane-1,3-diol.

### Step 4 Production of ethyl 3-[5-(4,4,5,5-tetramethyl-1,3,2-dioxaborolan-2-yl)-1H-benzimidazole-1-yl]propanoate

The title compound was prepared as a brown oil (125 mg) according to the aforementioned procedure described in Reference Example 105 Step 3 using ethyl 3-(5-bromo-1H-benzimidazol-1-yl)propanoate obtained in Reference Example 110 Step 3 (96 mg) instead of 1-(5-bromo-1H-benzimidazol-1-yl)-2-methylpropan-2-ol.

### Reference Example 111 1-(3-{[tert-butyl(dimethyl)silyl]oxy}propyl)-6-(4,4,5,5-tetramethyl-1,3,2-dioxaborolan-2-yl)-1H-benzimidazole

### Step 1 Production of 3-[(5-bromo-2-nitrophenyl)amino]propan-1-ol

To a solution of 4-bromo-2-fluoronitrobenzene (1.0 g) in acetonitrile (10 mL) was added N,N-diisopropylethylamine (883 mg) and 3-amino-1-propanol (684 mg), and the mixture was stirred at 80 °C for 2.5 hours. The solvent was evaporated under reduced pressure, and the resulting residue was purified by column chromatography to give 1.2 g of the title compound as a yellow solid.

### Step 2 Production of 3-(6-bromo-1H-benzimidazol-1-yl)propan-1-ol

To a suspension of 3-[(5-bromo-2-nitrophenyl)amino]propan-1-ol obtained in Reference Example 111 Step 1 (1.2 g) in ethanol (12 mL) was added CF 105 R/W (240 mg), and the mixture was stirred at room temperature for 4 hours under 3 atm hydrogen atmosphere. The reaction mixture was filtered through Celite, and the filtrate was concentrated under reduced pressure. To the resulting residue was successively added toluene (11 mL), methyl orthoformate (694 mg) and p-toluenesulfonic acid monohydrate (124 mg), and the mixture was stirred 50 °C for an hour. The solvent was evaporated under reduced pressure, and the resulting residue was purified by column chromatography to give 920 mg of the title compound as a brown solid.

### Step 3 Production of 6-bromo-1-(3-{[tert-butyl(dimethyl)silyl]oxy}propyl)-1H-benzimidazole

The title compound was prepared as a gray powder (1.17 g) according to the aforementioned procedure described in Reference Example 5 Step 2 using 3-(6-bromo-1H-benzimidazol-1-yl)propan-1-ol obtained in Reference Example 111 Step 2 (920 mg) instead of 2-[(5-bromopyridin-2-yl)amino] ethanol.

### Step 4 Production of 1-(3-{[tert-butyl(dimethyl)silyl]oxy}propyl)-6-(4,4,5,5-tetramethyl-1,3,2-dioxaborolan-2-yl)-1H-benzimidazole

The title compound was prepared as a pale brown oil (405 mg) according to the aforementioned procedure described in Reference Example 105 Step 3 using 6-bromo-1-(3-{[tert-butyl(dimethyl)silyl]oxy}propyl)-1H-benzimidazole obtained in Reference Example 111 Step 3 (300 mg) instead of 1-(5-bromo-1H-benzimidazol-1-yl)-2-methylpropan-2-ol.

### Reference Example 112 2-(trimethylsilyl)ethyl 3-[6-(4,4,5,5-tetramethyl-1,3,2-dioxaborolan-2-yl)-1H-benzimidazol-1-yl]propanoate

### Step 1 Production of ethyl 3-(6-bromo-1H-benzimidazol-1-yl)propanoate

The title compound was prepared as a brown oil (874 mg) according to the aforementioned procedure described in Reference Example 111 Step 1 and Step 2 using ethyl 3-aminopropanoate hydrochloride (1.4 g) instead of 3-amino-1-propanol.

### Step 2 Production of 3-(6-bromo-1H-benzimidazol-1-yl)propanoic acid

Ethyl 3-(6-bromo-1H-benzimidazol-1-yl)propanoate obtained in Reference Example 112 Step 1 (774 mg) was dissolved in ethanol (20 mL), and 2N aqueous sodium hydroxide solution (5 mL) was added, and the mixture was stirred at room temperature for an hour. Etanol was evaporated under reduced pressure, and the resulting residue was neutralized with 1N aqueous hydrochloric acid solution. The precipitated solid was collected by filtration and washed with water to give 613 mg of the title compound as a white powder.

### Step 3 Production of 2-(trimethylsilyl)ethyl 3-(6-bromo-1H-benzimidazol-1-yl) propanoate

To a suspension of 3-(6-bromo-1H-benzimidazol-1-yl)propanoic acid obtained in Reference Example 112 Step 2 (613 mg) in dichloromethane (12 mL) was successively added triethylamine (1.15 g), 2-trimethylsilylethanol (350 mg), WSCD·HCl (656 mg) and DMAP (418 mg), and the mixture was stirred at room temperature overnight. The solvent was evaporated under reduced pressure, and the resulting residue was purified by column chromatography to give 683 mg of the title compound as a pale brown oil.

### Step 4 Production of 2-(trimethylsilyl)ethyl 3-[6-(4,4,5,5-tetramethyl-1,3,2-dioxaborolan-2-yl)-1H-benzimidazol-1-yl]propanoate

The title compound was prepared as a pale yellow oil (131 mg) according to the aforementioned procedure described in Reference Example 105 Step 3 using 2-(trimethylsilyl)ethyl 3-(6-bromo-1H-benzimidazol-1-yl) propanoate obtained in Reference Example 112 Step 3 (150 mg) instead of 1-(5-bromo-1H-benzimidazol-1-yl)-2-methylpropan-2-ol.

### Reference Example 113 1-(4-{[tert-butyl(dimethyl)silyl]oxy}butyl)-6-(4,4,5,5-tetramethyl-1,3,2-dioxaborolan-2-yl)-1H-benzimidazole

The title compound was prepared as a pale brown oil according to the aforementioned procedure described in Reference Example 111 using 4-amino-1-butanol instead of 3-amino-1-propanol.

### Reference Example 114 1-(4-{[tert-butyl(dimethyl)silyl]oxy}butyl)-5-(4,4,5,5-tetramethyl-1,3,2-dioxaborolan-2-yl)-1H-benzimidazole

The title compound was prepared as a pale brown oil according to the aforementioned procedure described in Reference Example 111 using 4-bromo-1-fluoro-2-nitrobenzene instead of 4-bromo-2-fluoronitrobenzene, and using 4-amino-1-butanol instead of 3-amino-1-propanol.

### Reference Example 115 2-(trimethylsilyl)ethyl 3-[5-(4,4,5,5-tetramethyl-1,3,2-dioxaborolan-2-yl)-1H-benzimidazol-1-yl]propanoate

### Step 1 Production of ethyl N-(4-bromo-2-nitrophenyl)- β-alaninate

A suspension of 4-bromo-1-fluoro-2-nitrobenzene (500 mg), ethyl 3-aminopropanoate hydrochloride (349 mg) and potassium carbonate (1.25 g) in DMF (5 mL) was stirred at 60 °C for an hour. Saturated aqueous ammonium chloride solution was added to the reaction mixture, and the mixture was extracted with ethyl acetate. The organic layer was dried over Magnesium sulfate, and evaporated under reduced pressure. The resulting residue was purified by column chromatography to give 798 mg of the title compound as a brown oil.

### Step 2 Production of ethyl 3-(5-bromo-1H-benzimidazol-1-yl)propanoate

The title compound was prepared as a brown oil (993 mg) according to the aforementioned procedure described in Reference Example 111 Step 2 using Ethyl N-(4-bromo-2-nitrophenyl)-β-alaninate obtained in Reference Example 115 Step 1 instead of 3-[(5-bromo-2-nitrophenyl)amino]propan-1-ol.

### Step 3 Production of 2-(trimethylsilyl)ethyl 3-[5-(4,4,5,5-tetramethyl-1,3,2-dioxaborolan-2-yl)-1H-benzimidazol-1-yl]propanoate

The title compound was prepared as a pale brown solid (693 mg) according to the aforementioned procedure described in Reference Example 112 Step 2, 3 and 4 using ethyl 3-(5-bromo-1H-benzimidazol-1-yl)propanoate obtained in Reference Example 115 Step 2 instead of 3-(6-bromo-1H-benzimidazol-1-yl)propanoate.

### Reference Example 116 1-(pyridin-3-ylmethyl)-6-(4,4,5,5-tetramethyl-1,3,2-dioxaborolan-2-yl)-1H-benzimidazole

### Step 1 Production of 6-bromo-1-(pyridine-3-ylmethyl)-1H-benzimidazole

The title compound was prepared as a pale brown solid (330 mg) according to the aforementioned procedure described in Reference Example 111 Step 1, 2 using 3-picolylamine (294 mg) instead of 3-amino-1-propanol.

### Step 2 Production of 1-(pyridin-3-ylmethyl)-6-(4,4,5,5-tetramethyl-1,3,2-dioxaborolan-2-yl-)-1H-benzimidazole

The title compound was prepared as a pale brown amorphous form (107 mg) according to the aforementioned procedure described in Reference Example 105 Step 3 using 6-bromo-1-(pyridine-3-ylmethyl)-1H-benzimidazole obtained in Reference Example 116 Step 1 instead of 1-(5-bromo-1H-benzimidazol-1-yl)-2-methylpropan-2-ol.

### Reference Example 117 1-(pyridin-4-ylmethyl)-6-(4,4,5,5-tetramethyl-1,3,2-dioxaborolan-2-yl)-1H-benzimidazole

### Step 1 Production of 6-bromo-1-(pyridin-4-ylmethyl)-1H-benzimidazole

The title compound was prepared as a pale brown solid (191 mg) according to the aforementioned procedure described in Reference Example 111 Step 1, 2 using 4-picolylamine (294 mg) instead of 3-amino-1-propanol.

### Step 2 Production of 1-(pyridin-4-ylmethyl)-6-(4,4,5,5-tetramethyl-1,3,2-dioxaborolan-2-yl)-1H-benzimidazole

The title compound was prepared as a brown powder (103 mg) according to the aforementioned procedure described in Reference Example 105 Step 3 using 6-bromo-1-(pyridin-4-ylmethyl)-1H-benzimidazole (100 mg) obtained in Reference Example 117 Step 1 instead of 1-(5-bromo-1H-benzimidazol-1-yl)-2-methylpropan-2-ol.

### Reference Example 118 3-[5-(4,4,5,5-tetramethyl-1,3,2-dioxaborolan-2-yl)-1H-benzimidazol-1-yl]propanenitrile

### Step 1 Production of 3-(5-bromo-1H-benzimidazol-1-yl)propanenitrile

The title compound was prepared as a pale brown powder according to the aforementioned procedure described in Reference Example 111 using 4-bromo-1-fluoro-2-nitrobenzene instead of 4-bromo-2-fluoronitrobenzene, and using 3-aminopropionitrile instead of 3-amino-1-propanol.

### Step 2 Production of 3-[5-(4,4,5,5-tetramethyl-1,3,2-dioxaborolan-2-yl)-1H-benzimidazol-1-yl]propanenitrile

The title compound was prepared as a pale brown powder (55 mg) according to the aforementioned procedure described in Reference Example 105 Step 3 using 3-(5-bromo-1H-benzimidazol-1-yl)propanenitrile (54 mg) obtained in Reference Example 118 Step 1 instead of 1-(5-bromo-1H-benzimidazol-1-yl)-2-methylpropan-2-ol.

### Reference Example 119 1-[1-({[tert-butyl(dimethyl)silyl]oxy}methyl)cyclohexyl]-6-(4,4,5,5-tetramethyl-1,3,2-dioxaborolan-2-yl)-1H-benzimidazole

### Step 1 Production of (1-aminocyclohexyl)methanol

To a suspension of lithium aluminium hydride (398 mg) in THF (5 mL) was added 1-aminocyclohexanecarboxylic acid (500 mg) at 0 °C, and the mixture was stirred at room temperature overnight. To the mixture was added water (0.4 mL), 15 % aqueous sodium hydroxide solution (0.4 mL) and water (1.2 mL) at 0 °C, and the mixture was stirred at room temperature for an hour. The reaction mixture was filtered through Celite, and the filtrate was concentrated under reduced pressure to give 438 mg of the title compound as a colorless oil.

### Step 2 Production of 1-[1-({[tert-butyl(dimethyl)silyl]oxy}methyl)cyclohexyl]-6-(4,4,5,5-tetramethyl-1,3,2-dioxaborolan-2-yl)-1H-benzimidazole

The title compound was prepared as a pale brown oil according to the aforementioned procedure described in Reference Example 111 using (1-aminocyclohexyl)methanol obtained in Reference Example 119 Step 1 (351 mg) instead of 3-amino-1-propanol.

### Reference Example 120 1-(4,4-difluorocyclohexyl)-6-(4,4,5,5-tetramethyl-1,3,2-dioxaborolan-2-yl)-1H-benzimidazole

### Step 1 Production of 6-bromo-1-(4,4-difluorocyclohexyl)-1H-benzimidazole

The title compound was prepared as a brown amorphous form (112 mg) according to the aforementioned procedure described in Reference Example 111 Step 1, 2 using 4,4-difluorocyclohexylamine hydrochloride (386 mg) instead of 3-amino-1-propanol.

### Step 2 Production of 1-(4,4-difluorocyclohexyl)-6-(4,4,5,5-tetramethyl-1,3,2-dioxaborolan-2-yl)-1H-benzimidazole

The title compound was prepared as a pale brown powder (105 mg) according to the aforementioned procedure described in Reference Example 105 Step 3 using 6-bromo-1-(4,4-difluorocyclohexyl)-1H-benzimidazole (112 mg) obtained in Reference Example 120 Step 1 instead of 1-(5-bromo-1H-benzimidazol-1-yl)-2-methylpropan-2-ol.

### Reference Example 121 1-benzyl-5-(4,4,5,5-tetramethyl-1,3,2-dioxaborolan-2-yl)-1H-benzimidazole

### Step 1 Production of N-benzyl-4-bromo-2-nitroaniline

To a solution of 4-bromo-1-fluoro-2-nitrobenzene (500 mg) in acetonitrile (3 mL) was added triethylamine (345 mg) and benzylamine (255 mg) and the mixtute was at 50 °C for 3.5 hours. The solvent was evaporated under reduced pressure, and the resulting residue was purified by column chromatography to give 703 mg of the title compound as a brown powder.

### Step 2 Production of 1-benzyl-5-bromo-1H-benzimidazole

The title compound was prepared as a brown powder (173 mg) according to the aforementioned procedure described in Reference Example 104 Step 2 using N-benzyl-4-bromo-2-nitroaniline obtained in Reference Example 121 Step 1 (326 mg) instead of 2-[(4-bromo-2-nitrophenyl)amino]propane-1,3-diol.

### Step 3 Production of 1-benzyl-5-(4,4,5,5-tetramethyl-1,3,2-dioxaborolan-2-yl)-1H-benzimidazole

The title compound was prepared as a yellow powder (287 mg) according to the aforementioned procedure described in Reference Example 105 Step 3 using 1-benzyl-5-bromo-1H-benzimidazole

(244 mg) obtained in Reference Example 121 Step 2 instead of 1-(5-bromo-1H-benzimidazol-1-yl)-2-methylpropan-2-ol.

### Reference Example 122 6-bromo-1-(trans-4-methoxycyclohexyl)-1H-benzimidazole

### Step 1 Production of trans-4-(6-bromo-1H-benzimidazol-1-yl)cyclohexanol

The title compound was prepared according to the aforementioned procedure described in Reference Example 104 Step 1 and 2 using 2,4-dibromo-1-nitrobenzene instead of 1,4-dibromo-2-nitrobenzene, and using trans-4-aminocyclohexanol instead of 2-aminopropane-1,3-diol.

### Step 2 Production of 6-bromo-1-(trans-4-methoxycyclohexyl)-1H-benzimidazole

To a solution of trans-4-(6-bromo-1H-benzimidazol-1-yl)cyclohexanol obtained in Reference Example 122 Step 1 (600 mg) in THF (20 mL) was added sodium hydride (100 mg) and methyl iodide (1.24 mL) at 0°C and the mixture was stirred at room temperature for 6 hours. Water was added to the reaction mixture, and extracted with ethyl acetate. The solvent was evaporated under reduced pressure, and the resulting residue was purified by column chromatography to give 350 mg of the title compound.

### Reference Example 123 6-bromo-1-(tetrahydro-2H-pyran-4-yl)-1H-benzimidazole

The title compound was prepared according to the aforementioned procedure described in Reference Example 111 Step 1 and 2 using tetrahydro-2H-pyran-4-amine instead of 3-amino-1-propanol.

### Reference Example 124 trans-2-(6-bromo-1H-benzimidazol-1-yl)cyclopentanol

The title compound was prepared according to the aforementioned procedure described in Reference Example 111 Step 1 and 2 using trans-2-aminocyclopentanol instead of 3-amino-1-propanol.

### Reference Example 125 6-bromo-1-ethyl-1H-benzimidazole

The title compound was prepared as a yellow oil (560 mg) according to the aforementioned procedure described in Reference Example 111 Step 1 and 2 using ethylamine (2M solution in THF, 2.7 mL) instead of 3-amino-1-propanol.

### Reference Example 126 6-bromo-1-(1,3-bis{[tert-butyl(dimethyl)silyl]oxy}propan-2-yl)-1H-benzimidazole

### Step 1 Production of 2-[(5-bromo-2-nitrophenyl)amino]propane-1,3-diol

The title compound was prepared (950 mg) according to the aforementioned procedure described in Reference Example 111 Step 1 using 2-aminopropane-1,3-diol instead of 3-amino-1-propanol.

### Step 2 Production of Production of N-(1,3-bis{[tert-butyl(dimethyl)silyl]oxy}propan-2-yl)-N-(5-bromo-2-nitrophenyl)amine

The title compound was prepared (1.83 g) according to the aforementioned procedure described in Reference Example 5 Step2, using 2-[(5-bromo-2-nitrophenyl)amino]propane-1,3-diol obtained in Reference Example 126 step 1 (950 mg) instead of 2-[(5-bromopyridin-2-yl)amino]ethanol.

### Step 3 Production of 6-bromo-1-(1,3-bis{[tert-butyl(dimethyl)silyl]oxy}propan-2-yl)-1H-benzimidazole

The title compound was prepared (1.15 g) according to the aforementioned procedure described in Reference Example 111 Step 2 using N-(1,3-bis{[tert-butyl(dimethyl)silyl]oxy}propan-2-yl)-N-(5-bromo-2-nitrophenyl)amine (1.83 g) obtained in Reference Example 126 Step 2 instead of 3-[(5-bromo-2-nitrophenyl)amino]propan-1-ol.

### Reference Example 127 1-(6-bromo-1H-benzimidazol-1-yl)-2-methylpropan-2-ol

The title compound was prepared (365 mg) according to the aforementioned procedure described in Reference Example 111 Step 1 and 2 using 1-amino- 2-methylpropan-2-ol hydrochloride obtained in Reference Example 105 Step 1 (890 mg) instead of 3-amino-1-propanol.

### Reference Example 128 6-bromo-1-(cis-4-{[tert-butyl(dimethyl)silyl]oxy}cyclohexyl)-1H-benzimidazole

### Step 1 Production of 4-(6-bromo-1H-benzimidazol-1-yl)cyclohexanone

To a solution of trans-4-(6-bromo-1H-benzimidazol-1-yl)cyclohexanol obtained in Reference Example 122 Step 1 (906 mg) in dichloromethane (10 mL) was added Dess-Martin reagent (3.88 g), and the mixture was stirred at room temperature overnight. Water was added to the reaction mixture, and extracted with chloroform. The solvent was evaporated under reduced pressure, and the insoluble solid in chloroform was filtered off. The filtrate was evaporated under reduced pressure. The resulting residue was washed with ethyl acetate to give 724 mg of the title compound.

### Step 2 Production of 4-(6-bromo-1H-benzimidazol-1-yl)cyclohexanol

To a solution of 4-(6-bromo-1H-benzimidazol-1-yl)cyclohexanone obtained in Reference Example 128 Step 1 (720 mg) in THF (20 mL) was added lithium tri-sec-butylborohydride (also referred to as L-Selectride, 3.48 mL, 1M solution in THF) at -78 °C, and the mixture was stirred at the same temperature for 2 hours. Water was added to the mixture at -78 °C and then warmed to room temperature. The mixture was extracted with ethyl acetate, and the solvent was evaporated under reduced pressure. The resulting residue was purified by column chromatography to give 561 mg of the title compound as a mixture of cis- and trans- isomers

### Step 3 Production of 6-bromo-1-(cis-4-{[tert-butyl(dimethyl)silyl]oxy}cyclohexyl)-1H-benzimidazole

The title compound was prepared (347 mg) according to the aforementioned procedure described in Reference Example 5 Step 2, using 4-(6-bromo-1H-benzimidazol-1-yl)cyclohexanol obtained in Reference Example 128 Step 2 (561 mg) instead of 2-[(5-bromopyridin-2-yl)amino] ethanol.

### Reference Example 129 trans-2-(6-bromo-1H-benzimidazol-1-yl)cyclohexanol

The title compound was prepared (830 mg) according to the aforementioned procedure described in Reference Example 111 Step 1 and 2 using trans-2-aminocyclohexanol hydrochloride (826 mg) instead of 3-amino-1-propanol.

### Reference Example 130 2-(5-bromo-1H-benzimidazol-1-yl)ethanol

2-Aminoethanol (1 mL) was added to 1,4-dibromo-2-nitrobenzene (500 mg), and the mixture was reacted under microwave irradiation (Biotage INITIATOR, at 80 °C for 30 minutes).The reaction mixture was diluted with ethyl acetate, washed with saturated aqueous sodium hydrogen carbonate solution , and the solvent was evaporated under reduced pressure.The resulting residue was dissolved in ethanol-acetic acid (1 :1, 10 mL), iron (653 mg) was added, and the reaction mixutre was stirred at 80 °C for 30 minutes. To the reaction mixture was added methyl orthoformate (420 µL), and the mixture was stirred at 80 °C for 30 minutes. The reaction mixture was filtered through Celite, and the filtrate was diluted with ethyl acetate.. The organic layer was washed with 4N aqueous sodium hydroxide solution, dried over sodium sulfate. The solvent was evaporated under reduced pressure, to give 480 mg of the title compound as a brown solid.

### Reference Example 131 5-bromo-1-(2-methoxyethyl)-1H-benzimidazole

To a solution of 2-(5-romo-1H-benzimidazol-1-yl)ethanol obtained Reference Example 130 (150 mg) in THF (3 mL) was added sodium hydride (33 mg) at 0 °C and the mixture was stirred at 0 °C for 30 minutes. Methyl iodide (58 µL) was added at the same temperature, and the mixture was further stirred at room temperature for 3 hours. Water was added to the reaction mixture, and extracted with ethyl acetate, dried over sodium sulfate. The solvent was evaporated under reduced pressure, and the resulting residue was purified by column chromatography to give 137 mg of the title compound as a colorless oil.

### Reference Example 132 5-bromo-1-ethyl-1H-benzimidazole

To a solution of 1,4-dibromo-2-nitrobenzene (1.0 g), in methanol (2.0 mL) was added ethylamine (2M solution in methanol, 4 mL), and the mixture was reacted under microwave irradiation (Biotage INITIATOR, at 110 °C for an hour). The reaction mixture was diluted with ethyl acetate, washed with saturated aqueous sodium hydrogen carbonate solution, and the solvent was evaporated under reduced pressure. The resulting residue was dissolved in methanol-acetic acid (1 : 2, 15 mL), iron (990 mg) was added and the mixture was stirred at 80 °C for 30 minutes. Methyl orthoformate (4.2 mL) was added to the mixture, and the mixture was stirred at 80 °C for 20 minutes. The solvent was evaporated under reduced pressure, and 4N aqueous sodium hydroxide solution was added to the mixture and filterd through Celite. The filtrate was diluted with water, extracted with ethyl acetate, dried over sodium sulfate. The solvent was evaporated to give 800 mg of the title compound as a pale brown solid.

### Reference Example 133 5-bromo-1-cyclopropyl-1H-benzimidazole

To 1,4-dibromo-2-nitrobenzene (500 mg) was successively added tert-butanol (4 mL), cyclopropylamine (0.7 mL) and potassium carbonate (790 mg), and the mixture was reacted under microwave irradiation (Biotage INITIATOR, at 140 °C for 30 minutes). DMF (2 mL) was added to the mixture and reacted under microwave irradiation again (Biotage INITIATOR, at 180 °C for 30 minutes). Water was added to the reaction mixture, and the mixture was extracted with diethyl ether. The organic layer was dried over sodium sulfate, and evaporated under reduced pressure. The resulting residue was dissolved in ethanol-acetic acid (1 : 1, 10 mL) and iron (320 mg) was added, and the reaction mixutre was stirred at 100 °C for 2 hours. Methyl orthoformate (840 µL) was added to the reaction mixture, and the mixture was stirred 100 °C for 30 minuutes. The reaction mixture was filtered through Celite, and the filtrate was concentrated under reduced pressure.The resulting residue was diluted with ethyl acetate, and washed with 4N aqueous sodium hydroxide solution, dried over Sodium sulfate. The solvent was evaporated under reduced pressure, and the resulting residue was purified by column chromatography to give 185 mg of the title compound as a yellow oil.

### Reference Example 134 1-[trans-2-{[tert-butyl(dimethyl)silyl]oxy}cyclohexyl]-5-(4,4,5,5-tetramethyl-1,3,2-dioxaborolan-2-yl)-1H-benzimidazole

### Step 1 Production of trans-2-(5-bromo-1H-benzimidazol-1-yl)cyclohexanol

To 1,4-dibromo-2-nitrobenzene (1.0 g) was successively added N-methylpyrrolidone (8.5 mL) , , trans-2-aminocyclohexanol hydrochloride (1.2 g) and N,N-diisopropylethylamine (6.5 mL), and the mixture was reacted under microwave irradiation (Biotage INITIATOR, at 200 °C for an hour). Water was added to the reaction mixture, and extracted with ethyl acetate. The organic layer was dried over Sodium sulfate, and the solvent was evaporated under reduced pressure. The resulting residue was purified by column chromatography to give 776 mg of a brown solid. To the obtained solid was added ethanol-acetic acid (1 : 2, 12 mL) and iron (634 mg), and the reaction mixutre was stirred at 100 °C for 30 minutes. Methyl orthoformate (2.1 mL) was added to the reaction mixture, and the mixture was stirred 100 °C for an hour. The reaction mixture was filtered through Celite, and the filtrate was concentrated under reduced pressure. The resulting residue was diluted with ethyl acetate, and washed with 4N aqueous sodium hydroxide solution, and the organic layer was dried over sodium sulfate. The solvent was evaporated under reduced pressure, and the resulting residue was purified by column chromatography to give 468 mg of the title compound as a brown solid.

### Step 2 Production of 5-bromo-1-[trans-2-{[tert-butyl(dimethyl)silyl]oxy}cyclohexyl]-1H-benzimidazole

To a solution of trans-2-(5-bromo-1H-benzimidazol-1-yl)cyclohexanol obtained in Reference Example 134 Step 1 (468 mg) in THF (7 mL) was successively added N,N,-diisopropylethylamine (0.81 mL), TBSCl (1.0 g) and imidazole (325 mg) and the mixture was stirred at room temperature overnight. Saturated aqueous sodium hydrogen carbonate solution was added to the mixture, and extracted with ethyl acetate. The solvent was evaporated under reduced pressure, and the resulting residue was purified by column chromatography to give 700 mg of the title compound as a yelow oil.

### Step 3 Production of 1-[trans-2-{[tert-butyl(dimethyl)silyl]oxy}cyclohexyl]-5-(4,4,5,5-tetramethyl-1,3,2-dioxaborolan-2-yl)-1H-benzimidazole]

The title compound was prepared according to the aforementioned procedure described in Reference Example 105 Step 3 using 5-bromo-1-[trans-2-{[tert-butyl(dimethyl)silyl]oxy}cyclohexyl]-1H-benzimidazole obtained in Reference Example 134 Step 2 instead of 1-(5-bromo-1H-benzimidazol-1-yl)-2-methylpropan-2-ol.

### Reference Example 135 1-methyl-6-(4,4,5,5-tetramethyl-1,3,2-dioxaborolan-2-yl)-1H-benzimidazole

Step 1 Production of 6-chloro-1-methyl-1H-benzimidazole 5-Chloro-N-methyl-2-nitroaniline (5.0 g) was dissolved in ethanol-water (4 : 1, 130 mL), iron (1.1 g) and ammonium chloride (800 mg) were added to the solution and the mixutre was stirred at 80 °C for 2 hours. The solvent was concentrated under reduced pressure, and the resulting residue was diluted with ethyl acetate and filtered through Celite. The filtrate was evaporated under reduced pressure again and methyl orthofomate (75 mL), TFA (100µL) was added to the resulting residue and the mixture was stirred at 100 °C for 2 hours. The solvent was concentrated under reduced pressure, and the resulting residue was diluted with ethyl acetate and washed with saturated aqueous sodium hydrogen carbonate solution. The solvent was evaporated under reduced pressure, and the resulting residue was purified by column chromatography to give 4.5 g of the title compound as a brown solid.

### Step 2 Production of 1-methyl-6-(4,4,5,5-tetramethyl-1,3,2-dioxaborolan-2-yl)-1H-benzimidazole

To a mixture of 6-chloro-1-methyl-1H-benzimidazole obtained in Reference Example 135 Step 1 (2.0 g), Pin₂B₂ (4.0 g), palladium acetate (293 mg), potassium phosphate (7.6 g) and 2-dicyclohexylphosphino-2'-(N,N-dimethylamino)biphenyl (hereinafter referred to as Davephos) (950 mg) was added toluene (60 mL), and the interior of a vessel was purged with argon. The reaction mixture was stirred at 100 °C for a day. The reaction mixture was filtered through Celite, and the filtrate was concentrated under reduced pressure. The resulting residue was purified by column chromatography to give 1.75 g of the title compound as a white powder.

### Reference Example 136 4-(5-romo-4-methylpyridin-2-yl)thiomorpholine 1,1-dioxide

To a mixture of 2,5-dibromo-4-methylpyridine (251 mg), thiomorpholine 1,1-dioxide (112 mg), Xantphos (96 mg), NaOtBu (240 mg) and Pd₂(dba)₃ (76 mg) was added 1,4-dioxane (5.0 mL), and the interior of a vessel was purged with argon. The reaction mixture was stirred at 60 °C for an hour, and the reaction mixture was filtered through Celite, and the filtrate was concentrated under reduced pressure. The resulting residue was purified by column chromatography to give 265 mg of the title compound as a pale yellow solid.

### Reference Example 137 1-(trans-4-{[tert-butyl(dimethyl)silyl]oxy}cyclohexyl)-6-(4,4,5,5-tetramethyl-1,3,2-dioxaborolan-2-yl)-1,3-dihydro-2H-benzimidazol-2-one

### Step 1 Production of trans-4-[(5-Bromo-2-nitrophenyl)amino]cyclohexanol

The title compound was prepared as a yellow powder (3.8 g) according to the aforementioned procedure described in Reference Example 111 Step 1 using trans-4-aminocyclohexanol (2.4 g) instead of 3-amino-1-propanol.

### Step 2 Production of 6-bromo-1-(trans-4-{[tert-butyl(dimethyl)silyl]oxy}cyclohexyl)-1,3-dihydro-2H-benzimidazol-2-one

To a suspension of trans-4-[(5-bromo-2-nitrophenyl)amino]cyclohexanol obtained in Reference Example 137 Step 1 (500 mg) in ethanol (10 mL) was added CF 105 R/W (100 mg), and the mixture was stirred at room temperature for 3 hours under 3 atm hydrogen atmosphere. The reaction mixture was filtered through Celite, and the filtrate was concentrated under reduced pressure. The resulting residue was dissolved in DMF (10 mL), followed by the addition of urea (286 mg), and the mixture was stirred stirred at 120 °C overnight. Water was added to the reaction mixture, and extracted with ethyl acetate. The organic layer was washed with saturated brine, dried over magnesium sulfate, and the solvent was evaporated under reduced pressure. The resulting residue was dissolved in DMF (10 mL) and then imidazole (325 mg) and TBSCl (479 mg) was added to the solution. The mixture was stirred at room temperature overrnight. To the reaction mixture was added water and the mixture was extracted with ethyl acetate. The organic layer was washed with saturated brine, dried over magnesium sulfate, and then the solvent was evaporated under reduced pressure. The resulting residue was purified by column chromatography to give 168 mg of the title compound as a brown solid.

### Step 3 Production of 1-(trans-4-{[tert-butyl(dimethyl)silyl]oxy}cyclohexyl)-6-(4,4,5,5-tetramethyl-1,3,2-dioxaborolan-2-yl)-1,3-dihydro-2H-benzimidazol-2-one

The title compound was prepared as a pale yellow solid (121 mg) according to the aforementioned procedure described in Reference Example 105 Step 3 using 6-bromo-1-(trans-4-{[tert-butyl(dimethyl)silyl]oxy}cyclohexyl)-1,3-dihydro-2H-benzimidazol-2-one (180 mg) obtained in Reference Example 137 Step 2 instead of 1-(5-bromo-1H-benzimidazol-1-yl)-2-methylpropan-2-ol.

### Reference Example 138 4-[7-(4,4,5,5-tetramethyl-1,3,2-dioxaborolan-2-yl)-isoquinolin-1-yl]piperazin-2-on

### Step 1 Production of 4-(7-bromoisoquinolin-1-yl)piperazin-2-on

7-Bromo-1-chloroisoquinoline (622 mg) and piperazin-2-on (513 mg) were dissolved in 2-ethoxyethanol (10 mL), then N,N-diisopropylethylamine (893µL) was added, and the mixture was stirred at 120 °C for 1 day. The reaction mixture was diluted with water, extracted with ethyl acetate, and the solvent was evaporated under reduced pressure.The resulting residue was purified by column chromatography to give 222 mg of the title compound as a yellow solid.

### Step 2 Production of 4-[7-(4,4,5,5-tetramethyl-1,3,2-dioxaborolan-2-yl)-isoquinolin-1-yl]piperazin-2-on

To a mixture of 4-(7-bromoisoquinolin-1-yl)piperazin-2-on in Reference Example 138 Step 1 (222 mg), Pin₂B₂ (276 mg), potassium acetate (213 mg) and PdCl₂(dppf)-CH₂Cl₂ (59 mg) was added 1,4-dioxane (7 mL), and the interior of a vessel was purged with argon. The reaction mixture was stirred at 80 °C overnight, and then filtered through Celite, and the filtrate was concentrated under reduced pressure. The resulting residue was purified by column chromatography to give 175 mg of the title compound as a brown solid.

### Reference Example 139 1-(trans-4-{[tert-butyl(dimethyl)silyl]oxy}cyclohexyl)-2-methyl-6-(4,4,5,5-tetramethyl-1,3,2-dioxaborolan-2-yl)-1H-benzimidazole

### Step 1 Production of trans-4-[(2-amino-5-bromophenyl)amino]cyclohexanol

To a suspension of trans-4-[(5-bromo-2-nitrophenyl)amino]cyclohexanol obtained in Reference Example 137 Step 1 (600 mg) in ethanol (12 mL) was added CF 105 R/W (150 mg), and the mixture was stirred at room temperature for 4 hours under 3 atm hydrogen atmosphere. The reaction mixture was filtered through Celite, and the filtrate was concentrated under reduced pressure to give 599 mg of the title compound as a black amorphous form.

### Step 2 Production of trans-4-(6-bromo-2-methyl-1H-benzimidazol-1-yl)cyclohexanol

trans-4-[(2-Amino-5-bromophenyl)amino]cyclohexanol obtained in Reference Example 139 Step 1 (329 mg) was dissolved in minimum amount of methanol, and toluene (5.0 mL), trimethyl orthoacetate (208 mg) and p-toluenesulfonic acid monohydrate (33 mg) was successively added to the solution, and the mixture was was stirred at 80 °C for 2 hours. The solvent was evaporated under reduced pressure, and the resulting residue was purified by column chromatography to give 265 mg of the title compound as a pale brown amorphous form.

### Step 3 Production of 6-bromo-1-(trans-4-{[tert-butyl(dimethyl)silyl]oxy}cyclohexyl)-2-methyl-1H-benzimidazole

The title compound was prepared as a pale brown amorphous form (305 mg) according to the aforementioned procedure described in Reference Example 5 Step2, using trans-4-(6-bromo-2-methyl-1H-benzimidazol-1-yl)cyclohexanol obtained in Reference Example 139 step 2 (265 mg) instead of 2-[(5-bromopyridin-2-yl)amino]ethanol.

### Step 4 Production of 1-(trans-4-{[tert-butyl(dimethyl)silyl]oxy}cyclohexyl)-2-methyl-6-(4,4,5,5-tetramethyl-1,3,2-dioxaborolan-2-yl)-1H-benzimidazole

The title compound was prepared as a pale brown solid (76 mg) according to the aforementioned procedure described in Reference Example 105 Step 3 using 6-bromo-1-(trans-4-{[tert-butyl(dimethyl)silyl]oxy}cyclohexyl)-2-methyl-1H-benzimidazole obtained in Reference Example 139 Step 3 (90 mg) instead of 1-(5-bromo-1H-benzimidazol-1-yl)-2-methylpropan-2-ol.

### Reference Example 140 1-(trans-4-{[tert-butyl(dimethyl)silyl]oxy}cyclohexyl)-2-ethyl-6-(4,4,5,5-tetramethyl-1,3,2-dioxaborolan-2-yl)-1H-benzimidazole

### Step 1 Production of 5-bromo-N-(trans-4-{[tert-butyl(dimethyl)silyl]oxy}cyclohexyl)-2-nitroaniline

The title compound was prepared as a pale brown powder (3.71 g) according to the aforementioned procedure described in Reference Example 5 Step2 using trans-4-[(5-Bromo-2-nitrophenyl)amino]cyclohexanol obtained in Reference Example 137 step 1 (2.59 g) instead of 2-[(5-bromopyridin-2-yl)amino]ethanol.

### Step 2 Production of 4-bromo-N²-(trans-4-{[tert-butyl(dimethyl)silyl]oxy}cyclohexyl)benzene-1,2-diamine

The title compound was prepared as a brown oil (2.76 g) according to the aforementioned procedure described in Reference Example 139 Step 1 using 5-bromo-N-(trans-4-{[tert-butyl(dimethyl)silyl]oxy}cyclohexyl)-2-nitroaniline obtained in Reference Example 140 step 1 (3.53 g) instead of trans-4-[(5-bromo-2-nitrophenyl)amino]cyclohexanol.

### Step 3 Production of 6-bromo-1-(trans-4-{[tert-butyl(dimethyl)silyl]oxy}cyclohexyl)-2-ethyl-1H-benzimidazole

To a solution of 4-bromo-N²-(trans-4-{[tert-butyl(dimethyl)silyl]oxy}cyclohexyl)benzene-1,2-diamine obtained in Reference Example 140 Step 2 (250 mg) and N,N-diisopropylethylamine (162 mg) in dichloromethane (5 mL) was added propionyl chloride (64 mg) at 0 °C and the mixture was stirred at room temperature for 2 days. The reaction mixture was diluted with saturated aqueous ammonium chloride solution, extracted with ethyl acetate. The organic layer was washed with saturated brine, dried over magnesium sulfate, and the solvent was evaporated under reduced pressure. To the resulting residue was added actic acid (5 mL), p-toluenesulfonic acid monohydrate (24 mg), and the mixture was stirred at 95 °C for 4 hours. The solvent was vaporated under reduced pressure, and the resulting residue was purified by column chromatography to give 223 mg of the title compound as a pale brown powder.

### Step 4 Production of 1-(trans-4-{[tert-butyl(dimethyl)silyl]oxy}cyclohexyl)-2-ethyl-6-(4,4,5,5-tetramethyl-1,3,2-dioxaborolan-2-yl)-1H-benzimidazole

The title compound was prepared as a pale brown solid (249 mg) according to the aforementioned procedure described in Reference Example 105 Step 3 using 6-bromo-1-(trans-4-{[tert-butyl(dimethyl)silyl]oxy}cyclohexyl)-2-ethyl-1H-benzimidazole obtained in Reference Example 140 Step 3 (223 mg) instead of 1-(5-bromo-1H-benzimidazol-1-yl)-2-methylpropan-2-ol.

### Reference Example 141 1-(trans-4-{[tert-butyl(dimethyl)silyl]oxy}cyclohexyl)-2-(propan-2-yl)-6-(4,4,5,5-tetramethyl-1,3,2-dioxaborolan-2-yl)-1H-benzimidazole

The title compound was prepared as a pale yellow powder according to the aforementioned procedure described in Reference Example 140 Step 3 , 4 using isobutyryl chloride instead of propionyl chloride.

### Reference Example 142 [1-(trans-4-{[tert-butyl(dimethyl)silyl]oxy}cyclohexyl)-6-(4,4,5,5-tetramethyl -1,3,2-dioxaborolan-2-yl)-1H-benzimidazol-2-yl]methyl acetate

The title compound was prepared as a pale brown powder according to the aforementioned procedure described in Reference Example 140 Step 3 , using acetoxyacetyl chloride instead of propionyl chloride.

### Reference Example 143 tert-butyl 4-[7-(4,4,5,5-tetramethyl-1,3,2-dioxaborolan-2-yl)-isoquinolin-1-yl]piperazine-1-carboxylate

### Step 1 Production of tert- butyl 4-(7-bromoisoquinolin-1-yl)piperazin-1-carboxylate

A suspension of 7-bromo-1-chloroisoquinoline (194 mg), tert-butyl piperazine-1-carboxylate (298 mg), potassium carbonate (220 mg) in DMSO (4 mL) was stirred at 110 °C overnight. The reaction mixture was diluted with water, and extracted with ethyl acetate. The solvent was evaporated under reduced pressure and the resulting residue was purified by column chromatography to give 277 mg of the title compound as a yellow oil.

### Step 2 Production of tert- butyl 4-[7-(4,4,5,5-tetramethyl-1,3,2-dioxaborolan-2-yl)-isoquinolin-1-yl]piperazine-1-carboxylate

To a mixture of tert- butyl 4-(7-bromoisoquinolin-1-yl)piperazine-1-carboxylate in Reference Example 143 Step 1 (277 mg), Pin₂B₂ (269 mg), potassium acetate (208 mg) and PdCl₂(dppf)·CH₂Cl₂ (58 mg) was added 1,4-dioxane (5 mL), and the interior of a vessel was purged with argon. The reaction mixture was stirred at 80 °C for 2 hours, and then filtered through Celite, and the filtrate was concentrated under reduced pressure. The resulting residue was purified by column chromatography to give 280 mg of the title compound as a brown oil.

### Reference Example 144 1-(4-{[tert-butyl(dimethyl)silyl]oxy}piperidin-1-yl)-7-(4,4,5,5-tetramethyl-1,3,2-dioxaborolan-2-yl) isoquinoline

### Step 1 Production of 1-(7-bromoisoquinolin-1-yl)piperidin-4-ol

The title compound was prepared as a yellow oil (28 mg) according to the aforementioned procedure described in Reference Example 143 Step 1 using piperidin-4-ol (13 mg) instead of tert-butyl piperazine-1-carboxylate.

### Step 2 Production of 7-bromo-1-(4-{[tert-butyl(dimethyl)silyl]oxy}piperidin-1-yl)isoquinoline

The title compound was prepared as a yellow oil (31 mg) according to the aforementioned procedure described in Reference Example 5 Step2, using 1-(7-bromoisoquinolin-1-yl)piperidin-4-ol obtained in Reference Example 144 step 1 (28 mg) instead of 2-[(5-bromopyridin-2-yl)amino]ethanol.

### Step 3 Production of 1-(4-{[tert-butyl(dimethyl)silyl]oxy}piperidin-1-yl)-7-(4,4,5,5-tetramethyl-1,3,2-dioxaborolan-2-yl) isoquinoline

The title compound was prepared as a yellow oil (28 mg) according to the aforementioned procedure described in Reference Example 143 Step 2 using 7-bromo-1-(4-{[tert-butyl(dimethyl)silyl]oxy}piperidin-1-yl)isoquinoline (31 mg) obtained in Reference Example 144 Step 2 instead of tert-butyl 4-(7-bromoisoquinolin-1-yl)piperazine-1-carboxylate.

### Reference Example 145 1-[(3R)-3-{[tert-butyl(dimethyl)silyl]oxy}pyrrolidin-1-yl]-7-(4,4,5,5-tetramethyl-1,3,2-dioxaborolan-2-yl)isoquinoline

The title compound was prepared as a yellow oil (114 mg) according to the aforementioned procedure described in Reference Example 144 using (R)-pyrrolidin-3-ol (70 mg) instead of piperidin-4-ol.

### Reference Example 146 1-(3-{[tert-butyl(dimethyl)silyl]oxy}azetidin-1-yl)-7-(4,4,5,5-tetramethyl-1,3,2-dioxaborolan-2-yl) isoquinoline

The title compound was prepared as a pale yellow powder (22 mg) according to the aforementioned procedure described in Reference Example 144 using azetidin-3-ol hydrochloride (88 mg) instead of piperidin-4-ol.

### Reference Example 147 1-[-(2S)-2-({[tert-butyl(dimethyl)silyl]oxy}methyl)pyrrolidin-1-yl]-7(4,4,5,5-tetramethyl-1,3,2-dioxaborolan-2-yl)isoquinoline

The title compound was prepared as a pale yellow oil (163 mg) according to the aforementioned procedure described in Reference Example 144 using (S)-pyrrolidin-2-yl methanol (81 mg) instead of piperidin-4-ol.

### Reference Example 148 1-[(3R)-3-fluoropyrrolidin-1-yl]-7(4,4,5,5-tetramethyl-1,3,2-dioxaborolan-2-yl)isoquinoline

The title compound was prepared as a pale yellow oil (94 mg) according to the aforementioned procedure described in Reference Example 143 using (R)-3-fluoropyrrolidine hydrochloride (100 mg) instead of tert-butyl piperazine-1-carboxylate.

### Reference Example 149 tert-butyl {(3R)-1-[7-(4,4,5,5-tetramethyl-1,3,2-dioxaborolan-2-yl)isoquinolin-1-yl]pyrrolidin-3-yl}carbamate

The title compound was prepared as a brown oil (38 mg) according to the aforementioned procedure described in Reference Example 143 using (R)-tert-butyl pyrrolidine carbamate (149 mg) instead of tert-butyl piperazine-1-carboxylate.

### Reference Example 150 1-[7-(4,4,5,5-tetramethyl-1,3,2-dioxaborolan-2-yl)-isoquinolin-1-yl]piperidine-4-carbonitrile

The title compound was prepared as a pale yellow oil (120 mg) according to the aforementioned procedure described in Reference Example 143 using piperidine-4-carbonitrile hydrochloride (117 mg) instead of tert-butyl piperazine-1-carboxylate.

### Reference Example 151 1-[7-(4,4,5,5-tetramethyl-1,3,2-dioxaborolan-2-yl)isoquinolin-1-yl]imidazolidin-2-on

### Step 1 Production of N-(7-bromoisoquinoline-1-yl)ethane-1,2-diamine

The title compound was prepared as a pale yellow oil (197 mg) according to the aforementioned procedure described in Reference Example 143 Step 1 using ethylenediamine (132 mg) instead of tert-butyl piperazine-1-carboxylate.

### Step 2 Production of 1-(7-bromoisoquinolin-1-yl)imidazolidin-2-on

A solution of N-(7-bromoisoquinoline-1-yl)ethane-1,2-diamine obtained in Reference Example 151 Step 1 (110 mg) and 1,1'-carbonyldiimidazole (100 mg) in dichloroethane (2 mL) was stirred at 80 °C overnight. The reaction mixture was diluted with water, and extracted with ethyl acetate. The solvent was evaporated under reduced pressure and the resulting residue was purified by column chromatography to give 66 mg of the title compound as a white powder.

### Step 3 Production of 1-[7-(4,4,5,5-tetramethyl-1,3,2-dioxaborolan-2-yl)-isoquinolin-1-yl]imidazolidin-2-on

The title compound was prepared as a pale white powder (54 mg) according to the aforementioned procedure described in Reference Example 143 Step 2 using 1-(7-bromoisoquinolin-1-yl)imidazolidin-2-on obtained in Reference Example 151 Step 2 (66 mg) instead of tert-butyl 4-(7-bromoisoquinolin-1-yl)piperazine-1-carboxylate.

### Reference Example 152 tert-butyl {trans-4-[6-(4,4,5,5-tetramethyl-1,3,2-dioxaborolan-2-yl)-1H-benzimidazol-1-yl]cyclohexyl}carbamate

### Step 1 Production of tert-butyl [trans-4-(6-bromo-1H-benzimidazol-1-yl)cyclohexyl]carbamate

The title compound was prepared as a brown powder (247 mg) according to the aforementioned procedure described in Reference Example 111 Step 1, 2 using tert-butyl (trans-4-aminocyclohexyl)carbamate (731 mg) instead of 3-amino-1-propanol.

### Step 2 Production of tert-buty {trans-4-[6-(4,4,5,5-tetramethyl-1,3,2-dioxaborolan-2-yl)-1H-benzimidazol-1-yl]cyclohexyl}carbamate

The title compound was prepared as a pale brown amorphous form (73 mg) according to the aforementioned procedure described in Reference Example 105 Step 3 using tert-butyl [trans-4-(6-bromo-1H-benzimidazol-1-yl)cyclohexyl]carbamate obtained in Reference Example 152 Step 1 (100 mg) instead of 1-(5-bromo-1H-benzimidazol-1-yl)-2-methylpropan-2-ol.

### Reference Example 153 tert-butyl 4-[6-(4,4,5,5-tetramethyl-1,3,2-dioxaborolan-2-yl)-1H-benzimidazol-1-yl]piperidine-1-carboxylate

### Step 1 Production of tert-butyl 4-(6-bromo-1H-benzimidazol-1-yl)piperidine-1-carboxylate

The title compound was prepared as a brown powder (382 mg) according to the aforementioned procedure described in Reference Example 111 Step 1 and 2 using tert-butyl 4-aminopiperidine-1-carboxylate (409 mg) instead of 3-amino-1-propanol.

### Step 2 Production of tert-butyl 4-[6-(4,4,5,5-tetramethyl-1,3,2-dioxaborolan-2-yl)-1H-benzimidazol-1-yl]piperidine-1-carboxylate

The title compound was prepared as a brown oil (153 mg) according to the aforementioned procedure described in Reference Example 105 Step 3 using tert-butyl 4-(6-bromo-1H-benzimidazol-1-yl)piperidine-1-carboxylate obtained in Reference Example 153 Step 1 (150 mg) instead of 1-(5-bromo-1H-benzimidazol-1-yl)-2-methylpropan-2-ol.

### Reference Example 154 1-({6-[(4-methoxybenzyl)oxy]pyridin-3-yl}methyl)-6-(4,4,5,5-tetramethyl-1,3,2-dioxaborolan-2-yl)-1H-benzimidazole

### Step 1 Production of 6- [(4-methoxybenzyl)oxy]pyridine-3-carbonitrile

To a solution of 4-methoxybenzyl alcohol (698 mg) in DMF (10 mL) was added sodium hydride (217 mg) under ice water cooling, and the mixture was stirred at room temperature for 15 minutes. 2-chloro-5-cyanopyridine (500 mg) was added to the reaction mixture under ice water cooling, and the mixture was stirred at room temperature for 2 days. Water was added to the reaction mixture, and the mixture was extracted with ethyl acetate. The organic layer was washed with saturated brine, dried over magnesium sulfate, and the solvent was evaporated under reduced pressure. The resulting residue was purified by column chromatography to give 573 mg of the title compound as a white powder.

### Step 2 Production of 1-{6-[(4-methoxybenzyl)oxy]pyridin-3-yl}methanamine

To a suspension of 6- [(4-methoxybenzyl)oxy]pyridine-3-carbonitrile obtained in Reference Example 154 Step 1 (200 mg) and cobalt(II) chloride (216 mg) in methanol (5.0 mL) was added sodium borohydride (315 mg) under ice water cooling, and the mixture was stirred at room temperature for an hour. Water was added to the reaction mixture, and the mixture was extracted with ethyl acetate. The organic layer was washed with saturated brine, dried over magnesium sulfate, and the solvent was evaporated under reduced pressure to give 176 mg of the title compound as a gray oil.

### Step 3 Production of 6-bromo-1-({6-[(4-methoxybenzyl)oxy)pyridin-3-yl}methyl)-1H-benzimidazole

The title compound was prepared as a pale brown powder according to the aforementioned procedure described in Reference Example 111 Step 1 and 2 using 1-{6-[(4-methoxybenzyl) oxy]pyridin-3-yl}methanamine obtained in Reference Example 154 Step 2 instead of 3-amino-1-propanol.

### Step 4 Production of 1-({6-[(4-methoxybenzyl)oxy)pyridin-3-yl}methyl)-6-(4,4,5,5-tetramethyl-1,3,2-2-dioxaborolan-yl)-1H-benzimidazole

The title compound was prepared as a yellow amorphous form (65 mg) according to the aforementioned procedure described in Reference Example 105 Step 3 using 6-bromo-1-({6-[(4-methoxybenzyl)oxy)pyridine-3-yl}methyl)-1H-benzimidazole obtained in Reference Example 154 Step 3 (112 mg) instead of 1-(5-bromo-1H-benzimidazol-1-yl)-2-methylpropan-2-ol.

### Reference Example 155 2-(trans-4-{[tert-butyl(dimethyl)silyl]oxy}cyclohexyl)-6-(4,4,5,5-tetramethyl-1,3,2-dioxaborolan-2-yl)-1H-benzimidazole

### Step 1 Production of trans-4 - (acetoxy)cyclohexanecarboxylic acid

To a solution of trans-4-hydroxycyclohexanecarboxylic acid (300 mg) and triethyl amine (263 mg) in THF (5 mL) was added acetyl chloride (196 mg) under ice water cooling, and the mixture was stirred stirred at room temperature overnight. to the reaction mixture was added concentrated hydrochloric acid (0.25 mL), diluted with water, and extracted with diethyl ether. The organic layer was washed with saturated brine, dried over magnesium sulfate. The solvent was evaporated under reduced pressure to give 333 mg of the title compound as a white amorphous form.

### Step 2 Production of trans-4-[(4-bromo-2-nitrophenyl)carbamoyl]cyclohexyl acetate

trans-4 -(Acetoxy)cyclohexanecarboxylic acid obtained in Reference Example 155 Step 1 (64 mg) was dissolved in dichlorometane (4 mL), and oxalyl chloride (87 mg) and DMF (one drop) was successively added to the mixture, and the mixture was stirred at room temperature for an hour The solvent was evaporated under reduced pressure, and the resulting residue was dissolved in dichloromethane (2 mL), 4-bromo-2-nitroaniline (75 mg) and N,N-diisopropylethylamine (222 mg) was added to the solution, and the mixture was stirrrred at room temperature for 2 hours. The solvent was evaporated under reduced pressure and the resulting residue was purified by column chromatography to give 47 mg of the title compound as a pale yellow powder.

### Step 3 Production of trans-4-(6-bromo-1H-benzimidazol-2-yl)cyclohexyl acetate

To a suspension of trans-4-[(4-bromo-2-nitrophenyl)carbamoyl]cyclohexyl acetate obtained in Reference Example 155 Step 2 (47 mg) in ethanol (5 mL) was added CF 105 R/W (20 mg), and the mixture was stirred at room temperature for 4 hours under 3 atm hydrogen atmosphere. The reaction mixture was filtered through Celite, and the filtrate was concentrated under reduced pressure. To the resultingresulting residue was added acetic acid (1.5 mL), and the mixture was heated under reflux for an hour. The solvent was evaporated under reduced pressure and the resulting residue was purified by column chromatography to give 33 mg of the title compound as a pale yellow powder.

### Step 4 Production of trans-4-(6-bromo-1H-benzimidazol-2-yl)cyclohexanol

To a solution of trans-4-(6-bromo-1H-benzimidazol-2-yl)cyclohexyl acetate obtained in Reference Example 155 Step 3 (33 mg) in methanol-water (10 : 1, 2.2 mL) was added potassium carbonate (54 mg), and the mixture was stirred at room temperature for 1.5 hours. The reaction mixture was diluted with water, and extracted with ethyl acetate. The organic layer was washed with saturated brine, dried over magnesium sulfate. The solvent was evaporated under reduced pressure and the resulting residue was purified by column chromatography to give 28 mg of the title compound as a yellow powder.

### Step 5 Production of 6-bromo-2-(trans-4-{[tert-butyl(dimethyl)silyl]oxy}cyclohexyl)-1H-benzimidazole

The title compound was prepared as a pale yellow oil (64 mg) according to the aforementioned procedure described in Reference Example 5 Step2 using trans-4-(6-bromo-1H-benzimidazol-2-yl)cyclohexanol obtained in Reference Example 155 step 4 (36 mg) instead of 2-[(5-bromopyridin-2-yl)amino]ethanol.

### Step 6 Production of 2-(trans-4-{[tert-butyl(dimethyl)silyl]oxy}cyclohexyl)-6-(4,4,5,5-tetramethyl-1,3,2-dioxaborolan-2-yl)-1H-benzimidazole

The title compound was prepared as a yellow amorphous form (14 mg) according to the aforementioned procedure described in Reference Example 105 Step 3 using 6-bromo-2-(trans-4-{[tert-butyl(dimethyl)silyl]oxy}cyclohexyl)-1H-benzimidazole obtained in Reference Example 155 Step 5 (64 mg) instead of 1-(5-bromo-1H-benzimidazol-1-yl)-2-methylpropan-2-ol.

### Reference Example 156 trans-4-[6-(4,4,5,5-tetramethyl-1,3,2-dioxaborolan-2-yl)-1H-benzimidazol-1-yl]cyclohexanecarbonitrile

### Step 1 Production of methyl trans-4-(6-bromo-1H-benzimidazol-1-yl)cyclohexanecarboxylate

The title compound was prepared as a yellow powder (568 mg) according to the aforementioned procedure described in Reference Example 111 Step 1 and 2 using trans methyl 4-aminocyclohexanecarboxylate (440 mg) instead of 3-amino-1-propanol.

### Step 2 Production of trans-4-(6-bromo-1H-benzimidazol-1-yl)cyclohexanecarboxamide

To a solution of methyl trans-4-(6-bromo-1H-benzimidazol-1-yl)cyclohexanecarboxylate obtained in Reference Example 156 Step 1 (203 mg) in methanol (2.0 mL) was added 2N aqueous sodium hydroxide solution (2 mL), and the mixture was stirred at room temperature for an hour. The solvent was evaporated under reduced pressure and 1N hydrochloric acid was added to the residue, the precipitated solid was filtered. To a suspension of the resulting solid described above and HOBt (135 mg) in dichloromethane (6 mL) was successively added triethyl amine (742 µL), ammonium chloride (178 mg) and WSCD·HCl (191 mg), and the mixture was stirred at room temperature for 6 hours. The solvent was evaporated under reduced pressure and the resulting residue was purified by column chromatography to give 237 mg of the title compound as a yellow oil.

### Step 3 Production of trans-4-(6-bromo-1H-benzimidazol-1-yl)cyclohexanecarbonitrile

To a solution of trans-4-(6-bromo-1H-benzimidazol-1-yl)cyclohexanecarboxamide obtained Reference Example 156 Step 2 (237 mg) and pyridine (118 µL) in 1,4-dioxane (7 mL) was added trifluoroacetic anhydride (123µL), and the mixture was stirred at room temperature for 5 hours. The solvent was evaporated under reduced pressure and the resulting residue was purified by column chromatography to give 207 mg of the title compound as a yellow oil.

### Step 4 Production of trans-4-[6-(4,4,5,5-tetramethyl-1,3,2-dioxaborolan-2-yl)-1H-benzimidazol-1 - yl]cyclohexanecarbonitrile

The title compound was prepared as a yellow oil (154 mg) according to the aforementioned procedure described in Reference Example 105 Step 3 using trans-4-(6-bromo-1H-benzimidazol-1-yl)cyclohexanecarbonitrile obtained in Reference Example 156 Step 3 (207 mg) instead of 1-(5-bromo-1H-benzimidazol-1-yl)-2-methylpropan-2-ol.

### Reference Example 157 4-[3-fluoro-5-(4,4,5,5-tetramethyl-1,3,2-dioxaborolan-2-yl)pyridin-2-yl]thiomorpholine 1,1-dioxide

### Step 1 Production of 4-(5-bromo-3-fluoropyridin-2-yl)thiomorpholine 1,1-dioxide

To a solution of 4-(5-bromopyridin-2-yl)thiomorpholine-1,1-dioxide obtained in Reference Example 6 (233 mg) in DMF (5 mL) was added Selectfluor (registered trademark, 566 mg), and the mixture was at 80 °C overnight. The reaction mixture was diluted with water, and extracted with ethyl acetate. The solvent was evaporated under reduced pressure and the resulting residue was purified by column chromatography to give 113 mg of the title compound as a yellow oil.

### Step 2 Production of 4-[3-fluoro-5-(4,4,5,5-tetramethyl-1,3,2-dioxaborolan-2-yl)pyridin-2-yl]thiomorpholine 1,1-dioxide

The title compound was prepared as a white powder (125 mg) according to the aforementioned procedure described in Reference Example 105 Step 3 using 4-(5-bromo-3-fluoropyridin-2-yl)thiomorpholine 1,1-dioxide obtained in Reference Example 157 Step 1 (113 mg) instead of 1-(5-bromo-1H-benzimidazol-1-yl)-2-methylpropan-2-ol.

### Example 1 2-{[4-cyclopropyl-6'-(morpholin-4-yl)-2,3'-bipyridin-6-yl]amino}pyridine-4-carbonitrile hydrochloride

### Step 1 Production of 6-chloro-4-cyclopropyl-6'-(morpholin-4-yl)-2,3'-bipyridine

To a mixture of 2,6-dichloro-4-cyclopropylpyridine (60 mg), 4-[5-(4,4,5,5-tetramethyl-1,3,2-dioxaborolan-2-yl)-pyridin-2-yl]morpholine (102 mg) and sodium carbonate (102 mg) were added 1,4-dioxane (6 mL) and water (3 mL) and the interior of a vessel was purged with argon. PdCl₂(dppf)·CH₂Cl₂ (163 mg) was added to the mixture, and the reaction mixture was stirred at 100 °C for 1.5 hours. The reaction mixture was diluted with ethyl acetate, followed by carring out an operation of extraction. The organic layer was successively washed with water and saturated brine, evaporated under reduced pressure. The resulting residue was purified by column chromatography to give 53 mg of the title compound as a white solid.

### Step 2 Production of 2-{[4-cyclopropyl-6'-(morpholin-4-yl)-2,3'-bipyridin-6-yl]amino}pyridine-4-carbonitrile hydrochloride

To a mixture of 6-chloro-4-cyclopropyl-6'-(morpholin-4-yl)-2,3'-bipyridine obtained in Example 1 Step 1 (53 mg), 2-amino-4-cyanopyridine (20 mg), 2-dicyclohexylphosphino-2',4',6'-triisopropylbiphenyl (hereinafter referred to as X-Phos) (96 mg), NaOtBu (25 mg) and Pd₂(dba)₃·CHCl₃ (53 mg) was added 1,4-dioxane (3 mL), and the interior of a vessel was purged with argon. The reaction mixture was stirred at 100 °C for 0.5 hour, and the solvent was evaporated under reduced pressure. The resulting residue was purified by column chromatography to give 55 mg of a brown solid. To a suspension of the obtained solid in methanol (1 mL) was added 4N hydrogen chloride-dioxane solution (35 µL), and the mixture was stirred at room temperature for 15 minutes. The solvent was evaporated under reduced pressure, and the obtained solid was washed with acetone : ethyl acetate (1 : 1) to give 44 mg of the title compound as a yellow solid.
MS 399 (M + 1)

### Example 2 2-({4-cyclopropyl-6'-[(2-hydroxyethyl)amino]-2,3'-bipyridin-6-yl}amino)pyridine-4-carbonitrile hydrochloride

### Step 1 Production of N-(2-{[tert-butyl(dimethyl)silyl]oxy}ethyl)-6-chloro-4-cyclopropyl-2,3'-bipyridine-6'-amine

To a mixture of 5-bromo-N-(2-{[tert-butyl(dimethyl)silyl]oxy}ethyl)pyridine-2-amine obtained in reference Example 5 (718 mg), Pin₂B₂ (606 mg), X-Phos (207 mg), potassium acetate (640 mg) and Pd₂(dba)₃·CHCl₃ (113 mg) was added 1,4-dioxane (20 mL), and the interior of a vessel was purged with argon. The reaction mixture was stirred at 100 °C for 2 hours. The reaction mixture was filtered through Celite, and the filtrate was concentrated under reduced pressure. To the resulting residue was successively added 2,6-dichloro-4-cyclopropylpyridine obtained in Reference Example 2 (360 mg), sodium carbonate (610 mg), 1,4-dioxane (15 mL), water (6 mL) and PdCl₂(dppf)·CH₂Cl₂ (80 mg), and the interior of a vessel was purged with argon. The reaction mixture was stirred at 100 °C for 2 hours. The reaction mixture was diluted with ethyl acetate, followed by carring out an operation of extraction. The organic layer was successively washed with water and saturated brine, evaporated under reduced pressure. The resulting residue was purified by column chromatography to give 254 mg of the title compound as a brown oil.

### Step 2 Production of 2-[(6-chloro-4-cyclopropyl-2,3'-bipyridin-6'-yl)amino]ethanol

To N-(2-{[tert-butyl(dimethyl)silyl]oxy}ethyl)-6-chloro-4-cyclopropyl-2,3'-bipyridine-6'-amine obtained in Example 2 Step 1 (254 mg) was added 2N hydrogen chloride-ethanol solution (2 mL), and the mixture was stirred at room temperature for an hour. The reaction mixture was neutralized with aqueous sodium hydroxide solution, and extracted with ethyl acetate. The organic layer was washed with saturated brine and evaporated under reduced pressure and the resulting residue was purified by column chromatography to give 105 mg of the title compound as a yellow oil.

### Step 3 Production of 2-({4-cyclopropyl-6'-[(2-hydroxyethyl)amino]-2,3'-bipyridin-6-yl}amino)pyridine-4-carbonitrile hydrochloride

To a mixture of 2-[(6-chloro-4-cyclopropyl-2,3'-bipyridin-6'-yl)amino]ethanol obtained in Reference Example 2 Step 2 (48 mg), 2-amino-4-cyanopyridine (20 mg), X-Phos (95 mg), NaOtBu (24 mg) and Pd₂(dba)₃·CHCl₃ (52 mg) was added 1,4-dioxane (5 mL), and the interior of a vessel was purged with argon.The reaction mixture was stirred at 100 °C for 15 minutes and the solvent was evaporated under reduced pressure. The resulting residue was purified by column chromatography to give 33 mg of the title compound as a brown powder. To a suspension of the obtained solid in methanol (1 mL) was added 2N hydrogen chloride-ethanol solution (44 µL), and the mixture was stirred at room temperature for 10 minutes. The solvent was evaporated under reduced pressure, and the obtained solid was washed with acetone to give 27 mg of the title compound as a yellow solid.
MS 373 (M + 1)

### Example 3 2-{[4-cyclopropyl-6'-(1,1-dioxidethiomorpholin-4-yl)-2,3'-bipyridin-6-yl]amino}pyridine-4-carbonitrile hydrochloride

To a mixture of 4-(5-bromopyridin-2-yl)thiomorpholine 1,1-dioxide obtained in Reference Example 6 (153 mg), Pin₂B₂ (147 mg), X-Phos (52 mg), potassium acetate (155 mg) and Pd₂(dba)₃·CHCl₃ (27 mg) was added 1,4-dioxane (12 mL), and the interior of a vessel was purged with argon. The reaction mixture was stirred at 100 °C for an hour. The reaction mixture was filtered through Celite, and the filtrate was concentrated under reduced pressure. To the resultingresulting residue was successively added 2-[(6-chloro-4-cyclopropylpyridin-2-yl)amino]pyridine-4-carbonitrile obtained in Reference Example 3 (95 mg), 2-dicyclohexylphosphino-2',6'-dimethoxybiphenyl (hereinafter referred to as S-Phos)(62 mg) potassium phosphate (223 mg), 1,4-dioxane (6 mL), water (3 mL), palladium acetate (16 mg) and the interior of a vessel was purged with argon. The reaction mixture was stirred at 100 °C for an hour. The reaction mixture was diluted with ethyl acetate and water, then extracted twice with ethyl acetate. The organic layer was washed with saturated brine, dried over magnesium sulfate, and evaporated under reduced pressure. The resulting residue was purified by column chromatography to give 141 mg of the title compound as a pale yellow solid. To a suspension of the obtained solid in methanol (5 mL) was added 4N hydrogen chloride-dioxane solution (87 µL), and the mixture was stirred at room temperature for 10 minutes. The solvent was evaporated under reduced pressure, and the obtained solid was washed with ethyl acetate to give 129 mg of the title compound as a yellow solid.
MS 447 (M + 1)
Elementary analysis as C₂₃H₂₂N₆O₂S·HCl + 2H₂O
Calcd. (%) C: 53.22.; H: 5.24.; N: 16.19
Found. (%) C: 53.56.; 5.55.; N: 16.00

### Example 4 2-{[4-cyclopropyl-6'-(4-oxopiperidin-1-yl)-2,3'-bipyridin-6-yl]amino}pyridine-4-carbonitrile hydrochloride

To a mixture of 1-(5-bromopyridin-2-yl)piperidin-4-on obtained in Reference Example 7 (128 mg), Pin₂B₂ (135 mg), X-Phos (48 mg), potassium acetate (150 mg) and Pd₂(dba)₃·CHCl₃ (26 mg) was added 1,4-dioxane (5 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (100 mg), S-Phos (61 mg), potassium phosphate (236 mg), 1,4-dioxane (6 mL), water (2 mL), Palladium(II) acetate (17 mg), and the interior of a vessel was purged with argon. The reaction mixture was stirred at 100 °C for 30 minutes. Thesolvent was wvaporated under reduced pressure. The resulting residue was purified by column chromatography to give 130 mg of the title compound as a pale yellow solid. To a suspension of the obtained solid in methanol (3 mL) was added 2N hydrogen chloride-ethanol solution (158 µL), and the mixture was stirred at room temperature for 10 minutes. The solvent was evaporated under reduced pressure, and the obtained solid was washed with acetone to give 75 mg of the title compound as a yellow solid.
MS 411 (M + 1)

### Example 5 2-{[4-cyclopropyl-6'-(3-oxopiperazin-1-yl)-2,3'-bipyridin-6-yl]amino}pyridine-4-carbonitrile hydrochloride

### Step 1 Production of tert-butyl 4-{6-[(4-cyanopyridin-2-yl)amino]-4-cyclopropyl-2,3'-bipyridin-6'-yl}-2-oxopiperazine-1-carboxylate

To a mixture of tert-butyl 4-(5-bromopyridin-2-yl)-2-oxopiperazine-1-carboxylate obtained in Reference Example 8 (237 mg), Pin₂B₂ (178 mg), X-Phos (64 mg), potassium acetate (196 mg), Pd₂(dba)₃·CHCl₃ (35 mg) was added 1,4-dioxane (2 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (140 mg), S-Phos (85 mg), potassium phosphate (330 mg), 1,4-dioxane (2 mL), water (0.5 mL) and palladium(II) acetate (24 mg), and the interior of a vessel was purged with argon. The reaction mixture was stirred at 100 °C for 30 minutes, then the solvent was evaporated under reduced pressure. The resulting residue was purified by column chromatography to give 116 mg of the title compound as a yellow solid.

### Step 2 Production of 2-{[4-cyclopropyl-6'-(3-oxopiperazin-1-yl)-2,3'-bipyridin-6-yl]amino}pyridine-4-carbonitrile hydrochloride

tert-butyl 4-{6-[(4-cyanopyridin-2-yl)amino]-4-cyclopropyl-2,3'-bipyridine-6'-yl}-2-oxopiperazine-1-carboxylate was dissolved in TFA (1 mL), and the mixture was stirred at room temperature for an hour. The solvent was evaporated under reduced pressure and the resulting residue was purified by column chromatography to give 42 mg of the title compound as a pale yellow solid. To a suspension of the obtained solid in methanol (1 mL) was added 2N hydrogen chloride-ethanol solution (51 µL), and the mixture was stirred at room temperature for 15 minutes. The solvent was evaporated under reduced pressure, and the obtained solid was washed with acetone to give 40 mg of the title compound as a yellow solid.
MS 412 (M + 1)

### Example 6 2-{[4-cyclopropyl-6'-(3-hydroxyazetidin-1-yl)-2,3'-bipyridin-6-yl]amino}pyridine-4-carbonitrile hydrochloride

### Step 1 Production of 2-{[6'-(3-{[tert-butyl(dimethyl)silyl]oxy}azetidin-1-yl)-4-cyclopropyl-2,3'-bipyridin-6-yl]amino}pyridine-4-carbonitrile

To a mixture of 5-bromo-2-(3-{[tert-butyl(dimethyl)silyl]oxy}azetidin-1-yl)pyridine obtained in Reference example 9 (210 mg), Pin₂B₂ (163 mg), X-Phos (59 mg), potassium acetate (180 mg) and Pd₂(dba)₃·CHCl₃ (32 mg) was added 1,4-dioxane (2 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (100 mg), S-Phos (61 mg), potassium phosphate (236 mg), 1,4-dioxane (2 mL), water (0.5 mL) and palladium(II) acetate (17 mg), and the interior of a vessel was purged with argon. The reaction mixture was stirred at 100 °C for 30 minutes, then the solvent was evaporated under reduced pressure. The resulting residue was purified by column chromatography to give 180 mg of the title compound as a pale yellow solid.

### Step 2 Production of 2-{[4-cyclopropyl-6'-(3-hydroxyazetidin-1-yl)-2,3'-bipyridin-6-yl]amino}pyridine-4-carbonitrile hydrochloride

To a solution of 2-{[6'-(3-{[tert-butyl(dimethyl)silyl]oxy}azetidin-1-yl)-4-cyclopropyl-2,3'-bipyridin-6-yl]amino}pyridine-4-carbonitrile obtained in Example 6 Step 1 (180 mg) in THF (6 mL) was added tetrabutylammonium fluoride

(3 mL 1M solution in THF), and the mixture was stirred at 70 °C for an hour. The solvent was evaporated under reduced pressure and the resulting residue was purified by column chromatography to give 95 mg of the title compound as a pale yellow solid. To a suspension of the obtained solid in methanol (2 mL) was added 2N hydrogen chloride-ethanol solution (124 µL), and the mixture was stirred at room temperature for 10 minutes. The solvent was evaporated under reduced pressure, and the obtained solid was washed with acetone to give 90 mg of the title compound as a yellow solid.
MS 385 (M + 1)

### Example 7 2-(4-{6-[(4-cyanopyridin-2-yl)amino]-4-cyclopropyl-2,3'-bipyridin-6'-yl}piperazin-1-yl)acetamide hydrochloride

### Step 1 Production of 2-{[4-cyclopropyl-6'-(piperazin-1-yl)-2,3'-bipyridin-6-yl]amino}pyridine-4-carbonitrile hydrochloride

To a mixture of tert- butyl 4-(5-bromopyridin-2-yl)piperazine-1-carboxylate obtained in Reference Example 10 (800 mg), Pin₂B₂ (653 mg), X-Phos (224 mg), potassium acetate (688 mg) and Pd₂(dba)₃·CHCl₃ (124 mg) was added 1,4-dioxane (48 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (422 mg), S-Phos (256 mg), potassium phosphate (995 mg), 1,4-dioxane (24 mL), water (12 mL) and palladium(II) acetate (70 mg), and the interior of a vessel was purged with argon. The reaction mixture was stirred at 100 °C for an hour. The reaction mixture was diluted with water, extracted 3 times with ethyl acetate, and the combined organic layes werewashed with saturated brine, dried over magnesium sulfate, the solvent was evaporated under reduced pressure and the resulting residue was purified by column chromatography. To a suspension of the obtained solid in methanol (24 mL) was added 4N hydrogen chloride-dioxane (12 mL), and the mixture was stirred at room temperature for 1.5 hours. The solvent was evaporated under reduced pressure, and the obtained compound was washed with ethyl acetate to give 502 mg of the title compound as a yellow solid.

### Step 2 Production of tert-butyl (4-{6-[(4-cyanopyridin-2-yl)amino]-4-cyclopropyl-2,3'-bipyridin-6'-yl}piperazin-1-yl) acetate

To a suspension of 2-{[4-cyclopropyl-6'-(piperazin-1-yl)-2,3'-bipyridin-6-yl]amino}pyridine-4-carbonitrile hydrochloride obtained in Example 7 Step 1 (250 mg) in acetonitrile (1.5 mL) was added triethylamine (192 µL) and tert-butyl 2-bromoacetate (93 µL), and the mixture was stirred at room temperature overnight. Triethylamine (192 µ) and tert-butyl 2-bromoacetate (186 µL) were added to the reaction mixture again , and the mixture was stirred at 50 °C for 2 hours, and then heated under reflux for 30 minutes. Cloroform and water was added to the reaction mixture, followed by carrying out an operation of extraction . The aqueous layer was extrcted twice with chloroform. The combined organic layers were washed with saturated brine, dried over magnesium sulfate, and the solvent was evaporated under reduced pressure and the resulting residue was purified by column chromatography to give 212 mg of the title compound as a yellow solid.

### Step 3 Production of (4-{6-(4-cyanopyridin-2-yl)amino]-4-cyclopropyl-2,3'-bipyridin-6'-yl}piperazin-1-yl) acetic acid hydrochloride

To tert-butyl (4-{6-[(4-cyanopyridin-2-yl)amino]-4-cyclopropyl-2,3'-bipyridin-6'-yl}piperazin-1-yl) acetate were successively added 1,4-dioxane (4 mL) and 4N hydrogen chloride-dioxane (2 mL), and the mixture was stirred at room temperature overnight. The precipitated solid was collected by filtration and washed with ethyl acetate to give 107 mg of the title compound as a yellow solid.

### Step 4 Production of 2-(4-{6-[(4-cyanopyridin-2-yl)amino]-4-cyclopropyl-2,3'-bipyridin-6'-yl}piperazin-1-yl)acetamide hydrochloride

To (4-{6-(4-cyanopyridin-2-yl)amino]-4-cyclopropyl-2,3'-bipyridin-6'-yl}piperazin-1-yl) acetic acid hydrochloride obtained in Example 7 Step 3 (35 mg) in DMF were successively added DNF (1mL), N,N-diisopropylethylamine (49 µL) and ammonium chlorride (8 mg), N-[(dimethylamino)(3H-[1,2,3]triazolo [4,5-b]pyridin-3-yloxy)methylidyne]-N- methylmethanaminium hexafluorophosphate (HATU) (53 mg), and the mixture was stirred at room temperature for 60 hours. The reaction mixture was diluted with ethyl acetate, and the precipitated solid was collected by filtration and washed with water and ethyl acetate to give 18 mg of the title compound as a pale yellow solid. To a suspension of the obtained solid in methanol (1 mL) was added 4N hydrogen chloride-dioxane (11 µL), and the mixture was stirred at room temperature for 10 minutes. The solvent was evaporated under reduced pressure, and the obtained solid was washed with ethyl acetate to give 18 mg of the title compound as a yellow solid.
MS 455 (M + 1)

### Example 8 2-({4-cyclopropyl-6'-[(4R)-4-hydroxy-2-oxopyrrolidin-1-yl]-2,3'-bipyridin-6-yl}amino)pyridine-4-carbonitrile hydrochloride

### Step 1 Production of 2-({6'-[(4R)-4-{[tert-butyl(dimethyl)silyl]oxy}-2-oxopyrrolidin-1-yl]-4-cyclopropyl-2,3'-bipyridin-6-yl}amino)pyridine-4-carbonitrile

To a mixture of (4R)-1-(5-bromopyridin-2-yl)-4-{[tert-butyl(dimethyl)silyl]oxy}pyrrolidin-2-on obtained in Reference Example 11 (223 mg) , Pin₂B₂ (160 mg), X-Phos (58 mg), potassium acetate (177 mg) and Pd₂(dba)₃·CHCl₃ (31 mg) was added 1,4-dioxane (2 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (100 mg), S-Phos (61 mg), potassium phosphate (236 mg), 1,4-dioxane (2 mL), water (0.5 mL) and palladium(II) acetate (17 mg), and the interior of a vessel was purged with argon. The reaction mixture was stirred at 100 °C for 30 minutes. The solvent was evaporated under reduced pressure, and the resulting residue was purified by column chromatography to give 230 mg of the title compound as a pale yellow solid.

### Step 2 Production of 2-({4-cyclopropyl-6'-[(4R)-4-hydroxy-2-oxopyrrolidin-1-yl]-2,3'-bipyridin-6-yl}amino)pyridine-4-carbonitrile hydrochloride

To a solution of 2-({6'-[(4R)-4-{[tert-butyl(dimethyl)silyl]oxy}-2-oxopyrrolidin-1-yl]-4-cyclopropyl-2,3'-bipyridin-6-yl}amino)pyridine-4-carbonitrile obtained in Example 8 Step1 (230 mg) in THF (2 mL) was added tetrabutylammonium fluoride (1.0 mL 1M solution in THF), and the mixture was stirred at 70 °C for an hour. The solvent was evaporated under reduced pressure and the resulting residue was purified by column chromatography to give 80 mg of the title compound as a pale yellow solid. To a suspension of the obtained solid in methanol (2 mL) was added 2N hydrogen chloride-ethanol solution (97 µL), and the mixture was stirred at room temperature for 10 minutes. The solvent was evaporated under reduced pressure, and the obtained solid was washed with acetone to give 50 mg of the title compound as a pale yellow solid.
MS 413 (M + 1)

### Example 9 2-{[6'-(4-acetyl-1,4-diazepan-1-yl)-4-cyclopropyl-2,3'-bipyridin-6-yl]amino}pyridine-4-carbonitrile hydrochloride

To a mixture of 1-[4-(5-bromopyridin-2-yl)-1,4-diazepan-1-yl]ethanone obtained in Reference Example 12 (197 mg) Pin₂B₂ (177 mg), X-Phos (63 mg), potassium acetate (195 mg) and Pd₂(dba)₃·CHCl₃ (35 mg) was added 1,4-dioxane (3 mL), and the interior of a vessel was purged with argon. The reaction mixture was stirred at 100 °C for an hour. The reaction mixture was filtered through Celite, and the filtrate was concentrated under reduced pressure. To the resultingresulting residue was successively added 2-[(6-chloro-4-cyclopropylpyridin-2-yl)amino]pyridine-4-carbonitrile obtained in Reference Example 3 (100 mg), S-Phos (61 mg), potassium phosphate (236 mg), 1,4-dioxane (3 mL), water (1 mL) and palladium(II) acetate (17 mg), and the interior of a vessel was purged with argon. The reaction mixture was stirred at 100 °C for 30 minutes. The solvent was evaporated under reduced pressure, and the resulting residue was purified by column chromatography to give 90 mg of the title compound as a pale yellow solid. To a suspension of the obtained solid in methanol (2 mL) was added 2N hydrogen chloride-ethanol (99 µL), and the mixture was stirred at room temperature for 10 minutes. The solvent was evaporated under reduced pressure, and the obtained solid was washed with ethyl acetate/ acetone (1 : 1) to give 85 mg of the title compound as a yellow solid.
MS 454 (M + 1)

### Example 10 4-{6-[(4-cyanopyridin-2-yl)amino]-4-cyclopropyl-2,3'-bipyridin-6'-yl}-1,4-diazepane-1-carboxamide hydrochloride

### Step 1 Production of tert-butyl 4-{6-[(4-cyanopyridin-2-yl)amino]-4-cyclopropyl-2,3'-bipyridin-6'-yl}-1,4-diazepane-1-carboxylate

To a mixture of tert-butyl-4-(5-bromopyridine-2-yl)-1,4-diazepane-1-carboxylate obtained in Reference Example 13 (294 mg), Pin₂B₂ (233 mg), X-Phos (78 mg), potassium acetate (243 mg) and Pd₂(dba)₃·CHCl₃ (43 mg) was added 1,4-dioxane (15 mL), and the interior of a vessel was purged with argon. The reaction mixture was stirred at 100 °C for 1.5 hours. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (150 mg), S-Phos (93 mg), potassium phosphate (354 mg), 1,4-dioxane (14 mL), water (7.0 mL), palladium(II) acetate (26 mg), and the interior of a vessel was purged with argon. The reaction mixture was stirred at 100 °C for 3 hours. Ethyl acetate and water was added to the mixture, followed by carrying out an operation of extraction . The aqueous layer was extracted with ethyl acetate twice. The combined organic layers were washed with saturated brine, dried over magnesium sulfate, and the solvent was evaporated under reduced pressure and the resulting residue was purified by column chromatography to give 238 mg of the title compound as a yellow amorphous form.

### Step 2 Production of 2-{[4-cyclopropyl-6'-(1,4-diazepan-1-yl)-2,3'-bipyridin-6-yl]amino}pyridine-4-carbonitrile

To a solution of tert-butyl 4-{6-[(4-cyanopyridin-2-yl)amino]-4-cyclopropyl-2,3'-bipyridin-6'-yl}-1,4-diazepane-1-carboxylate obtained in Example 10 Step 1 (235 mg) in dichloromethane (3 mL) was added TFA (2 mL) at 0 °C. After strring for 30 minutes at room temperature, the solvent was evaporated under reduced pressure. The resulting residue was diluted with water, and saturated aqueous sodium hydrogen carbonate solution was added to the reaction mixture, and pH of the aqueous layer was adjusted to 7-8. The aqueous layer was successively extracted with chloroform-methanol (10 : 1) and chloroform, the combined organic layers were dried over magnesium sulfate. The solvent was evaporated under reduced pressure to give 150 mg of the title compound as a pale yellow solid.

### Step 3 Production of 4-{6-[(4-cyanopyridin-2-yl)amino]-4-cyclopropyl-2,3'-bipyridin-6'-yl}-1,4-diazepane-1-carboxamide hydrochloride

To 2-{[4-cyclopropyl-6'-(1,4-diazepan-1-yl)-2,3'-bipyridin-6-yl]amino}pyridine-4-carbonitrile obtained in Example 10 Step 2 (148 mg) was successively added dichloromethane (2 mL), triethylamine (73 µL), trimethylsilyl isocyanate (73 µL) and the mixture was stirred at room temperature overnight. The reaction mixture was diluted with water, extracted three times with chloroform-methanol (10 : 1), dried over magnesium sulfate, and the solvent was evaporated under reduced pressure. The resulting residue was sprinkled over silaca gel, and purified by column chromatography to give 49 mg of the compound as a white solid. To a suspension of the obtained solid in methanol (5 mL) was added 4N hydrogen chloride-dioxane (30 µL), and the mixture was stirred at room temperature for 20 minutes. The solvent was evaporated under reduced pressure, and the obtained solid was washed with ethyl acetate to give 51 mg of the title compound as a pale yellow solid.
MS 455 (M + 1)

### Example 11 2-({4-cyclopropyl-6'-[(4S)-4-hydroxy-2-oxopyrrolidin-1-yl]-2,3'-bipyridin-6-yl}amino)pyridine-4-carbonitrile hydrochloride

### Step 1 Production of 2-({6'-[(4S)-4-([tert-butyl(dimethyl)silyl]oxy)-2-oxopyrrolidin-1-yl]-4-cyclopropyl-2,3'-bipyridin-6-yl}amino)pyridine-4-carbonitrile

To a mixture of (4S)-1-(5-bromopyridin-2-yl)-4-{[tert-butyl(dimethyl)silyl]oxy}pyrrolidin-2-on obtained in Reference Example 14 (240 mg), Pin₂B₂ (173 mg), X-Phos (62 mg), potassium acetate (191 mg) and Pd₂(dba)₃·CHCl₃ (34 mg) was added 1,4-dioxane (3 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (135 mg), S-Phos (83 mg), potassium phosphate (320 mg), 1,4-dioxane (3 mL), water (1 mL) and palladium(II) acetate (23 mg), and the interior of a vessel was purged with argon. The reaction mixture was stirred at 100 °C for 40 minutes. The reaction mixture was concentrated under reduced pressure.The resulting residue was purified by column chromatography to give 280 mg of the title compound as a brown solid.

### Step 2 Production of 2-({4-cyclopropyl-6'-[(4S)-4-hydroxy-2-oxopyrrolidin-1-yl]-2,3'-bipyridin-6-yl}amino)pyridine-4-carbonitrile hydrochloride

To a solution of 2-({6'-[(4S)-4-([tert-butyl(dimethyl)silyl]oxy)-2-oxopyrrolidin-1-yl]-4-cyclopropyl-2,3'-bipyridin-6-yl}amino)pyridine-4-carbonitrile obtained in Example 11 Step 1 (280 mg) in THF (2 mL) was added tetrabutylammonium fluoride (1 mL, 1M solution in THF), and the mixture was stirred at 70 °C for 30 minutes. The solvent was evaporated under reduced pressure and the resulting residue was purified by column chromatography to give 88 mg of the compound as a pale yellow solid. To a suspension of the obtained solid in methanol (2 mL) was added 2N hydrogen chloride-ethanol solution (107 µL), and the mixture was stirred at room temperature for 10 minutes. The solvent was evaporated under reduced pressure, and the obtained solid was washed with acetone to give 77 mg of the title compound as a pale yellow solid.
MS 413 (M + 1)

### Example 12 2-({4-cyclopropyl-6'-[(trans-4-hydroxycyclohexyl)amino]-2,3'-bipyridin-6-yl}amino)pyridine-4-carbonitrile hydrochloride

### Step 1 Production of 2-({6'-[(trans-4-{[tert-butyl(dimethyl)silyl]oxy}cyclohexyl)amino]-4-cyclopropyl-2,3' -bipyridin-6-yl}amino)pyridine-4-carbonitrile

To a mixture of 5-bromo-N-(trans-4-{[tert-butyl(dimethyl)silyl]oxy}cyclohexyl)pyridine-2-amine obtained in Reference Example 15 (250 mg), Pin₂B₂ (181 mg), X-Phos (62 mg), potassium acetate (191 mg) and Pd₂(dba)₃·CHCl₃ (34 mg) was added 1,4-dioxane (3 mL), and the interior of a vessel was purged with argon. The reaction mixture was stirred at 100 °C for an hour. The reaction mixture was filtered through Celite, and the filtrate was concentrated under reduced pressure. To the resulting residue was successively added 2-[(6-chloro-4-cyclopropylpyridin-2-yl)amino]pyridine-4-carbonitrile obtained in Reference Example 3 (135 mg), S-Phos (83 mg), potassium phosphate (320 mg), 1,4-dioxane (3 mL), water (1 mL), andpalladium(II) acetate (23 mg), and the interior of a vessel was purged with argon. The reaction mixture was stirred at 100 °C for 40 minutes. The reaction mixture was filtered through Celite, and the filtrate was concentrated under reduced pressure.The resulting residue was purified by column chromatography to give 250 mg of the title compound as a pale yellow amorphous form.

### Step 2 Production of 2-({4-cyclopropyl-6'-[(trans-4-hydroxycyclohexyl)amino]-2,3'-bipyridin-6-yl}amino)pyridine-4-carbonitrile hydrochloride

To a solution of 2-({6'-[(trans-4-{[tert-butyl(dimethyl)silyl]oxy}cyclohexyl)amino]-4-cyclopropyl-2,3' -bipyridin-6-yl}amino)pyridine-4-carbonitrile obtained in Reference Example 12 Step1 (250 mg) in THF (2 mL) was added tetrabutylammonium fluoride (1 mL 1M solution in THF), and the mixture was stirred at 70 °C for an hour. The solvent was evaporated under reduced pressure and the resulting residue was purified by column chromatography to give 107 mg of the compound as a brown amorphous form. To a suspension of the obtained solid in methanol (2 mL) was added 2N hydrogen chloride-ethanol solution (125 µL), and the mixture was stirred at room temperature for 15 minutes. The solvent was evaporated under reduced pressure, and the obtained solid was washed with acetone-ethyl acetate to give 88 mg of the title compound as a yellow solid.
MS 427 (M + 1)

### Example_ 13 2-({6-[1-(2-aminoethyl)-1H-benzimidazol-5-yl]-4-cyclopropylpyridin-2-yl}amino)pyridine-4-carbonitrile hydrochloride

### Step 1 Production of tert-butyl {2-[5-(6-chloro-4-cyclopropylpyridin-2-yl)-1H-benzimidazol-1-yl]ethyl}carbamate

To a mixture of tert-Butyl [2-(5-bromo-1H-benzimidazol-1-yl)ethyl]carbamate obtained in Reference Example 16 (600 mg), Pin₂-B₂ (580 mg), potassium acetate (870 mg) and PdCl₂(dppf)-CH₂Cl₂ (72 mg) was added 1,4-dioxane (4.0 mL), and the interior of a vessel was purged with argon, and the mixture was reacted under microwave irradiation (Biotage INITIATOR, at 160 °C for 20 minutes). The reaction mixture was diluted with ethyl acetate, filtered through Celite, and the filtrate was concentrated under reduced pressure. resultingThe resulting residue was dissolved in 1,4-dioxane-water (3 : 1, 9 mL) and was successively added with 2,6-dichloro-4-cyclopropylpyridine obtained in Reference Example 2 (330 mg), PdCl₂ (dppf)·CH₂Cl₂ (43 mg) and potassium carbonate (730 mg) and the reaction mixture was stirred at 100 °C for 2 hours. The reaction mixture was diluted with ethyl acetate and filtered through a small pad of NH silica gel and the filtrate was concentrated under reduced pressure. The resulting residue was purified by column chromatography to give 550 mg of the title compound as a yellow oil.

### Step 2 Production of tert-butyl [2-(5{6-[(4-cyanopyridin-2-yl)amino]-4-cyclopropylpyridin-2-yl}-1H-benzimidazol-1-yl)ethyl]carbamate

To a mixture of tert-butyl {2-[5-(6-chloro-4-cyclopropylpyridin-2-yl)-1H-benzimidazol-1-yl]ethyl}carbamate obtained in Example 13 Step1 (57 mg), 2-amino-4-cyanopyridine (18 mg), X-Phos (78 mg), NaOt-Bu (20 mg) and Pd₂(dba)₃·CHCl₃ (42 mg) was added 1,4-dioxane (1.4 mL) and the interior of a vessel was purged with argon. The reaction mixture was stirred at 100 °C for 20 minutes. The reaction mixture was diluted with ethyl acetate and filtered through a small pad of NH silica gel and the filtrate was concentrated under reduced pressure. The resulting residue was purified by column chromatography to give 34 mg of the title compound as a yellow oil.

### Step 3 Production of 2-({6-[1-(2-aminoethyl)-1H-benzimidazol-5-yl]-4-cyclopropylpyridin-2-yl}amino)pyridine-4-carbonitrile hydrochloride

To tert-butyl [2-(5{6-[(4-cyanopyridin-2-yl)amino]-4-cyclopropylpyridin-2-yl}-1H-benzimidazol-1-yl)ethyl]carbamate obtained in Example 13 Step2 (34 mg) was added TFA (1 mL) and the mixture was stirred at room temperature for 2 hours. The reaction mixture was neutralized with 4N aqueous sodium hydroxide solution, and extracted twice with ethyl acetate. The organic layer was dried over sodium sulfate and the solvent was evaporated under reduced pressure. The resulting residue was purified by column chromatography to give 20 mg of the title compound as a yellow solid. To a solution of the obtained solid in ethyl acetate (1.0 mL) was added 4N hydrogen chloride-ethyl acetate solution (19 µL). The precipitated solid was collected by filtration and washed with ethanol to give 7 mg of the title compound as a yellow solid.
MS 396 (M + 1)

### Example 14 2-({4-cyclopropyl-6-[1-(trans-4-hydroxycyclohexyl)-1H-benzimidazol-5-yl]pyridin-2-yl}amino)pyridine-4-carbonitrile hydrochloride

### Step 1 Production of tert-Butyl {6-[1-(trans-4-{[tert-butyl(dimethyl)silyl]oxy}cyclohexyl)-1H-benzimidazol-5-yl]-4-cyclopropylpyridin-2-yl}(4-cyanopyridin-2-yl) carbamate

To a mixture of 5-Bromo-1-(trans-4-{[tert-butyl(dimethyl)silyl]oxy}cyclohexyl)-1H-benzimidazole obtained in Reference Example 17 (126 mg), Pin₂B₂ (102 mg), PdCl₂(dppf)·CH₂Cl₂ (13 mg) and potassium acetate (151 mg) was added 1,4-dioxane (5 mL), and the interior of a vessel was purged with argon. The reaction mixture was stirred at 100 °C overnight. The reaction mixture was filtered through Celite, and the filtrate was concentrated under reduced pressure.To a solution of the resultingresulting residue in 1,4-dioxane (3 mL) and water (1 mL) was successively added tert-butyl (6-chloro-4-cyclopropylpyridin-2-yl) (4-cyanopyridin-2-yl) carbamate obtained in Reference Example 4 (114 mg), S-Phos (50 mg), potassium phosphate (196 mg) and palladium(II) acetate (24 mg) and the reaction mixture was stirred at 100 °C for 3 hours. The reaction mixture was diluted with ethyl acetate and filtered through Celite, and the filtrate was concentrated under reduced pressure.The resulting residue was purified by column chromatography to give 137 mg of the title compound as a yellow oil.

### Step 2 Production of 2-({4-cyclopropyl-6-[1-(trans-4-hydroxycyclohexyl)-1H-benzimidazol-5-yl]pyridin-2-yl}amino)pyridine-4-carbonitrile hydrochloride

To a solution of tert-Butyl {6-[1-(trans-4-{[tert-butyl(dimethyl)silyl]oxy}cyclohexyl)-1H-benzimidazol-5-yl]-4-cyclopropylpyridin-2-yl}(4-cyanopyridin-2-yl) carbamate obtained in Example 14 Step 1 (137 mg) in dichloromethane (3 mL) was added TFA (5 mL), and the mixture was stirred at room temperature overnight. TFA (2 mL) was added again and the mixture was stirred at room temperature for an hour. TFA (5 mL) was further added and the mixture was stirred at room temperature for 2 hours. The solvent was evaporated under reduced pressure and the resulting residue was purified by column chromatography to give 97 mg of the compound as a pale yellow solid. 40 mg of the resulting compound was dissolved in chloroform-methanol (1 : 1, 5 mL), 1N aqueous hydrochloric acid solution (89 µL) was added to the solution. The solvent was evaporated under reduced pressure, and the obtained solid was washed with ethyl acetate and diethyl ether to give 40 mg of the title compound as a pale yellow solid.
MS 451 (M + 1)
Elementary analysis as C₂₇H₂₆N₆O·HCl + 2H₂O
Calcd. (%) C: 62.00.; H: 5.97.; N: 16.07
Found. (%) C: 62.32.; H: 6.16.; N: 15.96

### Example 15 2-{[4-cyclopropyl-6-(1-methyl-1H-benzimidazol-5-yl)pyridin-2-yl]amino}pyridine-4-carbonitrile hydrocloride

### Step 1 Production of 5-(6-chloro-4-cyclopropylpyridin-2-yl)-1-methyl-1H-benzimidazole

To a mixture of 5-bromo-1-methyl-1H-benzimidazole obtained in Reference Example 18 (1.0 g) Pin₂B₂ (1.8 g), PdCl₂(dppf)-CH₂Cl₂ (311 mg), potassium acetate (2.3 g) was added 1,4-dioxane (10 mL), and the interior of a vessel was purged with argon, and the mixture was reacted under microwave irradiation (Biotage INITIATOR, at 160 °C for 20 minutes). The reaction mixture was filtered through Celite, and the filtrate was concentrated under reduced pressure. To the resulting residue was successively added PdCl₂(dppf)·CH₂Cl₂ (194 mg), 2,6-dichloro-4-cyclopropylpyridine obtained in Reference Example 2 (900 mg), potassium carbonate (2.0 g) and 1,4-dioxane-water (3 : 1, 25 mL) and the interior of a vessel was purged with argon. The reaction mixture was stirred at 100 °C for 1.5 hours, filtered through Celite and the filtrate was concentrated under reduced pressure.The resulting residue was purified by column chromatography to give 560 mg of the title compound as a pale yellow solid.

### Step 2 Production of 2-{ [4-cyclopropyl-6-(1-methyl-1H-benzimidazol-5-yl)pyridin-2-yl]amino}pyridine-4-carbonitrile hydrocloride

To a mixture of 5-(6-chloro-4-cyclopropylpyridin-2-yl)-1-methyl-1H-benzimidazol obtained in Example 15 Step1 (114 mg), 2-amino-4-cyanopyridine (52 mg), X-Phos (229 mg), NaOt-Bu (58 mg) and Pd₂(dba)₃·CHCl₃ (124 mg) was added 1,4-dioxane (1.4 mL) and the interior of a vessel was purged with argon. The reaction mixture was stirred at 100 °C for 15 minutes. The reaction mixture was filtered through a small pad of NH silica gel and the filtrate was concentrated under reduced pressure. The resulting residue was purified by column chromatography to give 68 mg of the compound as a yellow solid. To a solution of the obtained solid in ethyl acetate (1.8 mL) was added 4N hydrogen chloride-ethyl acetate solution (69 µL). The precipitated solid was collected by filtration and washed with ethyl acetate to give 74 mg of the title compound as a yellow solid.
MS 367 (M + 1)

### Example 16 2-({4-cyclopropyl-6-[1-(tetrahydro-2H-pyran-4-yl)-1H-benzimidazol-5-yl]pyridin-2-yl}amino)pyridine-4-carbonitrile hydrochloride

The title compound was prepared as a pale yellow solid (52 mg) according to the aforementioned procedure described in Example 14, using 5-bromo-1-(tetrahydro-2H-pyran-4-yl)-1H-benzimidazole obtained in Reference Example 19.
MS 437 (M + 1)

### Example 17 6-[(4-cyanopyridin-2-yl)amino]-4-cyclopropyl-2,3'-bipyridine-6'-carbonitrile hydrochloride

The title compound was prepared as a yellow solid (20 mg) according to the aforementioned procedure described in Example 1, using 5-(4,4,5,5-tetramethyl-1,3,2-dioxaborolan-2-yl)pyridine-2-carbonitrile.
MS 339 (M + 1)

### Example 18 2-[(6'-amino-4-cyclopropyl-2,3'-bipyridin-6-yl)amino]pyridine-4-carbonitrile hydrochloride

To 2-[(6-chloro-4-cyclopropylpyridin-2-yl)amino]pyridine-4-carbonitrile obtained in Reference Example 3 (90 mg) was successively added 5-(4,4,5,5-tetramethyl-1,3,2-dioxaborolan-2-yl)pyridine-2-amine (88 mg), S-Phos (28 mg), potassium phosphate (212 mg), 1,4-dioxane (3 mL), water (1 mL) and Pd₂(dba)₃·CHCl₃ (35 mg), and the interior of a vessel was purged with argon. The reaction mixture was stirred at 100 °C for an hour. The reaction mixture was diluted with etyl acetate and washed with saturated brine. The organic layer was dried over magnesium sulfate, and the solvent was evaporated under reduced pressure. The resulting residue was purified by column chromatograph to give 43 mg of the compound as a yellow solid. To a suspension of the obtained solid in methanol (3 mL) was added 2N hydrogen chloride-ethanol solution (65 µL), and the mixture was stirred at room temperature for 10 minutes. The solvent was evaporated under reduced pressure, and the obtained solid was washed with ethyl acetate to give 40 mg of the title compound as a yellow solid.
MS 329 (M + 1)

### Example 19 2-({6'-[(2-aminoethyl)amino]-4-cyclopropyl-2,3'-bipyridin-6-yl}amino)pyridine-4-carbonitrile dihydrochloride

### Step 1 Production of tert-butyl [2-({6-[(4-cyanopyridine-2-yl)amino]-4-cyclopropyl-2,3'-bipyridine-6'-yl}amino)ethyl] carbamate

The title compound was prepared as a yellow powder (95 mg) according to the aforementioned procedure described in Example 5 Step 1, using tert-butyl {2-[(5-bromopyridin-2-yl)amino]ethyl}carbamate obtained in Reference Example 20.

### Step 2 Production of 2-({6'-[(2-aminoethyl)amino]-4-cyclopropyl-2,3'-bipyridin-6-yl}amino)pyridine-4-carbonitrile dihydrochloride

To tert-butyl [2-({6-[(4-cyanopyridine-2-yl)amino]-4-cyclopropyl-2,3'-bipyridin-6'-yl}amino)ethyl] carbamate obtained in Example 19 Step 1 (95 mg) was successively added methanol (2 mL) and 4N hydrogen chloride-dioxane solution (1 ml), and the mixture was stirred at room temperature for 80 minutes. The solvent was evaporated under reduced pressure, and the obtained solid was washed with acetone-ethyl acetate (1 : 1) to give 80 mg of the title compound as a yellow solid.
MS 372 (M + 1)

### Example 20 2-{[4-cyclopropyl-6'-(4-hydroxypiperidin-1-yl)-2,3'-bipyridin-6-yl]amino}pyridine-4-carbonitrile hydrochloride

The title compound was prepared as a yellow solid (85 mg) according to the aforementioned procedure described in Example 6, using 5-bromo-2-(4-{[tert-butyl(dimethyl)silyl]oxy}piperidin-1-yl)pyridine obtained in Reference Example 21.
MS 413 (M + 1)

### Example 21

### 2-({4-cyclopropyl-6'-[(2-methoxyethyl)amino]-2,3'-bipyridin-6-yl}amino)pyridine-4-carbonitrile hydrochloride

The title compound was prepared as a yellow solid (87 mg) according to the aforementioned procedure described in Example 4, using 5-bromo-N-(2-methoxyethyl)pyridin-2-amine obtained in Reference Example 22.
MS 387 (M + 1)

### Example 22 2-({4-cyclopropyl-6'-[(3R)-3-hydroxypyrrolidin-1-yl]-2,3'-bipyridin-6-yl}amino)pyridine-4-carbonitrile hydrochloride

The title compound was prepared as a yellow solid (95 mg) according to the aforementioned procedure described in Example 6, using 5-bromo-2-[(3R)-3-{[tert-butyl(dimethyl)silyl]oxy}pyrrolidine-1-yl]pyridine obtained in Reference Example 23.
MS 399 (M + 1)

### Example 23 2-({4-cyclopropyl-6'-[(3-hydroxypropyl)amino]-2,3'-bipyridin-6-yl}amino)pyridine-4-carbonitrile hydrochloride

The title compound was prepared as a yellow solid (82 mg) according to the aforementioned procedure described in Example 6 using 5-bromo-N-(3-{[tert-butyl(dimethyl)silyl]oxy}propyl)pyridine-2-amine obtained in Reference Example 24.
MS 387 (M + 1)

### Example 24 2-({4-cyclopropyl-6'-[(3-hydroxy-2,2-dimethylpropyl)amino]-2,3'-bipyridin-6-yl}amino)pyridine-4- carbonitrile hydrochloride

The title compound was prepared as a yellow solid (80 mg) according to the aforementioned procedure described in Example 6 using 5-Bromo-N-(3-{[tert-butyl(dimethyl)silyl]oxy}-2,2-dimethylpropyl)pyridine-2-amine obtained in Reference Example 25.
MS 415 (M + 1)

### Example 25 2-({4-cyclopropyl-6'-[(3S)-3-hydroxypyrrolidin-1-yl]-2,3'-bipyridin-6-yl}amino)pyridine-4-carbonitrile hydrochloride

The title compound was prepared as a yellow solid (38 mg) according to the aforementioned procedure described in Example 6 using 5-bromo-2-[(3S)-3-{[tert-butyl(dimethyl)silyl]oxy]pyrrolidin-1-yl}pyridine obtained in Reference Example 26.
MS 399 (M + 1)

### Example 26 2-({4-cyclopropyl-6'-[4-(hydroxymethyl)piperidin-1-yl]-2,3'-bipyridin-6-yl}amino)pyridine-4-carbonitrile hydrochloride

The title compound was prepared as a yellow solid (70 mg) according to the aforementioned procedure described in Example 6 using 5-bromo-2-[4-({[tert-butyl(dimethyl)silyl]oxy}methyl)piperidin-1-yl]pyridine obtained in Reference Example 27.
MS 427 (M + 1)

### Example 27 2-{[4-cyclopropyl-6'-(thiomorpholin-4-yl)-2,3'-bipyridin-6- yl]amino}pyridine-4-carbonitrile hydrochloride

The title compound was prepared as a yellow solid (95 mg) according to the aforementioned procedure described in Example 4 using 4-(5-bromopyridin-2-yl)thiomorpholine obtained in Reference Example 28.
MS 415 (M + 1)

### Example 28 2-{[6'-(4-aminopiperidin-1-yl)-4-cyclopropyl-2,3'-bipyridin-6-yl]amino}pyridine-4-carbonitrile dihydrochloride

The title compound was prepared as a yellow solid (130 mg) according to the aforementioned procedure described in Example 19 using tert-butyl [1-(5-bromopyridin-2-yl)piperidin-4-yl]carbamate obtained in Reference Example 29.
MS 412 (M + 1)

### Example 29 2-({4-cyclopropyl-6'-[4-(methylamino)piperidin-1-yl]-2,3'-bipyridin-6-yl}amino)pyridine-4-carbonitrile dihydrochloride

The title compound was prepared as a yellow solid (100 mg) according to the aforementioned procedure described in Example 19 using tert-butyl [1-(5-Bromopyridin-2-yl)piperidin-4-yl] methylcarbamate obtained in Reference Example 30.
MS 426 (M + 1)

### Example 30 2{[4-cyclopropyl-6'-(4-fluoropiperidin-1-yl)-2,3'-bipyridin-6-yl]amino}pyridine-4-carbonitrile hydrochloride

The title compound was prepared as a yellow solid (65 mg) according to the aforementioned procedure described in Example 4 using 5-bromo-2-(4-fluoropiperidin-1-yl)pyridine obtained in Reference Example 31.
MS 415 (M + 1)

### Example 31 2-({6'-[bis(2-hydroxyethyl)amino]-4-cyclopropyl-2,3'-bipyridin-6-yl} amino)pyridine-4-carbonitrile hydrochloride

The title compound was prepared as a yellow solid (95 mg) according to the aforementioned procedure described in Example 6 using 5-bromo-N,N-bis(2-{[tert-butyl(dimethyl)silyl]oxy}ethyl)pyridin-2-amine obtained in Reference Example 32.
MS 417 (M + 1)

### Example 32 2-{[4-cyclopropyl-6'-(1,4-diazepan-1-yl)-2,3'-bipyridin-6-yl]amino}pyridine-4-carbonitrile dihydrochloride

The title compound was prepared as a yellow solid (80 mg) according to the aforementioned procedure described in Example 5 using tert-butyl 4-(5-bromopyridin-2-yl)-1,4-diazepane-1-carboxylate obtained in Reference Example 33.
MS 412 (M + 1)

### Example 33 2-({4-cyclopropyl-6'-[(3S)-3-hydroxypiperidin-1-yl]-2,3'-bipyridin-6-yl}amino)pyridine-4-carbonitrile hydrochloride

The title compound was prepared as a yellow solid (87 mg) according to the aforementioned procedure described in Example 6 using 5-bromo-2-[(3S)-3-{[tert-butyl(dimethyl)silyl]oxy}piperidin-1-yl]pyridine obtained in Reference Example 34.
MS 413 (M + 1)

### Example 34 2-({4-cyclopropyl-6'-[(3R)-3-hydroxypiperidin-1-yl]-2,3'-bipyridin-6-yl}amino)pyridine-4-carbonitrile hydrochloride

The title compound was prepared as a yellow solid (76 mg) according to the aforementioned procedure described in Example 6 using 5-bromo-2-[(3R)-3-{[tert-butyl(dimethyl)silyl]oxy}piperidin-1-yl]pyridine obtained in Reference Example 35.
MS 413 (M + 1)

### Example 35 2-({4-cyclopropyl-6-[2-(4-hydroxypiperidin-1-yl)pyrimidin-5-yl)pyridin-2-yl}amino)pyridine-4-carbonitrile hydrochloride

The title compound was prepared as a yellow solid (55 mg) according to the aforementioned procedure described in Example 6 using 5-bromo-2-(4-{[tert-butyl(dimethyl)silyl]oxy}piperidin-1-yl)pyrimidine obtained in Reference Example 36.
MS 414 (M + 1)

### Example 36 2-{[4-cyclopropyl-6-(1-methyl-1H-indazol-5-yl)pyridin-2-yl]amino}pyridine-4-carbonitrile hydrochloride

To 2-[(6-chloro-4-cyclopropylpyridin-2-yl)amino]pyridine-4-carbonitrile obtained in Reference Example 3 (100 mg) was successively added (1-methyl-1H-indazol-5-yl)boronic acid (98 mg), S-Phos (91 mg), potassium phosphate (236 mg), 1,4-dioxane (6 mL), water (2 mL) and palladium acetate (25 mg), and the interior of a vessel was purged with argon. The reaction mixture was stirred at 100 °C for 30 minutes. The reaction mixture was diluted with ethyl acetate and washed with water. The solvent was evaporated under reduced pressure and the resulting residue was purified by column chromatography to give 61 mg of the compound as a white solid. To a suspension of the obtained solid in methanol (3 mL) was added 2N hydrogen chloride-dioxane solution (83 µL), and the mixture was stirred at room temperature for 10 minutes. The solvent was evaporated under reduced pressure, and the obtained solid was washed with acetone to give 60 mg of the title compound as a yellow solid.
MS 367 (M + 1)

### Example 37 2-{[4-cyclopropyl-6-(1-methyl-1H-indazol-6-yl)pyridin-2-yl]amino}pyridine-4-carbonitrile hydrochloride

The title compound was prepared as a yellow solid (85 mg) according to the aforementioned procedure described in Example 36 using (1-methyl-1H-indazol-6-yl)boronic acid instead of (1-methyl-1H-indazol-5-yl)boronic acid MS 367 (M + 1)

### Example 38 2-{[4-cyclopropyl-6'-(4-methyl-1,4-diazepan-1-yl)-2,3'-bipyridin-6-yl]amino}pyridine-4-carbonitrile dihydrochloride

The title compound was prepared as a yellow solid (90 mg) according to the aforementioned procedure described in Example 4 using 1-(5-bromopyridin-2-yl)-4-methyl-1,4-diazepane obtained in Reference Example 37
MS 426 (M + 1)

### Example 39 1-{6-[(4-cyanopyridin-2-yl)amino]-4-cyclopropyl-2,3'-bipyridin-6'-yl}-N-methylpyrrolidine-2-carboxamide hydrochloride

### Step 1 Production of (S)-tert-butyl 1-{6-[(4-cyanopyridin-2-yl)amino]-4-cyclopropyl-2,3'-bipyridin-6'-yl}pyrrolidine-2-carboxylate

The title compound was prepared as a yellow amorphous form (275 mg) according to the aforementioned procedure described in Example 5 Step 1, using (S)-tert-butyl 1-(5-bromopyridin-2-yl)pyrrolidine-2-carboxylate obtained in Reference Example 38

### Step 2 Production of (S)-1-{6-[(4-cyanopyridin-2-yl)amino]-4-cyclopropyl-2,3'-bipyridin-6'-yl}pyrrolidin-2-carboxylicacid trifluoroacetate

(S)-tert-butyl 1-{6-[(4-cyanopyridin-2-yl)amino]-4-cyclopropyl-2,3'-bipyridine-6'-yl}pyrrolidin-2-carboxylate obtained in Example 39 Step 1 (257 mg) was dissolved in TFA (1.0 mL) and the mixture was stirred at room temperature for an hour. TFA was evaporated under reduced pressure , followed by azeotropic distillation 2 times with toluene to give 430 mg of the title compound as a brown amorphous form.

### Step 3 Production of 1-{6-[(4-cyanopyridin-2-yl) amino]-4-cyclopropyl-2,3'-bipyridin-6'-yl}-N-methylpyrrolidine-2-carboxamide hydrochloride

To a mixture of (S)-1-{6-[(4-cyanopyridin-2-yl)amino]-4-cyclopropyl-2,3'-bipyridin-6'-yl}pyrrolidin-2-carbonxylic acid trifluoroacetate obtained in Example 39 Step 2 (72 mg), methylamine hydrochloride (27 mg) and N-[(dimethylamino)(3H-[1,2,3]triazolo[4,5-b]pyridin-3-yloxy)methylidyne]-N-methylmethanaminium hexafluorophosphate (HATU) (76 mg) was successively added DMF (1 mL) and N,N-diisopropylethylamine (190 µL) and the mixture was stirred at room temperature for 24 hours. The reaction mixture was diluted with water, and the aqueous layer was extracted with chloroform. The solvent was evaporated under reduced pressure and the resulting residue was purified by column chromatography to give 25 mg of the compound as a pale yellow solid. To a suspension of the obtained solid in methanol (1 mL) was added 2N hydrogen chloride-ethanol (28 µL), and the mixture was stirred at room temperature for 10 minutes. The solvent was evaporated under reduced pressure, and the obtained solid was washed with acetone to give 20 mg of the title compound as a yellow solid.
MS 440 (M + 1)

### Example 40 (S)-1-{6-[(4-cyanopyridin-2-yl)amino]-4-cyclopropyl-2,3'-bipyridin-6'-yl}pyrrolidine-2-carboxamide hydrochloride

The title compound was prepared as a yellow solid (42 mg) according to the aforementioned procedure described in Example 39 Step 3 using ammonium chloride instead of methylamine hydrochloride
MS 426 (M + 1)

### Examples 41 2-({4-cyclopropyl-6-[4-(4-hydroxypiperidin-1-yl)phenyl)pyridin-2-yl}amino)pyridine-4-carbonitrile hydrochloride

The title compound was prepared as a yellow solid (55 mg) according to the aforementioned procedure described in Example 6 using 1-(4-bromophenyl)-4-{[tert-butyl(dimethyl)silyl]oxy}piperidine obtained in Reference Example 39 (209 mg) instead of 5-bromo-2-(3-{[tert-butyl(dimethyl)silyl]oxylazetidin-1-yl)pyridine
MS 426 (M + 1)

### Example 42 N-[(3S)-1-{6-[(4-cyanopyridin-2-yl)amino]-4-cyclopropyl-2,3'-bipyridin-6'-yl}pyrrolidin-3-yl]acetamide hydrochloride

The title compound was prepared as a yellow solid (87 mg) according to the aforementioned procedure described in Example 4 using N-[(3S)-1-(5-bromopyridin-2-yl)pyrrolidin-3-yl]acetamide obtained in Reference Example 40 (200 mg) instead of 1-(5-bromopyridin-2-yl)piperidin-4-on MS 440 (M + 1)

### Example 43 4-{6-[(4-cyanopyridin-2-yl)amino]-4-cyclopropyl-2,3'-bipyridin-6'-yl}-N-ethylpiperazine-1-carboxamide hydrochloride

### Step 1 Production of tert-butyl 4-(6-chloro-4-cyclopropyl-2,3'-bipyridin-6'-yl)piperazine-1-carboxylate

The title compound was prepared as a yellow amorphous form (352 mg) according to the aforementioned procedure described in Example 2 Step 1 using tert-butyl 4-(5-bromopyridin-2-yl)piperazine-1-carboxylate obtained in Reference Example 10 (685 mg) instead of 5-bromo-N-(2-{[tert-butyl(dimethyl)silyl]oxy}ethyl)pyridin-2-amine

### Step 2 Production of 6-chloro-4-cyclopropyl-6'-(piperazin-1-yl)-2,3'-bipyridine

tert-Butyl 4-(6-chloro-4-cyclopropyl-2,3'-bipyridin-6'-yl)piperazine-1-carboxylate obtained in Example 43 Step 1 (352 mg) was dissolved in TFA (2 mL) and the mixture was stirred at room temperature for 2 hours. The solvent was evaporated under reduced pressure and the resulting residue was purified by column chromatography to give 190 mg of the title compound as a white amorphous form.

### Step 3 Production of 4-(6-chloro-4-cyclopropyl-2,3'-bipyridin-6'-yl)-N-ethylpiperazine-1-carboxamide

To a solution of 6-chloro-4-cyclopropyl-6'-(piperazin-1-yl)-2,3'-bipyridine obtained in Example 43 Step 2 (50 mg) in dichloromethane (1 mL) was added ethylisocyanate (25 µL) and the mixture was stirred at room temperature for an hour. The reaction solution was directly purified by column chromatography to give 57 mg of the title compound as a pale yellow solid.

### Step 4 Production of 4-{6-[(4-cyanopyridin-2-yl)amino]-4-cyclopropyl-2,3'-bipyridin-6'-yl}-N-ethylpiperazine-1-carboxamide hydrochloride

The title compound was prepared as a yellow solid (42 mg) according to the aforementioned procedure described in Example 1 Step 2 using 4-(6-chloro-4-cyclopropyl-2,3'-bipyridin-6'-yl)-N-ethylpiperazine-1-carboxamide obtained in Example 43 Step 3 (57 mg) instead of 6-chloro-4-cyclopropyl-6'-(morpholin-4-yl)-2,3'-bipyridine
MS 469 (M + 1)

### Example 44 4-{6-[(4-cyanopyridin-2-yl)amino]-4-cyclopropyl-2,3'-bipyridin-6'-yl}-N-(propan-2-yl)piperazine-1-carboxamide hydrochloride

### Step 1 Production of 4-(6-chloro-4-cyclopropyl-2,3'-bipyridin-6'-yl)-N-(propan-2-yl)piperazine-1-carboxamide

To a solution of 6-chloro-4-cyclopropyl-6'-(piperazin-1-yl)-2,3'-bipyridine obtained in Example 43 Step 2 (50 mg) in dichloromethane (1 mL) was added isopropylisocyanate (47 µL) and the mixture was stirred at room temperature for an hour. The reaction solution was directly purified by column chromatography to give 71 mg of the title compound as a pale yellow solid.

### Step 2 Production of 4-{6-[(4-cyanopyridin-2-yl)amino]-4-cyclopropyl-2,3'-bipyridin-6'-yl}-N-(propan-2-yl)piperazine-1-carboxamide hydrochloride

The title compound was prepared as a yellow solid (56 mg) according to the aforementioned procedure described in Example 1 Step 2 using 4-(6-chloro-4-cyclopropyl-2,3'-bipyridin-6'-yl)-N-(propan-2-yl)piperazine-1-carboxamide obtained in Example 44 Step 1 (71 mg) instead of 6-chloro-4-cyclopropyl-6'-(morpholin-4-yl)-2,3'-bipyridine
MS 483 (M + 1)

### Example 45 1-[(3R)-1-{6-[(4-cyanopyridin-2-yl)amino]-4-cyclopropyl-2,3'-bipyridin-6'-yl}pyrrolidin-3-yl]urea hydrochloride

### Step 1 Production of tert-butyl [(3R)-1-{6-[(4-cyanopyridin-2-yl)amino]-4-cyclopropyl-2,3'-bipyridin-6'-yl}pyrrolidin-3-yl] carbamate

The title compound was prepared as a yellow solid (200 mg) according to the aforementioned procedure described in Example 5 Step 1 using tert-butyl [(3R)-1-(5-bromopyridin-2-yl)pyrrolidin-3-yl]carbamate obtained in Reference Example 44 (266 mg) instead of tert-butyl 4-(5-bromopyridin-2-yl)-2-oxopiperazine-1-carboxylate

### Step 2 Production of 2-({6'-[(3R)-3-aminopyrrolidin-1-yl]-4-cyclopropyl-2,3'-bipyridin-6-yl}amino)pyridine-4-carbonitrile

To a solution of tert-butyl [(3R)-1-{6-[(4-cyanopyridin-2-yl)amino]-4-cyclopropyl-2,3'-bipyridin-6'-yl}pyrrolidin-3-yl] carbamate obtained in Example 45 Step 1 (200 mg) in dichloromethane (1 mL) was added TFA (1 mL) and the mixture was stirred at room temperature for an hour. The solvent was evaporated under reduced pressure and the resulting residue was purified by column chromatography to give 150 mg of the title compound as a yellow solid.

### Step 3 Production of 1-[(3R)-1-{6-[(4-cyanopyridin-2-yl)amino]-4-cyclopropyl-2,3'-bipyridin-6'-yl}pyrrolidin-3-yl]urea hydrochloride

To a solution of 2-({6'-[(3R)-3-aminopyrrolidin-1-yl]-4-cyclopropyl-2,3'-bipyridin-6-yl}amino)pyridine-4-carbonitrile obtained in Example 45 Step 2 (150 mg) in THF (2.0 mL) was added trimethylsilylisocyanate (3 X 60 µL) and the mixture was stirred stirred at room temperature for a day. The reaction mixture was diluted with water, extracted with chloroform, and the solvent was evaporated under reduced pressure. The resulting residue was purified by column chromatography to give 12 mg of the compound as a yellow solid. To a suspension of the obtained solid in methanol (1 mL) was added 2N hydrogen chloride-ethanol (14 µL), and the mixture was stirred at room temperature for 10 minutes. The solvent was evaporated under reduced pressure, and the obtained solid was washed with acetone to give 8 mg of the title compound as a yellow solid.
MS 441 (M + 1)

### Example 46 4-{6-[(4-cyanopyridin-2-yl)amino]-4-cyclopropyl-2,3'-bipyridin-6'-yl}piperazine-1-carbothioamide hydrochloride

### Step 1 Production of tert-butyl 4-{6-[(4-cyanopyridin-2-yl)amino]-4-cyclopropyl-2,3'-bipyridin-6'-yl}piperazine-1-carboxylate

To a mixture of tert-butyl 4-(5-bromopyridin-2-yl)piperazine-1-carboxylate obtained in Reference Example 10 (493 mg), Pin₂B₂ (385 mg), X-Phos (138 mg), potassium acetate (425 mg) and Pd₂(dba)₃·CHCl₃ (75 mg) was added 1,4-dioxane (10 mL) and the interior of a vessel was purged with argon. The reaction mixture was stirred at 100 °C for an hour. The reaction mixture was filtered through Celite, and the filtrate was concentrated under reduced pressure. To the resultingresulting residue was successively added 2-[(6-chloro-4-cyclopropylpyridin-2-yl)amino]pyridine-4-carbonitrile obtained in Reference Example 3 (300 mg), S-Phos (182 mg), potassium phosphate (706 mg), 1,4-dioxane (8 mL), water (2 mL) and palladium(II) acetate (50 mg), and the interior of a vessel was purged with argon. The reaction mixture was stirred at 100 °C for 30 minutes. The reaction mixture was concentrated under reduced pressure and the resulting residue was purified by column chromatography to give 600 mg of the title compound as a pale yellow solid.

### Step 2 Production of 2-{[4-cyclopropyl-6'-(piperazin-1-yl)-2,3'-bipyridin-6-yl]amino}pyridine-4-carbonitrile

To a solution of tert-butyl 4-{6-[(4-cyanopyridin-2-yl)amino]-4-cyclopropyl-2,3'-bipyridin-6'-yl}piperazin-1-carboxylate obtained in Example 46 Step 1 (600 mg) in dichloromethane (1.0 mL) was added TFA (1.0 mL) and the mixture was stirred at room temperature for an hour. The solvent was evaporated under reduced pressure and saturated aqueous sodium hydrogen carbonate solution was added to the resulting residue and extracted with chloroform-methanol (10 : 1). The organic layer was dried over magnesium sulfate and the solvent was evaporated under reduced pressure. The resulting residue was washed with diethyl ether to give 250 mg of the title compound as a pale yellow solid.

### Step 3 Production of 4-{6-[(4-cyanopyridin-2-yl)amino]-4-cyclopropyl-2,3'-bipyridin-6'-yl}piperazine-1-carbothioamide hydrochloride

To a solution of 2-{[4-cyclopropyl-6'-(piperazin-1-yl)-2,3'-bipyridin-6-yl]amino}pyridine-4-carbonitrile obtained in Example 46 Step 2 (111 mg) in 1,4-dioxane (2.0 mL) was successively added trimethylsilyl isocyanate (240 µL) and triethylamine (200 µL) and the mixture was stirred at 100 °C for 1 day. The solvent was evaporated under reduced pressure and the resulting residue was purified by column chromatography to give 19 mg of the compound as a yellow solid. To a suspension of the obtained solid in methanol (1 mL) was added 2N hydrogen chloride-ethanol (21 µL), and the mixture was stirred at room temperature for 10 minutes. The solvent was evaporated under reduced pressure, and the obtained solid was washed with acetone to give 15 mg of the title compound as a yellow solid.
MS 457 (M + 1)

### Example 47 (R)-1-{6-[(4-cyanopyridin-2-yl)amino]-4-cyclopropyl-2,3'-bipyridin-6'-yl}pyrrolidine-2-carboxamide hydrochloride

The title compound was prepared as a yellow solid (40 mg) according to the aforementioned procedure described in Example 4 using1-(5-bromopyridin-2-yl)pyrrolidin-2-carboxamide obtained in Reference Example 42 (80 mg) instead of 1-(5-bromopyridin-2-yl)piperidin-4-on MS 426 (M + 1)

### Example 48 2-({4-cyclopropyl-6'-[(2S)-2-(hydroxymethyl)pyrrolidin-1-yl]-2,3'-bipyridin-6-yl}amino)pyridine-4- carbonitrile hydrochloride

The title compound was prepared as a yellow solid (62 mg) according to the aforementioned procedure described in Example 6 using 5-bromo-2-[(2S)-2-({[tert-butyl(dimethyl)silyl]oxy}methyl)pyrrolidin-1-yl]pyridine obtained in Reference Example 43 (241 mg) instead of 5-bromo-2-(3-{[tert-butyl(dimethyl)silyl]oxy}azetidin-1-yl)pyridine
MS 413 (M + 1)

### Example 49 2-({6'-[(3R)-3-aminopyrrolidin-1- yl]-4-cyclopropyl-2,3'-bipyridin-6-yl}amino)pyridine-4-carbonitrile dihydrochloride

### Step 1 Production of tert-butyl [(3R)-1-{6-[(4-cyanopyridin-2-yl)amino]-4-cyclopropyl-2,3'-bipyridin-6'-yl}pyrrolidin-3-yl]carbamate

The title compound was prepared as a yellow solid (130 mg) according to the aforementioned procedure described in Example 5 Step 1 using tert-butyl [(3R)-1-(5-bromopyridin-2-yl)pyrrolidin-3-yl]carbamate obtained in Reference Example 44 (190 mg) instead of tert-butyl 4-(5-bromopyridin-2-yl)-2-oxopiperazine-1-carboxylate.

### Step 2 Production of 2-({6'-[3-aminopyrrolidin-1- yl]-4-cyclopropyl-2,3'-bipyridin-6-yl}amino)pyridine-4-carbonitrile dihydrochloride

To a solution of tert-butyl [(3R)-1-{6-[(4-cyanopyridin-2-yl)amino]-4-cyclopropyl-2,3'-bipyridin-6'-yl}pyrrolidin-3-yl]carbamate obtained in Example 49 Step 1 in methanol (2 mL) was added 2N aqueous hydrochloric acid solution (2 mL) and the mixture was stirred at room temperature for 30 minutes. The solvent was evaporated under reduced pressure followd by azeotropic distillation with ethanol four times. The obtained residure was washed with acetone to give 70 mg of the title compound as a yellow solid.
MS 398 (M + 1)

### Example 50 N-[(3R)-1-{6-[(4-cyanopyridin-2-yl)amino]-4-cyclopropyl-2,3'-bipyridin-6'-yl}pyrrolidin-3-yl]acetamide hydrochloride

The title compound was prepared as a yellow solid (45 mg) according to the aforementioned procedure described in Example 4 using N-[(3R)-1-(5-bromopyridin-2-yl)pyrrolidin-3-yl]acetamide obtained in Reference Example 45 (158 mg) instead of 1-(5-bromopyridin-2-yl)piperidin-4-on
MS 440 (M + 1)

### Example 51 2-({4-cyclopropyl-6'-[(3R)-3-fluoropyrrolidin-1-yl]-2,3'-bipyridin-6-yl}amino)pyridine-4-carbonitrile hydrochloride

The title compound was prepared as a yellow solid (24 mg) according to the aforementioned procedure described in Example 4 using 5-bromo-2-[(3R)-3-fluoropyrrolidin-1-yl]pyridine obtained in Reference Example 46 (136 mg) instead of 1-(5-bromopyridin-2-yl)piperidin-4-on
MS 401 (M + 1)

### Example 52 2-({4-cyclopropyl-6'-[(3R)-3-(dimethylamino)pyrrolidin-1-yl]-2,3'-bipyridin-6-yl}amino)pyridine-4-carbonitrile hydrochloride

The title compound was prepared as a yellow solid (7 mg) according to the aforementioned procedure described in Example 4 using (3R)-1-(5-bromopyridin-2-yl)-N,N-dimethylpyrrolidin-3-amine obtained in Reference Example 47 (150 mg) instead of 1-(5-bromopyridin-2-yl)piperidin-4-on
MS 426 (M + 1)

### Example 53 2{[4-cyclopropyl-6'-(2-oxopyrrolidin-1-yl)-2,3'-bipyridin-6-yl]amino}pyridine-4-carbonitrile hydrochloride

The title compound was prepared as a yellow solid (82 mg) according to the aforementioned procedure described in Example 4 using 1-(5-bromopyridin-2-yl)pyrrolidin-2-on obtained in Reference Example 48 (134 mg) instead of 1-(5-bromopyridin-2-yl)piperidin-4-on
MS 397 (M + 1)

### Example 54 2-[(4-cyclopropyl-6'-methyl-2,3'-bipyridin-6-yl)amino]pyridine-4-carbonitrile hydrochloride

The title compound was prepared as a yellow solid (11 mg) according to the aforementioned procedure described in Example 1 using 2-methyl-5-(4,4,5,5-tetramethyl-1,3,2-dioxaborolan-2-yl)pyridine (600 mg) in stead of 4-[5-(4,4,5,5-tetramethyl-1,3,2-dioxaborolan-2-yl)pyridine-2-yl]morpholine
MS 328 (M + 1)

### Example 55 2-{[4-cyclopropyl-6-(1H-indazol-6-yl)pyridin-2-yl]amino}pyridine-4-carbonitrile hydrochloride

The title compound was prepared as a yellow solid (3 mg) according to the aforementioned procedure described in Example 1 using 6-(4,4,5,5-tetramethyl-1,3,2-dioxaborolan-2-yl)-1H-indazole (169 mg) in stead of 4-[5-(4,4,5,5-tetramethyl-1,3,2-dioxaborolan-2-yl)pyridine-2-yl]morpholine
MS 353 (M + 1)

### Example 56 2-{[4-cyclopropyl-6-(1H-indazol-5-yl)pyridin-2-yl]amino}pyridine-4-carbonitrile hydrochloride

### Step 1 Production of 5-(6-chloro-4-cyclopropylpyridin-2-yl)-1H-indazole

The title compound was prepared as a yellow solid (81 mg) according to the aforementioned procedure described in Example 1 Step 1 using 5-(4,4,5,5-tetramethyl-1,3,2-dioxaborolan-2-yl)-1H-indazole (164 mg) in stead of 4-[5-(4,4,5,5-tetramethyl-1,3,2-dioxaborolan-2-yl)pyridin-2-yl]morpholine

### Step 2 Production of tert-butyl 5-(6-chloro-4-cyclopropylpyridin-2-yl)-1H-indazol-1-carboxylate

To 5-(6-chloro-4-cyclopropylpyridin-2-yl)-1H-indazole obtained in Reference Example 56 Step 1 (80 mg) was successively added THF (1.5 mL), triethylamine (148 µL), Boc₂O (194 mg) and DMAP (18 mg) and the mixture was stirred at room temperature overnight.The reaction mixture was diluted with saturated aqueous sodium hydrogen carbonate solution, and extracted with chloroform. The organic layer was dried over Magnesium sulfate and evaporated under reduced pressure. The resulting residue was purified by column chromatography to give 103 mg of the title compound as a pale yellow solid.

### Step 3 Production of tert-butyl 5-{6-[(4-cyanopyridin-2-yl)amino]-4-cyclopropylpyridin-2-yl}-1H-indazole -1-carboxylate

The title compound was prepared as a yellow solid (48 mg) according to the aforementioned procedure described in Example 13 Step 2 using tert-butyl 5-(6-chloro-4-cyclopropylpyridin-2-yl)-1H-indazole-1-carboxylate obtained in Example 56 Step 2 (100 mg) in stead of tert-butyl {2-[5-(6-chloro-4-cyclopropylpyridin-2-yl)-1H-benzimidazol-1-yl]ethyl}carbamate

### Step 4 Production of 2-{[4-cyclopropyl-6-(1H-indazol-5-yl)pyridin-2-yl]amino}pyridine-4-carbonitrile hydrochloride

To tert-butyl 5-{6-[(4-cyanopyridin-2-yl)amino]-4-cyclopropylpyridin-2-yl}-1H-indazole-1-carboxylate obtained in Example 56 Step 3 (48 mg) was added dichloromethane (3.0 mL) and TFA (27 µL) and the mixture was stirred at room temperature overnight. Triethylamine (100 µL) was added to the reaction mixture and the solvent was evaporated under reduced pressure. The resulting residue was purified by column chromatography to give 29 mg of the compound as a pale pale yellow solid. To a suspension of the obtained solid in methanol (1 mL) was added 4N hydrogen chloride-dioxane solution (23 µL), and the mixture was stirred at room temperature for 30 minutes. The solvent was evaporated under reduced pressure, and the obtained solid was washed with ethyl acetate to give 14 mg of the title compound as a yellow solid.
MS 353 (M + 1)

### Example 57 4-{6-[(4-cyanopyridin-2-yl)amino]-4-cyclopropyl-2,3'-bipyridin-6'-yl}piperazine-1-carboxamide hydrochloride

To a suspension of 2-{[4-cyclopropyl-6'-(piperazin-1-yl)-2,3'-bipyridin-6-yl]amino}pyridine-4-carbonitrile hydrochloride obtained in Example 7 Step 1 (40 mg) in dichloromethane (1 mL) was added triethylamine (15µL) and trimethylsilyl isocyanate (15 µL) at 0 °C and the mixture was stirred at room temperature for 3 hours. The reaction mixture was diluted with saturated aqueous sodium hydrogen carbonate solution, and extracted with chloroform-methanol (10 : 1). The organic layer was dried over magnesium sulfate and evaporated under reduced pressure. The resulting residue was purified by column chromatography to give 28 mg of the title compound as a pale yellow solid. To a suspension of the obtained solid in methanol (1 mL) was added 4N hydrogen chloride-dioxane solution (17 µL), and the mixture was stirred at room temperature for 10 minutes. The solvent was evaporated under reduced pressure, and the obtained solid was washed with ethyl acetate to give 29 mg of the title compound as a yellow solid.
MS 441 (M + 1)

### Example 58 2-({4-cyclopropyl-6'-[4-(methanesulfonyl)piperazin-1-yl]-2,3'-bipyridin-6-yl}amino)pyridine-4- carbonitrile hydrochloride

The title compound was prepared as a yellow solid (82 mg) according to the aforementioned procedure described in Example 4 using 1-(5-bromopyridin-2-yl)-4-(methanesulfonyl)piperazine obtained in Reference Example 49 (176 mg) instead of 1-(5-bromopyridin-2-yl)piperidin-4-on
MS 476 (M + 1)

### Example 59 2-{[6'-(4-acetylpiperazin-1-yl)-4-cyclopropyl-2,3'-bipyridin-6-yl]amino}pyridine-4-carbonitrile hydrochloride

The title compound was prepared as a yellow solid (40 mg) according to the aforementioned procedure described in Example 4 using 1-[4-(5-bromopyridin-2-yl)-piperazin-1-yl]ethanone obtained in Reference Example 50 (156 mg) instead of 1-(5-bromopyridin-2-yl)piperidin-4-on
MS 440 (M + 1)

### Example 60 1-{6-[(4-cyanopyridin-2-yl)amino]-4-cyclopropyl-2,3'-bipyridin-6'-yl} piperidine-4-carbonxylic acid hydrochloride

### Step 1 Production of 2-(trimethylsilyl)ethyl 1-{6-[(4-cyanopyridin-2-yl)amino]-4-cyclopropyl-2,3'-bipyridin-6'-yl}piperidine-4-carboxylate

The title compound was prepared as a pale yellow solid (154 mg) according to the aforementioned procedure described in Example 5 Step 1 using 2-(trimethylsilyl)ethyl 1-(5-bromopyridin-2-yl)piperidine-4-carboxylate obtained in Reference Example 51 (212 mg) instead of tert-butyl 4-(5-bromopyridin-2-yl)-2-oxopiperazine-1-carboxylate

### Step 2 Production of 1-{6-[(4-cyanopyridin-2-yl)amino]-4-cyclopropyl-2,3'-bipyridin-6'-yl} piperidine-4-carbonxylic acid hydrochloride

To a solution of 2-(trimethylsilyl)-ethyl 1-{6-[(4-cyanopyridin-2-yl)amino]-4-cyclopropyl-2,3'-bipyridin-6'-yl}piperidine-4-carboxylate (136 mg) obtained in Example 60 Step1 (136 mg) in THF (3 mL) was added tetrabutylammonium fluoride (1.9 mL, 1M solution in THF), and the mixture was stirred at 50 °C for 4 hours. The solvent was evaporated under reduced pressure and to the resulting residue was added water. The precipitated solid was collected by filtration and washed with water, hexane-ethyl acetate (30 : 1) to give 90 mg of the compound as a yellow solid. To a suspension of the obtained solid (22 mg) in 1,4-dioxane (1 mL) was added 4N hydrogen chloride-dioxane solution (14 µL), and the mixture was stirred at room temperature for 10 minutes. The solvent was evaporated under reduced pressure and the obtained solid was washed with ethyl acetate to give 23 mg of the title compound as a yellow solid.
MS 441 (M + 1)

### Example 61 1-{6-[(4-cyanopyridin-2-yl)amino]-4-cyclopropyl-2,3'-bipyridin-6'-yl}piperidine-4-carboxamide hydrochloride

The title compound was prepared as a yellow solid (21 mg) according to the aforementioned procedure described in Example 7 Step 4, using 1-{6-[(4-cyanopyridin-2-yl)amino]-4-cyclopropyl-2,3'-bipyridin-6'-yl} piperidine-4-carboxylic acid hydrochloride obtained in Example 60 Step 2 (22 mg) instead of (4-{6-(4-cyanopyridin-2-yl)amino]-4-cyclopropyl-2,3'-bipyridin-6'-yl}piperazin-1-yl) acetic acid hydrochloride
MS 440 (M + 1)

### Example 62 1-{6-[(4-cyanopyridin-2-yl)amino]-4-cyclopropyl-2,3'-bipyridin-6'-yl}-4-(4-fluorophenyl)piperidine-4-carboxamide hydrochloride

The title compound was prepared as a yellow solid (84 mg) according to the aforementioned procedure described in Example 4 using 1-(5-bromopyridin-2-yl)-4-(4-fluorophenyl)piperidine-4-carboxamide obtained in Reference Example 52 (208 mg) instead of 1-(5-bromopyridin-2-yl)piperidin-4-on
MS 534 (M + 1)

### Example 63 2-(4-{6-[(4-cyanopyridin-2-yl)amino]-4-cyclopropyl-2,3'-bipyridin-6'-yl}piperazin-1-yl)-Nethylacetamide hydrochloride

The title compound was prepared as a yellow solid (15 mg) according to the aforementioned procedure described in Example 7 Step 4, using methylamine hydrochloride (10 mg) instead of ammonium chloride
MS 469 (M + 1)

### Example 64 1-(1-{6-[(4-cyanopyridin-2-yl)amino]-4-cyclopropyl-2,3'-bipyridin-6'-yl}piperidin-4-yl)urea hydrochloride

The title compound was prepared as a yellow solid (41 mg) according to the aforementioned procedure described in Example 57 using 2-{[6'-(4-aminopiperidin-1-yl)-4-cyclopropyl-2,3'-bipyridin-6-yl]amino}pyridine-4-carbonitrile dihydrochloride obtained in Example 28 (100 mg) instead of 2-{[4-cyclopropyl-6'-(piperazin-1-yl)-2,3'-bipyridin-6-yl]amino}pyridine-4-carbonitrile hydrochloride
MS 455 (M + 1)

### Example 65 4-({6-[(4-cyanopyridin-2-yl)amino]-4-cyclopropyl-2,3'-bipyridin-6'-yl}amino)piperidine-1-carboxamide hydrochloride

The title compound was prepared as a yellow solid (97 mg) according to the aforementioned procedure described in Example 4 using 4-[(5-bromopyridin-2-yl)amino]piperidine-1-carboxamide obtained in Reference Example 53 (200 mg) instead of 1-(5-bromopyridin-2-yl)piperidin-4-on
MS 455 (M + 1)

### Example 66 2-{[4-cyclopropyl-6'-(2-oxopiperazin-1-yl)-2,3'-bipyridin-6-yl]amino}pyridine-4-carbonitrile hydrochloride

### Step 1 Production of tert-butyl 4-{6-[(4-cyanopyridin-2-yl)amino]-4-cyclopropyl-2,3'-bipyridin-6'-yl}-3-oxopiperazine-1-carboxylate

The title compound was prepared as a brown oil (230 mg) according to the aforementioned procedure described in Example 5 Step 1, using tert-butyl 4-(5-bromopyridin-2-yl)-3-oxopiperazine-1-carboxylate obtained in Reference Example 54 (171 mg) instead of tert-butyl 4-(5-bromopyridin-2-yl)-2-oxopiperazine-1-carboxylate

### Step 2 Production of 2-{[4-cyclopropyl-6'-(2-oxopiperazin-1-yl)-2,3'-bipyridin-6-yl]amino}pyridine-4-carbonitrile hydrochloride

2N Hydrogen chloride-ethanol solution (2 mL) was added to tert-butyl 4-{6-[(4-cyanopyridin-2-yl)amino]-4-cyclopropyl-2,3'-bipyridin-6'-yl}-3-oxopiperazine-1-carboxylate obtained in Example 66 Step 1 (230 mg) and the mixture was stirred at room temperature for 3 hours. Afrer evaporation of the solvent under reduced pressure, the resulting residue was purified by column chromatography followed by washing with methanol to give 38 mg of the compound as a white solid. To a suspension of the obtained material in methanol (1 mL) was added 2N hydrogen chloride-ethanol solution (46 µL), and the mixture was stirred at room temperature for 10 minutes. The solvent was evaporated under reduced pressure and the obtained solid was washed with acetone to give 30 mg of the title compound as a yellow solid.
MS 412 (M + 1)

### Example 67 4-{6-[(4-cyanopyridin-2-yl)amino]-4-cyclopropyl-2,3'-bipyridin-6'-yl}-3-oxopiperazine-1-carboxamide hydrochloride

The title compound was prepared as a yellow solid (48 mg) according to the aforementioned procedure described in Example 4 using 4-(5-bromopyridin-2-yl)-3-oxopiperazine-1-carboxamide obtained in Reference Example 55 (130 mg) instead of 1-(5-bromopyridin-2-yl)piperidin-4-on
MS 455 (M + 1)

### Example 68 2-{[4-cyclopropyl-5'-(1,1-dioxidethiomorpholin-4-yl)-2,3'-bipyridin-6-yl]amino}pyridine-4-carbonitrile hydrochloride

The title compound was prepared as a yellow solid (75 mg) according to the aforementioned procedure described in Example 4 using 4-(5-bromopyridin-3-yl)thiomorpholine 1,1-dioxide obtained in Reference Example 56 (194 mg) instead of 1-(5-bromopyridin-2-yl)piperidin-4-on
MS 447 (M + 1)

### Example 69 4-{6-[(4-cyanopyridin-2-yl)amino]-4-cyclopropyl-2,3'-bipyridin-5'-yl}-1,4-diazepane-1-carboxamide hydrochloride

To a mixture of 4-(5-bromopyridin-3-yl)-1,4-diazepane-1-carboxamide obtained in Reference Example 57 (166 mg), Pin₂B₂ (155 mg), X-Phos (53 mg), potassium acetate (164 mg) and Pd₂(dba)₃·CHCl₃ (29 mg) was added 1,4-dioxane (3 mL), and the interior of a vessel was purged with argon. The reaction mixture was stirred at 100 °C for 2 hours. To the resultingreaction mixture were successively added 2-[(6-chloro-4-cyclopropylpyridin-2-yl)amino]pyridine-4-carbonitrile obtained in Reference Example 3 (100 mg) and a solution of potassium phosphate (236 mg) in water (1 mL) and the mixture was stirred at 100 °C for 2 hours. The solvent was evaporated under reduced pressure and the resulting residue was purified by column chromatography to give 155 mg of the compound as a yellow solid.

To a suspension of the obtained solid in methanol (2 mL) was added 2N hydrogen chloride-ethanol solution (171 µL), and the mixture was stirred at room temperature for 10 minutes. The solvent was evaporated under reduced pressure, and the obtained solid was washed with acetone to give 111 mg of the title compound as a yellow solid.
MS 455 (M + 1)

### Example 70 2-({4-cyclopropyl-6'-[(3-oxopiperazin-1-yl)methyl]-2,3'-bipyridin-6-yl}amino)pyridine-4-carbonitrile hydrochloride

### Step 1 Production of tert-butyl (4-cyanopyridin-2-yl){4-cyclopropyl-6'-[(3-oxopiperazin-1-yl)methyl]-2,3'-bipyridin-6-yl} carbamate

To a mixture of 4-[(5-bromopyridin-2-yl)methyl]piperazin-2-on obtained in Reference Example 58 (120 mg), Pin₂B₂ (124 mg), X-Phos (43 mg), potassium acetate (131 mg) and Pd₂(dba)₃·CHCl₃ (23 mg) was added 1,4-dioxane (5 mL), and the interior of a vessel was purged with argon. The reaction mixture was stirred at 100 °C for an hour. The reaction mixture was filtered through Celite, and the filtrate was concentrated under reduced pressure. To the resultingresulting residue was successively added tert-butyl (6-chloro-4-cyclopropylpyridin-2-yl) (4-cyanopyridin-2-yl) carbamate obtained in Reference Example 4 (137 mg), S-Phos (61 mg), potassium phosphate (236 mg), 1,4-dioxane (6.0 mL), water (2.0 mL), palladium(II) acetate (17 mg), and the interior of a vessel was purged with argon. The reaction mixture was stirred at 100 °C for 30 minutes. The reaction mixture was concentrated under reduced pressure and the resulting residue was purified by column chromatography to give 79 mg of the title compound as a yellow amorphous form.

### Step 2 2-({4-cyclopropyl-6'-[(3-oxopiperazin-1-yl)methyl]-2,3'-bipyridin-6-yl}amino)pyridine-4-carbonitrile hydrochloride

To tert-butyl (4-cyanopyridin-2-yl){4-cyclopropyl-6'-[(3-oxopiperazin-1-yl)methyl]-2,3'-bipyridin-6-yl} carbamate obtained in Example 70 Step 1 (79 mg) was successively added methanol (0.3 mL) and methanesulfonic acid (0.2 mL) and the mixture was stirred at room temperature for 30 minutes. Saturated aqueous sodium hydrogen carbonate solution was added to the mixture and extracted with chloroform. The solvent was evaporated under reduced pressure and the resulting residue was purified by column chromatography to give 55 mg of the compound as a white solid. To a suspension of the obtained solid in methanol (1.0 mL) was added 2N hydrogen chloride-ethanol (65 µL), and the mixture was stirred at room temperature for 10 minutes. The solvent was evaporated under reduced pressure, and the obtained solid was washed with acetone to give 40 mg of the title compound as a yellow solid.
MS 426 (M + 1)

### Example 71 2-{[4-cyclopropyl-6'-(hydroxymethyl)-2,3'-bipyridin-6-yl]amino}pyridine-4-carbonitrile hydrochloride

### Step 1 Production of tert-Butyl (4-cyanopyridin-2-yl)[4-cyclopropyl-6'-(hydroxymethyl)-2,3'-bipyridin-6-yl]carbamate

To a mixture of (5-bromopyridin-2-yl) methanol obtained in Reference Example 60 (99 mg), Pin₂B₂ (91 mg), X-Phos (62 mg), potassium acetate (96 mg) and Pd₂(dba)₃·CHCl₃ (34 mg) was added 1,4-dioxane (3.0 mL), and the interior of a vessel was purged with argon. The reaction mixture was stirred at 100 °C for 2 hours. The reaction mixture was filtered through Celite, and the filtrate was concentrated under reduced pressure. To the resulting mixture was successively added tert-butyl (6-chloro-4-cyclopropylpyridin-2-yl) (4-cyanopyridin-2-yl) carbamate obtained in Reference Example 4 (100 mg) and a solution of potassium phosphate (173 mg) in water (1 mL) and the reaction mixture was stirred at 100 °C for 2 hours. The solvent was evaporated under reduced pressure and the resulting residue was purified by column chromatography to give 125 mg of the compound as a yellow oil.

### Step 2 Production of 2-{[4-cyclopropyl-6'-(hydroxymethyl)-2,3'-bipyridin-6-yl]amino}pyridine-4-carbonitrile hydrochloride

The title compound was prepared as a pale yellow solid (33 mg) according to the aforementioned procedure described in Example 70 Step 2 using tert-butyl (4-cyanopyridin-2-yl)[4-cyclopropyl-6'-(hydroxymethyl)-2,3'-bipyridin-6-yl]carbamate obtained in Reference Example 71 Step 1 (125 mg) instead of tert-butyl (4-cyanopyridin-2-yl){4-cyclopropyl-6'-[(3-oxopiperazin-1-yl)methyl]-2,3'-bipyridin-6-yl} carbamate
MS 344 (M + 1)

### Example 72 2-({4-cyclopropyl-6'-[(1,1-dioxidethiomorpholin-4-yl)methyl]-2,3'-bipyridin-6-yl}amino)pyridine-4-carbonitrile hydrochloride

The title compound was prepared as a pale yellow solid (125 mg) according to the aforementioned procedure described in Example 69 using 4-[(5-bromopyridin-2-yl)methyl] thiomorpholine 1,1-dioxide obtained in Reference Example 61 (125 mg) instead of 4-(5-bromopyridin-3-yl)-1,4-diazepane-1-carboxamide
MS 461 (M + 1)

### Example 73 2-({4-cyclopropyl-5'-[(3-oxopiperazin-1-yl)methyl]-2,3'-bipyridin-6-yl}amino)pyridine-4-carbonitrile hydrochloride

The title compound was prepared as a pale yellow solid (102 mg) according to the aforementioned procedure described in Example 71 using 4-[(5-bromopyridin-3-yl)methyl]piperazin-2-on obtained in Reference Example 62 (110 mg) instead of (5-bromopyridin-2-yl) methanol
MS 426 (M + 1)

### Example 74 2-({4-cyclopropyl-5'-[(1,1-dioxidethiomorpholin-4-yl)methyl]-2,3'-bipyridin-6-yl}amino)pyridine-4- carbonitrile hydrochloride

The title compound was prepared as a pale yellow solid (105 mg) according to the aforementioned procedure described in Example 69 using 4-[(5-bromopyridin-3-yl)methyl]thiomorpholine 1,1-dioxide obtained in Reference Example 63 (174 mg) instead of 4-(5-bromopyridin-3-yl)-1,4-diazepane-1-carboxamide
MS 461 (M + 1)

### Example 75 4-({6-[(4-cyanopyridin-2-yl)amino]-4-cyclopropyl-2,3'-bipyridin-5'-yl}methyl])-1,4-diazepane-1-carboxamide hydrochloride

The title compound was prepared as a yellow solid (175 mg) according to the aforementioned procedure described in Example 69 using 4-[(5-bromopyridin-3-yl)methyl]-1,4-diazepane-1-carboxamide obtained in Reference Example 64 (222 mg) instead of 4-(5-bromopyridin-3-yl)-1,4-diazepane-1-carboxamide
MS 469 (M + 1)

### Example 76 4-(6-[(4-cyanopyridin-2-yl)amino]-4-cyclopropyl-2,4'-bipyridin-2'-yl}-1,4-diazepane-1-carboxamide hydrochloride

The title compound was prepared as a yellow solid (30 mg) according to the aforementioned procedure described in Example 69 using 4-(4-bromopyridin-2-yl)-1,4-diazepane-1-carboxamide obtained in Reference Example 65 (60 mg) instead of 4-(5-bromopyridin-3-yl)-1,4-diazepane-1-carboxamide
MS 455 (M + 1)

### Example 77 4-({6-[(4-cyanopyridin-2-yl)amino]-4-cyclopropyl-2,3'-bipyridin-5'-yl}amino)piperidine-1-carboxamide hydrochloride

To a mixture of 4-[(5-chloropyridin-3-yl)amino]piperidine-1-carboxamide obtained in Reference Example 66 (180 mg), Pin₂B₂ (198 mg), X-Phos (68 mg), potassium acetate (209 mg) and Pd₂(dba)₃·CHCl₃ (37 mg) was added 1,4-dioxane (3 mL), and the interior of a vessel was purged with argon. The reaction mixture was stirred at 100 °C for an hour. The reaction mixture was filtered through Celite, and the filtrate was concentrated under reduced pressure. To the resultingresulting residue was successively added 2-[(6-chloro-4-cyclopropylpyridin-2-yl)amino]pyridine-4-carbonitrile obtained in Reference Example 3 (128 mg), (oxybis (2,1-phenylene))bis(diphenylphosphine)(hereinafter referred to as DPE-Phos) (39 mg), potassium phosphate (301 mg), 1,4-dioxane (3 mL), water (1 mL) and palladium(II) acetate (11 mg), and the interior of a vessel was purged with argon. The reaction mixture was stirred at 100 °C for an hour, then the solvent was evaporated under reduced pressure. The resulting residue was purified by column chromatography to give 35 mg of the compound as a pale yellow solid. To a suspension of the obtained solid in methanol (1 mL) was added 2N hydrogen chloride-ethanol (39 µL), and the mixture was stirred at room temperature for 10 minutes. The solvent was evaporated under reduced pressure, and the obtained solid was washed with acetone to give 30 mg of the title compound as a yellow solid.
MS 455 (M + 1)

### Example 78 4-(3-{6-[(4-cyanopyridin-2-yl)amino]-4-cyclopropylpyridin-2-yl}phenyl)-1,4-diazepane-1-carboxamide hydrochloride

The title compound was prepared as a yellow solid (27 mg) according to the aforementioned procedure described in Example 69 using 4-(3-bromophenyl)-1,4-diazepane-1-carboxamide obtained in Reference Example 67 (240 mg) instead of 4-(5-bromopyridin-3-yl)-1,4-diazepane-1-carboxamide
MS 454 (M + 1)

### Example 79 2-[(4-cyclopropyl-6-{3-[(3-oxopiperazin-1-yl)methyl]phenyl]pyridin-2-yl)amino]pyridine-4-carbonitrile hydrochloride

The title compound was prepared as a yellow solid (47 mg) according to the aforementioned procedure described in Example 69 using 4-(3-bromobenzyl)piperazin-2-on obtained in Reference Example 68 (130 mg) instead of 4-(5-bromopyridin-3-yl)-1,4-diazepane-1-carboxamide
MS 425 (M + 1)

### Example 80 2-[(4-cyclopropyl-6-{4-[(3-oxopiperazin-1-yl)methyl]phenyl}pyridin-2-yl]amino]pyridine-4-carbonitrile hydrochloride

The title compound was prepared as a yellow solid (60 mg) according to the aforementioned procedure described in Example 69 using 4-(4-bromobenzyl)piperazin-2-on obtained in Reference Example 68 (130 mg) instead of 4-(5-bromopyridin-3-yl)-1,4-diazepane-1-carboxamide
MS 425 (M + 1)

### Example 81 2-{[4-cyclopropyl-5'-(piperazin-1-ylmethyl)-2,3'-bipyridin-6-yl]amino}pyridine-4-carbonitrile hydrochloride

### Step 1 Production of tert-Butyl 4-({6-[(tert-butoxycarbonyl)(4-cyanopyridin-2-yl)amino]-4-cyclopropyl-2,3'-bipyridine-5'-yl}methyl)piperazine-1-carboxylate

To a mixture of tert-butyl 4-[(5-bromopyridin-3-yl)methyl]piperazine-1-carboxylate obtained in Reference Example 70 (860 mg), Pin₂B₂ (644 mg), potassium acetate (711 mg) and PdCl₂(dppf)·CH₂Cl₂ (99 mg), was added 1,4-dioxane (15 mL) and the interior of a vessel was purged with argon. The reaction mixture was stirred at 100 °C for an hour. The reaction mixture was filtered through Celite, and the filtrate was concentrated under reduced pressure. To the resultingresulting residue was successively added tert-butyl (6-chloro-4-cyclopropylpyridin-2-yl) (4-cyanopyridin-2-yl) carbamate obtained in Reference Example 4 (746 mg), DPE-Phos (163 mg), potassium phosphate (1.28 g), 1,4-dioxane (15 mL), water (5 mL), palladium(II) acetate (46 mg), and the interior of a vessel was purged with argon. The reaction mixture was stirred at 100 °C for an hour. The reaction mixture was concentrated under reduced pressure and the resulting residue was purified by column chromatography to give 700 mg of the compound as a pale yellow amorphous form.

### Step 2 Production of 2-{[4-cyclopropyl-5'-(piperazin-1-ylmethyl)-2,3'-bipyridine-6-yl]amino}pyridine-4-carbonitrile

To tert-butyl 4-({6-[(tert-butoxycarbonyl)(4-cyanopyridin-2-yl)amino]-4-cyclopropyl-2,3'-bipyridine-5'-yl}methyl)piperazin-1-carboxylate obtained in Example 81 Step 1 (700 mg) were successively added acetonitrile-methanol (10 : 1, 5.5 mL) and methanesulfonic acid (1.49 mL) and the mixture was stirred at room temperature for an hour. The reaction mixture was neutralized with 10% aqueous sodium hydroxide solution, and the aqueous layer was extracted 3 times with chloroform-methanol (5 : 1). The solvent was evaporated under reduced pressure and the resulting residue was purified by column chromatography to give 430 mg of the compound as a pale yellow solid.

### Step 3 Production of 2-{[4-cyclopropyl-5'-(piperazin-1-ylmethyl)-2,3'-bipyridin-6-yl] amino}pyridine-4-carbonitrile hydrochloride

To a suspension of 2-{[4-cyclopropyl-5'-(piperazin-1-ylmethyl)-2,3'-bipyridine-6-yl]amino}pyridine-4-carbonitrile obtained in Example 81 Step 2 (54 mg) in methanol (1 mL) was added 2N hydrogen chloride-ethanol (66 µL), and the mixture was stirred at room temperature for 10 minutes. The solvent was evaporated under reduced pressure, and the obtained solid was washed with acetone to give 45 mg of the title compound as a pale yellow solid.
MS 412 (M + 1)

### Example 82 2-({5'-[(4-acetylpiperazin-1-yl)methyl]-4-cyclopropyl-2,3'-bipyridin-6-yl}amino)pyridine-4-carbonitrile hydrochloride

To a solution of 2-{[4-cyclopropyl-5'-(piperazin-1-ylmethyl)-2,3'-bipyridine-6-yl]amino}pyridine-4-carbonitrile obtained in Refence Example 81 Step 2 (60 mg) in dichloromethane-THF (1 : 1, 2 mL) was successively added triethylamine (41 µL) and acetyl chloride (13 µL), and the reaction mixture was stirred at room temperature for an hour. .The mixture was diluted with chloroform, and the reaction solution was directly purified by column chromatography to give 65 mg of the compound as a white solid. To a suspension of the obtained solid in methanol (1 mL) was added 2N hydrogen chloride-ethanol (72 µL), and the mixture was stirred at room temperature for 10 minutes. The solvent was evaporated under reduced pressure, and the obtained solid was washed with acetone to give 60 mg of the title compound as a pale yellow solid.
MS 454 (M + 1)

### Example 83 4-({6-[(4-cyanopyridin-2-yl)amino]-4-cylopropyl-2,3'-bipyridin-5'-yl}methyl)piperazine-1-carboxamide hydrochloride

To 2-{[4-cyclopropyl-5'-(piperazin-1-ylmethyl)-2,3'-bipyridine-6-yl]amino}pyridine-4-carbonitrile obtained in Example 81 Step 2 (83 mg) was successively added ethanol (1 mL), potassium cyanate (50 mg) and acetic acid (172 µL) and the mixture was stirred at room temperature for 17 hours. Saturated aqueous sodium hydrogen carbonate solution was added to the mixture and the mixture was extracted with chloroform. The solvent was evaporated under reduced pressure and the resulting residue was purified by column chromatography to give 104 mg of the compound as a pale yellow solid. To a suspension of the obtained solid in methanol (2 mL) was added 2N hydrogen chloride-ethanol (114 µL), and the mixture was stirred at room temperature for 10 minutes. The solvent was evaporated under reduced pressure, and the obtained solid was washed with acetone to give 80 mg of the title compound as a white solid.
MS 455 (M + 1)

### Example 84 2-({4-cyclopropyl-6-[3-(piperazin-1-ylmethyl) phenyl]pyridin-2-yl}amino)pyridine-4-carbonitrile hydrochloride

### Step 1 Production of tert-butyl 4-(3-{6-[(tert-butoxycarbonyl)(4-cyanopyridin-2-yl)amino]-4-cyclopropylpyridin-2-yl}benzyl)piperazine-1-carboxylate

The title compound was prepared as a yellow oil (155 mg) according to the aforementioned procedure described in Example 71 Step 1 using tert- butyl 4-(3-bromobenzyl)piperazine-1-carboxylate obtained in Reference Example 71 (125 mg) instead of (5-bromopyridin-2-yl) methanol

### Step 2 Production of 2-({4-cyclopropyl-6-[3-(piperazin-1-ylmethyl) phenyl]pyridin-2-yl}amino)pyridine-4-carbonitrile hydrochloride

The title compound was prepared as a pale yellow solid (38 mg) according to the aforementioned procedure described in Example 81 Step 2 and 3 using tert-butyl 4-(3-{6-[(tert-butoxycarbonyl)(4-cyanopyridin-2-yl)amino]-4-cyclopropylpyridin-2-yl}benzyl)piperazine-1-carboxylate obtained in Reference Example 84 Step 1 (155 mg) instead of tert-butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 411 (M + 1)

### Example 85 2-{[4cyclopropyl-5'-(piperidin-4-ylamino)-2,3'-bipyridin-6-yl]amino}pyridine-4-carbonitrile hydrochloride

### Step 1 Production of tert-butyl 4-({6-[(tert-butoxycarbonyl)(4-cyanopyridin-2-yl)amino]-4-cyclopropyl-2,3'-bipyridine-5'-yl}amino)piperidine-1-carboxylate

The title compound was prepared as a brown oil (120 mg) according to the aforementioned procedure described in Example 71 Step 1 using tert- butyl 4-[(5-bromopyridin-3-yl)amino]piperidine-1-carboxylate obtained in Reference Example 72 (130 mg) instead of (5-bromopyridin-2-yl) methanol

### Step 2 Production of 2-{[4cyclopropyl-5'-(piperidin-4-ylamino)-2,3'-bipyridin-6-yl]amino}pyridine-4-carbonitrile hydrochloride

The title compound was prepared as a pale yellow solid (40 mg) according to the aforementioned procedure described in Example 81 Step 2 and 3 using tert-Butyl 4-({6-[(tert-butoxycarbonyl)(4-cyanopyridin-2-yl)amino]-4-cyclopropyl-2,3'-bipyridine-5'-yl}amino)piperidine-1-carboxylate obtained in Example 85 Step 1 (120 mg) instead of tert-Butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 412 (M + 1)

### Example 86 2-{[4-cyclopropyl-2'-(piperazin-1-ylmethyl)-2,4'-bipyridin-6-yl]amino}pyridine-4-carbonitrile hydrochloride

### Step 1 Production of tert-butyl 4-({6-[(tert-butoxycarbonyl)(4-cyanopyridin-2-yl)amino]-4-cyclopropyl-2,4'-bipyridin-2'-yl}methyl)piperazin-1-carboxylate

The title compound was prepared as a yellow amorphous form (175 mg) according to the aforementioned procedure described in Example 71 Step 1 using tert-butyl 4-[(4-bromopyridin-2-yl)methyl]piperazine-1-carboxylate obtained in Reference Example 73 (193 mg) instead of (5-bromopyridin-2-yl) methanol

### Step 2 Production of 2-{[4-cyclopropyl-2'-(piperazin-1-ylmethyl)-2,4'-bipyridin-6-yl]amino}pyridine-4-carbonitrile hydrochloride

The title compound was prepared as a pale yellow solid (95 mg) according to the aforementioned procedure described in Example 81 Step 2 and 3 using tert-butyl 4-({6-[(tert-butoxycarbonyl)(4-cyanopyridin-2-yl)amino]-4-cyclopropyl-2,4'-bipyridin-2'-yl}methyl)piperazin-1-carboxylate obtained in Example 86 Step 1 (175 mg) instead of tert-butyl 4-({6-[(tert-butoxycarbonyl)(4-cyanopyridin-2-yl)amino]-4-cyclopropyl-2,3'-bipyridine-5'-yl}methyl)piperazin-1-carboxylate
MS 412 (M + 1)

### Example 87 2-({4-cyclopropyl-2'-[(3-oxopiperazin-1-yl)methyl]-2,4'-bipyridin-6-yl}amino)pyridine-4-carbonitrile hydrochloride

### Step 1 Production of tert-butyl (4-cyanopyridin-2-yl){4-cyclopropyl-2'-[(3-oxopiperazin-1-yl)methyl]-2,4'-bipyridin-6- yl}carbamate

The title compound was prepared as a pale brown oil (105 mg) according to the aforementioned procedure described in Example 71 Step 1 using 4-[(4-bromopyridine-2-yl)methyl]piperazin-2-on obtained in Reference Example 74 (138 mg) instead of (5-bromopyridin-2-yl) methanol.

### Step 2 Production of 2-({4-cyclopropyl-2'-[(3-oxopiperazin-1-yl)methyl]-2,4'-bipyridin-6-yl}amino)pyridine-4-carbonitrile hydrochloride

The title compound was prepared as a pale yellow solid (35 mg) according to the aforementioned procedure described in Example 70 Step 2 using tert-butyl (4-cyanopyridin-2-yl){4-cyclopropyl-2'-[(3-oxopiperazin-1-yl)methyl]-2,4'-bipyridin-6-yl}carbamate obtained in Reference Example 87 Step 1 (105 mg) instead of tert-butyl (4-cyanopyridin-2-yl){4-cyclopropyl-6'-[(3-oxopiperazin-1-yl)methyl]-2,3'-bipyridin-6-yl}carbamate
MS 426 (M + 1)

### Example 88 2-({4-cyclopropyl-5'-[(3R)-pyrrolidin-3-ylamino]-2,3'-bipyridin-6-yl}amino)pyridine-4-carbonitrile hydrochloride

### Step 1 Production of tert-butyl (3R)-3-({6-[(tert-butoxycarbonyl)(4-cyanopyridin-2-yl)amino]-4-cyclopropyl-2,3'-bipyridin-5'-yl}amino)pyrrolidine-1-carboxylate

The The title compound was prepared as a pale brown amorphous form (120 mg) according to the aforementioned procedure described in Example 71 Step 1 using tert-butyl (3R)-3-[(5-bromopyridin-3-yl)amino]pyrrolidine-1-carboxylate obtained in Reference Example 75 (165 mg) instead of (5-bromopyridin-2-yl) methanol.

### Step 2 Production of 2-({4-cyclopropyl-5'-[(3R)-pyrrolidin-3-ylamino]-2,3'-bipyridin-6-yl}amino)pyridine-4-carbonitrile hydrochloride

The title compound was prepared as a pale yellow solid (22 mg) according to the aforementioned procedure described in Example 81 Step 2 and 3 using tert-butyl (3R)-3-({6-[(tert-butoxycarbonyl)(4-cyanopyridin-2-yl)amino]-4-cyclopropyl-2,3'-bipyridin-5'-yl}amino)pyrrolidine-1-carboxylate obtained in Example 88 Step 1 (120 mg) instead of tert-butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 398 (M + 1)

### Example 89 2-({4-cyclopropyl-5'-[(3S)-pyrrolidin-3-ylamino]-2,3'-bipyridin-6-yl}amino)pyridine-4-carbonitrile hydrochloride

### Step 1 Production of tert-butyl (3S)-3-({6-[(tert-butoxycarbonyl)(4-cyanopyridin-2-yl)amino]-4-cyclopropyl-2,3'-bipyridin-5'-yl}amino)pyrrolidine-1-carboxylate

The title compound was prepared as a pale yellow amorphous form (80 mg) according to the aforementioned procedure described in Example 71 Step 1 using tert-butyl (3S)-3-[(5-bromopyridin-3-yl)amino]pyrrolidine-1-carboxylate obtained in Reference Example 75 (125 mg) instead of (5-bromopyridin-2-yl) methanol.

### Step 2 Production of 2-({4-cyclopropyl-5'-[(3S)-pyrrolidin-3-ylamino]-2,3'-bipyridin-6-yl}amino)pyridine-4-carbonitrile hydrochloride

The title compound was prepared as a pale yellow solid (21 mg) according to the aforementioned procedure described in Example 81 Step 2 and 3 using tert-butyl (3S)-3-({6-[(tert-butoxycarbonyl)(4-cyanopyridin-2-yl)amino]-4-cyclopropyl-2,3'-bipyridin-5'-yl}amino)pyrrolidine-1-carboxylate obtained in Example 89 Step 1 (80 mg) instead of tert-butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 398 (M + 1)

### Example 90 2-({5'-[(2-aminoethyl)amino]-4-cyclopropyl-2,3'-bipyridin-6-yl}amino)pyridine-4-carbonitrile hydrochloride

### Step 1 Production of tert-butyl [5'-({2-[(tert-butoxycarbonyl)aminolethyl}amino)-4-cyclopropyl-2,3'-bipyridin-6-yl](4-cyanopyridin-2-yl)carbamate

The title compound was prepared as a yellow amorphous form (106 mg) according to the aforementioned procedure described in Example 71 Step 1 using tert-butyl {2-[(5-bromopyridin-3-yl)amino]ethyl}carbamate obtained in Reference Example 77 (180 mg) instead of (5-bromopyridin-2-yl) methanol.

### Step 2 Production of 2-({5'-[(2-aminoethyl)amino]-4-cyclopropyl-2,3'-bipyridin-6-yl}amino)pyridine-4-carbonitrile hydrochloride

The title compound was prepared as a pale yellow solid (36 mg) according to the aforementioned procedure described in Example 81 Step 2 and 3 using tert-butyl [5'-({2-[(tert-butoxycarbonyl)amino]ethyl}amino)-4-cyclopropyl-2,3'-bipyridin-6-yl](4-cyanopyridin-2-yl)carbamate obtained in Example 90 Step 1 (106 mg) instead of tert-butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 372 (M + 1)

### Example 91 2-({4-cyclopropyl-5'-[(piperidin-4-yloxy)methyl]-2,3'-bipyridin-6-yl}amino)pyridine-4-carbonitrile hydrochloride

### Step 1 Production of tert-butyl 4-({6-[(tert-butoxycarbonyl)(4-cyanopyridin-2-yl)amino]-4-cyclopropyl-2,3'-bipyridin-5'-yl}methoxy)piperidine-1-carboxylate

The title compound was prepared as a pale yellow amorphous form (200 mg) according to the aforementioned procedure described in Example 71 Step 1 using tert-butyl 4-[(5-bromopyridin-3-yl)methoxy]piperidine-1-carboxylate obtained in Reference Example 78 (146 mg) instead of (5-bromopyridin-2-yl) methanol.

### Step 2 Production of 2-({4-cyclopropyl-5'-[(piperidin-4-yloxy)methyl]-2,3'-bipyridin-6-yl}amino)pyridine-4-carbonitrile hydrochloride

The title compound was prepared as a pale yellow solid (60 mg) according to the aforementioned procedure described in Example 81 Step 2 and 3 using tert-butyl 4-({6-[(tert-butoxycarbonyl)(4-cyanopyridin-2-yl)amino]-4-cyclopropyl-2,3'-bipyridin-5'-yl}methoxy)piperidine-1-carboxylate obtained in Example 91 Step 1 (200 mg) instead of tert-bButyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 427 (M + 1)

### Example 92 2-[(4-cyclopropyl-5'-{[N-methyl-N-(piperidin-4-yl)amino]methyl}-2,3'-bipyridin-6-yl)amino]pyridine-4-carbonitrile hydrochloride

### Step 1 Production of tert-butyl 4-[{6-[((tert-butoxycarbonyl)(4-cyanopyridin-2-yl)amino]-4-cyclopropyl-2,3'-bipyridin-5'-yl}methyl)(methyl)amino]piperidine-1-carboxylate

The title compound was prepared as a pale yellow amorphous form (275 mg) according to the aforementioned procedure described in Example 71 Step 1 using tert-butyl 4-{[(5-bromopyridin-3-yl)methyl](methyl)amino}piperidine-1- carboxylate obtained in Reference Example 79 (228 mg) instead of (5-bromopyridin-2-yl) methanol.

### Step 2 Production of 2-[(4-cyclopropyl-5'-{[N-methyl-N-(piperidin-4-yl)amino]methyl}-2,3'-bipyridin-6-yl)amino]pyridine-4- carbonitrile hydrochloride

The title compound was prepared as a pale yellow solid (136 mg) according to the aforementioned procedure described in Example 81 Step 2 and 3 using tert-butyl 4-[{6-[((tert-butoxycarbonyl)(4-cyanopyridin-2-yl)amino]-4-cyclopropyl- 2,3'-bipyridin-5'-yl}methyl)(methyl)amino]piperidine-1-carboxylate obtained in Example 92 Step 1 (275 mg) instead of tert-butyl 4-({6-[(tert-butoxycarbonyl)(4-cyanopyridin-2-yl)amino]-4-cyclopropyl-2,3'-bipyridine-5'-yl}methyl)piperazine-1-carboxylate
MS 440 (M + 1)

### Example 93 2-({4-cyclopropyl-5'-[(piperidin-4-ylamino)methyl]-2,3'-bipyridin-6-yl}amino)pyridine-4-carbonitrile hydrochloride

### Step 1 Production of tert-butyl 4-[(tert-butoxycarbonyl)({6-[(tert-butoxycarbonyl)(4-cyanopyridin-2-yl)amino]-4-cyclopropyl-2,3'-bipyridin-5'-yl}methyl)amino]piperidine-1-carboxylate

The title compound was prepared as a pale yellow amorphous form (315 mg) according to the aforementioned procedure described in Example 71 Step 1 using tert-butyl 4-{[(5-bromopyridin-3-yl)methyl](tert-butoxycarbonyl)amino}piperidine -1-carboxylate obtained in Reference Example 80 (240 mg) instead of (5-bromopyridin-2-yl)methanol.

### Step 2 Production of 2-({4-cyclopropyl-5'-[(piperidin-4-ylamino)methyl]-2,3'-bipyridin-6-yl}amino)pyridine-4-carbonitrile hydrochloride

The title compound was prepared as a pale yellow solid (129 mg) according to the aforementioned procedure described in Example 81 Step 2 and 3 using tert-butyl 4-[(tert-butoxycarbonyl)({6-[(tert-butoxycarbonyl)(4-cyanopyridin-2-yl)amino]-4-cyclopropyl-2,3'-bipyridin-5'-yl}methyl)amino]piperidine-1-carboxylate obtained in Example 93 Step 1 (315 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 426 (M + 1)

### Example 94 2-({4-cyclopropyl-5'-[(piperidin-4-ylmethyl)amino]-2,3'-bipyridin-6-yl}amino)pyridine-4-carbonitrile hydrochloride

### Step 1 Production of tert-butyl 4-[({6-[(tert-butoxycarbonyl)(4-cyanopyridin-2-yl)amino]-4-cyclopropyl-2,3'-bipyridine-5'-yl}amino)methyl]piperidine-1-carboxylate

The title compound was prepared as a pale yellow amorphous form (120 mg) according to the aforementioned procedure described in Example 71 Step 1 using tert-butyl 4-{[(5-bromopyridin-3-yl)amino]methyl}piperidine-1-carboxylate obtained in Reference Example 81 (140 mg) instead of (5-bromopyridin-2-yl) methanol.

### Step 2 Production of 2-({4-cyclopropyl-5'-[(piperidin-4-ylmethyl)amino]-2,3'-bipyridin-6-yl}amino)pyridine-4-carbonitrile hydrochloride

The title compound was prepared as a pale yellow solid (60 mg) according to the aforementioned procedure described in Example 81 Step 2 and 3 using tert-butyl 4-[({6-[(tert-butoxycarbonyl)(4-cyanopyridin-2-yl)amino]-4-cyclopropyl-2,3'-bipyridine-5'-yl}amino)methyl]piperidine-1-carboxylate obtained in Example 94 Step 1 (120 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 426 (M + 1)

### Example 95 2-{[5'-(azetidin-3-ylamino)-4-cyclopropyl-2,3'-bipyridin-6-yl]amino}pyridine-4-carbonitrile hydrochloride

### Step 1 Production of tert-butyl 3-({6-[(tert-butoxycarbonyl)(4-cyanopyridin-2-yl)amino]-4-cyclopropyl-2,3'-bipyridine-5'-yl}amino)azetidine -1-carboxylate

The title compound was prepared as a pale brown amorphous form (182 mg) according to the aforementioned procedure described in Example 71 Step 1 using tert-butyl 3-[(5-bromopyridin-3-yl)amino]azetidine-1-carboxylate obtained in Reference Example 82 (310 mg) instead of (5-bromopyridin-2-yl) methanol.

### Step 2 Production of 2-{[5'-(azetidin-3-ylamino)-4-cyclopropyl-2,3'-bipyridin-6-yl]amino}pyridine-4-carbonitrile hydrochloride

The title compound was prepared as a pale yellow solid (63 mg) according to the aforementioned procedure described in Example 81 Step 2 and 3 using tert-butyl 3-({6-[(tert-butoxycarbonyl)(4-cyanopyridin-2-yl)amino]-4-cyclopropyl-2,3'-bipyridine-5'-yl}amino)azetidine -1-carboxylate obtained in Example 95 Step 1 (182 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 384 (M + 1)

### Example 96 2-{[4-cyclopropyl-5'-(piperidin-4-yloxy)-2,3'-bipyridin-6-yl]amino}pyridine-4-carbonitrile hydrochloride

### Step 1 Production of tert-butyl 4-({6-[(tert-butoxycarbonyl)(4-cyanopyridin-2-yl)amino]-4-cyclopropyl-2,3'-bipyridine-5'-yl}oxy)piperidine-1-carboxylate

The title compound was prepared as a pale brown amorphous form (200 mg) according to the aforementioned procedure described in Example 71 Step 1 using tert-butyl 4-[(5-bromopyridin-3-yl)oxylpiperidine-1-carboxylate obtained in Reference Example 83 (140 mg) instead of (5-bromopyridin-2-yl) methanol.

### Step 2 Production of 2-{[4-cyclopropyl-5'-(piperidin-4-yloxy)-2,3'-bipyridin-6-yl]amino}pyridine-4-carbonitrile hydrochloride

The title compound was prepared as a pale yellow solid (82 mg) according to the aforementioned procedure described in Example 81 Step 2 and Step 3 using tert-butyl 4-({6-[(tert-butoxycarbonyl)(4-cyanopyridin-2-yl)amino]-4-cyclopropyl-2,3'-bipyridine-5'-yl}oxy)piperidine-1-carboxylate obtained in Example 96 Step 1 (200 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 413 (M + 1)

### Example 97 2-[(4-cyclopropyl-5'-{[(3S)-pyrrolidin-3-ylmethyl]amino}-2,3'-bipyridin-6-yl)amino]pyridine-4-carbonitrile hydrochloride

### Step 1 tert-butyl (3R)-3-[({6-[(tert-butoxycarbonyl)(4-cyanopyridin-2-yl)amino]-4-cyclopropyl-2,3'-bipyridin-5'-yl}amino)methyl]pyrrolidine-1- carboxylate

The title compound was prepared as a pale yellow amorphous form (167 g) according to the aforementioned procedure described in Example 71 Step 1 using tert-butyl (3R)-3-{[(5-bromopyridin-3-yl)amino]methyl}pyrrolidine-1-carboxylate obtained in Reference Example 84 (240 mg) instead of (5-bromopyridin-2-yl) methanol.

### Step 2 Production of 2-[(4-cyclopropyl-5'-{[(3S)-pyrrolidin-3-ylmethyl]amino}-2,3'-bipyridin-6-yl)amino]pyridine-4-carbonitrile hydrochloride

The title compound was prepared as a pale yellow solid (73 mg) according to the aforementioned procedure described in Example 81 Step 2 and 3 using tert-butyl (3R)-3-[({6-[(tert-butoxycarbonyl)(4-cyanopyridin-2-yl)amino]-4-cyclopropyl-2,3'-bipyridin-5'-yl}amino)methyl]pyrrolidine-1-carboxylate obtained in Example 97 Step 1 (167 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 412 (M + 1)

### Example 98 2-{[4-cyclopropyl-5'-(1,4-diazepan-1-yl)-2,3'-bipyridin-6-yl]amino}pyridine-4-carbonitrile hydrochloride

### Step 1 Production of tert-butyl 4-{6-[(4-cyanopyridin-2-yl)amino]-4-cyclopropyl-2,3'-bipyridin-5'-yl}-1,4-diazepane-1-carboxylate

To a mixture of tert-butyl 4-(5-bromopyridin-3-yl)-1,4-diazepane-1-carboxylate obtained in Reference Example 85 (245 mg), Pin₂B₂ (192 mg), X-Phos (66 mg), potassium acetate (200 mg) and Pd₂(dba)₃·CHCl₃ (36 mg) was added 1,4-dioxane (3 mL), and the interior of a vessel was purged with argon. The reaction mixture was stirred at 100 °C for 2 hours. To the reaction mixture were successively added 2-[(6-chloro-4-cyclopropylpyridin-2-yl)amino]pyridine-4-carbonitrile obtained in Reference Example 3 (125 mg) and a solution of potassium phosphate (292 mg) in water (1 mL) and the mixture was further stirred at 100 °C for 2 hours. The solvent was evaporated under reduced pressure and the resulting residue was purified by column chromatography to give 94 mg of the compound as a pale yellow solid.

### Step 2 Production of 2-{[4-cyclopropyl-5'-(1,4-diazepan-1-yl)-2,3'-bipyridin-6-yl]amino}pyridine-4-carbonitrile hydrochloride

The title compound was prepared as a pale yellow solid (63 mg) according to the aforementioned procedure described in Example 81 Step 2 and 3 using tert-butyl 4-{6-[(4-cyanopyridin-2-yl)amino]-4-cyclopropyl-2,3'-bipyridin-5'-yl}-1,4-diazepane-1-carboxylate obtained in Example 98 Step 1 (94 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 412 (M + 1)

### Example 99 2-({4-cyclopropyl-5'-[(1-methylpiperidin-4-yl)oxy]-2,3'-bipyridin-6-yl}amino)pyridine-4-carbonitrile hydrochloride

The title compound was prepared as a pale yellow solid (68 mg) according to the aforementioned procedure described in Example 69 using 3-bromo-5-[(1-methylpiperidin-4-yl)oxy]pyridine obtained in Reference Example 86 (268 mg) instead of 4-(5-bromopyridin-3-yl)-1,4-diazepane-1-carboxamide
MS 427 (M + 1)

### Example 100 2-({4-cyclopropyl-5'-[2-(piperazin-1-yl)ethoxy]-2,3'-bipyridin-6-yl}amino)pyridine-4-carbonitrile hydrochloride

### Step 1 Production of tert-butyl 4-[2-({6-[(tert-butoxycarbonyl)(4-cyanopyridin-2-yl)amino]-4-cyclopropyl-2,3'-bipyridin-5'-yl}oxy)-ethyl]piperazine-1-carboxylate

The title compound was prepared as a pale yellow amorphous form (483 g) according to the aforementioned procedure described in Example 71 Step 1 using tert-butyl 4-{2-[(5-bromopyridin-3-yl)-oxy]ethyl}piperazine-1-carboxylate obtained in Reference Example 87 (490 mg) instead of (5-bromopyridin-2-yl) methanol.

### Step 2 2-({4-cyclopropyl-5'-[2-(piperazin-1-yl)ethoxy]-2,3'-bipyridin-6-yl}amino)pyridine-4-carbonitrile hydrochloride

The title compound was prepared as a pale yellow solid (70 mg) according to the aforementioned procedure described in Example 81 Step 2 and 3 using tert-butyl 4-[2-({6-[(tert-butoxycarbonyl)(4-cyanopyridin-2-yl)amino]-4-cyclopropyl-2,3'-bipyridin-5'-yl}oxy)-ethyl]piperazine-1-carboxylate obtained in Example 100 Step 1 (120 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 442 (M + 1)

### Example 101 2-({4-cyclopropyl-5'-[2-(morpholin-4-yl)ethoxy]-2,3'-bipyridine-6-yl}amino)pyridine-4-carbonitrile hydrochloride

The title compound was prepared as a pale yellow solid (100 mg) according to the aforementioned procedure described in Example 69 using 4-{2-[(5-bromopyridin-3-yl)-oxy]ethyl}morpholine obtained in Reference Example 88 (182 mg) instead of 4-(5-bromopyridin-3-yl)-1,4-diazepane-1-carboxamide
MS 443 (M + 1)

### Example 102 2-{[5'-(azetidin-3-yloxy)-4-cyclopropyl-2,3'-bipyridin-6-yl]amino}pyridine-4-carbonitrile hydrochloride

### Step 1 Production of tert-butyl 3-({6-[(tert-butoxycarbonyl)(4-cyanopyridin-2-yl)amino]-4-cyclopropyl-2,3'-bipyridin-5'-yl}oxy)azetidine-1-carboxylate

The title compound was prepared as a yellow oil (267 g) according to the aforementioned procedure described in Example 71 Step 1 using tert-butyl 3-[(5-bromopyridin-3-yl)oxy]azetidine-1-carboxylate obtained in Reference Example 89 (150 mg) instead of (5-bromopyridin-2-yl) methanol.

### Step 2 Production of 2-{[5'-(azetidin-3-yloxy)-4-cyclopropyl-2,3'-bipyridin-6-yl]amino}pyridine-4-carbonitrile hydrochloride

The title compound was prepared as a yellow solid (20 mg) according to the aforementioned procedure described in Example 81 Step 2 and 3 using tert-butyl 3-({6-[(tert-butoxycarbonyl)(4-cyanopyridin-2-yl)amino]-4-cyclopropyl-2,3'-bipyridin-5'-yl}oxy)azetidine-1-carboxylate obtained in Example 102 Step 1 (267 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 385 (M + 1)

### Example 103 2-[4-({6-[(4-cyanopyridin-2-yl)amino]-4-cyclopropyl-2,3'-bipyridine-5'-yl}oxy)-piperidin-1-yl]acetamide hydrochloride

### Step 1 Production of tert-butyl (5'-{[1-(2-amino-2-oxoethyl)piperidin-4-yl}oxy}-4-cyclopropyl-2,3'-bipyridin-6-yl)(4-cyanopyridin-2-yl)carbamate

The title compound was prepared as a yellow oil (234 g) according to the aforementioned procedure described in Example 71 Step 1 using 2-{4-[(5-bromopyridin-3-yl)oxy]piperidin-1-yl}acetamide obtained in Reference Example 90 (164 mg) instead of (5-bromopyridin-2-yl) methanol.

### Step 2 Production of 2-[4-({6-[(4-cyanopyridin-2-yl)amino]-4-cyclopropyl-2,3'-bipyridine-5'-yl}oxy)-piperidin-1-yl]acetamide hydrochloride

The title compound was prepared as a pale yellow solid (130 mg) according to the aforementioned procedure described in Example 81 Step 2 and 3 using tert-butyl (5'-{[1-(2-amino-2-oxoethyl)piperidin-4-yl)oxy}-4-cyclopropyl-2,3'-bipyridin-6-yl)(4-cyanopyridin-2-yl)carbamate obtained in Example 103 Step 1 (234 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 470 (M + 1)

### Example 104 4-({6-[(4-cyanopyridin-2-yl)amino]-4-cyclopropyl-2,3'-bipyridin-5'-yl}oxy)piperidine-1-carboxamide hydrochloride

### Step 1 Production of tert-butyl {5'-[(1-carbamoylpiperidin-4-yl)oxy]-4-cyclopropyl-2,3'-bipyridin-6-yl}(4-cyanopyridin-2-yl)carbamate

The title compound was prepared as a white amorphous form (230 g) according to the aforementioned procedure described in Example 71 Step 1 using 4-[(5-bromopyridin-3-yl)oxy]piperidine-1-carboxamide obtained in Reference Example 91 (193 mg) instead of (5-bromopyridin-2-yl) methanol.

### Step 2 Production of 4-({6-[(4-cyanopyridin-2-yl)amino]-4-cyclopropyl-2,3'-bipyridin-5'-yl}oxy)piperidine-1-carboxamide hydrochloride

The title compound was prepared as a yellow solid (88 mg) according to the aforementioned procedure described in Example 81 Step 2 and 3 using tert-butyl {5'-[(1-carbamoylpiperidin-4-yl)oxy]-4-cyclopropyl-2,3'-bipyridin-6-yl}(4-cyanopyridin-2-yl)carbamate obtained in Example 104 Step 1 (229 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate
MS 448 (M + 1)

### Example 105 2-({4-cyclopropyl-6-[2-(1,1-dioxidethiomorpholin-4-yl)pyrimidin-5-yl]pyridin-2-yl}amino)pyridine-4-carbonitrile hydrochloride

The title compound was prepared as a pale yellow solid (140 mg) according to the aforementioned procedure described in Example 4 using 4-(5-bromopyrimidin-2-yl)thiomorpholine 1,1-dioxide obtained in Reference Example 92 (180 mg)
MS 456 (M + 1)

### Example 106 4-(5-{6-[(4-cyanopyridin-2-yl)amino]-4-cyclopropylpyridin-2-yl}pyrimidin-2-yl)-1,4-diazepane-1-carboxamide hydrochloride

The title compound was prepared as a pale yellow solid (63 mg) according to the aforementioned procedure described in Example 4 using 4-(5-bromopyrimidin-2-yl)-1,4-diazepane-1-carboxamide obtained in Reference Example 92 (166 mg)
MS 456 (M + 1)

### Example 107 2-({4-cyclopropyl-5'-[2-(dimethylamino)ethoxy]-2,3'-bipyridin-6-yl}amino)pyridine-4-carbonitrile hydrochloride

The title compound was prepared as a pale yellow solid (130 mg) according to the aforementioned procedure described in Example 69 using 2-[(5-bromopyridin-3-yl)oxy]-N,N-dimethylethaneamine obtained in Reference Example 94 (130 mg) instead of 4-(5-bromopyridin-3-yl)-1,4-diazepane-1-carboxamide
MS 401 (M + 1)

### Example 108 2-({4-cyclopropyl-5'-[2-(dimethylamino)-2-methylpropoxy]-2,3'-bipyridin-6-yl}amino)pyridine-4-carbonitrile hydrochloride

The title compound was prepared as a pale yellow solid (85 mg) according to the aforementioned procedure described in Example 69 using 1-[(5-chloropyridin-3-yl)oxy]-N,N, 2-trimethylpropan-2-amine obtained in Reference Example 95 (130 mg) instead of 4-(5-bromopyridin-3-yl)-1,4-diazepane-1-carboxamide MS 429 (M + 1)

### Example 109 2-({6-[(4-cyanopyridin-2-yl)amino]-4-cyclopropyl-2,3'-bipyridin-5'-yl}oxy)acetamide hydrochloride

### Step 1 Production of tert-butyl [5'-(2-amino-2-oxoethoxy)-4-cyclopropyl-2,3'-bipyridin-6-yl](4-cyanopyridin-2-yl)carbamate

The title compound was prepared as a yellow amorphous form (110 mg) according to the aforementioned procedure described in Example 71 Step 1 using 2-[(5-bromopyridin-3-yl)oxy]acetamide obtained in Reference Example 96 (72 mg) instead of (5-bromopyridin-2-yl) methanol.

### Step 2 Production of 2-({6-[(4-cyanopyridin-2-yl)amino]-4-cyclopropyl-2,3'-bipyridin-5'-yl}oxy)acetamide hydrochloride

The title compound was prepared as a yellow solid (30 mg) according to the aforementioned procedure described in Example 81 Step 2 and 3 using tert-butyl [5'-(2-amino-2-oxoethoxy)-4-cyclopropyl-2,3'-bipyridin-6-yl](4-cyanopyridin-2-yl)carbamate obtained in Example 109 Step 1 (110 mg) instead of tert-butyl 4-({6-[(tert-butoxycarbonyl)(4-cyanopyridin-2-yl)amino]-4-cyclopropyl-2,3'-bipyridin-5'-yl}methyl)piperazine-1-carboxylate MS 387 (M + 1)

### Example 110 2-{[5'-(4-acetyl-1,4-diazepan-1-yl)-4-cyclopropyl-2,3'-bipyridin-6-yl]amino}pyridine-4-carbonitrile hydrochloride

The title compound was prepared as a yellow solid (56 mg) according to the aforementioned procedure described in Example 69 using 1-[4-(5-bromopyridin-3-yl)-1,4-diazepan-1-yl]ethanone obtained in Reference Example 97 (145 mg) instead of 4-(5-bromopyridin-3-yl)-1,4-diazepane-1-carboxamide
MS 454 (M + 1)

### Example 111 2-{[4-cyclopropyl-5'-(3-hydroxypyrrolidin-1-yl)-2,3'-bipyridin-6-yl]amino}pyridine-4-carbonitrile hydrochloride

### Step 1 Production of tert-butyl [5'-(3-{[tert-butyl(dimethyl)silyl]oxy}pyrrolidin-1-yl)-4-cyclopropyl-2,3'-bipyridin-6-yl](4-cyanopyridine-2-yl)carbamate

The title compound was prepared as a brown oil (149 mg) according to the aforementioned procedure described in Example 71 Step 1 using 3-bromo-5-(3-{[tert-butyl(dimethyl)silyl]oxy}pyrrolidin-1-yl)pyridine obtained in Reference Example 98 (116 mg) instead of (5-bromopyridin-2-yl) methanol.

### Step 2 Production of 2-{[4-cyclopropyl-5'-(3-hydroxypyrrolidin-1-yl)-2,3'-bipyridin-6-yl]amino}pyridine-4-carbonitrile hydrochloride

To tert-butyl [5'-(3-{[tert-butyl(dimethyl)silyl]oxy}pyrrolidin-1-yl)-4-cyclopropyl-2,3'-bipyridine-6-yl](4-cyanopyridine-2-yl)carbamate obtained in Example 111 Step 1 (147 mg) was successively added acetonitrile (1.5 mL) and methanesulfonic acid (78 µL) and the mixture was stirred at room temperature for 22 hours. Saturated aqueous sodium hydrogen carbonate solution was added to the reaction mixture and the precipitated solid was collected by filtration and washed with water and methanol to give 52 mg of the compound as a yellow solid. To a suspension of the obtained solid in methanol (1 mL) was added 1N aqueous hydrochloric acid solution (137 µL). The solvent was evaporated under reduced pressure, and the obtained solid was washed with acetone-ethanol (1 : 1) to give 39 mg of the title compound as a yellow solid.
MS 399 (M + 1)

### Example 112 2-({4-cyclopropyl-5'-[(3S)-3-hydroxypiperidin-1-yl]-2,3'-bipyridin-6-yl}amino)pyridine-4-carbonitrile hydrochloride

The title compound was prepared as a yellow solid (53 mg) according to the aforementioned procedure described in Example 111 using 3-bromo-5-[(3S)-3-{[tert-butyl(dimethyl)silyl]ox}piperidin-1-yl]pyridine obtained in Reference Example 99 (131 mg) instead of 3-bromo-5-(3-{[tert-butyl(dimethyl)silyl]oxy}pyrrolidin-1-yl)pyridine.
MS 413 (M + 1)

### Example 113 2-({4-cyclopropyl-5'-[(3R)-3-hydroxypiperidin-1-yl]-2,3'-bipyridin-6-yl}amino)pyridine-4-carbonitrile hydrochloride

The title compound was prepared as a pale yellow solid (43 mg) according to the aforementioned procedure described in Example 111 using 3-bromo-5-[(3R)-3-{[tert-butyl(dimethyl)silyl]oxy}piperidin-1-yl]pyridine obtained in Reference Example 100 (131 mg) instead of 3-bromo-5-(3-{[tert-butyl(dimethyl)silyl]oxy}pyrrolidin-1-yl)pyridine.
MS 413 (M + 1)

### Example 114 2-{[4-cyclopropyl-5'-(1-methyl-1H-pyrazol-4-yl)-2,3'-bipyridin-6-yl]amino}pyridine-4-carbonitrile hydrochloride

The title compound was prepared as a pale yellow solid (84 mg) according to the aforementioned procedure described in Example 111 using 3-bromo-5-(1-methyl-1H-pyrazol-4-yl)pyridine obtained in Reference Example 101 (78 mg) instead of 3-bromo-5-(3-{[tert-butyl(dimethyl)silyl]oxy}pyrrolidin-1-yl)pyridine.
MS 394 (M + 1)

### Example 115 2-{[4-cyclopropyl-5'-(3-oxopiperazin-1-yl)-2,3'-bipyridin-6-yl]amino}pyridine-4-carbonitrile

### Step 1 Production of tert-butyl 4-{6-[(tert-butoxycarbonyl)(4-cyanopyridin-2-yl)amino]-4-cyclopropyl - 2,3'-bipyridin-5'-yl}-2-oxopiperazin-1-carboxylate

The title compound was prepared as a yellow oil (33 mg) according to the aforementioned procedure described in Example 71 Step 1 using tert-butyl 4-(5-bromopyridin-3-yl)-2-oxopiperazine-1-carboxylate obtained in Reference Example 102 (73 mg) instead of (5-bromopyridin-2-yl) methanol.

### Step 2 Production of 2-{[4-cyclopropyl-5'-(3-oxopiperazin-1-yl)-2,3'-bipyridin-6-yl]amino}pyridine-4-carbonitrile

To tert-butyl 4-{6-[(tert-butoxycarbonyl)(4-cyanopyridin-2-yl)amino]-4-cyclopropyl -2,3'-bipyridin-5'-yl}-2-oxopiperazin-1-carboxylate obtained in Example 115 Step 1 (32 mg) were successively added acetonitrile (1 mL)and methanesulfonic acid (34 µL) and the mixture was stirred at room temperature for 17 hours. Saturated aqueous sodium hydrogen carbonate solution was added to the mixture and extracted with chloroform two times. The organic laye was dried over magnesium sulfate and the solvent was evaporated under reduced pressure. The resulting residue was washed with methanol to give 9 mg of the title compound as a white solid.
MS 412 (M + 1)

### Example 116 2{[4-cyclopropyl-5'-(4-hydroxypiperidin-1-yl)-2,3'-bipyridin-6-yl]amino}pyridine-4-carbonitrile hydrochloride

The title compound was prepared as yellow solid (67 mg) according to the aforementioned procedure described in Example 111 using 3-bromo-5-(4-{[tert-butyl(dimethyl)silyl]oxy}piperidin-1-yl)pyridine obtained in Reference Example 103 (120 mg) instead of 3-bromo-5-(3-{[tert-butyl(dimethyl)silyl]oxy}pyrrolidin-1-yl)pyridine.
MS 413 (M + 1)
Elementary analysis as C₂₄H₂₄N₆O·HCl + 1H₂O
Calcd. (%) C: 61.73.; H: 5.83.; N: 18.00
Found. (%) C: 61.78.;H: 5.97.; N: 17.67

### Example 117 2-({4-cyclopropyl-6-[1-(1,3-dihydroxypropan-2-yl)-1H-benzimidazol-5-yl)pyridin-2-yl}amino) pyridine-4-carbonitrile hydrochloride

### Step 1 Production of 2-{[4-cyclopropyl-6-(1-{1,3-bis[tert-butyl(dimethyl)siloxy]propan-2-yl}-1H-benzimidazol-5-yl)pyridin-2-yl]amino}pyridine-4-carbonitrile

To a mixture of 5-bromo-1-{1,3-bis[tert-butyl(dimethyl)siloxy]propan-2-yl}-1H-benzimidazole obtained in Reference Example 104 (284 mg), Pin₂B₂ (187 mg) and potassium acetate (279 mg), PdCl₂(dppf)·CH₂Cl₂ (23 mg) were added 1,4-dioxane (3 mL) and the interior of a vessel was purged with argon. The reaction mixture was stirred at 100 °C for 6 hours. The reaction mixture was filtered through Celite and the filtrate was concentrated under reduced pressure. To the resulting residue was successively added 2-[(6-chloro-4-cyclopropylpyridin-2-yl)amino]pyridine-4-carbonitrile obtained in Reference Example 3 (100 mg), DPE-Phos (71 mg), potassium phosphate (278 mg), 1,4-dioxane (3 mL), water (1 mL), palladium(II) acetate (29 mg) and the interior of a vessel was purged with argon. The reaction mixture was stirred at 100 °C for 2 hours. The reaction mixture was filtered through Celite and the filtrate was concentrated under reduced pressure. The resulting residue was purified by column chromatography to give 132 mg of the compound as a white solid.

### Step 2 Production of 2-({4-cyclopropyl-6-[1-(1,3-dihydroxypropan-2-yl)-1H-benzimidazol-5-yl)pyridin-2-yl}amino) pyridine-4-carbonitrile hydrochloride

To 2-{[4-cyclopropyl-6-(1-{1,3-bis[tert-butyl(dimethyl)siloxy]propan-2-yl}-1H-benzimidazol-5-yl)pyridin-2-yl]amino}pyridine-4-carbonitrile obtained in Example 117 Step 1 (132 mg) were successively added methanol (4.5 mL) and methanesulfonic acid (194 mg) and the mixture was stirred at room temperature for 2 hours. Saturated aqueous sodium hydrogen carbonate solution was added to the mixture and extracted with ethyl acetate. The organic layer was washed with saturated brine, dried over magnesium sulfate and the solvent was evaporated under reduced pressure. The resulting residue was successively washed with dichloromethane, ethyl acetate to give 62 mg of the compound as a yellow solid. To the obtained solid was added chloroform-methanol (1 : 1) and 1N aqueous hydrochloric acid solution (145 µL). The solvent was evaporated under reduced pressure. The residue was subjected to azeotropic distillation with ethyl acetate. The obtained solid was washed with diethyl ether to give 64 mg of the title compound as a pale yellow solid.
MS 427 (M + 1)

### Example 118 2-({4-cyclopropyl-6-[1-(2-hydroxy-2-methylpropyl)-1H-benzimidazol-5-yl)pyridin-2-yl}amino)pyridine-4-carbonitrile hydrochloride

To a mixture of 2-Methyl-1-[5-(4,4,5,5-tetramethyl-1,3,2-dioxaborolan-2-yl)-1H-benzimidazol-1-yl]propan-2-ol obtained in Reference Example 105 (228 mg), 2-[(6-chloro-4-cyclopropylpyridin-2-yl)amino]pyridine-4-carbonitrile obtained in Reference Example 3 (150 mg), DPE-Phos (89 mg) and potassium phosphate (352 mg), was successively added 1,4-dioxane (4.5 mL), water (1.5 mL) and palladium(II) acetate (29 mg) and the interior of a vessel was purged with argon. The reaction mixture was stirred at 100 °C for an hour. The reaction mixture was filtered through Celite and the filtrate was concentrated under reduced pressure. The resulting residue was purified by column chromatography to give 55 mg of the compound as a yellow solid. To the obtained solid was added chloroform-methanol (1 : 1) and 1N aqueous hydrochloric acid solution (130 µL). The solvent was evaporated under reduced pressure. The residue was subjected to azeotropic distillation with ethyl acetate. The obtained solid was washed with diethyl ether to give 54 mg of the title compound as a pale yellow solid.
MS 425 (M + 1)

### Example 119 2-({4-cyclopropyl-6-[1-(3-hydroxy-2,2-dimethylpropyl)-1H-benzimidazol-5-yl)pyridin-2-yl}amino)pyridine-4-carbonitrile hydrochloride

### Step 1 tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridine - 2-yl) carbamate

To a mixture of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole obtained in Reference Example 106 (106 mg), tert-butyl___(6-chloro-4-cyclopropylpyridin-2-yl) (4-cyanopyridin-2-yl) carbamate obtained in Reference Example 4 (80 mg), DPE-Phos (17 mg) and potassium phosphate (138 mg) were successively added 1,4-dioxane (1.6 mL), water (0.4 mL) and palladium(II) acetate (4.8 mg) and the interior of a vessel was purged with argon. The reaction mixture was stirred at 100 °C for 2 hours. The reaction mixture was filtered through Celite and the filtrate was concentrated under reduced pressure. The resulting residue was purified by column chromatography to give 113 mg of the title compound as a yellow oil.

### Step 2 2-({4-cyclopropyl-6-[1-(3-hydroxy-2,2-dimethylpropyl)-1H-benzimidazol-5-yl)pyridin-2-yl}amino)pyridine-4-carbonitrile hydrochloride

To tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate obtained in Example 119 Step 1 (113 mg) were successively added acetonitrile-methanol (10 : 1, 0.55 mL) and methanesulfonic acid (83 mg) and the mixture was stirred at room temperature overnight. The reaction mixture was added with water (2 mL) at 0°C and neutralized with 2N aqueous sodium hydroxide solution. The reaction mixture was stirred at 0 °C for an hour and the precipitated solid was collected by filtration. The obtained solid was purified by column chromatography to give 41 mg of the compound as a yellow solid. To the obtained solid were added chloroform-methanol (1 : 1) and 1N aqueous hydrochloric acid solution (93 µL). The mixture was evaporated under reduced pressure and subjected to azeotropic distillation with ethyl acetate. The obtained solid was washed with diethyl ether to give 37 mg of the title compound as a pale yellow solid.
MS 439 (M + 1)

### Example 120 2-[(4-cyclopropyl-6-{1-[(1-hydroxycyclohexyl)methyl]-1H-benzimidazol-5-yl}pyridin-2-yl]amino] pyridine-4-carbonitrile hydrochloride

The title compound was prepared as yellow solid (69 mg) according to the aforementioned procedure described in Example 119 using 1-{[5-(4,4,5,5-tetramethyl-1,3,2-dioxaborolan-2-yl)-1H-benzimidazol-1-yl]methyl}cyclohexanol obtained in Reference Example 107 (85 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 465 (M + 1)

### Example 121 2-({4-cyclopropyl-6-[1-(3-hydroxypropyl)-1H-benzimidazol-5-yl)pyridin-2-yl}amino)pyridine-4-carbonitrile hydrochloride

The title compound was prepared as yellow solid (41 mg) according to the aforementioned procedure described in Example 119 using 1-(3-{[tert-butyl(dimethyl)silyl]oxy}propyl)-5-(4,4,5,5-tetramethyl-1,3,2- dioxaborolan-2-yl)-1H-benzimidazole obtained in Reference Example 108 (99 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 411 (M + 1)

### Example 122 2-({4-cyclopropyl-6-[1-(trans-4-hydroxycyclohexyl)-1H-benzimidazol-6-yl)pyridin-2-yl}amino)pyridine-4- carbonitrile hydrochloride

The title compound was prepared as pale yellow solid (13 mg) according to the aforementioned procedure described in Example 119 using 1-(trans-4-{[tert-butyl(dimethyl)silyl]oxy}cyclohexyl)-6-(4,4,5,5-tetramethyl-1,3,2-dioxaborolan-2-yl)-1H-benzimidazole obtained in Reference Example 109 (116 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 451 (M + 1)

### Example 123 ethyl 3-(5-{6-[(4-cyanopyridin-2-yl)amino]-4-cyclopropylpyridin-2-yl}-1H-benzimidazol-1-yl)propanonate hydrochloride

### Step 1 Production of ethyl 3-(5-{6-[(tert-butoxycarbonyl)(4-cyanopyridin-2-yl)amino]-4-cyclopropyl pyridin-2-yl}-1H-benzimidazol-1-yl)propanonate

To a mixture of ethyl 3-[5-(4,4,5,5-tetramethyl-1,3,2-dioxaborolan-2-yl)-1H-benzimidazol-1-yl]propanonate obtained in Reference Example 110 (55 mg), tert-butyl (6-chloro-4-cyclopropylpyridin-2-yl)(4-cyanopyridin-2-yl) carbamate obtained in Reference Example 4 (54 mg), DPE-Phos (12 mg), potassium phosphate (92 mg), were successively added 1,4-dioxane (2 mL) and palladium(II) acetate (3 mg) and the interior of a vessel was purged with argon. The reaction mixture was stirred at 100 °C for 2 hours. The reaction mixture was filtered through Celite and the filtrate was concentrated under reduced pressure. The resulting residue was purified by column chromatography to give 33 mg of the compound as a yellow amorphous form.

### Step 2 Production of ethyl 3-(5-{6-[(4-cyanopyridine-2-yl)amino]-4-cyclopropylpyridin-2-yl}-1H-benzimidazol-1-yl)propanonate hydrochloride

To ethyl 3-(5-{6-[(tert-butoxycarbonyl)(4-cyanopyridin-2-yl)amino]-4-cyclopropyl pyridin-2-yl}-1H-benzimidazol-1-yl)propanonate obtained in Example 123 Step 1 (33 mg) were successively added acetonitrile-methanol (10 : 1, 2.2 mL) and methanesulfonic acid (114 mg) and the mixture was stirred at room temperature overnight. Water (2.0 mL) was added to the mixture at 0 °C, neutralized with 4N aqueous sodium hydroxide solution. The mixture was extracted with ethyl acetate and the organic layer was washed with saturated brine, dried over magnesium sulfate. The solvent was evaporated under reduced pressure and the obtained solid was purified by column chromatography to give 17 mg of the compound as a yellow solid. To the obtained solid was added chloroform-methanol (1 : 1) and 1N aqueous hydrochloric acid solution (38 µL). The mixture was evaporated under reduced pressure and subjected to azeotropic distillation with ethyl acetate. The obtained solid was washed with diethylether to give 10 mg of the title compound as a pale yellow solid.
MS 453 (M + 1)

### Example 124 2-({4-cyclopropyl-6-[1-(3-hydroxypropyl)-1H-benzimidazol-6-yl)] pyridin-2-yl}amino)pyridine-4-carbonitrile hydrochloride

The title compound was prepared as pale yellow solid (85 mg) according to the aforementioned procedure described in Example 119 using 1-(3-{[tert-butyl(dimethyl)silyl]oxy}propyl)-6-(4,4,5,5-tetramethyl-1,3,2-dioxaborolan-2-yl)-1H-benzimidazole obtained in Reference Example 111 (219 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 411 (M + 1)

### Example 125 3-(6-{6-[(4-cyanopyridin-2-yl)amino]-4-cyclopropylpyridin-2-yl}-1H-benzimidazol-1-yl)-N,N-dimethylpropanamide hydrochloride

### Step 1 Production of 2-(trimethylsilyl)ethyl 3-(6-{6-[(tert-butoxycarbonyl)(4-cyanopyridin-2-yl) amino]-4-cyclopropylpyridin-2-yl}-1H-benzimidazol-1-yl)propanonate

To a mixture of 2-(trimethylsilyl)ethyl 3-[6-(4,4,5,5-tetramethyl-1,3,2-dioxaborolan-2-yl)-1H-benzimidazol-1-yl]propanonate obtained in Reference Example 112 (131 mg), tert-butyl (6-chloro-4-cyclopropylpyridin-2-yl)(4-cyanopyridin-2-yl) carbamate obtained in Reference Example 4 (106 mg), S-Phos (54 mg) and potassium phosphate (182 mg), were successively added 1,4-dioxane (4 mL), water (1 mL), Pd₂(dba)₃·(26 mg) and the interior of a vessel was purged with argon. The reaction mixture was stirred at 100 °C for an hour. The reaction mixture was filtered through Celite and the filtrate was concentrated under reduced pressure. The resulting residue was purified by column chromatography to give 130 mg of the compound as a brown oil.

### Step 2 Production of tert-butyl (4-cyanopyridin-2-yl)(4-cyclopropyl-6-{1-[3-(dimethylamino)-3-oxopropyl]-1H-benzimidazol-6-yl}pyridin-2-yl)carbamate

To a solution of 2-(trimethylsilyl)ethyl 3-(6-{6-[(tert-butoxycarbonyl)(4-cyanopyridin-2-yl) amino]-4-cyclopropylpyridin-2-yl}-1H-benzimidazol-1-yl)propanonate obtained in Example 125 Step 1 (130 mg) in THF (4 mL) was added tetrabutylammonium fluoride (416 µL, 1M solution in THF), and the mixture was stirred at room temperature for 4 hours. The solvent was evaporated under reduced pressure To a mixture of the resulting residue and HOBt (42 mg) in dichloromethane (3 mL) was successively added triethylamine (105 mg), dimethylamine hydrochloride (34 mg) and WSCD·HCl (60 mg) and the mixture was stirred at room temperature overnight. The solvent was evaporated under reduced pressure and the resulting residue was purified by column chromatography to give 48 mg of the title compound as a pale yellow amorphous form.

### Step 3 Production of 3-(6-{6-[(4-cyanopyridin-2-yl)amino]-4-cyclopropylpyridin-2-yl}-1H-benzimidazol-1-yl)-N,N-dimethylpropanamide hydrochloride

The title compound was prepared as pale yellow solid (13 mg) according to the aforementioned procedure described in Example 119 Step 2 using tert-butyl (4-cyanopyridin-2-yl)(4-cyclopropyl-6-{1-[3-(dimethylamino)-3-oxopropyl]-1H-benzimidazol-6-yl}pyridin-2-yl)carbamate obtained in Example 125 Step 2 (48 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 452 (M + 1)

### Example 126 methyl 3-(6-{6-[(4-cyanopyridin-2-yl)amino]-4-cyclopropylpyridin-2-yl}-1H-benzimidazol-1-yl) propanonate hydrochloride

To 2-(trimethylsilyl)ethyl 3-(6-{6-[(tert-butoxycarbonyl)(4-cyanopyridin-2-yl) amino]-4-cyclopropylpyridin-2-yl}-1H-benzimidazol-1-yl)propanonate obtained in Example 125 Step 1 (150 mg) was successively added acetonitrile-methanol (10 : 1, 2.2 mL) and methanesulfonic acid (231 mg) and the mixture was stirred at room temperature overnight. Water (4 mL) was added to the mixture at 0 °C, neutralized with 2N aqueous sodium hydroxide solution (pH7). The reaction mixture was stirred at 0 °C for an hour and the precipitated solid was collected by filtration, washed with water. The obtained yellow solid was purified by column chromatography to give 30 mg of the compound as a white solid. To the obtained solid were added chloroform-methanol (1 : 1) and 1N aqueous hydrochloric acid solution (69 µL). The solvent was evaporated under reduced pressure and subjected to azeotropic distillation with ethyl acetate. The obtained solid was washed with diethylether to give 21 mg of the title compound as a pale yellow solid.
MS 439 (M + 1)

### Example 127 2-({4-cyclopropyl-6-[1-(4-hydroxybutyl)-1H-benzimidazol-6-yl]pyridin-2-yl}amino)pyridine-4-carbonitrile hydrochlroride

The title compound was prepared as pale yellow solid (3 mg) according to the aforementioned procedure described in Example 119 using 1-(4-{[tert-butyl(dimethyl)silyl]oxy}butyl)-6-(4,4,5,5-tetramethyl-1,3,2-dioxaborolan-2-yl)-1H-benzimidazole obtained in Reference Example 113 (112 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 425 (M + 1)

### Example 128 2-({4-[1-cyclopropyl-6-(4-hydroxybutyl)-1H-benzimidazol-5-yl)pyridin-2-yl}amino)pyridine-4-carbonitrile hydrochloride

The title compound was prepared as pale yellow solid (74 mg) according to the aforementioned procedure described in Example 119 using 1-(4-{[tert-butyl(dimethyl)silyl]oxy}butyl)-5-(4,4,5,5-tetramethyl-1,3,2-dioxaborolan-2-yl)-1H-benzimidazole obtained in Reference Example 114 (206 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 425 (M + 1)

### Example 129 3-(5-{6-[(4-cyanopyridin-2-yl)amino]-4-cyclopropylpyridin-2-yl}-1H-benzimidazol-1-yl)propanamide hydrochloride

### Step 1 Production of 2-(trimethylsilyl)ethyl 3-(5-{6-[(tert-butoxycarbonyl)(4-cyanopyridin-2-yl)amino]-4-cyclopropylpyridin-2-yl}-1H-benzimidazol-1-yl)propanonate

The title compound was prepared as brown amorphous form (722 mg) according to the aforementioned procedure described in Example 125 Step 1 using 2-(trimethylsilyl)ethyl 3-[5-(4,4,5,5-tetramethyl-1,3,2-dioxaborolan-2-yl)-1H-benzimidazol-1-yl]propanonate obtained in Reference Example 115 (693 mg) instead of 3-[5-(4,4,5,5-tetramethyl-1,3,2-dioxaborolan-2-yl)-1H-benzimidazol-1-yl]propanenitrile.

### Step 2 Production of 3-(5-{6-[(tert-butoxycarbonyl)(4-cyanopyridin-2-yl)amino]-4-cyclopropylpyridin-2-yl}-1H-benzimidazol-1-yl)propanoic acid

To a solution of 2-(trimethylsilyl)ethyl 3-(5-{6-[(tert-butoxycarbonyl)(4-cyanopyridin-2-yl)amino]-4-cyclopropylpyridin-2-yl}-1H-benzimidazol-1-yl)propanonate obtained in Example 129 Step 1 (150 mg) in THF (4 mL) was added tetrabutylammonium fluoride (480 µL, 1M solution in THF) and the mixture was stirred at room temperature for 3.5 hours. The solvent was evaporated under reduced pressure and the resulting residue was purified by column chromatography to give 81 mg of the compound as a pale yellow amorphous form.

### Step 3 Production of tert-butyl {6-[1-(3-amino-3-oxopropyl)-1H-benzimidazol-5-yl]-4-cyclopropylpyridin-2-yl}(4-cyanopyridin-2-yl) carbamate

To a suspension of 3-(5-{6-[(tert-butoxycarbonyl)(4-cyanopyridin-2-yl)amino]-4-cyclopropylpyridin-2-yl}-1H-benzimidazol-1-yl)propanoic acid obtained in Example 129 Step 2 (81 mg) and HOBt (31 mg) in dichloromethane (2 mL) was successively added triethylamine (234 mg), ammonium chloride (82 mg) and WSCD·HCl (44 mg) and the mixture was stirred at room temperature for 2 hours. Triethylamine (234 mg), ammonium chloride (82 mg) was further added and the mixture was stirred at room temperature for 2 days. The solvent was evaporated under reduced pressure and the resulting residue was purified by column chromatography to give 49 mg of the compound as a white amorphous form.

### Step 4 Production of 3-(5-{6-[(4-cyanopyridin-2-yl)amino]-4-cyclopropylpyridin-2-yl}-1H-benzimidazol-1-yl)propanamide hydrochloride

The title compound was prepared as pale yellow solid (24 mg) according to the aforementioned procedure described in Example 119 Step 2 using tert-butyl {6-[1-(3-amino-3-oxopropyl)-1H-benzimidazol-5-yl]-4-cyclopropylpyridin-2-yl}(4-cyanopyridin-2-yl) carbamate obtained in Example 129 Step 3 (49 mg) instead of tert-butyl {6-[1-(3-{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 424 (M + 1)

### Example 130 2-({4-cyclopropyl-6-[1-(pyridin-3-ylmethyl)-1H-benzimidazol-6-yl]pyridin-2-yl}amino)pyridine-4-carbonitrile hydrochloride

The title compound was prepared as pale yellow solid (75 mg) according to the aforementioned procedure described in Example 119 using 1-(pyridin-3-ylmethyl)-6-(4,4,5,5-tetramethyl-1,3,2-dioxaborolan-2-yl)-1H-benzimidazole obtained in Reference Example 116 (107 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 444 (M + 1)

### Example 131 3-(5-{6-[(4-cyanopyridin-2-yl)amino]-4-cyclopropylpyridin-2-yl}-1H-benzimidazol-1-yl)-N,N-dimethylpropanamide hydrochloride

### Step 1 Production of tert-butyl (4-cyanopyridin-2-yl)(4-cyclopropyl-6-{1-[3-(dimethylamino)-3-oxopropyl]-1H-benzimidazol-5-yl}pyridin-2-yl)carbamate

The title compound was prepared as white amorphous form (40 mg) according to the aforementioned procedure described in Example 129 Step 3 using dimethylamine hydrochloride (70 mg) instead of ammonium chloride.

### Step 2 Production of 3-(5-{6-[(4-cyanopyridin-2-yl)amino]-4-cyclopropylpyridin-2-yl}-1H-benzimidazol-1-yl)-N,N- dimethylpropanamide hydrochloride

To tert-butyl (4-cyanopyridin-2-yl)(4-cyclopropyl-6-{1-[3-(dimethylamino)-3-oxopropyl]-1H-benzimidazol-5-yl}pyridine-2-yl)carbamate obtained in Example 131 Step 1 (40 mg) were successively added acetonitrile (1 mL) and methanesulfonic acid (70 mg) and the mixture was stirred at room temperature for 3 hours. Water (1 mL) was added to the mixture at 0 °C, neutralized with 2N aqueous sodium hydroxide solution and extracted with ethyl acetate. The organic layer was washed with saturated brine, dried over magnesium sulfate and the solvent was evaporated under reduced pressure. The obtained solid was purified by column chromatography to give 27 mg of the compound as a yellow solid. To the obtained solid was added chloroform-methanol (1 : 1) and 1N aqueous hydrochloric acid solution (60 µL). The solvent was evaporated under reduced pressure and subjected to azeotropic distillation with ethyl acetate. The obtained solid was washed with diethylether to give 28 mg of the title compound as a pale yellow solid.
MS 439 (M + 1)

### Example 132 Production of 3-(5-{6-[(4-cyanopyridin-2-yl)amino]-4-cyclopropylpyridin-2-yl}-1H-benzimidazol-1-yl)-N-methylpropanamide hydrochloride

### Step 1 Production of tert-butyl (4-cyanopyridin-2-yl)(4-cyclopropyl-6-{1-[3-(methylamino)-3-oxo propyl]-1H-benzimidazol-5-yl} pyridin-2-yl)carbamate

The title compound was prepared as yelow amorphous form (72 mg) according to the aforementioned procedure described in Example 129 Step 3 using 2M methylamine solution in THF (429 µL) instead of ammonium chloride.

### Step 2 Production of 3-(5-{6-[(4-cyanopyridin-2-yl)amino]-4-cyclopropylpyridin-2-yl}-1H-benzimidazol-1-yl)-N-methylpropanamide hydrochloride

The title compound was prepared as pale yellow solid (8 mg) according to the aforementioned procedure described in Example 131 Step 2 using tert-butyl (4-cyanopyridin-2-yl)(4-cyclopropyl-6-{1-[3-(methylamino)-3-oxo propyl]-1H-benzimidazol-5-yl} pyridin-2-yl)carbamate obtained in Reference Example 132 Step 1 (72 mg) instead of tert-butyl (4-cyanopyridin-2-yl)(4-cyclopropyl-6-{1-[3-(dimethylamino)-3-oxopropyl]-1H-benzimidazol-5-yl}pyridine-2-yl)carbamate.
MS 438 (M + 1)

### Example 133 2-({4-cyclopropyl-6-[1-(pyridin-4-ylmethyl)-1H-benzimidazol-6-yl)pyridin-2-yl}amino)pyridine-4-carbonitrile hydrochloride

The title compound was prepared as pale yellow solid (41 mg) according to the aforementioned procedure described in Example 119 using 1-(pyridin-4-ylmethyl)-6-(4,4,5,5-tetramethyl-1,3,2-dioxaborolan-2-yl)-1H-benzimidazole obtained in Reference Example 117 (103 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 444 (M + 1)

### Example 134 2-({6-[1-(2-cyanoethyl)-1H-benzimidazol-5-yl]-4-cyclopropylpyridin-2-yl}amino)pyridine-4-carbonitrile hydrochloride

### Step 1 Production of tert-butyl {6-[1-(2-cyanoethyl)]-1H-benzimidazol-5-yl-4-cyclopropylpyridin-2-yl}(4-cyanopyridin-2-yl) carbamate

The title compound was prepared as pale yellow amorphous form (25 mg) according to the aforementioned procedure described in Example 125 Step 1 using 3-[5-(4,4,5,5-tetramethyl-1,3,2-dioxaborolan-2-yl)-1H-benzimidazol-1-yl]propanenitrile obtained in Reference Example 118 (55 mg) instead of 2-(trimethylsilyl)ethyl 3-[6-(4,4,5,5-tetramethyl-1,3,2-dioxaborolan-2-yl)-1H-benzimidazol-1-yl]propanoate.

### Step 2 Production of 2-({6-[1-(2-cyanoethyl)-1H-benzimidazol-5-yl]-4-cyclopropylpyridin-2-yl}amino)pyridine-4- carbonitrile hydrochloride

The title compound was prepared as pale yellow solid (3 mg) according to the aforementioned procedure described in Example 119 Step 2 using tert-butyl {6-[1-(2-cyanoethyl)]-1H-benzimidazol-5-yl-4-cyclopropylpyridin-2-yl}(4-cyanopyridin-2-yl) carbamate obtained in Example 134 Step 1 (45 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 406 (M + 1)

### Example 135 2-[(4-cyclopropyl-6-{1-[1-(hydroxymethyl)cyclohexyl]-1H-benzimidazol-6-yl}pyridin-2-yl)aminolpyridine-4- carbonitrile hydrochloride

The title compound was prepared as pale yellow solid (8 mg) according to the aforementioned procedure described in Example 119 using 1-[1-({[tert-butyl(dimethyl)silyl]oxy}methyl)cyclohexyl]-6-(4,4,5,5-tetramethyl-1,3,2-dioxaborolan-2-yl)-1H-benzimidazole obtained in Reference Example 119 (134 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 465 (M + 1)

### Example 136 2-({4-cyclopropyl-6-[1-(4,4-difluorocyclohexyl)-1H-benzimidazol-6-yl]pyridine-2-yl}amino)pyridine-4- carbonitrile hydrochloride

The title compound was prepared as pale yellow solid (54 mg) according to the aforementioned procedure described in Example 119 using 1-(4,4-difluorocyclohexyl)-6-(4,4,5,5-tetramethyl-1,3,2-dioxaborolan-2-yl)-1H-benzimidazole obtained in Reference Example 120 (105 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 471 (M + 1)

### Example 137 2-{[6-(1-benzyl-1H-benzimidazol-5-yl)-4-cyclopropylpyridin-2-yl]amino}pyridin-4-carbonitrile

### hydrochloride

The title compound was prepared as yellow solid (35 mg) according to the aforementioned procedure described in Example 123 using 1-benzyl-5-(4,4,5,5-tetramethyl-1,3,2-dioxaborolan-2-yl)-1H-benzimidazole obtained in Reference Example 121 (99 mg) instead of ethyl 3-[5-(4,4,5,5-tetramethyl-1,3,2-dioxaborolan-2-yl)-1H-benzimidazol-1-yl]propanonate.
MS 443 (M + 1)

### Example 138 2-({4-cyclopropyl-6-[1-(trans-4-methoxycyclohexyl)-1H-benzimidazol-6-yl)pyridin-2-yl}amino)pyridine-4-carbonitrile hydrochloride

### Step 1 Production of tert-butyl (4-cyanopyridin-2-yl){4-cyclopropyl-6-[1-(trans-4-methoxycyclohexyl)-1H-benzimidazol-6-yl]pyridin-2-yl} carbamate

To a mixture of 6-bromo-1-(trans-4-methoxycyclohexyl)-1H-benzimidazole obtained in Reference Example 122 (350 mg), Pin₂B₂ (308 mg), potassium acetate (325 mg) and PdCl₂(dppf)·CH₂Cl₂ (67 mg) was added 1,4-dioxane (6.5 mL) and the interior of a vessel was purged with argon. The reaction mixture was stirred at 100 °C for 1.5 hours. The reaction mixture was filtered through Celite and the filtrate was concentrated under reduced pressure. To the resulting residue were successively added tert-butyl_(6-chloro-4-cyclopropylpyridin-2-yl) (4-cyanopyridin-2-yl) carbamate obtained in Reference Example 4 (302 mg), DPE-Phos (72 mg), potassium phosphate (518 mg), 1,4-dioxane (5 mL), water (1.2 mL) and palladium(II) acetate (20 mg) and the interior of a vessel was purged with argon. The reaction mixture was stirred at 100 °C for 2 hours. The reaction mixture was filtered through Celite and the filtrate was concentrated under reduced pressure. The resulting residue was purified by column chromatography to give 178 mg of the title compound.

### Step 2 Production of 2-({4-cyclopropyl-6-[1-(trans-4-methoxycyclohexyl)-1H-benzimidazol-6-yl)pyridin-2-yl}amino)pyridine-4-carbonitrile hydrochloride

To tert-butyl (4-cyanopyridin-2-yl){4-cyclopropyl-6-[1-(trans-4-methoxycyclohexyl)-1H-benzimidazol-6-yl]pyridin-2-yl} carbamate obtained in Example 138 Step 1 (56 mg) was successively added acetonitrile-methanol (10 : 1, 3.3 mL) and methanesulfonic acid (1 mL) and the mixture was stirred at room for an hour. Saturated aqueous sodium hydrogen carbonate solution was added to the mixture at 0 °C and extracted with ethyl acetate. The solvent was evaporated under reduced pressure and the obtained solid was purified by column chromatography to give 36 mg of the compound as a free base. To the obtained solid was added chloroform-methanol (1 : 1, 1 mL) and 2N aqueous hydrochloric acid solution (39 µL). The solvent was evaporated under reduced pressure. The obtained solid was washed with ethyl acetate to give 25 mg of the title compound as a yellow solid.
MS 465 (M + 1)

### Examples 139 2-({4-cyclopropyl-6-[1-(tetrahydro-2H-pyran-4-yl)-1H-benzimidazol-6-yl)pyridin-2-yl}amino)pyridine -4-carbonitrile hydrochloride

The title compound was prepared as yellow solid (238 mg) according to the aforementioned procedure described in Example 138 using 6-bromo-1-(tetrahydro-2H-pyran-4-yl)-1H-benzimidazole obtained in Reference Example 123 (500 mg) instead of 6-bromo-1-(trans-4-methoxycyclohexyl)-1H-benzimidazole.
MS 437 (M + 1)

### Example 140 2-[(4-cyclopropyl-6-{1-[trans-2-hydroxycyclopentyl]-1H-benzimidazol-6-yl}pyridine-2-yl)amino]pyridine-4-carbonitrile hydrochloride

### Step 1 Production of tert-butyl (4-cyanopyridin-2-yl)(4-cyclopropyl-6-{1-[trans-2-hydroxycyclopentyl]-1H-benzimidazol-6-yl}pyridine-2-yl) carbamate

The title compound was prepared (1.27 g) according to the aforementioned procedure described in Example 138 Step 1 using trans-2-(6-bromo-1H-benzimidazol-1-yl)cyclopentanol obtained in Reference Example 124 (1.17 g) instead of 6-bromo-1-(trans-4-methoxycyclohexyl)-1H-benzimidazole.

### Step 2 Production of 2-[(4-cyclopropyl-6-{1-[trans-2-hydroxycyclopentyl]-1H-benzimidazol-6-yl}pyridine-2-yl)amino]pyridine-4-carbonitrile hydrochloride

The title compound was prepared as a yellow solid (19 mg) according to the aforementioned procedure described in Example 138 Step 2 using tert-butyl (4-cyanopyridin-2-yl)(4-cyclopropyl-6-{1-[trans-2-hydroxycyclopentyl]-1H-benzimidazol-6-yl}pyridin-2-yl) carbamate obtained in Example 140 Step 1 (40 mg) instead of tert-butyl (4-cyanopyridin-2-yl){4-cyclopropyl-6-[1-(trans-4-methoxycyclohexyl)-1H-benzimidazol-6-yl]pyridin-2-yl} carbamate.
MS 437 (M + 1)

### Example 141 trans-2-(6-{6-[(4-cyanopyridin-2-yl)amino]-4-cyclopropylpyridin-2-yl}-1H-benzimidazol-1-yl)cyclopentyl acetate hydrochloride

### Step 1 Production of trans-2-(6-{6-[(tert-butoxycarbonyl) (4-cyanopyridin-2-yl)amino]4-cyclopropylpyridin-2-yl}-1H-benzimidazol-1-yl)cyclopentyl acetate

To a solution of tert-butyl (4-cyanopyridin-2-yl)(4-cyclopropyl-6-{1-[trans-2-hydroxycyclopentyl]-1H-benzimidazol-6-yl}pyridine-2-yl) carbamate obtained in Example 140 Step 1 (54 mg), pyridine (73 µL) and DMAP (1 mg) in dichloromethane (0.5 mL) was added acetic anhydride (57 µL) and the mixture was stirred at 0 °C for 20 minutes. A saturated aqueous ammonium chloride solution was added to the reaction mixture and the mixture was extracted with ethyl acetate. The solvent was evaporated under reduced pressure and the resulting residue was purified by column chromatography to give 53 mg of the title compound.

### Step 2 Production of trans-2-(6-{6-[(4-cyanopyridin-2-yl)amino]-4-cyclopropylpyridin-2-yl}-1H-benzimidazol-1-yl)cyclopentyl acetate hydrochloride

The title compound was prepared as a yellow solid (19 mg) according to the aforementioned procedure described in Example 138 Step 2 using trans-2-(6-{6-[(tert-butoxycarbonyl) (4-cyanopyridin-2-yl)amino]-4-cyclopropylpyridin-2-yl}-1H- benzimidazol-1-yl)cyclopentyl acetate obtained in Example 141 Step 1 (53 mg) instead of tert-butyl (4-cyanopyridin-2-yl){4-cyclopropyl-6-[1-(trans-4-methoxycyclohexyl)-1H-benzimidazol-6-yl]pyridin-2-yl} carbamate.
MS 479 (M + 1)

### Example 142 Production of 2-{[4-cyclopropyl-6-(1-ethyl-1H-benzimidazol-6-yl)pyridin-2-yl]amino}pyridine-4-carbonitrile hydrochloride

The title compound was prepared as a yellow solid (116 mg) according to the aforementioned procedure described in Example 138 using 6-bromo-1-ethyl-1H-benzimidazole obtained in Reference Example 125 (162 mg) instead of 6-bromo-1-(trans-4-methoxycyclohexyl)-1H-benzimidazole.
MS 381 (M + 1)

### Example 143 2-({4-cyclopropyl-6-[1-(1,3-dihydroxypropan-2-yl)-1H-benzimidazol-6-yl]pyridin-2-yl}amino)pyridine-4-carbonitrile hydrochloride

The title compound was prepared as a yellow solid (38 mg) according to the aforementioned procedure described in Example 138 using 6-bromo-1-(1,3-bis{[tert-butyl(dimethyl)silyl]oxy}propan-2-yl)-1H-benzimidazole obtained in Reference Example 126 (345 mg) instead of 6-bromo-1-(trans-4-methoxycyclohexyl)-1H-benzimidazole.
MS 427 (M + 1)

### Example 144 2-({4-cyclopropyl-6-[1-(2-hydroxy-2-methylpropyl)-1H-benzimidazol-6-yl)pyridin-2-yl}amino)pyridine-4-carbonitrile hydrochloride

The title compound was prepared as a yellow solid (13 mg) according to the aforementioned procedure described in Example 138 using 1-(6-bromo-1H-benzimidazol-1-yl)-2-methylpropan-2-ol obtained in Reference Example 127 (194 mg) instead of 6-bromo-1-(trans-4-methoxycyclohexyl)-1H-benzimidazole.
MS 425 (M + 1)

### Example 145 2-({4-cyclopropyl-6-[1-(cis-4-hydroxycyclohexyl)-1H-benzimidazol-6-yl]pyridin-2-yl}amino)pyridine -4-carbonitrile hydrochloride

The title compound was prepared as a yellow solid (127 mg) according to the aforementioned procedure described in Example 138 using 6-bromo-1-(cis-4-{[tert-butyl(dimethyl)silyl]oxy}cyclohexyl)-1H-benzimidazole obtained in Reference Example 128 (294 mg) instead of 6-bromo-1-(trans-4-methoxycyclohexyl)-1H-benzimidazole.
MS 451 (M + 1)

### Example 146 2-[(4-cyclopropyl-6-{1-[trans-2-hydroxycyclohexyl]-1H-benzimidazol-6-yl}pyridin-2-yl]amino]pyridine-4-carbonitrile hydrochloride

The title compound was prepared as a yellow solid (6 mg) according to the aforementioned procedure described in Example 138 using trans-2-(6-bromo-1H-benzimidazol-1-yl)cyclohexanol obtained in Reference Example 129 instead of 6-bromo-1-(trans-4-methoxycyclohexyl)-1H-benzimidazole.
MS 451 (M + 1)

### Example 147 2-({4-cyclopropyl-6-[1-(2-hydroxyethyl)-1H-benzimidazol-5-yl)pyridin-2-yl}amino)pyridine-4-carbonitrile hydrochloride

### Step 1 Production of tert-butyl (4-cyanopyridin-2-yl){4-cyclopropyl-6-[1-(2-hydroxyethyl)-1H-benzimidazol-5-yl]pyridin-2-yl}carbamate

To a mixture of 2-(5-bromo-1H-benzimidazol-1-yl)ethanol obtained in Reference Example 130 (106 mg), Pin₂B₂ (145 mg), potassium acetate (216 mg) and PdCl₂(dppf)·CH₂Cl₂ (18 mg) was added 1,4-dioxane (2.2 mL) and the interior of a vessel was purged with argon and the mixture was reacted under microwave irradiation (Biotage INITIATOR, at 160 °C for 20 minutes). The reaction mixture was filtered through Celite and the filtrate was concentrated under reduced pressure. To the resulting residue was successively added 1,4-dioxane-water (3 : 1, 2.2 mL), tert-butyl (6-chloro-4-cyclopropylpyridin-2-yl) (4-cyanopyridin-2-yl) carbamate obtained in Reference Example 4 (80 mg), S-Phos (36 mg), potassium phosphate (140 mg) and palladium(II) acetate (9.9 mg) and the interior of a vessel was purged with argon. The reaction mixture was stirred at 100 °C for 3 hours. The reaction mixture was diluted with ethyl acetate, filtered through Celite and the filtrate was concentrated under reduced pressure. The resulting residue was purified by column chromatography to give 76 mg of the title compound as a yellow oil.

### Step 2 2-({4-cyclopropyl-6-[1-(2-hydroxyethyl)-1H-benzimidazol-5-yl)pyridin-2-yl}amino)pyridine-4-carbonitrile hydrochloride

To tert-butyl (4-cyanopyridin-2-yl){4-cyclopropyl-6-[1-(2-hydroxyethyl)-1H-benzimidazol-5-yl]pyridin-2-yl}carbamate obtained in Example 147 Step 1 (38 mg) was added TFA (1 mL) and the mixture was stirred at room temperature for 4 hours. The reaction mixture was neutralized with 4N aqueous sodium hydroxide solution, and extracted two times with ethyl acetate. The solvent was evaporated under reduced pressure and the resulting residue was purified by column chromatography to give 29 mg of the compound as a pale yellow oil. To a solution of the obtained solid in ethyl acetate was added 4N hydrogen chloride-ethyl acetate solution (18 µL). The precipitated solid was washed with ethyl acetate to give 5 mg of the title compound as a pale yellow solid.
MS 397 (M + 1)

### Example 148 2-({4-cyclopropyl-6-[1-(2-ethoxyethyl)-1H-benzimidazol-5-yl]pyridine-2-yl}amino)pyridin-4-carbonitrile hydrochloride

The title compound was prepared as a yellow solid (10 mg) according to the aforementioned procedure described in Example 147 using 5-bromo-1-(2-methoxyethyl)-1H-benzimidazole obtained in Reference Example 131 (65 mg) instead of 2-(5-bromo-1H-benzimidazol-1-yl)ethanol.
MS 411 (M + 1)

### Example 149 2-{[4-cyclopropyl-6-(1-ethyl-1H-benzimidazol-5-yl)pyridin-2-yl]amino}pyridine-4-carbonitrile hydrochloride

### Step 1 Production of tert-butyl(4-cyanopyridin-2-yl)[4-cyclopropyl-6-(1-ethyl-1H-benzimidazol-5-yl)pyridin-2-yl]carbamate

To a mixture of 5-bromo-1-ethyl-1H-benzimidazole obtained in Reference Example 132 (800 mg), Pin₂B₂ (1.17 g), PdCl₂(dppf)·CH₂Cl₂ (145 mg) and potassium acetate (1.7 g) was added 1,4-dioxane (17 mL) and the interior of a vessel was purged with argon. The reaction mixture was stirred at 100 °C for a day. The reaction mixture was filtered through Celite and the filtrate was concentrated under reduced pressure. To one eighth of the resulting residue were successively added 1,4-dioxane-water (3 : 1, 4 mL), tert-butyl (6-chloro-4-cyclopropylpyridin-2-yl) (4-cyanopyridin-2-yl) carbamate obtained in Reference Example 4 (82 mg), S-Phos (36 mg), potassium phosphate (140 mg) and palladium(II) acetate (10 mg) and the reaction mixture was stirred at 100 °C for 3 hours. The reaction mixture was diluted with ethyl acetate, filtered through Celite and the filtrate was concentrated under reduced pressure. The resulting residue was purified by column chromatography to give 95 mg of the title compound as a pale yellow amorphous form.

### Step 2 Production of 2-{[4-cyclopropyl-6-(1-ethyl-1H-benzimidazol-5-yl)pyridin-2-yl]amino}pyridine-4-carbonitrile hydrochloride

The title compound was prepared as a yellow solid (22 mg) according to the aforementioned procedure described in Example 147 Step 2 using tert-butyl(4-cyanopyridin-2-yl)[4-cyclopropyl-6-(1-ethyl-1H-benzimidazol-5-yl)pyridin-2-yl]carbamate obtained in Example 149 Step 1 (89 mg) instead of tert-butyl (4-cyanopyridin-2-yl){4-cyclopropyl-6-[1-(2-hydroxyethyl)-1H-benzimidazol-5-yl]pyridin-2-yl}carbamate.
MS 381 (M + 1)

### Example 150 2-{[4-cyclopropyl-6-(1-cyclopropyl-1H-benzimidazol-5-yl)pyridin-2-yl]amino}pyridin-4-carbonitrile hydrochloride

The title compound was prepared as a yellow solid (61 mg) according to the aforementioned procedure described in Example 15 using 5-bromo-1-cyclopropyl-1H-benzimidazole obtained in Refernce Example 133 (185 mg) instead of 5-bromo-1-methyl-1H-benzimidazole.
MS 393 (M + 1)

### Example 151 N-[2-(5-{6-[(4-cyanopyridin-2-yl)amino]-4-cyclopropylpyridin-2-yl}-1H-benzimidazol-1-yl)ethyl]acetamide hydrochloride

### Step 1 Production of 2-[5-(6-chloro-4-cyclopropylpyridin-2-yl)-1H-benzimidazol-1-yl]ethanamine

tert-Butyl {2-[5-(6-chloro-4-cyclopropylpyridin-2-yl)-1H-benzimidazol-1-yl]ethyl}carbamate obtained in Example 13 Step 1 (500 mg) was dissolved in TFA (3 mL) and the solution was stirred at room temperature for 4 hours. 4N aqueous sodium hydroxide solution was added to the mixture and extracted with ethyl acetate two times. The organic layer was dried over sodium sulfate and the solvent was evaporated under reduced pressure. The resulting residue was purified by column chromatography to give 273 mg of the title compound as a pale yellow oil.

### Step 2 Production of N-{2-[5-(6-chloro-4-cyclopropylpyridin-2-yl)-1H-benzimidazol-1-yl]ethyl}acetamide

To a solution of 2-[5-(6-chloro-4-cyclopropylpyridin-2-yl)-1H-benzimidazol-1-yl]ethanamine obtained in Example 151 Step 1 (91 mg) in THF (3 mL) was successively added pyridine (140 µL), acetic anhydride (83 µL) and the mixture was stirred at room temperature overnight. Saturated aqueous sodium hydrogen carbonate solution was added to the mixture, and the mixture was extracted with ethyl acetate. The solvent was evaporated under reduced pressure to give 55 mg of the title compound as a pale yellow oil.

### Step 3 Production of N-[2-(5-{6-[(4-cyanopyridin-2-yl)amino]-4-cyclopropylpyridin-2-yl}-1H-benzimidazol-1-yl)ethyl]acetamide hydrochloride

The title compound was prepared as a yellow solid (14 mg) according to the aforementioned procedure described in Example 15 Step 2 using N-{2-[5-(6-chloro-4-cyclopropylpyridin-2-yl)-1H-benzimidazol-1-yl]ethyl}acetamide obtained in Example 151 Step 2 (55 mg) instead of 5-(6-chloro-4-cyclopropylpyridin-2-yl)-1-methyl-1H-benzimidazole.
MS 438 (M + 1)

### Example 152 2-[(4-cyclopropyl-6-{1-[trans-2-hydroxycyclohexyl]-1H-benzimidazol-5-yl}pyridin-2-yl]amino]pyridine-4-carbonitrile hydrochloride

### Step 1 Production of 2-[6-{1-[trans-2-{[tert-butyl(dimethyl)silyl]oxy}cyclohexyl]-1H-benzimidazol-5-yl}-4-cyclopropylpyridin-2-yl]amino]pyridine-4-carbonitrile

To 2-[(6-chloro-4-cyclopropylpyridin-2-yl)amino]pyridine-4-carbonitrile obtained in Reference Example 3 (143 mg) and was successively added 1-[trans-2-{[tert-butyl(dimethyl)silyl]oxy}cyclohexyl]-5-(4,4,5,5-tetramethyl-1,3,2-dioxaborolan-2-yl)-1H-benzimidazole obtained in Reference Example 134 (363 mg), S-Phos (87 mg), potassium acetate (1.7 g), 1,4-dioxane (3 mL), water (1 mL) and palladium acetate (24 mg) and the interior of a vessel was purged with argon. The reaction mixture was stirred at 100 °C for 2 hours. The reaction mixture was diluted with ethyl acetate and washed with water. The organic layer was dried over magnesium sulfate and evaporated under reduced pressure. The resulting residue was purified by column chromatography to give 139 mg of the title compound as a brown oil.

### Step 2 Production of 2-[(4-cyclopropyl-6-{1-[trans-2-hydroxycyclohexyl]-1H-benzimidazol-5-yl}pyridin-2-yl]amino]pyridine-4-carbonitrile hydrochloride To

To a solution of 2-[6-{1-[trans-2-{[tert-butyl(dimethyl)silyl]oxy}cyclohexyl]-1H-benzimidazol-5-yl}-4-cyclopropylpyridin-2-yl]amino]pyridine-4-carbonitrile obtained in Example 152 Step 1 (137 mg) in THF (2 mL) was added tetrabutylammonium fluoride (486 µL, 1M solution in THF) and the mixture was stirred at 65 °C for an hour. Methanol (2 mL) was added to the mixture and the solvent was evaporated under reduced pressure. The resulting residue was purified by column chromatography to give 85 mg of the title compound as a pale yellow solid. To a suspension of the obtained solid in methanol (1 mL) was added 1N hydrogen chloride-ethanol solution (196 µL), and the mixture was stirred at room temperature for 10 minutes. The solvent was evaporated under reduced pressure, and the obtained solid was washed with acetone to give 80 mg of the title compound as a pale yellow solid.
MS 451 (M + 1)

### Example 153 2-{[4-cyclopropyl-6-(1-methyl-1H-benzimidazol-6-yl)pyridin-2-yl]amino}pyridine-4-carbonitrile hydrochloride

### Step 1 Production of tert-butyl (4-cyanopyridin-2-yl) [4-cyclopropyl-6-(1-methyl-1H-benzimidazol-6-yl)pyridine-2-yl]carbamate

To tert-butyl (6-chloro-4-cyclopropylpyridin-2-yl) (4-cyanopyridin-2-yl) carbamate obtained in Reference Example 4 (100 mg) was successively added 1-methyl-6-(4,4,5,5-tetramethyl-1,3,2-dioxaborolan-2-yl)-1H-benzimidazole obtained in Reference Example 135 (84 mg), S-Phos (44 mg), potassium phosphate (172 mg),1,4-dioxane (2.4 mL), water (0.6 mL) and palladium acetate (12 mg) and the interior of a vessel was purged with argon. The reaction mixture was stirred at 100 °C for 2.5 hours. The reaction mixture was diluted with ethyl acetate and washed with water. The organic layer was dried over magnesium sulfate and evaporated under reduced pressure. The resulting residue was purified by column chromatography to give 85 mg of the title compound as a yellow oil.

### Step 2 Production of 2-{[4-cyclopropyl-6-(1-methyl-1H-benzimidazol-6-yl)pyridin-2-yl]amino}pyridine-4-carbonitrile hydrochloride

To tert-butyl (4-cyanopyridin-2-yl) [4-cyclopropyl-6-(1-methyl-1H-benzimidazol-6-yl)pyridine-2-yl]carbamate obtained in Example 153 Step 1 (98 mg) was added TFA (1 mL) and the mixture was stirred at room temperature for an hour. The solvent was vaporated under reduced pressure. Saturated aqueous sodium hydrogen carbonate solution was added to the mixture and the mixture was stirred at room tempperature for an hour. The precipitated solid was filtered and washed with water to give 79 mg of the compound as a pale yellow solid. To a suspension of the obtained solid was successively added methanol and 1N hydrogen chloride-ethanol solution (216 µL), and the mixture was stirred at room temperature for 10 minutes. The solvent was evaporated under reduced pressure, and the obtained solid was washed with acetone-ethanol (10 : 1) to give 63 mg of the title compound as a pale yellow solid.
MS 367 (M + 1)

### Example 154 4-({6-[(4-cyanopyridine-2-yl)amino]-4-cyclopropyl-2,3'-bipyridin-6'-yl}methyl)-1,4-diazepane-1-carboxamide hydrochloride

The title compound was prepared as a pale yellow solid (97 mg) according to the aforementioned procedure described in Example 4 using 4-[(5-bromopyridin-2-yl)methyl]-1,4-diazepane-1-carboxamide obtained in Reference Example 59 (160 mg) instead of 1-(5-bromopyridin-2-yl)piperidin-4-on
MS 469 (M + 1)

### Example 155 2-{[4-cyclopropyl-6'-(1,1-dioxidethiomorpholin-4-yl)-4'-methyl-2,3'-bipyridin-6-yl]amino}pyridine-4-carbonitrile hydrochloride

### Step 1 Production of tert-butyl (4-cyanopyridin-2-yl)[4-cyclopropyl-6'-(1,1- dioxidethiomorpholin-4-yl)-4'-methyl-2,3'-bipyridin-6-yl]carbamate

To a mixture of 4-(5-bromo-4-methylpyridin-2-yl)thiomorpholine 1,1-dioxide obtained in Reference Example 136 (265 mg), Pin₂B₂ (265 mg), X-Phos (83 mg), potassium acetate (255 mg) and palladium acetate (19 mg) was added 1,4-dioxane (5 mL) and the interior of a vessel was purged with argon. The reaction mixture was stirred at 100 °C for 2 hours. To the reaction mixture was successively added tert-butyl (6-chloro-4-cyclopropylpyridin-2-yl) (4-cyanopyridin-2-yl) carbamate obtained in Reference Example 4 (321 mg) and a solution of potassium phosphate (552 mg) in water (3 mL), and the mixture was stirred at 100 °C for 2 hours. The reaction mixture was filtered through Celite and the filtrate was concentrated under reduced pressure. The resulting residue was purified by column chromatography to give 159 mg of the title compound as a yellow oil.

### Step 2 Production of 2-{[4-cyclopropyl-6'-(1,1-dioxidethiomorpholin-4-yl)-4'-methyl-2,3'-bipyridin-6-yl]amino}pyridine-4-carbonitrile hydrochloride

The title compound was prepared as yellow solid (44 mg) according to the aforementioned procedure described in Example 119 Step 2 using tert-butyl (4-cyanopyridin-2-yl)[4-cyclopropyl-6'-(1,1- dioxidethiomorpholin-4-yl)-4'-methyl-2,3'-bipyridin-6-yl]carbamate obtained in Example 155 Step 1 (159 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 461 (M + 1)

### Example 156 2-({4-cyclopropyl-6-[3-(trans-4-hydroxycyclohexyl)-2-oxo-2,3-dihydro-1H-benzimidazol-5-yl] pyridin- 2-yl}amino)pyridine-4-carbonitrile hydrochloride

### Step 1 Production of tert-butyl {6-[3-(trans-4-{[tert-butyl(dimethyl)silyl]oxy}cyclohexyl)-2-oxo-2,3-dihydro-1H-benzimidazol-5-yl]-4-cyclopropylpyridin-2-yl}(4-cyanopyridin-2-yl)carbamate

The title compound was prepared as pale brown solid (98 mg) according to the aforementioned procedure described in Example 125 Step 1 using 1-(trans-4-{[tert-butyl(dimethyl)silyl]oxy}cyclohexyl)-6-(4,4,5,5-tetramethyl-1,3,2-dioxaborolan-2-yl)-1,3-dihydro-2H-benzimidazol-2-one obtained in Reference Example 137 (121 mg) instead of 2-(trimethylsilyl)ethyl 3-[6-(4,4,5,5-tetramethyl-1,3,2-dioxaborolan-2-yl)-1H-benzimidazol-1-yl]propanoate.

### Step 2 Production of 2-({4-cyclopropyl-6-[3-(trans-4-hydroxycyclohexyl)-2-oxo-2,3-dihydro-1H-benzimidazol-5-yl] pyridin- 2-yl}amino)pyridine-4-carbonitrile hydrochloride

The title compound was prepared as pale yellow solid (9 mg) according to the aforementioned procedure described in Example 119 Step 2 using tert-butyl {6-[3-(trans-4-{[tert-butyl(dimethyl)silyl]oxy}cyclohexyl)-2-oxo-2,3-dihydro-1H-benzimidazol-5-yl]-4-cyclopropylpyridin-2-yl}(4-cyanopyridin-2-yl)carbamate obtained in Example 156 Step 1 (98 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 467 (M + 1)

### Example 157 2-({4-cyclopropyl-6-[2-ethyl-1-(trans-4-hydroxycyclohexyl)-1H-benzimidazol-6-yl)pyridin-2-yl} amino)pyridine-4-carbonitrile hydrochloride

The title compound was prepared as yellow solid (112 mg) according to the aforementioned procedure described in Example 119 using 1- (trans-4-{[tert-butyl(dimethyl)silyl]oxy}cyclohexyl)-2-ethyl-6-(4,4,5,5-tetramethyl-1,3,2-dioxaborolan-2-yl)-1H-benzimidazole obtained in Reference Example 140 (247 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 479 (M + 1)
Elementary analysis as C₂₉H₃₀N₆O·HCl + 3H₂O
Calcd. (%) C: 61.20.; H: 6.55.; N: 14.77
Found. (%) C: 60.84.;H: 6.52.; N: 14.79

### Example 158 2-({4-cyclopropyl-6-[1-(3-oxopiperazin-1-yl)isoquinolin-7-yl]pyridin-2-yl}amino)pyridine-4-carbonitrile hydrochloride

### Step 1 Production of tert-butyl (4-cyanopyridin-2-yl){4-cyclopropyl-6-[1-(3-oxopiperazin-1-yl)isoquinolin-7-yl]pyridin-2-yl}carbamate

The title compound was prepared as pale yellow amorphous form (116 mg) according to the aforementioned procedure described in Example 153 Step 1 using 4-[7-(4,4,5,5-tetramethyl-1,3,2-dioxaborolan-2-yl)-isoquinolin-1-yl]piperazin-2-on obtained in Reference Example 138 (175 mg) instead of 1-methyl-6-(4,4,5,5-tetramethyl-1,3,2-dioxaborolan-2-yl)-1H-benzimidazole.

### Step 2 Production of 2-({4-cyclopropyl-6-[1-(3-oxopiperazin-1-yl)isoquinolin-7-yl]pyridin-2-yl}amino)pyridine-4-carbonitrile hydrochloride

The title compound was prepared as pale yellow solid (46 mg) according to the aforementioned procedure described in Example 119 Step 2 using Production of tert-butyl (4-cyanopyridin-2-yl){4-cyclopropyl-6-[1-(3-oxopiperazin-1-yl)isoquinolin-7-yl]pyridin-2-yl}carbamate obtained in Example 158 Step 1 (116 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 462 (M + 1)
Elementary analysis as C₂₇H₂₃N₇O·HCl + 3H₂O + 0.15ethyl acetate
Calcd. (%) C: 58.65.; H: 5.56.; N: 17.35
Found. (%) C: 58.68.;H: 5.27.; N: 17.00

### Example 159 2-({4-cyclopropyl-6-[1-(trans-4-hydroxycyclohexyl)-2-methyl-1H-benzimidazol-6-yl]pyridin-2-yl}amino)pyridine-4-carbonitrile hydrochloride

The title compound was prepared as pale yellow solid (10 mg) according to the aforementioned procedure described in Example 119 using 1-(trans-4-{[tert-butyl(dimethyl)silyl]oxy}cyclohexyl)-2-methyl-6-(4,4,5,5-tetramethyl-1,3,2-dioxaborolan-2-yl)-1H-benzimidazole obtained in Reference Example 139 (76 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 465 (M + 1)

### Example 160 2-({4-cyclopropyl-6-[2-ethyl-1-(trans-4-hydroxycyclohexyl)-1H-benzimidazol-6-yl)pyridin-2-yl} amino)pyridine-4-carbonitrile methanesulfonate

2-({4-cyclopropyl-6-[2-ethyl-1-(trans-4-hydroxycyclohexyl)-1H-benzimidazol-6-yl)pyridin-2-yl} amino)pyridine-4-carbonitrile (185 mg) was dissolved in DMF (1.8 mL) at 70 °C. Methanesulfonic acid (25 µL) was added and the mixture was stirred at 70 °C for 30 minutes. The reaction mixture was cooled to 50 °C, and then acetone (3.6 mL) was added to the mixture and the mixture was stirred at 50 °C for 30 minutes. The reaction mixture was evaporated under reduced pressure, acetone was then added to the mixture, stirred under heating, followed by stirring at room temperature for 30 minutes. The precipitated solid was collected by filtration and washed with acetone. The obtained residue was dried under reduce pressure to give 106 mg of the title compound as a yellow solid.
Elementary analysis as C₂₉H₃₀N₆O·CH₄O₃S + 3.5 H₂O
Calcd. (%) C: 56.50.; H: 6.48.; N: 13.18
Found. (%) C: 56.57.;H: 6.41.; N: 13.04

### Example 161 2-({4-cyclopropyl-6-[1-(trans-4-hydroxycyclohexyl)-2-(propan-2-yl)-1H-benzimidazol-6-yl)pyridin-2-yl}amino)pyridine-4-carbonitrile hydrochloride

The title compound was prepared as pale yellow solid (105 mg) according to the aforementioned procedure described in Example 119 using 1-(trans-4-{[tert-butyl(dimethyl)silyl]oxy}cyclohexyl)-2-(propan-2-yl)-6-(4,4,5,5-tetramethyl-1,3,2-dioxaborolan-2-yl)-1H-benzimidazole obtained in Reference Example 141 (185 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 493 (M + 1)

### Example 162 2-({4-cyclopropyl-6-[1-(trans-4-hydroxycyclohexyl)-2-(hydroxymethyl)-1H-benzimidazol-6-yl)pyridin-2-yl}amino)pyridine-4-carbonitrile hydrochloride

### Step 1 Production of [6-{6-[(tert-butoxycarbonyl)(4-cyanopyridin-2-yl)amino]-4-cyclopropylpyridin-2-yl}-1-(trans-4-{[tert-butyl(dimethyl)silyl]oxy}cyclohexyl)-1H-benzimidazol-2-yl]methyl acetate

The title compound was prepared as pale yellow amorphous form (83 mg) according to the aforementioned procedure described in Example 119 Step 1 using [1-(trans-4-{[tert-butyl(dimethyl)silyl]oxy}cyclohexyl)-6-(4,4,5,5-tetramethyl-1,3,2-dioxaborolan-2-yl)-1H-benzimidazol-2-yl]methyl acetate obtained in Reference Example 142 (143 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.

### Step 2 Production of tert-butyl {6-[1-(trans-4-{[tert-butyl(dimethyl)silyl]oxy}cyclohexyl)-2-(hydroxymethyl)-1H-benzimidazol-6-yl]-4-cyclopropylpyridin-2-yl}(4-cyanopyridin-2-yl)carbamate

To a solution of [6-{6-[(tert-butoxycarbonyl)(4-cyanopyridin-2-yl)amino]-4-cyclopropylpyridin-2-yl}-1-(trans-4-{[tert-butyl(dimethyl)silyl]oxy}cyclohexyl)-1H-benzimidazol-2-yl]methyl acetate obtained in Example 162 Step 1 (136 mg) in methanol (5 mL) was added potassium carbonate (2.3 mg) at 0 °C and the mixture was stirred for an hour. The reaction mixture was diluted with water, extracted with ethyl acetate. The organic layer was dried over magnesium sulfate and the solvent was evaporated under reduced pressure. The resulting residue was purified by column chromatography to give 75 mg of the title compound as a pale yellow amorphous form.

### Step 3 Production of 2-({4-cyclopropyl-6-[1-(trans-4-hydroxycyclohexyl)-2-(hydroxymethyl)-1H-benzimidazol-6-yl)pyridin-2-yl}amino)pyridine-4-carbonitrile hydrochloride

The title compound was prepared as pale yellow solid (1 mg) according to the aforementioned procedure described in Example 119 Step 2 using tert-butyl {6-[1-(trans-4-{[tert-butyl(dimethyl)silyl]oxy}cyclohexyl)-2-(hydroxymethyl)-1H-benzimidazol-6-yl]-4-cyclopropylpyridin-2-yl}(4-cyanopyridin-2-yl)carbamate obtained in Example 162 Step 2 (11 mg) instead of tert-butyl {6-[1-(3{[tert-butyl(dimethyl)silyl]oxy}-2,2-dimethylpropyl)-1H-benzimidazol-5-yl]-4-cyclopropylpyridin-2-yl}(4-cyanopyridin-2-yl) carbamate.
MS 481 (M + 1)

### Example 163 2-({4-cyclopropyl-6-[1-(piperazin-1-yl)isoguinolin-7-yl]pyridin-2-yl}amino)pyridine-4-carbonitrile hydrochloride

The title compound was prepared as yellow solid (11 mg) according to the aforementioned procedure described in Example 158 using tert-butyl 4-[7-(4,4,5,5-tetramethyl-1,3,2-dioxaborolan-2-yl)-isoquinolin-1-yl]piperazin-1-carboxylate obtained in Reference Example 143 (280 mg) instead of 4-[7-(4,4,5,5-tetramethyl-1,3,2-dioxaborolan-2-yl)-isoquinolin-1-yl]piperazin-2-on.
MS 448 (M + 1)

### Example 164 2-(14-cyclopropyl-6-[1-(4-hydroxypiperidin-1-yl)isoquinolin-7-yl]pyridin-2-yl}amino)pyridine-4-carbonitrile hydrochloride

The title compound was prepared as yellow solid (6 mg) according to the aforementioned procedure described in Example 158 using t1-(4-{[tert-butyl(dimethyl)silyl]oxy}piperidin-1-yl)-7-(4,4,5,5-tetramethyl-1,3,2-dioxaborolan-2-yl)isoquinoline obtained in Reference Example 144 (280 mg) instead of 4-[7-(4,4,5,5-tetramethyl-1,3,2-dioxaborolan-2-yl)-isoquinolin-1-yl]piperazin-2-on.
MS 463 (M + 1)

### Example 165 2-[(4-cyclopropyl-6-{1-[(3R)-3-hydroxypyrrolidin-1-yl]isoquinolin-7-yl}pyridin-2-yl]amino]pyridine-4-carbonitrile hydrochloride

The title compound was prepared as yellow solid (29 mg) according to the aforementioned procedure described in Example 158 using 1-[(3R)-3-{[tert-butyl(dimethyl)silyl]oxy}pyrrolidin-1-yl]-7-(4,4,5,5-tetramethyl-1,3,2-dioxaborolan-2-yl)isoquinoline obtained in Reference Example 145 (114 mg) instead of 4-[7-(4,4,5,5-tetramethyl-1,3,2-dioxaborolan-2-yl)-isoquinolin-1-yl]piperazin-2-on.
MS 449 (M + 1)

### Example 166 2-({4-cyclopropyl-6-[1-(3-hydroxyazetidin-1-yl)isoguinolin-7-yl]pyridin-2-yl}amino)pyridine-4-carbonitrile hydrochloride

The title compound was prepared as yellow solid (4 mg) according to the aforementioned procedure described in Example 158 using 1-(3-{[tert-butyl(dimethyl)silyl]oxy}azetidin-1-yl)-7-(4,4,5,5-tetramethyl-1,3,2-dioxaborolan-2-yl)isoquinoline obtained in Reference Example 146 (22 mg) instead of 4-[7-(4,4,5,5-tetramethyl-1,3,2-dioxaborolan-2-yl)-isoquinolin-1-yl]piperazin-2-on.
MS 435 (M + 1)

### Example 167 2-[(4-cyclopropyl-6-{1-[(2S)-2-(hydroxymethyl)pyrrolidin-1-yl]isoguinolin-7-yl}pyridin-2-yl]amino]pyridine-4-carbonitrile hydrochloride

The title compound was prepared as yellow solid (47 mg) according to the aforementioned procedure described in Example 158 using 1-[-(2S)-2-({[tert-butyl(dimethyl)silyl]oxy}methyl)pyrrolidin-1-yl]-7(4,4,5,5-tetramethyl-1,3,2-dioxaborolan-2-yl)isoquinoline obtained in Reference Example 147 (163 mg) instead of 4-[7-(4,4,5,5-tetramethyl-1,3,2-dioxaborolan-2-yl)-isoquinolin-1-yl]piperazin-2-on.
MS 463 (M + 1)

### Example 168 2-[(4-cyclopropyl-6-{1-[(3R)-3-fluoropyrrolidin-1-yl]isoquinolin-7-yl}pyridin-2-yl)amino]pyridine-4- carbonitrile hydrochloride

The title compound was prepared as yellow solid (14 mg) according to the aforementioned procedure described in Example 158 using 1-[(3R)-3-fluoropyrrolidin-1-yl]-7(4,4,5,5-tetramethyl-1,3,2-dioxaborolan-2-yl)isoquinoline obtained in Reference Example 148 (94 mg) instead of 4-[7-(4,4,5,5-tetramethyl-1,3,2-dioxaborolan-2-yl)-isoquinolin-1-yl]piperazin-2-on.
MS 451 (M + 1)

### Example 169 2-[(6-{1-[(3R)-3-aminopyrrolidin-1-yl]isoquinolin-7-yl}-4-cyclopropylpyridin-2-yl)amino]pyridine-4-carbonitrile hydrochloride

The title compound was prepared as yellow solid (10 mg) according to the aforementioned procedure described in Example 158 using tert-butyl {(3R)-1-[7-(4,4,5,5-tetramethyl-1,3,2-dioxaborolan-2-yl)isoquinolin-1-yl]pyrrolidin-3-yl}carbamate obtained in Reference Example 149 (38 mg) instead of 4-[7-(4,4,5,5-tetramethyl-1,3,2-dioxaborolan-2-yl)-isoquinolin-1-yl]piperazin-2-on.
MS 448 (M + 1)

### Example 170 2-({6-[1-(4-cyanopiperidin-1-yl)isoquinolin-7-yl]-4-cyclopropylpyridin-2-yl}amino)pyridine-4-carbonitrile hydrochloride

The title compound was prepared as a pale yellow solid (45 mg) according to the aforementioned procedure described in Example 158 using 1-[7-(4,4,5,5-tetramethyl-1,3,2-dioxaborolan-2-yl)-isoquinolin-1-yl]piperidin-4-carbonitrile obtained in Reference Example 150 (120 mg) instead of 4-[7-(4,4,5,5-tetramethyl-1,3,2-dioxaborolan-2-yl)-isoquinolin-1-yl]piperazin-2-on.
MS 472 (M + 1)

### Example 171 2-({4-cyclopropyl-6-[1-(2-oxo-imidazolidin-1-yl)isoquinolin-7-yl]pyridin-2-yl}amino)pyridine-4-carbonitrile hydrochloride

The title compound was prepared as a yellow solid (8 mg) according to the aforementioned procedure described in Example 158 using 1-[7-(4,4,5,5-tetramethyl-1,3,2-dioxaborolan-2-yl)-isoquinolin-1-yl]imidazolidin-2-on obtained in Reference Example 151 (54 mg) instead of 4-[7-(4,4,5,5-tetramethyl-1,3,2-dioxaborolan-2-yl)-isoquinolin-1-yl]piperazin-2-on.
MS 448 (M + 1)

### Example 172 2-({6-[1-(trans-4-aminocyclohexyl)-1H-benzimidazol-6-yl]-4-cyclopropylpyridin-2-yl}amino)pyridine-4-carbonitrile hydrochloride

The title compound was prepared as pale yellow solid (29 mg) according to the aforementioned procedure described in Example 119 using tert-butyl {trans-4-[6-(4,4,5,5-tetramethyl-1,3,2-dioxaborolan-2-yl)-1H-benzimidazol-1-yl]cyclohexyl}carbamate obtained in Reference Example 152 (119 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 450 (M + 1)

### Example 173 2-({4-[1-cyclopropyl-6-(piperidin-4-yl)-1H-benzimidazol-6-yl)pyridin-2-yl}amino)pyridine-4-carbonitrile hydrochloride

The title compound was prepared as pale yellow solid (38 mg) according to the aforementioned procedure described in Example 119 using tert-butyl 4-[6-(4,4,5,5-tetramethyl-1,3,2-dioxaborolan-2-yl)-1H-benzimidazol-1-yl]piperidine-1-carboxylate obtained in Reference Example 153 (153 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 436 (M + 1)

### Example 174 2-[(4-cyclopropyl-6-{1-(6-oxo-1,6-dihydropyridin-3-yl)methyl]-1H-benzimidazol-6-yl}pyridin-2-yl)amino]pyridine-4-carbonitrile hydrochloride

The title compound was prepared as pale yellow solid (24 mg) according to the aforementioned procedure described in Example 119 using 1-({6-[(4-methoxybenzyl)oxy]pyridin-3-yl}methyl)₋6-(4,4,5,5-tetramethyl-1,3,2-dioxaborolan-2-yl)-1H-benzimidazole obtained in Reference Example 154 (65 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H- benzimidazole.
MS 460 (M + 1)

### Example 175_ 2-({4-cyclopropyl-6-[2-(trans-4-hydroxycyclohexyl)-1H-benzimidazol-6-yl)pyridin-2-yl}amino)pyridine-4- carbonitrile hydrochloride

The title compound was prepared as pale yellow solid (2 mg) according to the aforementioned procedure described in Example 119 using 2-(trans-4-{[tert-butyl(dimethyl)silyl]oxy}cyclohexyl)-6-(4,4,5,5-tetramethyl-1,3,2-dioxaborolan-2-yl)-1H-benzimidazole obtained in Reference Example 155 (14 mg) instead of 1-(3-{[tert-butyl(dimethyl)silyl]oxy}-2,2-dimethylpropyl)-5-(4,4,5,5-tetramethyl-1,3,2-dioxaborolan-2-yl)-1H-benzimidazole.
MS 451 (M + 1)

### Example 176 2-({6-[1-(trans-4-cyanocyclohexyl)-1H-benzimidazol-6-yl]-4-cyclopropylpyridin-2-yl}amino)pyridine-4-carbonitrile hydrochloride

The title compound was prepared as a yellow solid (17 mg) according to the aforementioned procedure described in Example 158 using trans-4-[6-(4,4,5,5-tetramethyl-1,3,2-dioxaborolan-2-yl)-1H-benzimidazol-1-yl]cyclohexanecarbonitrile obtained in Reference Example 156 (154 mg) instead of 4-[7-(4,4,5,5-tetramethyl-1,3,2-dioxaborolan-2-yl)-isoquinolin-1-yl]piperazin-2-on.
MS 460 (M + 1)

### Example 177 2-{[4-cyclopropyl-6'-(1,1-dioxidethiomorpholin-4-yl)-5'-fluoro-2,3'-bipyridin-6-yl]amino}pyridine-4-carbonitrile hydrochloride

The title compound was prepared as a yellow solid (36 mg) according to the aforementioned procedure described in Example 158 using 4-[3-fluoro-5-(4,4,5,5-tetramethyl-1,3,2-dioxaborolan-2-yl)pyridin-2-yl]thiomorpholine 1,1-dioxide obtained in Reference Example 157 (125 mg) instead of 4-[7-(4,4,5,5-tetramethyl-1,3,2-dioxaborolan-2-yl)-isoquinolin-1-yl]piperazin-2-on.
MS 465 (M + 1)

### Example 178 2-({4-cyclopropyl-6-[1-(trans-4-hydroxycyclohexyl)-1H-benzimidazol-5-yl)pyridin-2-yl}amino)pyridine-4-carbonitrile methanesulfonate

2-({4-Cyclopropyl-6-[1-(trans-4-hydroxycyclohexyl)-1H-benzimidazol-5-yl]pyridin-2-yl}amino)pyridine-4-carbonitrile (1.5 g) was dissolved in DMF (7.5 mL) at 70 °C. Methanesulfonic acid (216 µL) was added and the mixture was stirred at 70 °C for an hour. The reaction mixture was cooled to 50 °C, and then acetone (30 mL) was added to the mixture and the mixture was stirred at 50 °C for an hour.

After the mixture was cooled, and then cooled under ice water. The precipitated solid was collected by filtration and washed with acetone. The obtained solid was dried under reduced pressure to give 1.7 g of the title compound as a yellow solid.
Elementary analysis as C₂₇H₂₅N₆O • CH₄O₃S + 0.2 H₂O
Calcd. (%) C: 61.12.; H: 5.57.; N: 15.27
Found. (%) C: 61.11.;H: 5.40.; N: 15.34

### Example 179 2-{[4-cyclopropyl-6'-(1,1-dioxidethiomorpholin-4-yl)-2,3'-bipyridin-6-yl]amino}pyridine-4-carbonitrile dimethanesulfonate

2-{[4-Cyclopropyl-6'-(1,1-dioxidethiomorpholin-4-yl)-2,3'-bipyridin-6-yl]amino}pyridine-4-carbonitrile (2.0 g) was dissolved in DMF (20 mL) at 70 °C. Methanesulfonic acid (870 µL) was added and the mixture was stirred at 70 °C for an hour. The reaction mixture was cooled to 50 °C, and then acetone (40 mL) was added to the mixture and the mixture was stirred at 50 °C for 2 hours. After the mixture was cooled, and then cooled under ice water for 30 minutes. The precipitated solid was collected by filtration and washed with acetone. The obtained solid was dried under reduced pressure to give 2.8 g of the title compound as a yellow solid.
Elementary analysis as C₂₇H₂₂N₆O₂S • 2CH₄O₃S
Calcd. (%) C: 47.01.; H: 4.74.; N: 13.16
Found. (%) C: 46.83.;H: 4.75.; N: 13.23

### Example 180 2-{[4-cyclopropyl-5'-(4-hydroxypiperidin-1-yl)-2,3'-bipyridin-6-yl]amino}pyridine-4-carbonitrile dimethanesulfonate

2-{[4-Cyclopropyl-5'-(4-hydroxypiperidin-1-yl)-2,3'-bipyridin-6-yl]amino}pyridine-4-carbonitrile (130 mg) was dissolved in DMF (1.3 mL) at 70 °C. Methanesulfonic acid (61 µL) was added and the mixture was stirred at 70 °C for 30 minutes. The reaction mixture was cooled to 50 °C, and then acetone (2.6 mL) was added to the mixture and the mixture was stirred at 50 °C for 30 minutes. The solvent was evaporated under reduced pressure, acetone was added to the mixture, stirred at room temperature overnight. The precipitated solid was collected by filtration and washed with acetone, followed by drying under reduced pressure to give 143 mg of the title compound as a yellow solid.
Elementary analysis as C₂₄H₂₄N₆O+1 • 5H₂O
Calcd. (%) C: 49.43.; H: 5.58.; N: 13.30
Found. (%) C: 49.26.;H: 5.73.; N: 13.19

### Test Example 1: Test for Syk Tyrosine Kinase Inhibitory Activity

### 1. Preparation of Test Substance

A test substance was prepared at 10 mM in dimethyl sulfoxide (DMSO), and further diluted with DMSO to concentrations of 1000, 300, 100, 30, 10, 3, 1, 0.3, 0.1, 0.03, and 0.01 µM. The resulting solutions were further diluted to 20-fold with an assay buffer, whereby test substance solutions were prepared. As a negative control, a solution obtained by diluting DMSO to 20-fold with an assay buffer was used. As the assay buffer, a buffer containing 15 mM Tris-HCl (pH 7.5), 0.01 (v/v) % Tween-20, and 1 mM dithiothreitol was used.

### 2. Measurement of Syk Tyrosine Kinase Inhibitory Activity

The activity was measured using the ELISA method. Each of the test substance solutions was added to a streptavidine-coated 96-well plate (DELFIA Strip Plate 8 x 12 wells, PerkinElmer Co., Ltd.) at 10 µL per well (n=2), and a substrate solution (625 nM biotinylated peptide substrate, 25 µM ATP, 25 mM MgCl₂, 15 mM Tris-HCl (pH 7.5 ) , 0.01 (v/v) % Tween-20, 1 mM dithiothreitol) was added to the plate at 20 µL per well, and the resulting mixture was stirred. Finally, Syk tyrosine kinase (Carna Biosciences, Inc.) (previously diluted to 0.025 nM with the assay buffer) was added to the plate at 20 µL per well, and the resulting mixture was stirred. A reaction was allowed to proceed at 30°C for 1 hour. After the plate was washed 4 times with a washing buffer (50 mM Tris-HCl (pH 7.5, 150 mM NaCl, 0.02 (v/v) % Tween-20), a blocking buffer (0.1 % bovine serum albumin, 50 mM Tris-HCl (pH 7.5), 150 mM NaCl, 0.02 (v/v) % Tween-20) was added to the plate at 150 µL per well, and blocking was performed at 30°C for 30 minutes. Then, the blocking buffer was removed, and a horseradish peroxidase-labeled anti-phosphorylated tyrosine antibody (BD Biosciences, Inc.) (previously diluted to 10000-fold with the blocking buffer) was added to the plate at 100 µL per well, and the plate was incubated at 30°C for 30 minutes. After the plate was washed 4 times with the washing buffer, a 3,3',5,5'-tetramethylbenzidine solution (Sigma-Aldrich Co., Ltd.) was added to the plate at 100 µL per well to develop the color for 10 minutes. The reaction was stopped by adding 0.1 M sulfuric acid at 100 µL per well. The absorbance at 450 nm was measured using a microplate reader (Multiskan FC, Thermo Fisher Scientific K.K.).

### 3. Analysis of Measurement Results

A non-linear regression analysis was performed for the measured absorbance using the SAS system (SAS Institute, Inc.), and a concentration of each of the test substances inhibiting the tyrosine kinase activity at 50% (IC₅₀) was calculated. The results are shown in the following Tables 1 to 5. A case where IC₅₀ is less than 5 nM was evaluated as ***, a case where IC₅₀ is 5 nM or more and less than 50 nM was evaluated as **, and a case where IC₅₀ is 50 nM or more and less than 500 nM was evaluated as *, which are shown in the column of evaluation item.

**Table 1**

| Example No. | Evaluation |
|---|---|
| 1 | ** |
| 2 | ** |
| 3 | *** |
| 4 | ** |
| 5 | ** |
| 6 | ** |
| 7 | * |
| 8 | * |
| 9 | * |
| 10 | ** |
| 11 | * |
| 12 | * |
| 13 | ** |
| 14 | ** |
| 15 | ** |
| 16 | ** |
| 17 | * |
| 18 | ** |
| 19 | *** |
| 20 | ** |
| 21 | ** |
| 22 | ** |
| 23 | ** |
| 24 | ** |
| 25 | ** |
| 26 | ** |
| 27 | ** |
| 28 | ** |
| 29 | ** |
| 30 | * |
| 31 | ** |
| 32 | ** |
| 33 | ** |
| 34 | ** |
| 35 | * |
| 36 | ** |
| 37 | ** |
| 38 | ** |
| 39 | * |
| 40 | * |

**Table 2**

| Example No. | Evaluation |
|---|---|
| 41 | * |
| 42 | * |
| 43 | * |
| 44 | * |
| 45 | * |
| 46 | * |
| 47 | * |
| 48 | * |
| 49 | ** |
| 50 | * |
| 51 | * |
| 52 | * |
| 53 | * |
| 54 | * |
| 55 | ** |
| 56 | ** |
| 57 | ** |
| 58 | ** |
| 59 | ** |
| 60 | ** |
| 61 | * |
| 62 | ** |
| 63 | * |
| 64 | ** |
| 65 | ** |
| 66 | * |
| 67 | * |
| 68 | * |
| 69 | ** |
| 70 | * |
| 71 | * |
| 72 | ** |
| 73 | ** |
| 74 | ** |
| 75 | ** |
| 76 | ** |
| 77 | ** |
| 78 | ** |
| 79 | ** |
| 80 | * |

**Table 3**

| Example No. | Evaluavtion |
|---|---|
| 81 | ** |
| 82 | * |
| 83 | ** |
| 84 | *** |
| 85 | *** |
| 86 | ** |
| 87 | ** |
| 88 | *** |
| 89 | *** |
| 90 | *** |
| 91 | *** |
| 92 | *** |
| 93 | ** |
| 94 | ** |
| 95 | *** |
| 96 | *** |
| 97 | *** |
| 98 | *** |
| 99 | *** |
| 100 | ** |
| 101 | * |
| 102 | ** |
| 103 | ** |
| 104 | ** |
| 105 | * |
| 106 | ** |
| 107 | ** |
| 108 | * |
| 109 | ** |
| 110 | * |
| 111 | ** |
| 112 | *** |
| 113 | ** |
| 114 | * |
| 115 | *** |
| 116 | ** |
| 117 | ** |
| 118 | * |
| 119 | * |
| 120 | * |

**Table 4**

| Example No. | Evaluation |
|---|---|
| 121 | * |
| 122 | *** |
| 123 | * |
| 124 | ** |
| 125 | * |
| 126 | * |
| 127 | ** |
| 128 | ** |
| 129 | ** |
| 130 | * |
| 131 | * |
| 132 | * |
| 133 | ** |
| 134 | ** |
| 135 | ** |
| 136 | * |
| 137 | * |
| 138 | * |
| 139 | ** |
| 140 | ** |
| 141 | * |
| 142 | * |
| 143 | ** |
| 144 | * |
| 145 | ** |
| 146 | ** |
| 147 | * |
| 148 | * |
| 149 | * |
| 150 | * |
| 151 | ** |
| 152 | * |
| 153 | ** |
| 154 | * |
| 155 | ** |
| 156 | *** |
| 157 | *** |
| 158 | *** |
| 159 | *** |

**Table 5**

| Example No. | Evaluation |
|---|---|
| 161 | *** |
| 162 | *** |
| 163 | ** |
| 164 | ** |
| 165 | ** |
| 166 | ** |
| 167 | ** |
| 168 | * |
| 169 | ** |
| 170 | ** |
| 171 | ** |
| 172 | ** |
| 173 | *** |
| 174 | ** |
| 175 | * |
| 176 | ** |
| 177 | ** |

### Test Example 2: Evaluation of drug efficacy using momodel of leukemia obtained by transplantation of Ba/F3 cells that express TEL-Syk fusion protein into nude mouse

A test substance was orally administered to a model mouse of leukemia obtained by transplantation of Ba/F3 cells that express TEL-Syk fusion protein (e.g., Blood, 2001, 15, 97(4), 1050-1055) into 7 weeks old female nude mouse, and an effect of prolonging the survival period of each mouse was evaluated as the drug efficacy. The Ba/F3 cells that express TEL-Syk fusion protein were transplanted into each mouse by injection through the tail vein at 1 x 10⁶ cells/nude mouse. From the following day of the transplantation of the cells, a suspension of a test substance in methyl cellulose (Example 3: 5.0 mg/mL, 10 mg/mL, and 15 mg/mL, Example 5: 15 mg/mL and 30 mg/mL, Example 7: 15 mg/mL and 30 mg/mL, Example 10: 15 mg/mL and 30 mg/mL, Example 12: 5.0 mg/mL and 10 mg/mL, Example 14: 5.0 mg/mL, 7.5 mg/mL, and 10 mg/mL) was orally administered at a dose of 10 mL/kg of body weight twice daily repetitively for 11 days. After completion of administration, the number of survival days of each animal was measured. The results are shown in Figs. 1 to 6. Incidentally, in each drawing, the daily dose is shown.

As described above, the compound of the present invention or a pharmaceutically acceptable salt thereof has a high Syk tyrosine kinase inhibitory activity, and therefore a pharmaceutical composition containing the compound of the present invention or a pharmaceutically acceptable salt thereof as an active ingredient can be used as a preventive agent or a therapeutic agent for a disease associated with Syk tyrosine kinase, for example, an allergic disease (e.g., bronchial asthma, allergic rhinitis, allergic dermatitis, or allergic conjunctivitis), an autoimmune disease (e.g., chronic rheumatoid arthritis, idiopathic thrombocytopenic purpura, systemic lupus erythematosus, or multiple sclerosis) or a malignant tumor (e.g., a B-cell lymphoma (e.g., small-cell lymphoma), a B-cell leukemia (e.g., chronic lymphocytic leukemia), peripheral T-cell lymphoma not-otherwise specified, angioimmunoblastic T-cell lymphoma, anaplastic large-cell lymphoma, cutaneous anaplastic large-cell lymphoma, mycosis fungoides, enteropathy-associated T-cell lymphoma, extranodal NK-T-cell lymphoma, hepatosplenic T-cell lymphoma, subcutaneous panniculitis-like T-cell lymphoma, diffuse large-cell lymphoma, or follicular lymphoma).

### Formulation Example 1

### Tablets (tablets for oral administration)

### Formulation: in one tablet (80 mg)

Compound of the present invention of Example 1: 5.0 mg

Corn starch: 46.6 mg

Crystalline cellulose: 24.0 mg

Methyl cellulose: 4.0 mg

Magnesium stearate: 0.4 mg

The mixed powder containing the above components at the above ratio was formed into tablets by a conventional method to prepare tablets for oral administration.

### Formulation Example 2

### Tablets (tablets for oral administration)

### Formulation: in one tablet (80 mg)

Compound of the present invention of Example 2: 5.0 mg

Corn starch: 46.6 mg

Crystalline cellulose: 24.0 mg

Methyl cellulose: 4.0 mg

Magnesium stearate: 0.4 mg

The mixed powder containing the above components at the above ratio was formed into tablets by a conventional method to prepare tablets for oral administration.

## Claims

1. A pyridine derivative represented by the following general formula [1] or a pharmaceutically acceptable salt thereof: 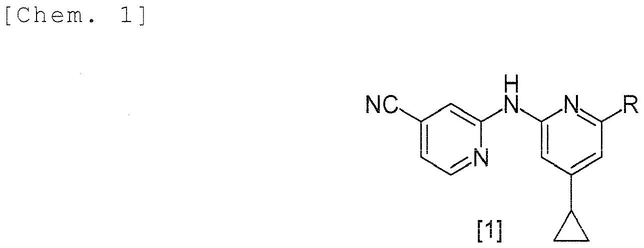 wherein
R represents aryl or heteroaryl, each of which may be substituted with one or two substituents selected from the group consisting of alkyl which may be substituted with hydroxy, hydroxy, halogen, and a group represented by the following general formula [2] :
[Chem. 2] -L¹-L²-L³-R^{A}
(wherein
L¹ and L³ each independently represent a single bond, alkylene, or cycloalkylene;
L² represents a single bond, O, or NR^{B};
R^{B} represents alkyl which may be substituted with hydroxy;
R^{A} represents:
(1) H,
(2) amino,
(3) cyano,
(4) hydroxy,
(5) alkoxy,
(6) aryl,
(7) monoalkylamino,
(8) dialkylamino,
(9) carbamoyl,
(10) alkyloxycarbonyl,
(11) monoalkylaminocarbonyl,
(12) dialkylaminocarbonyl,
(13) alkylcarbonylamino,
(14) alkyl which may be substituted with one or two hydroxy groups,
(15) heteroaryl which may be substituted with hydroxy or alkyl,
(16) cycloalkyl which may be substituted with one or two halogens, alkoxy, alkylcarbonyloxy, hydroxy, or hydroxyalkyl, or (17) a 4- to 7-membered saturated heterocyclic group, which has one or two heteroatoms, and may be substituted with one or two substituents selected from the group consisting of cyano, hydroxy, oxo, halogen, alkylcarbonyl, amino, monoalkylamino, dialkylamino, alkylcarbonylamino, carbamoylamino, monoalkylaminocarbonylamino, alkyl, hydroxyalkyl, hydroxycarbonylalkyl, carbamoylalkyl, monoalkylaminocarbonylalkyl, dialkylaminocarbonylalkyl, hydroxycarbonyl, carbamoyl, monoalkylaminocarbonyl, dialkylaminocarbonyl, aminothiocarbonyl, alkylsulfonyl, and aryl which may be substituted withhalogen).

2. The pyridine derivative or a pharmaceutically acceptable salt thereof according to claim 1, wherein aryl or heteroaryl represented by R is phenyl, pyridyl, pyrimidinyl, benzimidazolyl, indazolyl, or isoquinolyl.

3. The pyridine derivative or a pharmaceutically acceptable salt thereof according to claim 1 or 2, wherein R^{A} is cycloalkyl which may be substituted with hydroxy or hydroxyalkyl, or a 4- to 7-membered saturated heterocyclic group, which has one or two heteroatoms, and is substituted with one or two substituents selected from the group consisting of cyano, hydroxy, oxo, halogen, alkylcarbonyl, amino, monoalkylamino, dialkylamino, alkylcarbonylamino, carbamoylamino, monoalkylaminocarbonylamino, alkyl, hydroxyalkyl, hydroxycarbonylalkyl, carbamoylalkyl, monoalkylaminocarbonylalkyl, dialkylaminocarbonylalkyl, hydroxycarbonyl, carbamoyl, monoalkylaminocarbonyl, dialkylaminocarbonyl, aminothiocarbonyl, alkylsulfonyl, and aryl which may be substituted with halogen.

4. The pyridine derivative or a pharmaceutically acceptable salt thereof according to claim 1, which is a compound selected from the group consisting of the following compounds (1) to (170):
(1) 2-{[4-cyclopropyl-6'-(morpholin-4-yl)-2,3-bipyridin-6-yl]amino}pyridine-4-carbonitrile,
(2) 2-({4-cyclopropyl-6'-[(2-hydroxyethyl)amino]-2,3'-bipyridin-6-yl}amino)pyridine-4-carbonitrile,
(3) 2-{[4-cyclopropyl-6'-(1,1-dioxidethiomorpholin-4-yl)-2,3'-bipyridin-6-yl]amino}pyridine-4-carbonitrile,
(4) 2-{[4-cyclopropyl-6'-(4-oxopiperidin-1-yl)-2,3'-bipyridin-6-yl]amino}pyridine-4-carbonitrile,
(5) 2-{[4-cyclopropyl-6'-(3-oxopiperazin-1-yl)-2,3'-bipyridin-6-yl]amino}pyridine-4-carbonitrile,
(6) 2-{[4-cyclopropyl-6'-(3-hydroxyazetidin-1-yl)-2,3'-bipyridin-6-yl]amino}pyridine-4-carbonitrile,
(7) 2-(4-{6-[(4-cyanopyridin-2-yl)amino]-4-cyclopropyl-2,3'-bipyridin-6'-yl}piperazin-1-yl)acetamide,
(8) 2-({4-cyclopropyl-6'-[4-hydroxy-2-oxopyrrolidin-1-yl]-2,3'-bipyridin-6-yl}amino)pyridine-4-carbonitrile,
(9) 2-{[6'-(4-acetyl-1,4-diazepan-1-yl)-4-cyclopropyl-2,3'-bipyridin-6-yl]amino}pyridine-4-carbonitrile,
(10) 4-{6-[(4-cyanopyridin-2-yl)amino]-4-cyclopropyl-2,3'-bipyridin-6'-yl}-1,4-diazepane-1-carboxamide,
(11) 2-({4-cyclopropyl-6'-[(trans-4-hydroxycyclohexyl)amino]-2,3'-bipyridin-6-yl}amino)pyridine-4- carbonitrile),
(12) 2-({6-[1-(2-aminoethyl)-1H-benzimidazol-5-yl]-4-cyclopropylpyridin-2-yl}amino)pyridine-4- carbonitrile,
(13) 2-({4-cyclopropyl-6-[1-(trans-4-hydroxycyclohexyl)-1H-benzimidazol-5-yl]pyridin-2-yl}amino)pyridine-4-carbonitrile,
(14) 2-{[4-cyclopropyl-6-(1-methyl-1H-benzimidazol-5-yl)pyridin-2-yl]amino}pyridine-4-carbonitrile,
(15) 2-({4-cyclopropyl-6-[1-(tetrahydro-2H-pyran-4-yl)-1H-benzimidazol-5-yl]pyridin-2-yl}amino)pyridine-4-carbonitrile,
(16) 6-[(4-cyanopyridin-2-yl)amino]-4-cyclopropyl-2,3'-bipyridine-6'-carbonitrile
(17) 2-[(6'-amino-4-cyclopropyl-2,3'-bipyridin-6-yl)amino]pyridine-4-carbonitrile
(18) 2-({6'-[(2-aminoethyl)amino]-4-cyclopropyl-2,3'-bipyridin-6-yl}amino)pyridine-4-carbonitrile)
(19) 2-{[4-cyclopropyl-6'-(4-hydroxypiperidin-1-yl)-2,3'-bipyridin-6-yl]amino}pyridine-4-carbonitrile,
(20) 2-({4-cyclopropyl-6'-[(2-methoxyethyl)amino]-2,3'-bipyridin-6-yl}amino)pyridine-4-carbonitrile,
(21) 2-({4-cyclopropyl-6'-[3-hydroxypyrrolidin-1-yl]-2,3'-bipyridin-6-yl}amino)pyridine-4-carbonitrile,
(22) 2-({4-cyclopropyl-6'-[(3-hydroxypropyl)amino]-2,3'-bipyridin-6-yl}amino)pyridine-4-carbonitrile,
(23) 2-({4-cyclopropyl-6'-[(3-hydroxy-2,2-dimethylpropyl)amino]-2,3'-bipyridin-6-yl}amino)pyridine-4- carbonitrile,
(24) 2-({4-cyclopropyl-6'-[4-(hydroxymethyl)piperidin-1-yl]-2,3'-bipyridin-6-yl}amino)pyridine-4-carbonitrile,
(25) 2-{[4-cyclopropyl-6'-(thiomorpholin-4-yl)-2,3'-bipyridin-6- yl]amino}pyridine-4-carbonitrile,
(26) 2-{[6'-(4-aminopiperidin-1-yl)-4-cyclopropyl-2,3'-bipyridin-6-yl]amino}pyridine-4-carbonitrile,
(27) 2-({4-cyclopropyl-6'-[4-(methylamino)piperidin-1-yl]-2,3'-bipyridin-6-yl}amino)pyridine-4-carbonitrile,
(28) 2{[4-cyclopropyl-6'-(4-fluoropiperidin-1-yl)-2,3'-bipyridin-6-yl]amino}pyridine-4-carbonitrile,
(29) 2-({6'-[bis(2-hydroxyethyl)amino]-4-cyclopropyl-2,3'-bipyridin-6-yl} amino)pyridine-4-carbonitrile
(30) 2-{[4-cyclopropyl-6'-(1,4-diazepan-1-yl)-2,3'-bipyridin-6-yl]amino}pyridine-4-carbonitrile,
(31) 2-({4-cyclopropyl-6'-[3-hydroxypiperidin-1-yl]-2,3'-bipyridin-6-yl}amino)pyridine-4-carbonitrile,
(32) 2-({4-cyclopropyl-6-[2-(4-hydroxypiperidin-1-yl)pyrimidin-5-yl)pyridin-2-yl}amino)pyridine-4-carbonitrile,
(33) 2-{[4-cyclopropyl-6-(1-methyl-1H-indazol-5-yl)pyridin-2-yl]amino}pyridine-4-carbonitrile,
(34) 2-{[4-cyclopropyl-6-(1-methyl-1H-indazol-6-yl)pyridin-2-yl]amino}pyridine-4-carbonitrile,
(35) 2-{[4-cyclopropyl-6'-(4-methyl-1,4-diazepan-1-yl)-2,3'-bipyridin-6-yl]amino}pyridine-4-carbonitrile,
(36) 1-{6-[(4-cyanopyridin-2-yl)]-4-aminocyclopropyl-2,3'-bipyridin-6'-yl}-N-methylpyrrolidine-2-carboxamide,
(37) 1-{6-[(4-cyanopyridin-2-yl)amino]-4-cyclopropyl-2,3'-bipyridin-6'-yl}pyrrolidine-2-carboxamide,
(38) 2-({4-cyclopropyl-6-[4-(4-hydroxypiperidin-1-yl)phenyl)pyridin-2-yl}amino)pyridine-4-carbonitrile,
(39) N-[1-{6-[(4-cyanopyridin-2-yl)amino]-4-cyclopropyl-2,3'-bipyridin-6'-yl}pyrrolidin-3-yl]acetamide,
(40) 4-{6-[(4-cyanopyridin-2-yl)amino]-4-cyclopropyl-2,3'-bipyridin-6'-yl}-N-ethylpiperazine-1-carboxamide,
(41) 4-{6-[(4-cyanopyridin-2-yl)amino]-4-cyclopropyl-2,3'-bipyridin-6'-yl}-N-(propan-2-yl)piperazine-1-carboxamide,
(42) 1-[1-{6-[(4-cyanopyridin-2-yl)amino]-4-cyclopropyl-2,3'-bipyridin-6'-yl}pyrrolidin-3-yl]urea,
(43) 4-{6-[(4-cyanopyridin-2-yl)amino]-4-cyclopropyl-2,3'-bipyridin-6'-yl}piperazine-1-carbothioamide,
(44) 2-({4-cyclopropyl-6'-[2-(hydroxymethyl)pyrrolidin-1-yl]-2,3'-bipyridin-6-yl}amino)pyridine-4-carbonitrile,
(45) 2-({6'-[3-aminopyrrolidin-1-yl]-4-cyclopropyl-2,3'-bipyridin-6-yl}amino)pyridine-4-carbonitrile,
(46) 2-({4-cyclopropyl-6'-[3-fluoropyrrolidin-1-yl]-2,3'-bipyridin-6-yl}amino)pyridine-4-carbonitrile,
(47) 2-({4-cyclopropyl-6'-[3-(dimethylamino)pyrrolidin-1-yl]-2,3'-bipyridin-6-yl}amino)pyridine-4-carbonitrile
(48) 2{[4-cyclopropyl-6'-(2-oxopyrrolidin-1-yl)-2,3'-bipyridin-6-yl]amino}pyridine-4-carbonitrile,
(49) 2-[(4-cyclopropyl-6'-methyl-2,3'-bipyridin-6-yl)amino]pyridine-4-carbonitrile,
(50) 2-{[4-cyclopropyl-6-(1H-indazol-6-yl)pyridin-2-yl]amino}pyridine-4-carbonitrile,
(51) 2 {[4-cyclopropyl-6-(1H-indazol-5-yl)pyridin-2-yl]amino}pyridine-4-carbonitrile,
(52) 4-{6-[(4-cyanopyridin-2-yl)amino]-4-cyclopropyl-2,3'-bipyridin-6'-yl}piperazine-1-carboxamide,
(53) 2-({4-cyclopropyl-6'-[4-(methanesulfonyl)piperazin-1-yl]-2,3'-bipyridin-6-yl}amino)pyridine-4- carbonitrile,
(54) 2-{[6'-(4-acetylpiperazin-1-yl)-4-cyclopropyl-2,3'-bipyridin-6-yl]amino}pyridine-4-carbonitrile,
(55) 1-{6-[(4-cyanopyridin-2-yl)amino]-4-cyclopropyl-2,3'-bipyridin-6'-yl} piperidine-4-carbonxylic acid,
(56) 1-{6-[(4-cyanopyridin-2-yl)amino]-4-cyclopropyl-2,3'-bipyridin-6'-yl}piperidine-4-carboxamide,
(57) 1-{6-[(4-cyanopyridin-2-yl)amino]-4-cyclopropyl-2,3'-bipyridin-6'-yl}-4-(4-fluorophenyl)piperidine-4-carboxamide,
(58) 2-(4-{6-[(4-cyanopyridin-2-yl)amino]-4-cyclopropyl-2,3'-bipyridin-6'-yl}piperazin-1-yl)-Nethylacetamide,
(59) 1-(1-{6-[(4-cyanopyridin-2-yl)amino]-4-cyclopropyl-2,3'-bipyridin-6'-yl}piperidin-4-yl)urea,
(60) 4-({6-[(4-cyanopyridin-2-yl)amino]-4-cyclopropyl-2,3'-bipyridin-6'-yl}amino)piperidine-1- carboxamide,
(61) 2-{[4-cyclopropyl-6'-(2-oxopiperazin-1-yl)-2,3'-bipyridin-6-yl]amino}pyridine-4-carbonitrile,
(62) 4-{6-[(4-cyanopyridin-2-yl)amino]-4-cyclopropyl-2,3'-bipyridin-6'-yl}-3-oxopiperazine-1-carboxamide,
(63) 2-{[4-cyclopropyl-5'-(1,1- dioxidethiomorpholin-4-yl)-2,3'-bipyridin-6-yl]amino}pyridine-4-carbonitrile,
(64) 4-{6-[(4-cyanopyridin-2-yl)amino]-4-cyclopropyl-2,3'-bipyridin-5'-yl}-1,4-diazepane-1-carboxamide,
(65) 2-({4-cyclopropyl-6'-[(3-oxopiperazin-1-yl)methyl]-2,3'-bipyridin-6-yl}amino)pyridine-4-carbonitrile,
(66) 2-{[4-cyclopropyl-6'-(hydroxymethyl)-2,3'-bipyridin-6-yl]amino}pyridine-4-carbonitrile,
(67) 2-({4-cyclopropyl-6'-[(1,1-dioxidethiomorpholin-4-yl)methyl]-2,3'-bipyridin-6-yl}amino)pyridine-4-carbonitrile,
(68) 2-({4-cyclopropyl-5'-[(3-oxopiperazin-1-yl)methyl]-2,3'-bipyridin-6-yl}amino)pyridine-4-carbonitrile,
(69) 2-({4-cyclopropyl-5'-[(1,1-dioxidethiomorpholin-4-yl)methyl]-2,3'-bipyridin-6-yl}amino)pyridine-4-carbonitrile
(70) 4-({6-[(4-cyanopyridin-2-yl)amino]-4-cyclopropyl-2,3'-bipyridin-5'-yl}methyl])-1,4-diazepane-1-carboxamide,
(71) 4-{6-[(4-cyanopyridin-2-yl)amino]-4-cyclopropyl-2,4'-bipyridin-2'-yl}-1,4-diazepane-1-carboxamide,
(72) 4-({6-[(4-cyanopyridin-2-yl)amino]-4-cyclopropyl-2,3'-bipyridin-5'-yl}amino)piperidine-1-carboxamide,
(73) 4-(3-{6-[(4-cyanopyridin-2-yl)amino]-4-cyclopropylpyridin-2-yl}phenyl)-1,4-diazepane-1-carboxamide,
(74) 2-[(4-cyclopropyl-6-{3-[(3-oxopiperazin-1-yl)methyl]phenyl}pyridin-2-yl)amino]pyridine-4-carbonitrile,
(75) 2-[(4-cyclopropyl-6-{4-[(3-oxopiperazin-1-yl)methyl]phenyl}pyridin-2-yl]amino]pyridine-4-carbonitrile,
(76) 2-{[4-cyclopropyl-5'-(piperazin-1-ylmethyl)-2,3'-bipyridin-6-yl] amino}pyridine-4-carbonitrile,
(77) 2-({5'-[(4-acetylpiperazin-1-yl)methyl]-4-cyclopropyl-2,3'-bipyridin-6-yl}amino)pyridine-4-carbonitrile,
(78) 4-({6-[(4-cyanopyridin-2-yl)amino]-4-cyclopropyl-2,3'-bipyridin-5'-yl}methyl)piperazine-1-carboxamide,
(79) 2-({4-cyclopropyl-6-[3-(piperazin-1-ylmethyl) phenyl]pyridin-2-yl}amino)pyridine-4-carbonitrile,
(80) 2-{[4cyclopropyl-5'-(,piperidin-4-ylamino)-2,3'-bipyridin-6-yl]amino}pyridine-4-carbonitrile,
(81) 2-{[4-cyclopropyl-2'-(piperazin-1-ylmethyl)-2,4'-bipyridin-6-yl]amino}pyridine-4-carbonitrile,
(82) 2-({4-cyclopropyl-2'-[(3-oxopiperazin-1-yl)methyl]-2,4'-bipyridin-6-yl}amino)pyridine-4-carbonitrile,
(83) 2-({4-cyclopropyl-5'-[pyrrolidin-3-ylamino]-2,3'-bipyridin-6-yl}amino)pyridine-4-carbonitrile,
(84) 2-({5'-[(2-aminoethyl)amino]-4-cyclopropyl-2,3'-bipyridin-6-yl}amino)pyridine-4-carbonitrile,
(85) 2-({4-cyclopropyl-5'-[(piperidin-4-yloxy)methyl]-2,3'-bipyridin-6-yl}amino)pyridine-4-carbonitrile,
(86) 2-[(4-cyclopropyl-5'-{[N-methyl-N-(piperidin-4-yl)amino]methyl}-2,3'-bipyridin-6-yl)amino]pyridine-4-carbonitrile,
(87) 2-({4-cyclopropyl-5'-[(piperidin-4-ylamino)methyl]-2,3'-bipyridin-6-yl}amino)pyridine-4-carbonitrile,
(88) 2-({4-cyclopropyl-5'-[(piperidin-4-ylmethyl)amino]-2,3'-bipyridin-6-yl}amino)pyridine-4-carbonitrile
(89) 2-{[5'-(azetidin-3-ylamino)-4-cyclopropyl-2,3'-bipyridin-6-yl]amino}pyridine-4-carbonitrile,
(90) 2-{[4-cyclopropyl-5'-(piperidin-4-yloxy)-2,3'-bipyridin-6-yl]amino}pyridine-4-carbonitrile,
(91) 2-[(4-cyclopropyl-5'-{[pyrrolidin-3-ylmethyl]amino}-2,3'-bipyridin-6-yl)amino]pyridine-4-carbonitrile,
(92) 2-{[4-cyclopropyl-5'-(1,4-diazepan-1-yl)-2,3'-bipyridin-6-yl]amino}pyridine-4-carbonitrile,
(93) 2-({4-cyclopropyl-5'-[(1-methylpiperidin-4-yl)oxy]-2,3'-bipyridin-6-yl}amino)pyridine-4-carbonitrile,
(94) 2-({4-cyclopropyl-5'-[2-(piperazin-1-yl)ethoxy]-2,3'-bipyridin-6-yl}amino)pyridine-4-carbonitrile,
(95) 2-({4-cyclopropyl-5'-[2-(morpholin-4-yl)ethoxy]-2,3'-bipyridine-6-yl}amino)pyridine-4-carbonitrile,
(96) 2-{[5'-(azetidin-3-yloxy)-4-cyclopropyl-2,3'-bipyridin-6-yl]amino}pyridine-4-carbonitrile,
(97) 2-[4-({6-[(4-cyanopyridin-2-yl)amino]-4-cyclopropyl-2,3'-bipyridine-5'-yl}oxy)-piperidine-1-yl]acetamide,
(98) 4-({6-[(4-cyanopyridin-2-yl)amino]-4-cyclopropyl-2,3'-bipyridin-5'-yl}oxy)piperidine-1-carboxamide,
(99) 2-({4-cyclopropyl-6-[2-(1,1-dioxidethiomorpholin-4-yl)pyrimidin-5-yl]pyridin-2-yl}amino)pyridine-4-carbonitrile,
(100) 4-(5-{6-[(4-cyanopyridin-2-yl)amino]-4-cyclopropylpyridin-2-yl}pyrimidin-2-yl)-1,4-diazepane-1-carboxamide,
(101) 2-({4-cyclopropyl-5'-[2-(dimethylamino)ethoxy]-2,3'-bipyridin-6-yl}amino)pyridine-4-carbonitrile,
(102) 2-({4-cyclopropyl-5'-[2-(dimethylamino)-2-methylpropoxy]-2,3'-bipyridin-6-yl}amino)pyridine-4-carbonitrile,
(103) 2-({6-[(4-cyanopyridin-2-yl)amino]-4-cyclopropyl-2,3'-bipyridin-5'-yl}oxy)acetamide,
(104) 2-{[5'-(4-acetyl-1,4-diazepan-1-yl)-4-cyclopropyl-2,3'-bipyridin-6-yl]amino}pyridine-4-carbonitrile,
(105) 2-{[4-cyclopropyl-5'-(3-hydroxypyrrolidin-1-yl)-2,3'-bipyridin-6-yl]amino}pyridine-4-carbonitrile,
(106) 2-({4-cyclopropyl-5'-[3-hydroxypiperidin-1-yl]-2,3'-bipyridine-6-yl}amino)pyridine-4-carbonitrile,
(107) 2-{[4-cyclopropyl-5'-(1-methyl-1H-pyrazol-4-yl)-2,3'-bipyridin-6-yl]amino}pyridine-4-carbonitrile,
(108) 2-{[4-cyclopropyl-5'-(3-oxopiperazin-1-yl)-2,3'-bipyridin-6-yl]amino}pyridine-4-carbonitrile,
(109) 2-{[4-cyclopropyl-5'-(4-hydroxypiperidin-1-yl)-2,3'-bipyridin-6-yl]amino}pyridine-4-carbonitrile,
(110) 2-({4-cyclopropyl-6-[1-(1,3-dihydroxypropan-2-yl)-1H-benzimidazol-5-yl)pyridin-2-yl}amino) pyridine-4-carbonitrile,
(111) 2-({4-cyclopropyl-6-[1-(2-hydroxy-2-methylpropyl)-1H-benzimidazol-5-yl)pyridin-2-yl}amino)pyridine-4-carbonitrile
(112) 2-({4-cyclopropyl-6-[1-(3-hydroxy-2,2-dimethylpropyl)-1H-benzimidazol-5-yl)pyridin-2-yl}amino)pyridine-4-carbonitrile,
(113) 2-[(4-cyclopropyl-6-{1-[(1-hydroxycyclohexyl)methyl]-1H-benzimidazol-5-yl}pyridin-2-yl]amino] pyridine-4-carbonitrile,
(114) 2-({4-cyclopropyl-6-[1-(3-hydroxypropyl)-1H-benzimidazol-5-yl)pyridin-2-yl}amino)pyridine-4-carbonitrile,
(115) 2-({4-cyclopropyl-6-[1-(trans-4-hydroxycyclohexyl)-1H-benzimidazol-6-yl)pyridin-2-yl}amino)pyridine-4-carbonitrile,
(116) Ethyl 3-(5-{6-[(4-cyanopyridine-2-yl)amino]-4-cyclopropylpyridin-2-yl}-1H-benzimidazol-1-yl)propanonate,
(117) 2-({4-cyclopropyl-6-[1-(3-hydroxypropyl)-1H-benzimidazol-6-yl)] pyridin-2-yl}amino)pyridine-4-carbonitrile,
(118) 3-(6-{6-[(4-cyanopyridin-2-yl)amino]-4-cyclopropylpyridin-2-yl}-1H-benzimidazol-1-yl)-N,N-dimethylpropanamide,
(119) methyl 3-(6-{6-[(4-cyanopyridin-2-yl)amino]-4-cyclopropylpyridin-2-yl}-1H-benzimidazol-1-yl) propanonate,
(120) 2-({4-cyclopropyl-6-[1-(4-hydroxybutyl)-1H-benzimidazol-6-yl]pyridin-2-yl}amino)pyridine-4-carbonitrile,
(121) 2-({4-[1-cyclopropyl-6-(4-hydroxybutyl)-1H-benzimidazol-5-yl)pyridin-2-yl}amino)pyridine-4-carbonitrile,
(122) 3-(5-{6-[(4-cyanopyridin-2-yl)amino]-4-cyclopropylpyridin-2-yl}-1H-benzimidazol-1-yl)propanamide,
(123) 2-({4-cyclopropyl-6-[1-(pyridin-3-ylmethyl)-1H-benzimidazol-6-yl]pyridin-2-yl}amino)pyridine-4-carbonitrile,
(124) 3-(5-{6-[(4-cyanopyridin-2-yl)amino]-4-cyclopropylpyridin-2-yl}-1H-benzimidazol-1-yl)-N,N-dimethylpropanamide,
(125) 3-(5-{6-[(4-cyanopyridin-2-yl)amino]-4-cyclopropylpyridin-2-yl}-1H-benzimidazol-1-yl)-N-methylpropanamide,
(126) 2-({4-cyclopropyl-6-[1-(pyridin-4-ylmethyl)-1H-benzimidazol-6-yl)pyridin-2-yl}amino)pyridine-4-carbonitrile,
(127) 2-({6-[1-(2-cyanoethyl)-1H-benzimidazol-5-yl]-4-cyclopropylpyridin-2-yl}amino)pyridine-4- carbonitrile,
(128) 2-[(4-cyclopropyl-6-{1-[1-(hydroxymethyl)cyclohexyl]-1H-benzimidazol-6-yl}pyridin-2-yl)amino]pyridine-4- carbonitrile,
(129) 2-({4-cyclopropyl-6-[1-(4,4-difluorocyclohexyl)-1H-benzimidazol-6-yl]pyridine-2-yl}amino)pyridine-4-carbonitrile,
(130) 2{[6-(1-benzyl-1H-benzimidazol-5-yl)-4-cyclopropylpyridine-2-yl]amino}pyridin-4-carbonitrile,
(131) 2-({4-cyclopropyl-6-[1-(trans-4-methoxycyclohexyl)-1H-benzimidazol-6-yl)pyridin-2-yl}amino)pyridine-4-carbonitrile,
(132) 2-({4-cyclopropyl-6-[1-(tetrahydro-2H-pyran-4-yl)-1H-benzimidazol-6-yl)pyridin-2-yl}amino)pyridine -4-carbonitrile,
(133) 2-[(4-cyclopropyl-6-{1-[trans-2-hydroxycyclopentyl]-1H-benzimidazol-6-yl}pyridine-2-yl)amino]pyridine-4-carbonitrile,
(134) trans-2-(6-{6-[(4-cyanopyridin-2-yl)amino]-4-cyclopropylpyridin-2-yl}-1H-benzimidazol-1-yl)cyclopentyl acetate,
(135) 2-{[4-cyclopropyl-6-(1-ethyl-1H-benzimidazol-6-yl)pyridin-2-yl]amino}pyridine-4-carbonitrile,
(136) 2-({4-cyclopropyl-6-[1-(1,3-dihydroxydimethylmethan-2-yl)-1H-benzimidazol-6-yl]pyridin-2-yl}amino)pyridine-4-carbonitrile,
(137) 2-({4-cyclopropyl-6-[1-(2-hydroxy-2-methylpropyl)-1H-benzimidazol-6-yl)pyridin-2-yl}amino)pyridine-4-carbonitrile,
(138) 2-({4-cyclopropyl-6-[1-(cis-4-hydroxycyclohexyl)-1H-benzimidazol-6-yl]pyridin-2-yl}amino)pyridine-4-carbonitrile,
(139) 2-[(4-cyclopropyl-6-{1-[trans-2-hydroxycyclohexyl]-1H-benzimidazol-6-yl}pyridin-2-yl]amino]pyridine-4-carbonitrile,
(140) 2-({4-cyclopropyl-6-[1-(2-hydroxyethyl)-1H-benzimidazol-5-yl)pyridin-2-yl}amino)pyridine-4-carbonitrile,
(141) 2-({4-cyclopropyl-6-[1-(2-ethoxyethyl)-1H-benzimidazol-5-yl]pyridin-2-yl}amino)pyridin-4-carbonitrile,
(142) 2-{[4-cyclopropyl-6-(1-ethyl-1H-benzimidazol-5-yl)pyridin-2-yl]amino}pyridine-4-carbonitrile,
(143) 2-{[4-cyclopropyl-6-(1-cyclopropyl-1H-benzimidazol-5-yl)pyridin-2-yl]amino}pyridin-4-carbonitrile,
(144) N-[2-(5-{6-[(4-cyanopyridin-2-yl)amino]-4-cyclopropylpyridin-2-yl}-1H-benzimidazol-1-yl)ethyl]acetamide,
(145) 2-[(4-cyclopropyl-6-{1-[trans-2-hydroxycyclohexyl]-1H-benzimidazol-5-yl}pyridin-2-yl]amino]pyridine-4-carbonitrile,
(146) 2-{[4-cyclopropyl-6-(1-methyl-1H-benzimidazol-6-yl)pyridin-2-yl]amino}pyridine-4-carbonitrile,
(147) 4-({6-[(4-cyanopyridine-2-yl)amino]-4-cyclopropyl-2,3'-bipyridin-6'-yl}methyl)-1,4-diazepane-1-carboxamide,
(148) 2-{[4-cyclopropyl-6'-(1,1-dioxidethiomorpholin-4-yl)-4'-methyl-2,3'-bipyridin-6-yl]amino}pyridine-4-carbonitrile,
(149) 2-({4-cyclopropyl-6-[3-(trans-4-hydroxycyclohexyl)-2-oxo-2,3-dihydro-1H-benzimidazol-5-yl]pyridin- 2-yl}amino)pyridine-4-carbonitrile,
(150) 2-({4-cyclopropyl-6-[2-ethyl-1-(trans-4-hydroxycyclohexyl)-1H-benzimidazol-6-yl)pyridin-2-yl} amino)pyridine-4-carbonitrile,
(151) 2-({4-cyclopropyl-6-[1-(3-oxopiperazin-1-yl)isoquinolin-7-yl]pyridin-2-yl}amino)pyridine-4-carbonitrile,
(152) 2-({4-cyclopropyl-6-[1-(trans-4-hydroxycyclohexyl)-2-methyl-1H-benzimidazol-6-yl]pyridine-2-yl}amino)pyridine -4 - carbonitrile,
(153) 2-({4-cyclopropyl-6-[1-(trans-4-hydroxycyclohexyl)-2-(propan-2-yl)-1H-benzimidazol-6-yl)pyridin-2-yl}amino)pyridine-4-carbonitrile,
(154) 2-({4-cyclopropyl-6-[1-(trans-4-hydroxycyclohexyl)-2-(hydroxymethyl)-1H-benzimidazol-6-yl)pyridin-2-yl}amino)pyridine-4-carbonitrile,
(155) 2-({4-cyclopropyl-6-[1-(piperazin-1-yl)isoquinolin-7-yl]pyridin-2-yl}amino)pyridine-4-carbonitrile,
(156) 2-({4-cyclopropyl-6-[1-(4-hydroxypiperidin-1-yl)isoquinolin-7-yl]pyridin-2-yl}amino)pyridine-4-carbonitrile,
(157) 2-[(4-cyclopropyl-6-{1-[3-hydroxypyrrolidin-1-yl]isoquinolin-7-yl}pyridin-2-yl]amino]pyridine-4-carbonitrile,
(158) 2-({4-cyclopropyl-6-[1-(3-hydroxyazetidin-1-yl)isoquinolin-7-yl]pyridin-2-yl}amino)pyridine-4-carbonitrile,
(159) 2-[(4-cyclopropyl-6-{1-[2-(hydroxymethyl)pyrrolidin-1-yl]isoquinolin-7-yl}pyridin-2-yl]amino]pyridine-4-carbonitrile,
(160) 2-[(4-cyclopropyl-6-{1-[3-fluoropyrrolidin-1-yl]isoquinolin-7-yl}pyridin-2-yl)amino]pyridine-4-carbonitrile,
(161) 2-[(6-{1-[(3R)-3-aminopyrrolidin-1-yl]isoquinolin-7-yl}-4-cyclopropylpyridin-2-yl)amino]pyridine-4-carbonitrile,
(162) 2-({6-[1-(4-cyanopiperidin-1-yl)isoquinolin-7-yl]-4-cyclopropylpyridin-2-yl}amino)pyridine-4-carbonitrile,
(163) 2-({4-cyclopropyl-6-[1-(2-oxo-imidazolidin-1-yl)isoquinolin-7-yl]pyridin-2-yl}amino)pyridine-4-carbonitrile,
(164) 2-({6-[1-(trans-4-aminocyclohexyl)-1H-benzimidazol-6-yl]-4-cyclopropylpyridin-2-yl}amino)pyridine-4-carbonitrile,
(165) 2-({4-[1-cyclopropyl-6-(piperidin-4-yl)-1H-benzimidazol-6-yl)pyridin-2-yl}amino)pyridine-4-carbonitrile,
(166) 2-[(4-cyclopropyl-6-{1-(6-oxo-1,6-dihydropyridin-3-yl)methyl]-1H-benzimidazol-6-yl}pyridin-2-yl)amino]pyridine-4-carbonitrile,
(167) 2-({4-cyclopropyl-6-[2-(trans-4-hydroxycyclohexyl)-1H-benzimidazol-6-yl)pyridin-2-yl}amino)pyridine-4-carbonitrile,
(168) 2-({6-[1-(trans-4-cyanocyclohexyl)-1H-benzimidazol-6-yl]-4-cyclopropylpyridin-2-yl}amino)pyridine-4-carbonitrile,
(169) 2-{[4-cyclopropyl-6'-(1,1-dioxidethiomorpholin-4-yl)-5'-fluoro-2,3'-bipyridin-6-yl]amino}pyridine-4-carbonitrile,
(170) 2-({4-cyclopropyl-6-[1-(trans-4-hydroxycyclohexyl)-1H-benzimidazol-5-yl)pyridin-2-yl}amino)pyridine-4-carbonitrile.

5. A pharmaceutical composition as an active ingredient comprising the pyridine derivative or a pharmaceutically acceptable salt thereof according to any one of claims 1 to 4.

6. A Syk tyrosine kinase inhibitor as an active ingredient comprising the pyridine derivative or a pharmaceutically acceptable salt thereof according to any one of claims 1 to 4.

7. A preventive agent or a therapeutic agent for an allergic disease, an autoimmune disease, or a malignant tumor, comprising as an active ingredient the pyridine derivative or a pharmaceutically acceptable salt thereof according to any one of claims 1 to 4.

8. The preventive agent or therapeutic agent according to claim 7, wherein the allergic disease is bronchial asthma, allergic rhinitis, allergic dermatitis, or allergic conjunctivitis.

9. The preventive agent or therapeutic agent according to claim 7, wherein the autoimmune disease is chronic rheumatoid arthritis, idiopathic thrombocytopenic purpura, systemic lupus erythematosus, or multiple sclerosis.

10. The preventive agent or therapeutic agent according to claim 7, wherein the malignant tumor is a B-cell lymphoma, a B-cell leukemia, peripheral T-cell lymphoma not-otherwise specified, angioimmunoblastic T-cell lymphoma, anaplastic large-cell lymphoma, cutaneous anaplastic large-cell lymphoma, mycosis fungoides, enteropathy-associated T-cell lymphoma, extranodal NK-T-cell lymphoma, hepatosplenic T-cell lymphoma, subcutaneous panniculitis-like T-cell lymphoma, diffuse large-cell lymphoma, or follicular lymphoma.
